(19) Europäisches Patentamt
European Patent Office
Office européen des brevets

(11) EP 4 715 056 A1

(12) # EUROPEAN PATENT APPLICATION

published in accordance with Art. 153(4) EPC

(43) Date of publication:
**25.03.2026 Bulletin 2026/13**

(21) Application number: **24806583.1**

(22) Date of filing: **14.05.2024**

(51) International Patent Classification (IPC):
***C12N 15/80*** *(2006.01)* ***C12N 1/15*** *(2006.01)*
***C12P 7/48*** *(2006.01)* ***C07C 67/08*** *(2006.01)*
***C07C 69/604*** *(2006.01)* ***C08L 27/06*** *(2006.01)*

(52) Cooperative Patent Classification (CPC):
**C07C 67/08; C07C 69/604; C08L 27/06; C12N 15/80; C12P 7/48**

(86) International application number:
**PCT/CN2024/093180**

(87) International publication number:
**WO 2024/235233 (21.11.2024 Gazette 2024/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:
**15.05.2023 CN 202310546966**
**22.05.2023 CN 202310580674**
**22.05.2023 CN 202310580689**
**22.05.2023 CN 202310573624**
**22.05.2023 CN 202310575243**
**05.09.2023 CN 202311140617**
**05.09.2023 CN 202311140630**
**06.09.2023 CN 202311144969**

(71) Applicants:
- **Qingdao Institute of Bioenergy and Bioprocess Technology, Chinese Academy of Sciences**
  **Qingdao, Shandong 266101 (CN)**
- **Shandong Lukang Pharmaceutical Co., Ltd.**
  **Jining, Shandong 272104 (CN)**

(72) Inventors:
- **LU, Xuefeng**
  **Qingdao, Shandong 266101 (CN)**
- **WANG, Qinggang**
  **Qingdao, Shandong 266101 (CN)**
- **HUANG, Xuenian**
  **Qingdao, Shandong 266101 (CN)**
- **PENG, Xin**
  **Jining, Shandong 272104 (CN)**
- **HOU, Hongbin**
  **Qingdao, Shandong 266101 (CN)**
- **GUO, Qiang**
  **Jining, Shandong 272104 (CN)**
- **WANG, Min**
  **Qingdao, Shandong 266101 (CN)**
- **LI, Jibin**
  **Jining, Shandong 272104 (CN)**
- **GU, Meng**
  **Qingdao, Shandong 266101 (CN)**
- **DU, Zhiqiang**
  **Qingdao, Shandong 266101 (CN)**
- **WU, Maocheng**
  **Jining, Shandong 272104 (CN)**
- **GENG, Ce**
  **Qingdao, Shandong 266101 (CN)**
- **SI, Xuejian**
  **Jining, Shandong 272104 (CN)**
- **XU, Guangqiang**
  **Qingdao, Shandong 266101 (CN)**
- **GUO, Jian**
  **Qingdao, Shandong 266101 (CN)**
- **ZHANG, Yunpeng**
  **Qingdao, Shandong 266101 (CN)**
- **YIN, Jijin**
  **Jining, Shandong 272104 (CN)**
- **GUO, Qing**
  **Qingdao, Shandong 266101 (CN)**

(74) Representative: **Huang, Liwei**
**Cäcilienstraße 12**
**40597 Düsseldorf (DE)**

Remarks:
The complete document including Reference Table(s) and the Sequence Listing(s) can be downloaded from the EPO website

EP 4 715 056 A1

(54) **BIO-BASED TRANS-ACONITATE, PREPARATION METHOD THEREFOR AND USE THEREOF**

(57) The present disclosure provides a bio-based *trans*-aconitate, and a preparation method and applications thereof, belonging to the technical field of synthetic biology. On the one hand, the present disclosure provides a recombinant engineered strains for producing trans-Aconitic acid. The modified engineered strains can significantly improve the capability of producing *trans*-Aconitic acid through fermentation. The present disclosure further provides a submerged liquid fermentation process for producing *trans*-Aconitic acid, and the yield of *trans*-Aconitic acid is further increased by low dissolved oxygen control and appropriate timing of a sugar feeding process. The present disclosure further provides a process for synthesizing high-purity aconitate from *trans*-Aconitic acid product produced by fermentation, thereby solving the problem of removing pigment impurities from aconitic acid raw material produced by fermentation, and providing specific applications and products of aconitate in industrial production. The present disclosure provides a complete system from strain modification, fermentation production, product synthesis to applications, offers a complete technical route for bio-based *trans*-Aconitic acid and its industrial application, and has great economic values and application prospects.

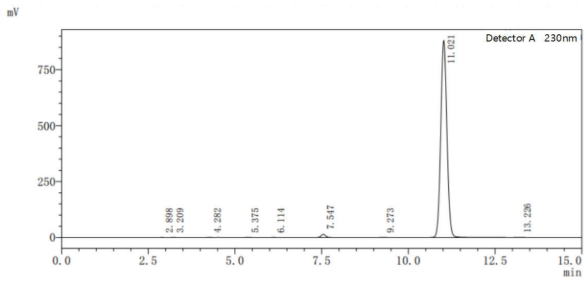


Fig. 23

## Description

The present disclosure claims the priorities of a Chinese patent application filed with the Chinese Patent Office on September 6, 2023, with an application number of 202311144969.1 and entitled "Engineered Strains with High Yield of trans-Aconitic Acid, Construction Method and Applications Thereof"; a Chinese patent application filed with the Chinese Patent Office on May 22, 2023, with an application number of 202310580689.9 and entitled "High Cold-resistant PVC Composite Material, Preparation Method and Applications Thereof"; a Chinese patent application filed with the Chinese Patent Office on May 15, 2023, with an application number of 202310546966.4 and entitled "Submerged Liquid Fermentation Process for Producing trans-Aconitic Acid"; a Chinese patent application filed with the Chinese Patent Office on May 22, 2023, with an application number of 202310575243.7 and entitled "Mixed Aconitate, Preparation Method Thereof and Composite Material Containing Mixed Aconitate"; a Chinese patent application filed with the Chinese Patent Office on May 22, 2023, with an application number of 202310580674.2 and entitled "Preparation Method of High-purity Aconitate"; a Chinese patent application filed with the Chinese Patent Office on May 22, 2023, with an application number of 202310573624.1 and entitled "High-strength Volatilization-resistant Composite Material, Preparation Method and Applications Thereof"; a Chinese patent application filed with the Chinese Patent Office on September 5, 2023, with an application number of 202311140630.4 and entitled "Recombinant *Aspergillus niger* with High Yield of trans-Aconitic Acid, Construction Method and Applications Thereof"; and a Chinese patent application filed with the Chinese Patent Office on September 5, 2023, with an application number of 202311140617.9 and entitled "Recombinant *Aspergillus terreus* Strains with High Yield of trans-Aconitic Acid and Applications Thereof", the contents of which are incorporated herein by reference in their entireties.

## TECHNICAL FIELD

The present disclosure relates to the technical field of synthetic biology, in particular to a bio-based trans-aconitate, a preparation method and applications thereof.

## BACKGROUND

As an unsaturated tricarboxylic acid, and owing to its unsaturated double bonds together with multiple carboxyl groups, trans-Aconitic acid (tAA, CAS: 4023-65-8) can be used as a monomer compound for preparing polymeric materials, and can also be used as a plasticizer in a plastic processing process after esterification. In addition, as a stereoisomer of cis-Aconitic acid (cAA, CAS: 585-84-2), which is a key intermediate in the tricarboxylic acid (TCA) cycle, tAA exhibits a certain inhibitory effect on aconitase, the key enzyme in the TCA cycle. The tAA can interfere with the TCA cycle, thereby affecting life activities, and exhibiting certain biological activities. The *tAA* can be used as biological pesticides to control plant-parasitic nematodes, brown planthoppers, and animal parasites such as *Leishmania.*

At present, trans-Aconitic acid is mainly produced industrially through chemical synthesis. The main steps include saponification reaction of propane-1,1,2,3-tetracarboxylic acid compounds, dehydrochlorination reaction, and sulfuric acid acidification of propylene tetracarboxylic acid. Many by-products such as isocitric acid lactone will be generated during this production process, resulting in great difficulty in subsequent separation and purification, this difficulty results in low yield, high cost, and environmental problems such as wastewater pollution caused by the synthesis process. Consequently, large-scale production has not been achieved. Therefore, the limited accessibility of *trans-Aconitic* acid feedstock is a major bottleneck for downstream applications and product development.

Furthermore, many attempts have been explored to prepare trans-Aconitic acid through fermentation production. For example, researchers have constructed aconitic acid-producing strains in *Escherichia coli,* but the total yield of trans-Aconitic acid and cis-Aconitic acid of the strains is only 360 mg/L (Li et al. Microb Cell Fact, 2020, 19:174). The inventors of this patent have previously developed a series of *Aspergillus terreus* strains for producing trans-Aconitic acid, achieving a titer of 20-25 g/L in shake-flask (Authorized patent numbers: CN110527637B; CN112011468B; CN112011469B; CN112011470B; CN112029671B). Later, a fungi cell factory based on *Aspergillus pseudoterreus* was reported to produce aconitic acid with the 10 g/L yield after 10 days of shake flask fermentation (US 2021/0163966A1). However, the above researches all remain at a laboratory research stage. No systematic research has been conducted on the submerged liquid fermentation or industrial-scale expanded fermentation process of trans-Aconitic acid-producing strains, such that the fermentation production of *trans-Aconitic* acid only remains at a laboratory research level and fails to generate actual economic or social benefits.

In addition, high content of pigment impurities in *trans-Aconitic* acid prepared by fermentation also limits its specific industrial applications. The crude aconitic acid obtained through fermentation contains 2% to 40% impurities during preparation. These impurities are mainly polysaccharides, pigments, and trace amounts of mycelia and proteins. Although the proportion of pigments is relatively small (typically 1-5% of the total impurities), their presence will greatly affect the product’s color. As a raw material for the synthesis of aconitates, if aconitic acid is not decolorized, the pigment

impurities will remain in the synthesized aconitates. When subsequent products are prepared from aconitates, the pigment impurities in the aconitates have a great coloring effect on the final product and also have a significant impact on the subsequent application of the product.

Additionally, the presence of polysaccharides also greatly affects the purity and yield of aconitates. Firstly, the monosaccharides derived from polysaccharides under acidic conditions compete with the alcohol for esterification with aconitic acid, leading to impure products and a reduced final yield. Secondly, polysaccharides undergo caramelization at high temperatures. The caramelization reaction will increase the chromaticity of aconitates and reduce the purity of aconitates, further affecting the use of aconitates.

At present, considering the decolorization and purification method of acids, a dissolution-precipitation method is used for acids with low solubility. A concentrated solution of the acid is prepared and heated. Due to its low solubility, the acid precipitates as the temperature decreases. At this time, the pigment impurities have not reached a precipitation limit, so effective purification can be achieved. For acids with high solubility, a salt-forming precipitation method is used. The acid is prepared into a concentrated solution, and salts (such as calcium salts) are added to form precipitates with the acid. A higher solubility allows the acid to precipitate as much as possible, while fewer pigment impurities precipitate and can be further washed away, so effective purification can also be achieved. However, Aconitic acid has high solubility. When the concentrated solution precipitates the product as the temperature decreases, pigment impurities will also precipitate together. When the salt-forming precipitation method is used, since the solubility of aconitic acid salts is still high, it is difficult to effectively precipitate the aconitic acid salts, and it is even more difficult to retain the precipitated product during further washing and purification. If decolorization and purification of aconitic acid need to be performed through extraction with polar organic solvents and then decolorization is performed with adsorbents, the above steps are cumbersome, and if the organic extract is not completely removed, it will affect the subsequent synthesis reaction of aconitates. Therefore, aconitic acid obtained from fermentation has the problem of difficulty in separating from pigments, then the synthesized aconitates will inevitably contain pigment impurities, thereby increasing the chromaticity and decreasing the purity of the aconitate product.

A Chinese patent with an application number of CN101391957A discloses a method for preparing tributyl citrate using a rare earth salt binary compound solid acid catalyst. In the method, activated carbon and hydrogen peroxide are simultaneously added to the crude tributyl citrate product without washing and solvent removal steps for decolorization. However, this method has poor effect and can effectively remove color from the citrate esters with high raw material purity and low chromaticity. For aconitates with high pigment content and strong pigment chroma, the pigments in the aconitates cannot be effectively removed through this method.

In view of this, the solution of the present disclosure is specially proposed to solve the above problems in the fermentation of *trans*-Aconitic acid, and the preparation and applications of *trans-aconitates.*

**SUMMARY**

The present disclosure aims to solve at least one of the technical problems existing in the prior art or related technologies.

On the one hand, the present disclosure provides a preparation method of bio-based trans-Aconitic acid, and the method includes:

(1) preparing a *trans*-Aconitic acid-producing engineered strains; (2) preparing seed; (3) performing product fermentation.

Optionally, the present disclosure provides a preparation method of bio-based trans-Aconitic acid, and the method includes:

(1) preparing a *trans*-Aconitic acid-producing engineered strains; (2) activating the producing strains; (3) preparing seed; (4) performing primary seed fermentation; (5) performing secondary seed fermentation; (6) performing product fermentation.

Further, the engineered strains express aconitate isomerases, and the aconitate isomerases include TbrA and/or Adi1.

The aconitate isomerase TbrA is derived from *Bacillus thuringiensis,* and the aconitate isomerase Adi1 is derived from *Ustilago maydis.*

Further, the amino acid sequence of the aconitate isomerase TbrA includes amino acid sequence shown as SEQ ID NO. 1 or amino acid sequence having at least 60% homology with the amino acid sequence shown as SEQ ID NO. 1; and/or the amino acid sequence of the aconitate isomerase Adi1 includes amino acid sequence shown as SEQ ID NO. 2 or amino acid sequence having at least 60% homology with the amino acid sequence shown as SEQ ID NO. 2.

Optionally, the aconitate isomerase gene encoding TbrA includes the sequence shown as SEQ ID NO. 3 or a nucleotide sequence having at least 60% sequence homology with the sequence shown as SEQ ID NO. 3.

Optionally, the aconitate isomerase gene encoding Adi1 includes the sequence shown as SEQ ID NO. 4 or a nucleotide sequence having at least 60% sequence homology with the sequence shown as SEQ ID NO. 4.

In an optional embodiment, the present disclosure uses *Aspergillus niger* for the first time to produce trans-Aconitic acid. Since the aconitase Aco of *Aspergillus niger* itself is subject to strict feedback inhibition regulation, a large amount of cis-Aconitic acid cannot be accumulated. In the present disclosure, by transferring aconitate isomerase into *Aspergillus niger,* the cis-Aconitic acid produced in *Aspergillus niger* is converted into trans-Aconitic acid, thereby avoiding the feedback inhibition regulation in a synthesis process of cis-Aconitic acid.

Further, the engineered strains also express aconitase.

In an optional embodiment, the present disclosure provides an aconitase that can efficiently catalyze the synthesis of cis-Aconitic acid, an aconitate isomerase that can efficiently catalyze the conversion of cis-Aconitic acid to trans-Aconitic acid, and an *Aspergillus niger* genetic engineered strains that can synthesize trans-Aconitic acid, so as to realize one-step production of *trans*-Aconitic acid.

Further, the aconitase is Aco.

Optionally, the aconitase is Aco (ATEG_03325) derived from *Aspergillus terreus.*

Further, the amino acid sequence of the aconitase Aco includes amino acid sequence shown as SEQ ID NO. 5 or an amino acid sequence having at least 60% homology with the amino acid sequence shown as SEQ ID NO. 5.

In one embodiment, the gene encoding the aconitase Aco includes the sequence shown as SEQ ID NO. 6 or a nucleotide sequence having at least 60% homology with the sequence shown as SEQ ID NO. 6.

Further, at least one terminus of the aconitate isomerase is fused with a mitochondrial targeting signal peptide (MTS).

In an optional embodiment, the present disclosure provides an engineered strains with a high yield of *trans*-Aconitic acid, a construction method and applications thereof. By combining a mitochondrial signal peptide with aconitate isomerase to form a gene expression element, and using genetic engineering techniques to introduce a gene expression cassette containing this element into the strains, the aconitate isomerase expressed in the constructed recombinant strains can be localized into mitochondria, such that the two steps of the reaction of *trans*-Aconitic acid synthesis are performed in the same compartment. This inhibits a metabolic pathway that converts cis-Aconitic acid to citric acid, and enables the cis-Aconitic acid produced in mitochondria to be rapidly converted into trans-Aconitic acid under the catalysis of aconitate isomerase, thereby preventing excessive precursor cis-Aconitic acid from being used for growth and metabolism, being more conducive to the production of *trans*-Aconitic acid, and further improving the efficiency of producing *trans*-Aconitic acid by fermentation in the recombinant strains.

Further, the mitochondrial targeting signal peptide is one or more selected from MTSacoAt, MTScitAt and MTSicdAn.

Further, the amino acid sequence of the mitochondrial targeting signal peptide contains amino acid sequence shown as SEQ ID NO. 7, SEQ ID NO. 8 or SEQ ID NO. 9.

Further, the N-terminus of the aconitate isomerase is fused with the mitochondrial targeting signal peptide.

Further, the engineered strains are selected from *Aspergillus terreus* or *Aspergillus niger;* the *Aspergillus niger* is one or more selected from *Aspergillus niger* MEFC1501, *Aspergillus niger* ATCC 1015, *Aspergillus niger* NRRL 41873, *Aspergillus niger* NRRL 27809, *Aspergillus niger* NRRL 31821, *Aspergillus niger* NRRL 6276, *Aspergillus niger* Co827, *Aspergillus niger 5016, Aspergillus niger* 3008, *Aspergillus niger* GCB75, *Aspergillus niger* ATCC 9142 and *Aspergillus niger* MTCC 282. The *Aspergillus terreus* is one or more selected from *Aspergillus terreus* CICC40205, *Aspergillus terreus* NRRL1960, *Aspergillus terreus* DSM23081, *Aspergillus terreus* TN484 and *Aspergillus terreus* TN484-M1.

Among common industrial chassis strains, *Aspergillus niger* is an excellent citric acid-producing strains. The produced citric acid is a direct precursor of cis-Aconitic acid, and *Aspergillus niger* does not synthesize itaconic acid, so there is no need to block the synthesis of itaconic acid. Compared with *Aspergillus terreus,* the pathway for synthesizing trans-Aconitic acid is simplified. In addition, *Aspergillus niger* is a traditional fermentation strains widely used in the food industry, and is recognized as generally regarded as safe (GRAS) by the US FDA and the World Health Organization. *Aspergillus niger* can be used as a cell factory for the large-scale production of proteins, organic acids, antigens, etc. Therefore, *Aspergillus niger* is a potential strain for large-scale production of trans-Aconitic acid. However, there have been no relevant reports on the production of trans-Aconitic acid using *Aspergillus niger* so far.

In addition, *Aspergillus tubingensis, Aspergillus phoenicis, Aspergillus usamii,* Candida and the like can also be used as potential hosts for producing trans-Aconitic acid.

Further, when the engineered strains are *Aspergillus terreus,* the cis-aconitate decarboxylase gene *(cadA)* is further knocked out.

Optionally, the nucleotide sequence of an upstream homology arm of the *cadA* gene is shown as SEQ ID NO. 137; the nucleotide sequence of the downstream homology arm of the *cadA* gene is shown as SEQ ID NO. 138.

Optionally, when the starting strains is *Aspergillus terreus,* in order to prevent the itaconic acid pathway from affecting the synthesis of trans-Aconitic acid, the cis-aconitate decarboxylase gene *(cadA)* is further knocked out to improve the production efficiency of *trans*-Aconitic acid.

Optionally, in the method for knocking out the cis-aconitate decarboxylase gene *(cadA),* the gene expression cassette is used to replace the nucleotide sequence of the *Aspergillus terreus* gene (*cadA*) with the nucleotide sequence of the mitochondrial targeting signal peptide and the aconitate isomerase.

It should be noted that the present disclosure does not limit the specific method for knocking out the *cadA* gene. For example, a separate gene knockout element can be used to knock out the *cadA* gene, and an additional gene expression element can be used to target and localize the aconitate isomerase into mitochondria. When this method is used, the *cadA* gene can be knocked out while the construction for expressing aconitate isomerase targeting the interior of mitochondria is achieved, thereby reducing the operation procedures in strains modification and lower the operation difficulty.

Optionally, a gene expression cassette containing a promoter, an aconitate isomerase gene expression element with the N-terminus fused with a mitochondrial targeting signal peptide, and a terminator sequence can be inserted into a genome of the starting strains through transformation. It should be noted that the insertion method can be random insertion, and the modified strains that does not affect the normal life activities of the strains can be screened out later, or directional insertion can be used. The insertion method is not specifically limited in the present disclosure. It should also be noted that it is known to those skilled in the art that the above genome inserted into the starting strains is not necessary, and the genome can also exist on a plasmid in a free state, as long as the successfully expressed aconitate isomerase can be targeted and localized into mitochondria.

It should be noted that since there is a synthesis pathway for itaconic acid formed by decarboxylation of precursor cis-Aconitic acid in *Aspergillus terreus,* in order to further improve the synthesis efficiency of *trans-Aconitic* acid, the key gene *cadA* in the itaconic acid synthesis pathway is knocked out; while *Aspergillus niger* does not have the key gene for the synthesis of itaconic acid, so it is only necessary to directly introduce aconitate isomerase that can be localized into mitochondria.

Further, the engineered strains also includes a promoter, and the promoter is one or more selected from PglaA promoter, PgpdAt promoter, PgpdAn promoter, PacoA promoter, PcitA promoter, PicdA promoter, PcadA promoter, PmfsA promoter, and PgasA promoter; more optionally, the promoter is a PglaA promoter.

Optionally, the sequence of the PglaA promoter is shown as SEQ ID NO. 10.

Optionally, the sequence of the PgpdAt promoter is shown as SEQ ID NO. 11.

Optionally, the sequence of the PgpdAn promoter is shown as SEQ ID NO. 12.

Optionally, the sequence of the PacoA promoter is shown as SEQ ID NO. 13.

Optionally, the sequence of the PcitA promoter is shown as SEQ ID NO. 14.

Optionally, the sequence of the PicdA promoter is shown as SEQ ID NO. 15.

Optionally, the sequence of the PcadA promoter is shown as SEQ ID NO. 16.

Optionally, the sequence of the PmfsA promoter is shown as SEQ ID NO. 17.

Optionally, the sequence of the PgasA promoter is shown as SEQ ID NO. 18.

Further, the engineered strains also includes a terminator, and the terminator includes TtrpC terminator and/or Tpgk terminator.

Optionally, the sequence of the TtrpC terminator is shown as SEQ ID NO. 19.

Optionally, the sequence of the Tpgk terminator is shown as SEQ ID NO. 20.

The promoter or terminator for terminating expression in the present disclosure can be a promoter or terminator commonly used in fungi, or a promoter or terminator specific to the host bacterium. Those skilled in the art can make adaptive selections according to actual conditions, and the present disclosure does not impose mandatory restrictions on the sequences of the promoter or terminator.

It will be understood by those skilled in the art that the construction of the engineered strains of the present disclosure can be completed using the existing gene editing technologies.

In an optional embodiment, the expression cassette of the target gene is located on a recombinant vector or introduced into a recombinant microorganism using a recombinant vector. For example, a mitochondrial targeting signal peptide sequence is added in front of the gene encoding aconitate isomerase by using the fusion PCR technology or the gene synthesis technology, and the mitochondrial targeting signal peptide sequence is introduced into the strains through PEG-mediated transformation or electroporation. The translated signal peptide will guide the aconitate isomerase sequence into mitochondria where it folds and performs its function. It should be noted that any conventional technical means in the art that can realize the targeted localization of aconitate isomerase into mitochondria should also be included within the scope of the present disclosure. Alternatively, the expression of aconitate isomerase or aconitase in the engineered strains can be realized through genetic recombination methods.

Those skilled in the art can select existing plasmids to construct recombinant vectors according to actual conditions, and perform routine optimization and modification on the constructed recombinant vectors. The present disclosure does not impose mandatory restrictions on the type of plasmid.

Those skilled in the art will appreciate that other commonly used expression elements can be adaptively added to assist the expression of the target gene based on the expression cassette described in the present disclosure, such as adding tags, fluorescent protein markers, resistance selection markers, etc.

In an optional embodiment, the recombinant vector and the recombinant microorganism contain a resistance selection marker gene. Those skilled in the art can select according to actual conditions, and the present disclosure does

not impose mandatory restrictions on the resistance selection marker gene.

Further, the dissolved oxygen (DO) level during the product fermentation process is maintained at 25% to 30%.

Further, sugar feeding is carried out between 30 and 60 h of the product fermentation process.

Further, in a DO control step during the product fermentation process, the aeration rate is controlled from 0.1 to 0.2 vvm; and the agitation speed is controlled from 50 to 150 rpm.

Further, a fermentation medium used in the product fermentation process contains a nutritional factor.

Further, the fermentation medium includes: 120-150 g/L glucose, 2-4 g/L ammonium sulfate, 0.05-0.15% defoamer, 1-3 g/L corn steep liquor powder, 0.005-0.015 g/L diammonium hydrogen phosphate, 0.01-0.06 g/L ferrous sulfate, 0.2-0.4 g/L magnesium sulfate, 0.02-0.04 g/L zinc sulfate, 0.02-0.04 g/L copper sulfate, 0.3-0.6 g/L sodium chloride, and 0.05-0.5 g/L nutritional factor.

Further, the fermentation medium is: 150 g/L glucose, 4 g/L ammonium sulfate, 0.10% defoamer, 2 g/L corn steep liquor powder, 0.01 g/L diammonium hydrogen phosphate, 0.06 g/L ferrous sulfate, 0.4 g/L magnesium sulfate, 0.04 g/L zinc sulfate, 0.04 g/L copper sulfate, 0.5 g/L sodium chloride, 0.4 g/L nutritional factor.

Further, the nutritional factors are one or more selected from L-tryptophan, L-methionine, L-leucine and biotin.

In the present disclosure, the formula of the fermentation medium is improved, which effectively increases the yield and production rate of trans-Aconitic acid and the conversion rate of glucose to trans-Aconitic acid.

The present disclosure relates to the improvement of the fermentation process of trans-Aconitic acid to further increase the yield of *trans-Aconitic* acid, including the optimal level and method of DO control.

On the other hand, the present disclosure also provides an engineered strains for producing trans-Aconitic acid, wherein the engineered strains express aconitate isomerases, and the aconitate isomerases include TbrA and/or Adil.

On the other hand, the present disclosure also provides a preparation method of high-purity aconitate, wherein the method includes:

(1) reacting the bio-based *trans*-Aconitic acid prepared by the method with alcohols to obtain a solution containing aconitate, neutralizing the solution containing aconitate, and then performing liquid separation to collect an upper organic phase;
(2) adding hydrogen peroxide to the upper organic phase to perform first-step decolorization, followed by washing, performing liquid separation and removing solvent to obtain crude aconitate product; and
(3) adding decolorizing agent to the crude aconitate product for second-step decolorization, and then filtering to obtain the high-purity aconitate, wherein the decolorizing agent is selected from at least one of activated carbon, diatomite, kaolin, basic aluminum oxide and neutral aluminum oxide.

Since the aconitic acid raw material obtained by fermentation contains a large number of pigments, and different types of pigments have obvious differences in solubility in organic solvents and water. The pigments in aconitic acid obtained by fermentation are divided into two types: fat-soluble pigments and water-soluble pigments. Fat-soluble pigments are mainly anthraquinone compounds, most of which have double bonds in their structures, have good compatibility with aconitates, and are difficult to be adsorbed. The water-soluble pigments enter the organic phase with a small amount of water during the washing process, and remain in the product after solvent removal, so the water-soluble pigments are difficult to be completely washed off in a water-washing stage.

They usually react (bleach) with oxidizing agents (such as hydrogen peroxide). In the preparation method, the first-step decolorization and the second-step decolorization are performed during the preparation of aconitates. During the first-step decolorization, the structures of fat-soluble pigments are mainly oxidized and destroyed. Through oxidation with hydrogen peroxide, double bonds in such pigments are destroyed, causing them to undergo oxidation reactions. The organic skeletons of the pigments are decomposed to produce substances such as hydroquinones and aldehydes. Therefore, the main structures of fat-soluble pigments are destroyed to effectively reduce the chromaticity. At the same time, while hydrogen peroxide removes the chromaticity of fat-soluble pigments, the absorbance of fat-soluble pigments adsorbed by the decolorizing agent is increased, such that the content of fat-soluble pigments in aconitates can be effectively reduced through the combination with the second-step decolorization. However, hydrogen peroxide is immiscible with aconitates, and it needs to be washed with clean water after decolorization. Therefore, first-step decolorization using hydrogen peroxide is performed before the washing and solvent removal steps, thereby not only fully decolorizing fat-soluble pigments but also ensuring that hydrogen peroxide is removed in the subsequent washing step. If the first-step decolorization is performed after the solvent removal step, the introduction of hydrogen peroxide will cause oil-water separation of aconitates, and hydrogen peroxide still needs to be further removed, resulting in cumbersome steps and reduced removal rate of fat-soluble pigments.

Second-step decolorization is performed after the solvent removal step, mainly to remove water-soluble pigments. The decolorizing agent used can be uniformly distributed in aconitates, and it only decolorizes aconitates uniformly in one phase, thereby effectively reducing the content of water-soluble pigments in aconitates. If the decolorizing agent is placed before the washing step or decolorized together with hydrogen peroxide, the decolorizing agent exhibits

different affinity for the aconitate and the solvent liquid phase, such that the decolorizing agent is absorbed by different liquid phases to form agglomerates. These agglomerates are dispersed in aconitates and are difficult to mix uniformly with aconitates, thereby resulting in insufficient contact with aconitates, reducing the decolorization effect, and reducing the removal rate of water-soluble pigments.

Further, the concentration of the hydrogen peroxide is 33% to 40%, and the weight of the hydrogen peroxide accounts for 1% to 5% of the weight of the upper organic phase.

Further, the weight ratio of the decolorizing agent to the crude aconitate product is 1:(2-100).

The double bonds in fat-soluble pigments are double bonds in alkyl chains, and they have no significant steric hindrance around them and are therefore easily destroyed. The double bonds of aconitate are located between two ester groups with large steric hindrance, making it difficult to be destroyed. Therefore, the above concentration and weight of hydrogen peroxide can quickly destroy the double bonds of fat-soluble pigments, thereby improving the removal rate of fat-soluble pigments. Moreover, the hydrogen peroxide with this concentration and weight will not destroy the double bonds in aconitates, and improves the yield of aconitates. The strong oxidizing property of hydrogen peroxide oxidizes the chromophoric functional groups in fat-soluble pigments into non-chromophoric functional groups, so the structures of fat-soluble pigment substances with destroyed double bonds change and absorb no light. The hydrogen peroxide at this concentration has the best bleaching effect on the pigments in aconitates. The amount of the decolorizing agent can ensure that the decolorizing agent is uniformly dispersed in aconitates, thereby improving the adsorption effect on fat-soluble pigments with changed structures and water-soluble pigments, and being beneficial to subsequent removal of the decolorizing agent. When the amount exceeds 5%, hydrogen peroxide will cause hydrolysis of aconitates, and affect the yield.

Further, the first-step decolorization using hydrogen peroxide is performed at 20°C to 70°C for 15 to 30 min.

Further, the second-step decolorization using the decolorizing agent is performed at 20°C to 70°C for 10 to 120 min.

Optionally, the fat-soluble pigments include anthraquinone compounds; the water-soluble pigments include terrein.

For the decolorization of pigment impurities carried in aconitic acid, temperature is an important influencing factor. The higher the temperature, the lower the viscosity of the product system and the better the fluidity, and the more uniformly the decolorizing agent is distributed in the system. However, the higher the temperature, the more intense the Brownian motion of molecules, the more difficult it is for the decolorizing agent to adsorb pigments, and instead, the removal rate of pigment impurities decreases. In the decolorization of citrate esters, since the structure of citric acid has hydroxyl groups, and hydroxyl groups easily form hydrogen bonds with hydroxyl groups and ester groups on other molecules, thereby causing aggregation of product molecules. The adsorption effect of activated carbon used is poor. Therefore, high temperature is needed to destroy the aggregation of molecules to achieve the purpose of decolorization.

The first-step decolorization temperature and second-step decolorization temperature in the present disclosure are relatively low. On the one hand, energy consumption in the decolorization step can be lowered. On the other hand, researches have proved that the lower the decolorization temperature, the lower the Brownian motion of molecules, and the better the decolorization effect. Therefore, the above decolorization temperature and time can effectively improve the removal efficiency and removal rate of pigment impurities, and further improve the purity of aconitates.

Further, the activated carbon has an iodine value of greater than 800 and a methylene blue value of greater than 10;

the particle size of diatomite is 200 to 300 mesh;
the particle size of kaolin is 200 to 300 mesh;
both particle sizes of basic aluminum oxide and neutral aluminum oxide are both 100 to 200 mesh, both specific surface areas of basic aluminum oxide and neutral aluminum oxide are 60 to 90 $m^2/g$, and both average pore diameter of basic aluminum oxide and neutral aluminum oxide are 10 to 20 nm.

Under the above settings of the decolorizing agent, the decolorizing agent is uniformly dispersed in the aconitate phase, so as to avoid agglomeration or aggregation of the decolorizing agent. Moreover, this decolorizing agent can increase the contact area with water-soluble pigments and fat-soluble pigments with destroyed double bonds, thereby facilitating to adsorb and store the above two types of pigments and other impurities in the pores of the decolorizing agent, improving the adsorption capacity for pigments, preventing the adsorbed pigments from escaping again, and further improving the removal effect of water-soluble pigments, fat-soluble pigments, and impurities.

Aluminum oxide itself has a large adsorption area and pores for adsorbing pigments. In the above system, since sugars and pigment impurities are generally acidic, when basic aluminum oxide and neutral aluminum oxide are selected as decolorizing agents, the adsorption capacity for sugars and pigment impurities can be enhanced, and the removal rate of pigment impurities and sugars is improved.

Optionally, the decolorizing agent is selected from activated carbon.

The purity of aconitates prepared by traditional methods can reach up to 95%, and the chromaticity is above 500 Pt-Co, and the chromaticity is tested by the platinum-cobalt colorimetry method. In the preparation method of the present disclosure, first-step decolorization and second-step decolorization are performed during the synthesis of aconitates, and the decolorization effect is improved by an order of magnitude, thereby not only reducing the chromaticity, but also reducing the impurity content, and increasing the purity of the product to be greater than or equal to 99% with a significant purification effect.

The preparation method of the present disclosure is applicable to the reaction between aconitic acid and any alcohol. The alcohols can be a single alcohol, in which case a single aconitate is prepared with identical substituents on each aconitic acid molecule. Alternatively, the alcohols can be mixed alcohols, in which case mixed aconitates are prepared, and for the substituents on a single aconitic acid molecule, there are three cases: all the three substituents are the same, two substituents are the same and one substituent is different, or all the three substituents are different.

Optionally, the alcohols in step (1) are selected from at least one of methanol, ethanol, 2-methoxyethanol, 2-ethoxyethanol, 2-butoxyethanol, propanol, isopropanol, n-butanol, isobutanol, tert-butanol, 4-methyl-2-butanol, 2-ethylbutanol, 3,3-dimethyl-2-butanol, 4-phenylbutanol, n-pentanol, isopentanol, tert-pentanol, 2-methylpentanol, 2-methyl-2-pentanol, 2-methyl-3-pentanol, 3-ethyl-3-pentanol, 4-methyl-1-pentanol, 4-methyl-2-pentanol, n-hexanol, 2-hexanol, 3-hexanol, 2-ethylhexanol, 3,5,5-trimethylhexanol, n-heptanol, 2-heptanol, 3-heptanol, 2-methylheptanol, 4-methyl-3-heptanol, 3-propylheptanol, 2,6-dimethyl-4-heptanol, n-octanol, 2-octanol, n-nonanol, 2-nonanol, 3-nonanol, 5-ethyl-2-nonanol, 2,6,8-trimethyl-4-nonanol, n-decanol, isodecanol, undecanol, dodecanol, cyclohexanol, cyclohexylmethanol, chloroethanol, 1-chloro-2-propanol, 3-chloro-1-propanol, 2-chloro-1-propanol, benzyl alcohol, and phenethyl alcohol.

Further, the reaction between the aconitic acid obtained by fermentation and alcohols is performed at 75°C to 145°C for 2 to 8 h.

Under the action of the catalyst in the present disclosure, the above reaction temperature and reaction time can not only improve the efficiency of the esterification reaction, but also prevent aconitic acid obtained from fermentation from reducing the purity of aconitates and increasing the chromaticity of aconitates.

When the temperature is lower than 75°C, the efficiency of the esterification reaction will decrease significantly; and an extended reaction time is required to achieve higher utilization rate or yield. Polysaccharides hardly undergo esterification reactions due to their large steric hindrance from bulky structures. However, a prolonged reaction time will cause acidic catalysts to promote the hydrolysis of polysaccharides into monosaccharides, such that monosaccharides with abundant hydroxyl groups compete with alcohols for esterification with aconitic acid. When the temperature exceeds 145°C, polysaccharides will undergo a caramelization reaction, and dark brown sugar-dehydrated polymers that deepen the color of aconitates will be produced from caramelization. A small amount of these sugar-dehydrated polymers can be absorbed by activated carbon, but excessive sugar-dehydrated polymers are difficult to remove and will block adsorption pores of activated carbon, thereby reducing its adsorption efficiency of the activated carbon. Therefore, aconitates with low chromaticity and high purity can be only obtained under this specific temperature and through effective treatment.

The esterification time in the present disclosure is also determined by aconitic acid from fermentation sources. Aconitic acid from fermentation sources significantly influences the reaction rate due to a high content of impurities. If the time is too short, the reaction will be incomplete, making it difficult to achieve a higher yield. If the time is too long, a large number of polysaccharides will hydrolyze in the reaction; the resulting monosaccharides from hydrolysis will then compete with alcohols for the esterification reaction, leading to low purity of the product. Therefore, aconitates with high purity, low chromaticity, and high yield can only be obtained by performing esterification reaction within this time period.

Optionally, in step (1), a water-carrying agent and a catalyst are added to a mixture of aconitic acid and alcohol solvent with a molar ratio of 1:(3-9), and the mixture is agitated and reacted at 75°C to 145°C for 2 to 8 h to obtain a solution containing aconitate. The solution containing aconitate is neutralized with an alkaline washing solution, and then the liquid is separated to obtain an upper organic phase.

Optionally, the agitation speed is 100 to 1200 rpm.

Optionally, the water-carrying agent is selected from at least one of toluene, benzene, and cyclohexane, optionally toluene, and a molar ratio of the water-carrying agent to aconitic acid is (0-5):1.

Optionally, the weight ratio of the catalyst to aconitic acid is 1:(2-100), and the catalyst is selected from homogeneous catalysts and heterogeneous catalysts. When a heterogeneous catalyst is used, no water-carrying agent needs to be added, and the molar ratio of the water-carrying agent to aconitic acid is 0:1. When a homogeneous catalyst is used, a water-carrying agent needs to be added, and the molar ratio of the water-carrying agent to aconitic acid is (1-5):1.

Optionally, the homogeneous catalyst is selected from at least one of p-toluenesulfonic acid, p-toluenesulfonic acid monohydrate, dinitrobenzoic acid, ethylenediaminetetraacetic acid, trichloroacetic acid, methanesulfonic acid, ethanesulfonic acid, propanesulfonic acid, butanesulfonic acid, alkyl sulfuric acid, dodecylbenzenesulfonic acid, amidosulfonic acid, tetrabutyl titanate, isopropyl titanate, stannous octoate, stannous chloride, antimony acetate, cobalt acetate, and manganese acetate, optionally p-toluenesulfonic acid or p-toluenesulfonic acid monohydrate. The above homogeneous catalysts can maximize the efficiency, completeness, and rapidity of the esterification reaction, reduce the

generation of by-products, and have high esterification reaction efficiency, good product quality, and low energy consumption. In the presence of this homogeneous catalyst, toluene, benzene, or cyclohexane is used as a water-carrying agent to carry water away from the reaction and separate the water, and at the same time, aconitic acid can be effectively mixed with alcohols to promote catalytic efficiency and improve the reaction speed.

Optionally, the heterogeneous catalyst is selected from at least one of sodium bisulfate monohydrate, sodium bisulfate, potassium bisulfate, copper sulfate, ferric sulfate, titanium sulfate, aluminum sulfate, jarosite, cerium sulfate, lanthanum sulfate, magnesium sulfate, cadmium sulfate, ferrous sulfate, ammonium ferric sulfate, cerous methanesulfonate, ferric chloride, tin tetrachloride, titanium chloride, rare earth chloride, potassium hydroxide, sodium hydroxide, potassium bicarbonate, sodium bicarbonate, sodium carbonate, anion exchange resin, strong acid cation exchange resin, weak acid cation exchange resin, potassium amidosulfonate, sodium amidosulfonate, tetrabutylammonium chloride, tetrabutylammonium bromide, and copper methanesulfonate, optionally sodium bisulfate monohydrate or sodium bisulfate. This heterogeneous catalyst can realize efficient, sufficient, and rapid completion of the esterification reaction under relatively low temperature, reduce the generation of by-products, and have high esterification reaction efficiency, good product quality, and low energy consumption. The heterogeneous catalyst is easy to separate and facilitates post-treatment. The recovered catalyst can be reused, and further reduces industrial costs.

Concentrated sulfuric acid is used as a catalyst in traditional esterification reactions. Concentrated sulfuric acid has both strong oxidizing property and strong acidity. Although concentrated sulfuric acid can catalyze the reaction, it will carbonize the mycelia and proteins in aconitic acid obtained from fermentation, making aconitates complex and difficult to handle. The above homogeneous catalysts and heterogeneous catalysts replace traditional concentrated sulfuric acid. The catalyst does not have both strong oxidizing property and acidity, and can effectively inhibit side reactions in the esterification reaction. For example, p-toluenesulfonic acid has strong acidity but no oxidizing property. Compared with concentrated sulfuric acid, sodium bisulfate itself has weaker acidity, fewer side reactions, and higher chromaticity and purity of the product. Therefore, the types of the above homogeneous catalysts and heterogeneous catalysts are set based on aconitic acid obtained from fermentation, so as to further improve the yield and purity of aconitates and reduce the chromaticity of aconitates in the esterification reaction between aconitic acid and alcohols.

Optionally, when a heterogeneous catalyst is used, after the reaction between aconitic acid and alcohols, filtration is performed to obtain the reaction solution.

Optionally, the reaction solution is subjected to three rounds of alkaline washing for neutralization using an alkaline washing solution, the residual alkaline washing solution and salts generated by the alkaline washing reaction in the solution are removed by three rounds of water washing, and the liquid is separated and the upper organic phase is collected.

Optionally, the solute of the alkaline washing solution is at least one of NaOH, $NaHCO_3$, and $Na_2CO_3$.

Optionally, the mass fraction of the solute in the alkaline washing solution is 5% to 25%.

Optionally, the temperature of the reaction for neutralizing the reaction solution by alkaline washing with the alkaline washing solution is 20°C to 70°C, and the reaction time is 15 to 30 min.

Optionally, the temperature for solvent removal in step (2) is 40°C to 100°C, and the vacuum degree is 0.01 mbar to 100 mbar.

The impurities in aconitic acid are mainly monosaccharides and polysaccharides. Monosaccharides have small molecular weights, such as mannose, glucose, and galactose. Their molecules contain a large number of hydroxyl groups, which compete with alcohols for the esterification reaction, thereby greatly reducing the purity of the product. Polysaccharides have large molecular weights and complex structures, contain a large number of hydroxyl groups, and have large steric hindrance. Although the rate of their reaction with aconitic acid is slow, once esterification occurs, their larger steric hindrance makes it difficult for the other two carboxylic acids to be esterified, thereby affecting the yield and purity of the product. Therefore, both monosaccharides and polysaccharides can greatly reduce the rate and purity of the esterification reaction of aconitic acid.

Therefore, at the above temperature, the rate of the reaction between aconitic acid and alcohols is much higher than the rate of the reaction between impurities (sugars) and alcohols. The reaction dosages of the catalyst and alcohols enable aconitic acid to undergo esterification with alcohols as much as possible, thereby increasing the reaction conversion rate of aconitic acid. Moreover, the above unreacted sugars and other impurities can also be removed through the first-step decolorization and the second-step decolorization processes, to further improve the purity of aconitates.

To solve the problem of low purity of aconitates produced due to high content of pigments and impurities in bio-based trans-Aconitic acid, the present disclosure provides a preparation method of high-purity aconitate. In this method, first-step decolorization is performed before the solvent removal step: first, the fat-soluble pigments in aconitates are oxidized and destroyed to reduce the chromaticity impact caused by fat-soluble pigments. After solvent removal, a decolorizing agent is used to selectively remove the water-soluble pigments and the destroyed fat-soluble pigments from the aconitates. The combination of the two can enhance the decolorization and impurity removal effects for aconitates, thereby obtaining high-purity aconitates and expanding the application range of aconitates.

On the other hand, the present disclosure also provides a high-purity aconitate prepared by the above method.
Further, the purity of the high-purity aconitate is not less than 99%.
Further, the chromaticity of the high-purity aconitate is not higher than 50 Pt-Co.
Further, the content of fat-soluble pigments in the high-purity aconitate is less than 1000 ppm, and the content of water-soluble pigments is less than 1000 ppm.
Further, the structural formula of the high-purity aconitate is as follows:

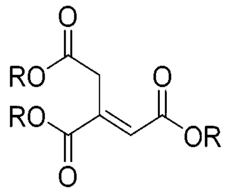
,

where R is an alkyl group.
Further, the structural formula of the high-purity aconitate is as follows:

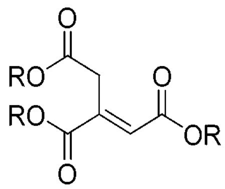
,

where R is an alkyl group having no more than 5 carbon atoms.
Further, the structural formula of the high-purity aconitate is as follows:

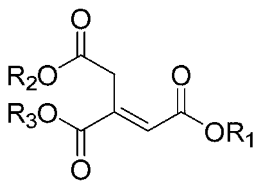
,

where $R_1$, $R_2$ and $R_3$ are each independently selected from one of alkyl groups having 1 to 5 carbon atoms, alkyl groups having more than 6 carbon atoms, alkyl groups having 1 to 5 carbon atoms and substituted by chlorine atoms or alkyl groups containing aromatic groups, and at least two of $R_1$, $R_2$ and $R_3$ are different.
Further, the structural formula of the high-purity aconitate is as follows:

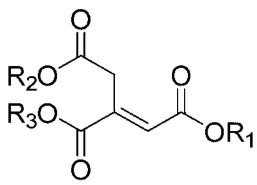
,

where $R_1$, $R_2$ and $R_3$ are independently selected from alkyl groups having 6 to 18 carbon atoms.
On the other hand, the present disclosure also provides a composition containing the above high-purity aconitate.
Further, the composition includes: 100-120 parts of PVC resin, 5-90 parts of high-purity aconitate, 0.5-10 parts of lubricant, and 2-10 parts of stabilizer.
Optionally, the high-purity aconitate is a mixed aconitate, and the structural formula of the mixed aconitate is as follows:

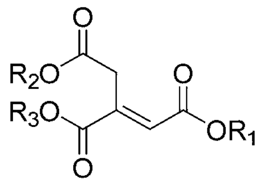

,

where $R_1$, $R_2$, and $R_3$ are each independently selected from one of alkyl groups having 1 to 5 carbon atoms, alkyl groups having more than 6 carbon atoms, alkyl groups having 2 to 5 carbon atoms and substituted by chlorine atoms, or aryl-containing alkyl groups, and at least two of $R_1$, $R_2$, and $R_3$ are different.

The above aconitate is a mixture of complex aconitates. At least two of $R_1$, $R_2$, and $R_3$ of the complex aconitates are different, which can make a single aconitate have different substituents, thereby coordinating the tolerance performance of the aconitate itself and the plasticizing performance on PVC, improving the quality of PVC products, and expanding the application range of PVC products.

Optionally, at least one of $R_1$, $R_2$, and $R_3$ is selected from alkyl groups having 1 to 5 carbon atoms, and at least one is selected from alkyl groups having more than 6 carbon atoms.

The shorter the alkyl chain of the substituent in the aconitate, the larger the proportion of polar ester groups, and the better the compatibility with PVC. The longer the alkyl chain, the better the flexibility, and the better the lubricity between PVC molecular chains, and theoretically, the better the plasticizing effect should be. However, the present disclosure found that if the alkyl chain is only short, the plasticizer can only be embedded between PVC molecular chains but cannot shield the strong interaction between PVC molecular chains (poor shielding effect), so the plasticizing effect is not necessarily good. If the substituted alkyl chain is only long, the proportion of polar ester groups becomes smaller and smaller, and the compatibility with PVC is poor. Even if it has a large steric hindrance to shield the interaction between PVC, it is difficult to be embedded between PVC molecular chains due to poor compatibility (poor solvent effect), and it is difficult to dissolve the "connection points" between PVC molecular chains, resulting in a decrease in plasticizing effect. The mixed aconitate contains both short-chain alkyl groups having 1 to 5 carbon atoms, which increases the polarity of the mixed aconitate, and improves its resistance to organic solvent extraction in PVC plastics, and has good compatibility with PVC. It also contains long-chain alkyl groups having more than 6 carbon atoms, and can improve its volatility resistance, migration resistance, and water solvent extraction resistance in PVC. The above short-chain alkyl groups and long-chain alkyl groups work synergistically, so as to obtain a mixed aconitate that can not only improve various plasticizing performances of PVC but also take into account the tolerance performance of the aconitate itself in PVC, and can also improve its resistance to extraction in different solvents.

Optionally, at least one of $R_1$, $R_2$, and $R_3$ is selected from butyl groups, and at least one is selected from isooctyl groups;

optionally, two of $R_1$, $R_2$ and $R_3$ are selected from butyl groups and one is selected from isooctyl groups, or two of $R_1$, $R_2$ and $R_3$ are selected from isooctyl groups and one is selected from butyl groups.

Butyl itself has a certain steric hindrance and can effectively shield the interaction between PVC molecular chains. When butyl is in the structure of aconitate, since it has not many alkyl groups, the polarity of the aconitate is large, and it has good compatibility with PVC. Butyl has a stronger shielding effect than alkyl groups with shorter chains and a better compatibility effect than alkyl groups with longer chains. Therefore, butyl aconitate has the best plasticizing effect. However, in addition to the plasticizing effect, materials also need better stability. Isooctyl (2-ethylhexyl) itself has a larger molecular chain, has good thermal stability, and has a branched chain, making it more difficult to migrate to the outside. Therefore, the combination of butyl and isooctyl can make the aconitate material have both plasticizing performance and stability performance. By adjusting the ratio of butyl to isooctyl, the focus of the aconitate plasticizer can be further adjusted, such as more efficient plasticization or more long-term stability.

Optionally, at least one of $R_1$, $R_2$, and $R_3$ is selected from alkyl groups having 1 to 3 carbon atoms, and at least one is selected from aryl-containing alkyl groups.

When at least one of $R_1$, $R_2$, and $R_3$ is selected from alkyl groups having 1 to 3 carbon atoms, the substituted alkyl chain itself is easy to volatilize and migrate. At this time, even if other substituents introduce long chains having more than 6 carbon atoms, it is difficult to change the limitation of the short-chain alkyl groups. By introducing aryl-containing alkyl groups in the molecular structure, a benzene ring contained has a large steric hindrance, and can make up for the insufficient spatial shielding effect and poor heat resistance of short-chain esters. The above mixed aconitate has good plasticizing performance, volatility resistance, migration resistance, and heat resistance.

Optionally, at least one of $R_1$, $R_2$, and $R_3$ is selected from alkyl groups having more than 10 carbon atoms, and at least one is selected from alkyl groups having 2 to 5 carbon atoms and substituted by chlorine atoms.

When at least one of $R_1$, $R_2$, and $R_3$ is selected from alkyl groups having more than 10 carbon atoms, the substituted alkyl chain itself is difficult to be compatible with PVC. Even if short chains are introduced, the compatibility cannot be effectively improved. Therefore, introducing alkyl groups substituted by Cl can make the molecules of the mixed

aconitate have Cl atoms. The Cl atoms can have a strong dipole moment with H on PVC, thereby increasing the compatibility with PVC and achieving the purpose of enhancing the interaction.

Optionally, a K value of the PVC resin is 55 to 77, and the polydispersity index is 3 to 5; optionally, the polydispersity index of the PVC resin is 3.5 to 4.5.

When the PVC resin with the above K value is used, the prepared PVC material has better toughness. The reason is that the macromolecular chains are entangled with each other and react to cross-link with the mixed aconitate, lubricant, and stabilizer components to form a stable cross-linked network structure, thereby having better toughness, plasticity, and tensile resistance.

The inventors found that when the polydispersity index of the PVC resin used is 3 to 5, the composite material has the optimal toughness and plasticity. This may be because when the polydispersity index of the PVC resin is 3 to 5, the material contains PVC resins with various molecular weights. Resin materials with different molecular weights are mixed to better react with the mixed aconitates containing different alkyl groups, thereby effectively increasing the dissolution amount of the mixed aconitates. Resin materials can be effectively combined to increase the interaction between molecular chains, making the prepared PVC material have high toughness, high stability, plasticity and good stability, and no phase separation easily occurs.

Optionally, the lubricant is selected from at least one of oxidized polyethylene wax, glycerol stearate, ethylene bis-stearamide synthetic wax, paraffin wax, and polyethylene wax, optionally oxidized polyethylene wax.

The compatibility between the above lubricant and PVC material is generally low. Therefore, after mixing, the lubricant will migrate to the product surface and form a certain protective layer. When used together with mixed aconitate which is a plasticizer resistant to extraction by various solvents, the lubricant exhibits a synergistic effect. The lubricant on the surface enables easier demolding of the product during processing, and prevents auxiliary agents from migrating outwards to a certain extent.

Optionally, the stabilizer is selected from at least one of organotin heat stabilizers, dibutyltin laurate, dibutyl/octyltin sulfate, octyltin maleate, and methyltin mercaptide, optionally octyltin maleate.

The above heat stabilizers are non-toxic, have good transparency and no odor, and are widely used in industries such as food and medical and health care with high safety requirements. When used with the plasticizer such as mixed aconitate which has various safety properties, the heat stabilizer and plasticizer have a synergistic effect, and together improve the tolerance performance of the material in complex environments.

Further, the composition includes the following components in parts by weight: 100 parts of PVC resin, 1-10 parts of cold-resistant modifier, 5-90 parts of high-purity aconitate, 0.5-10 parts of lubricant, and 3-20 parts of stabilizer.

Optionally, the high-purity aconitate is a single aconitate, and the structural formula of the single aconitate is as follows:

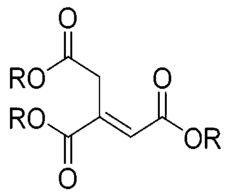,

where R is an alkyl group having no more than 5 carbon atoms.

A single aconitate is used, and each R substituent in its molecular structure is the same. The same substituents can make the aconitate have uniform compatibility and dispersibility in different systems, and uniform distribution allows the composite material to maintain long-term stability. If the temperature is relatively low and R has different molecular structures, R will show a more nonuniform distribution under lower free volume, resulting in poor local low-temperature performance of the material.

Optionally, the $T_g$ (glass transition temperature) of the high cold-resistant PVC composite material is not higher than 50°C.

Optionally, the $T_g$ of the high cold-resistant PVC composite material is not higher than 30°C.

Moreover, when R is an alkyl group having no more than 5 carbon atoms, and when it is added to a PVC material system as a plasticizer, the main structure of its double bond has strong rigidity similar to the benzene ring structure. At the same time, the three ester groups on the molecule and the rigid main body work together to make the plasticizer play a huge supporting role between the PVC molecular chains. The strong supporting effect significantly increases the free volume of the PVC material. The material can only shrink to the free volume required for vitrification at a much lower temperature; therefore, its glass transition temperature is noticeably reduced, and the low-temperature performance of the PVC composite material is improved. At the same time, the inventors found that the short-chain esters formed by alkyl groups having no more than 5 carbon atoms have strong polarity, which further enhances the compatibility with PVC molecular

chains and cold-resistant modifiers. Good compatibility is a prerequisite for exerting a supporting effect of the aconitate structure. Only when the aconitate molecules can enter between the PVC molecular chains and the molecular chains of the modified cold-resistant agent can their own supporting effect be exerted. Therefore, good compatibility and excellent low-temperature resistance are produced under the synergy of the short-chain ester of aconitic acid and the main rigid structure, and the two are indispensable. The single aconitate used in the present disclosure is a short-chain aconitate. Owing to its good compatibility, the single aconitate can effectively increase the maximum addition amount of the plasticizer, making the composite material achieve higher plasticity. On the one hand, the processing difficulty of the material can be effectively lowered. On the other hand, the application field and usage scope of the material are expanded.

Optionally, the acid value of the single aconitate is less than 0.15 mg KOH/g, the water content is less than 0.2%, and the purity is greater than 97%.

Optionally, the solubility of the single aconitate in 100 parts of PVC resin is greater than or equal to 50 parts, the single aconitate is liquid at 25°C, and the single aconitate is colorless and transparent or light yellow at 25°C.

Optionally, the purity of the single aconitate is not less than 99%, and the chromaticity is not higher than 50 Pt-Co.

The acid value of the single aconitate ensures that no large number of acidic substances will corrode and degrade the material during material processing, which would otherwise affect the basic properties and internal structure of the materials. Therefore, the compatibility and cold resistance of the composite material are stably improved. A low water content can prevent the phenomenon of insufficient effective components during material processing, thereby avoiding the situation that the material cannot achieve an expected plasticizing effect at the required parts. The higher the purity of the single aconitate, the less the impurity content in the single aconitate. If there are too many impurities, the actual addition amount of the single aconitate will be smaller and the contact with the PVC resin will be less, thereby reducing the compatibility of the single aconitate in PVC and increasing the $T_g$ of the composite material, and reducing the cold resistance. Therefore, the purity of the above single aconitate can further improve the compatibility and plasticizing performance of the single aconitate in PVC and the cold resistance of the composite material. Moreover, the chromaticity of the single aconitate which is not higher than 50 Pt-Co can reduce the coloring effect of the plasticizer on the PVC material, and further improve the appearance controllability of the composite material.

Optionally, the degree of polymerization of the PVC resin is 650 to 1800, the polydispersity index is 3 to 5, and the K value is 55 to 77;

optionally, the polydispersity index of the PVC resin is 3.5 to 4.5.

When the PVC resin with the above degree of polymerization is used, the prepared PVC material has better toughness. The reason is that the macromolecular chains are entangled with each other and react to cross-link with the single aconitate, lubricant, and stabilizer components to form a stable cross-linked network structure, so as to have better toughness, plasticity, tensile resistance, and cold resistance.

The inventors found that when the polydispersity index of the PVC resin used is 3 to 5, the composite material has the optimal toughness and plasticity. This may be because when the polydispersity index of the PVC resin is 3 to 5, the material contains PVC resins with various molecular weights. The resin materials with different molecular weights are mixed to better increase the free volume inside the PVC, effectively increase the dissolution amount of the aconitate plasticizer, and can be effectively combined to increase the interaction between the plasticizer and the molecular chains, thereby making the prepared PVC material have high toughness, high stability and plasticity, and good stability, and no phase separation easily occurs.

Optionally, the cold-resistant modifier is at least one of chlorinated polyethylene (CPE) and ethylene-vinyl acetate copolymer (EVA);

optionally, the chlorine content in the chlorinated polyethylene is not less than 25%, and the VA content in the ethylene-vinyl acetate copolymer is 5% to 40%.

The inventors found that cold-resistant modifiers themselves have a relatively low $T_g$. For example, the $T_g$ of CPE is about -25°C, and the $T_g$ of EVA is about -34°C. The chlorine content in chlorinated polyethylene is not less than 25%, and the VA content in the ethylene-vinyl acetate copolymer is 5% to 40%. Only when the above substances used as cold-resistant modifiers are added to the composite material can the cold resistance of PVC be effectively improved. However, the compatibility between the above cold-resistant modifier and PVC is only moderate. In contrast, the single aconitate used in the present disclosure can exert a strong solvent effect, to effectively improve the compatibility between the cold-resistant modifier and PVC and promote uniform mixing among the three components. The single aconitate not only plays a role in plasticizing PVC, but also acts as a compatibilizer for the cold-resistant modifier. Therefore, the aconitate and the cold-resistant modifier play a synergistic role, and the two further improve the low-temperature performance of the PVC composite material.

Optionally, the lubricant is 2 to 4 parts;

the lubricant is selected from at least one of oxidized polyethylene wax, glycerol stearate, ethylene bis-stearamide synthetic wax, paraffin wax, and polyethylene wax, optionally ethylene bis-stearamide synthetic wax.

The above lubricant can lubricate the PVC resin and the single aconitate, help the single aconitate to quickly contact the PVC resin, and further improve the dispersion speed of the single aconitate, and the lubricant is added to

lubricate the molecular chains of the PVC resin, thereby increasing the mobility of the molecular chains, and further facilitating the aconitate in reducing the $T_g$ of the composite material and improving the cold resistance of the composite material.

Optionally, the stabilizer is 4 to 7 parts;

the stabilizer is at least one of heat stabilizers and light stabilizers;
optionally, the weight ratio of the heat stabilizer to the light stabilizer is 2-10:0.5-4, optionally 3-5:1-2.

The above light stabilizer and heat stabilizer are combined to enhance the light stability and thermal stability of the composite material, so as to improve the light resistance and heat resistance of the composite material and expand the service temperature range of the composite material.

Optionally, the heat stabilizer is selected from calcium stearate and zinc stearate in a weight ratio of 0.1 to 5:1.

When calcium stearate and zinc stearate with the above weight ratio are used as heat stabilizers, they can work synergistically with the light stabilizer to further inhibit the degradation of the PVC resin, and the plasticizing effect is enhanced. The reason lies in that calcium stearate and zinc stearate are easily dispersed and have high compatibility in the system added with short-chain single aconitate. When the two are used in combination, they can be effectively dispersed in the system and will not migrate too much. Combined with the single aconitate with the specific structure, the prepared PVC composite material can have low hardness, high toughness, cold resistance, and plasticity.

Optionally, the light stabilizer is at least one of benzophenones, benzotriazoles, or triazines;

the benzophenones include at least one of UV-9, UV-531, UV-356, and UV-284;
the benzotriazoles include at least one of UV-327, UV-P, UV-320, UV-328, UV-350, UV-326, and UV-234.

Further, the composition includes the following components in parts by weight: 100 parts of base material, 5-90 parts of high-purity aconitate, 10-60 parts of inorganic filler, 1-5 parts of organotin compound, 0.5-2 parts of rare earth stearate, 0-2 parts of antistatic agent, and 0.5-2 parts of flame retardant; the base material is selected from at least one of cross-linked PVC resin, non-cross-linked PVC resin, cross-linked nitrile rubber, and thermoplastic nitrile rubber.

Optionally, the structural formula of the high-purity aconitate is as follows, and the high-purity aconitate is used as a plasticizer in the composition:

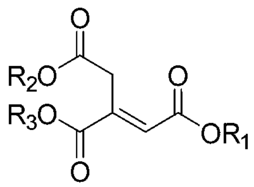
,

where $R_1$, $R_2$, and $R_3$ are independently selected from alkyl groups having 6 to 18 carbon atoms;
the plasticizer is a reaction product of aconitic acid and alkyl alcohols having 6 to 18 carbon atoms. The substituents $R_1$, $R_2$, and $R_3$ in the plasticizer can be either identical or different. When the length of the alkyl chain in the plasticizer is less than 6 carbon atoms, the relative molecular weight of the plasticizer decreases. Therefore, the mobility and diffusion ability of the plasticizer in the base material increase, making it easier for the plasticizer to volatilize from the base material, and resulting in poor volatility resistance of the composite material.

Therefore, when $R_1$, $R_2$ and $R_3$ in the plasticizer of the present disclosure are selected from alkyl groups having 6 to 18 carbon atoms, the substituent molecular chain of the plasticizer is longer, and the number of alkyl groups is also larger, thereby effectively increasing the relative molecular weight of the aconitate, making it more difficult to volatilize from the high-temperature environment, and improving the volatility resistance and migration resistance of the plasticizer in the base material. At the same time, the longer alkyl chain endows the plasticizer with a larger steric hindrance volume. When the longer alkyl chain is between the PVC molecular chains, it can effectively shield the interaction between the PVC molecular chains, thereby enhancing the mobility of the PVC molecular chains and achieving the plasticizing effect.

The main structure of the aconitate itself contains double bonds that cannot rotate, and similar to the benzene ring, this kind of rigid main structure can play a supporting role between the PVC molecular chains, further amplifying the shielding effect of the long-chain alkyl groups and achieving a good plasticizing effect. Therefore, under the combined action of the double-bond structure and the long-chain structure of aconitic acid, this kind of high-boiling-point long-chain aconitate can make the PVC material have good heat resistance and can also effectively plasticize PVC. The ratio of the above base material to the plasticizer can improve the plasticizing performance of the base material and improve the

stability of the base material. Compared with the commonly used bio-based citric acid plasticizers, the aconitate also increases the maximum addition amount of the plasticizer. The possible reason is that there is no active hydroxyl group in the aconitate, which reduces side reactions, then the maximum addition amount of the plasticizer is increased to a certain extent, and the mechanical properties and high-temperature resistance of the composite material are improved.

If the addition amount of the above plasticizer is too much, the mechanical properties of the composite material will be reduced. The possible reason lies in that the aconitate plasticizer is distributed between the molecular chains of the base material. Too much aconitate plasticizer is easy to form aggregates. Since the aconitate has ester groups, the aggregates can be polar. Too many polar aggregates increase the defects of the base material, and stress damage easily occurs at the aggregates, resulting in nonuniform distribution of the mechanical strength of the composite material. Too little plasticizer cannot play a role of plasticization, thereby failing to improve the volatility resistance, migration resistance, mechanical properties, and high-temperature resistance of the composite material.

Optionally, the amount of the plasticizer is 30-60 parts, more optionally 50 parts.

Optionally, the base material is selected from non-cross-linked PVC resin, and the weight-average molecular weight of the non-cross-linked PVC resin is 40,000 to 100,000, optionally 60,000 to 80,000.

When the non-cross-linked PVC resin with the above molecular weight is used, the molecular chains of the PVC resin itself can be entangled with each other, to improve the toughness of the composite material, and at the same time increase the compatibility with the plasticizer. The molecular chains of the PVC resin can be cross-linked with the plasticizer, organotin compound, rare earth stearate, antistatic agent, and flame-retardant components to form a stable cross-linked network structure, thereby improving the mechanical properties and high-temperature resistance of the composite material. If the molecular weight is too high, the mechanical properties of the composite material will be reduced.

Optionally, the polydispersity index of the non-cross-linked PVC resin is 3 to 5, optionally 3.5 to 4.5;
the K value of the non-cross-linked PVC resin is 55 to 77.

The inventors found that when the polydispersity index of the PVC resin used is 3 to 5, the composite material has the optimal toughness and strength. This may be because when the polydispersity index of the PVC resin is 3 to 5, the material contains PVC resins with long chains and short chains. The bent ends of the long-chain PVC resin base material complement the short-chain PVC resin base material. This facilitates the insertion of the aconitate plasticizer between the molecular chains of the PVC base material, enabling better reaction with aconitate plasticizers containing different alkyl groups. This also effectively increases the solubility of the aconitate plasticizer, promotes effective bonding with the resin, and enhances the intermolecular forces between molecular chains. As a result, the prepared composite material exhibits high toughness, high stability, and good plasticity, while maintaining excellent stability and being less prone to issues such as phase separation.

Optionally, the acid value of the plasticizer is less than 0.1 mg KOH/g, the water content is less than 0.2%, and the purity is greater than 97%.

Optionally, the purity of the plasticizer is not less than 99%, and the chromaticity is not higher than 50 Pt-Co.

The acid value of the plasticizer ensures that no large number of acidic substances will corrode and degrade the material during use, which would otherwise affect the basic properties and internal structure of the material. Therefore, the material maintains excellent thermal stability and migration resistance. A lower water content increases the effective components of the material, and water is easy to volatilize to the outside, especially at high temperatures. When the water content of the plasticizer is too high, the volatility resistance and migration resistance of the material will be greatly reduced, thereby reducing the thermal stability of the material. The higher the purity of the plasticizer, the more components that can play the plasticizing role, and the better the plasticizing effect. When the impurity content of the plasticizer increases, low-boiling-point impurities will volatilize and migrate to the outside under long-term use or high temperature, resulting in poor volatility resistance of the product. At the same time, even if the impurities do not migrate to the outside, they will not play a role of plasticization. Therefore, at a certain addition amount, a lower purity of the plasticizer will lead to a decrease in the volatility resistance and plasticizing effect of the material. Therefore, the purity of the above plasticizer can further improve the plasticizing property, volatility resistance, and migration resistance of the plasticizer in the base material, and improve the mechanical strength and high-temperature resistance of the composite material. The chromaticity of the plasticizer which is not higher than 50 Pt-Co can reduce the coloring effect of the plasticizer on the base material, and further improve the appearance controllability of the composite material.

Optionally, the solubility of the plasticizer in 100 parts of PVC resin is greater than or equal to 50 parts, the plasticizer is liquid at 25°C, and the plasticizer is colorless and transparent or light yellow at 25°C.

The more ester groups in the plasticizer, the better the compatibility with PVC, and the stronger the ability of the plasticizer to dissolve the "cross-linking points" between the PVC molecular chains (solvent effect). Although the long-chain alkyl groups will weaken the polarity of the plasticizer, more ester groups make the aconitate still have good compatibility and high solubility with PVC in the presence of long-chain alkyl groups.

Optionally, the inorganic filler is selected from at least one of talc powder, barium sulfate, calcium carbonate, titanium dioxide, and ceramic clay.

The inorganic filler itself can be added to improve the thermal stability of the material, but at the same time, it will increase the hardness of PVC and weaken the effect of the plasticizer. For ordinary plasticizers, the longer the alkyl chain, the better the high-temperature resistance. However, this also leads to poorer improvement in the mobility of PVC chains, resulting in nonuniform dispersion of inorganic fillers in PVC. Therefore, it is difficult for ordinary long-chain high-temperature-resistant plasticizers to balance the plasticizing effect and high-temperature resistance. However, the plasticizer in the present disclosure is a long-chain aconitate, which not only has long chains but also has a rigid main structure containing double bonds and three ester groups. The rigid main structure and long chains endow the material with a larger free volume between them, promote the mobility of molecular chains, and enable the material to effectively accommodate the inorganic filler. At the same time, the uniformly distributed inorganic filler increases the contact area with the PVC material, thereby further expanding the volume between molecular chains, making it easier for the aconitate to plasticize the material. Therefore, the plasticizer and the inorganic filler in the present disclosure have a synergistic effect. The plasticizing effect of the aconitate will not be significantly weakened due to the addition of the inorganic filler. The inorganic filler can be uniformly dispersed due to the aconitate to further increase the heat resistance.

Optionally, the particle size of the inorganic filler is 80 to 100 nm. If the particle size of the inorganic filler is too large, the specific surface area is too small, the reinforcing effect of the filler on the composite material is poor, and the appearance of the product will be influenced. If the particle size is too small, the surface energy will increase, the particles will agglomerate seriously, and it is difficult for the particles to be completely dispersed during processing, thereby resulting in a decrease in the performance of the composite material and the appearance of particles on the surface.

Pure PVC molecular chains have poor mobility. Even if inorganic fillers have an appropriate particle size, agglomeration may still occur, resulting in an insignificant increase in material performance. However, the plasticizer in the present disclosure is a long-chain aconitate, which can effectively enhance the mobility of PVC chains and achieve good dispersion of the three components. However, other plasticizers fail to achieve such an effect. Plasticizers that can improve molecular chain mobility generally have poor volatility resistance, however, plasticizers with good volatility resistance have either fewer ester groups or benzene rings with poor compatibility, which results in insufficient mobility imparted to the PVC molecular chains. Therefore, when the plasticizer in the present disclosure promotes the movement of PVC molecular chains, the inorganic fillers with a particle size of 80 to 100 nm can achieve an optimal dispersion effect and exhibit a synergistic reinforcing and plasticizing effect with the plasticizer.

Optionally, the weight ratio of the organotin compound to the rare earth stearate is (0.5-4):1, preferably (1-2):1.

Organotin compounds can effectively slow down the decomposition of HCl during the high-temperature processing of PVC, and improve the processing stability and thermal decomposition resistance of PVC materials. Rare earth stearates act as lubricants, and promote more uniform mixing of products and additives during processing. When composite materials are stored for a long time, processing additives are prone to precipitate, which may lead to a decrease in the stability of the materials. The composite material in the present disclosure uses organotin compounds and rare earth stearates in a weight ratio of (0.5-4):1, which improves the dispersion performance of the composite material system and enhances the mechanical properties and stability of the composite material. Additionally, the organotin compounds and rare earth stearates themselves have a sufficiently large molecular weight, making them difficult to migrate from the material to the outside. At the same time, their flexible alkyl chains can further enhance the effect of the plasticizer in shielding the interactions between PVC molecular chains under a large steric hindrance volume of the aconitate, and promote the mobility of PVC molecular chains. Meanwhile, both the two can absorb HCl decomposed from PVC during long-term use, and ensure long-term stability of the material during use.

As small molecules, plasticizers exist between macromolecular chains of the base material. The interactions between plasticizers and the base material molecular chains replace the interactions between the base material molecular chains, thereby increasing the free volume of the base material. This increase in the free volume enhances the dispersion effect of organotin compounds, inorganic fillers, rare earth stearates, antistatic agents, and flame retardants in the base material, enhances their bonding effect with the base material macromolecules, reduces the precipitation of the above components, and effectively improves the mechanical properties and stability of the composite material.

Optionally, the organotin compounds include methyltin mercaptide, di-n-butyltin maleate, di-n-octyltin bis(2-ethylhexyl mercaptoacetate), dimethyltin bis(2-ethylhexyl mercaptoacetate), di-n-butyltin dodecyl mercaptide, and di-n-octyltin β-mercaptopropionate.

The rare earth stearates include lanthanum stearate and cerium stearate.

Optionally, the antistatic agent is a quaternary ammonium salt antistatic agent.

Optionally, the quaternary ammonium salt antistatic agent is selected from at least one of stearyltrimethylammonium chloride, dilauryldimethylammonium chloride, didecyldimethylammonium chloride, dodecyltrimethylammonium chloride, tetradecyltrimethylammonium chloride, and cetyltrimethylammonium chloride.

The flame retardant is selected from at least one of chlorinated paraffin, chlorinated polyethylene, tricresyl phosphate, tris(2-chloroethyl) phosphate, antimony trioxide, zinc borate, and magnesium hydroxide.

Optionally, the composite material has a volatility loss rate of no more than 2.32% at 70°C for 24 h, a migration rate to PE of no more than 1.93%, and a migration rate to NR of no more than 2.02%.

Optionally, the composite material has a volatility loss rate of no more than 1.43% at 70°C for 24 h, a migration rate to PE of no more than 1.25%, and a migration rate to NR of no more than 1.36%.

More preferably, the composite material has a volatility loss rate of no more than 0.84% at 70°C for 24 h, a migration rate to PE of no more than 0.28%, and a migration rate to NR of no more than 0.29%.

On the other hand, the present disclosure also provides a preparation method of the composition, including the following steps:

S1: mixing the components of the composition to obtain a premix;
S2: adding the premix into a high-speed mixer, mixing at 30°C to 80°C for 10 to 60 min, then adding into two-roll open mixing mill, and mixing at 130°C to 190°C for 5 to 20 min to obtain a mixed compound; and
S3: placing the mixed compound in a plate vulcanizer, and vulcanizing at 140°C to 190°C under a pressure of 10 to 15 MPa for 5 to 15 min to obtain the composition.

On the other hand, the present disclosure also provides the applications of the high-purity aconitate or the composition in improving cold resistance, heat resistance, volatility resistance, stability and plasticizing performance.

On the other hand, the present disclosure also provides the applications of the high-purity aconitate or the composition in household appliance housings, printer housings, charging gun housings, automobile taillights, PVC plastic films used in winter greenhouses, refrigerator door seals, snow boot soles, snowboards and blood storage bags.

The beneficial effects of the present disclosure include but are not limited to:

1. The present disclosure provides a recombinant *Aspergillus niger* that produces trans-Aconitic acid by using *Aspergillus niger* as a starting strains and heterologously expressing aconitate isomerase, providing a safe engineered strains for efficient production of trans-Aconitic acid.
2. The present disclosure also provides a recombinant *Aspergillus niger* strains. By using *Aspergillus niger* as the starting strains, aconitase Aco from *Aspergillus terreus,* aconitate isomerase Adi1 from *Ustilago maydis Corola* or aconitate isomerase TbrA from *Bacillus thuringiensis* are heterologously expressed in *Aspergillus niger* to obtain an *Aspergillus niger* engineered strains that produces trans-Aconitic acid. Through fermentation, the strains can solve the source dilemma of traditional trans-Aconitic acid production which previously relied on plant extraction or chemical synthesis. Moreover, *Aspergillus niger* is used to synthesize trans-Aconitic acid.
3. The present disclosure also provides a high-yield trans-Aconitic acid strains. Through a mitochondrial targeting signal peptide, aconitate isomerase is localized and transported into mitochondria to exert its function, such that cis-Aconitic acid in mitochondria is efficiently converted into trans-Aconitic acid under the catalysis of aconitate isomerase. This improves the efficiency of producing trans-Aconitic acid by the strains and provides a new engineered strains for the efficient production of *trans-Aconitic* acid. This strains solves the problem in the prior art that the trans-Aconitic acid synthesis pathway is distributed across two compartments, which leads to the consumption and insufficient supply of the precursor cis-Aconitic acid. The aconitate isomerase localized inside the mitochondria can efficiently catalyze the cis-Aconitic acid reactant produced in mitochondria, thereby improving the production efficiency of the trans-Aconitic acid and having good application values.
4. The present disclosure also provides a submerged liquid fermentation process for trans-Aconitic acid. By adding an appropriate organic nitrogen source, efficient acid production by fungal mycelia is achieved; by increasing $Fe^{2+}$ supply, the conversion rate of glucose to aconitic acid is improved; by adopting a low dissolved oxygen control of 25 to 30% and an early sugar feeding process at 30 to 60 h, the yield of trans-Aconitic acid is effectively increased. The original fermentation yield of trans-Aconitic acid (20- 25 g/L) is increased to 60-65 g/L, which is 2.8-3 times of the original yield, showing significant social and economic benefits. Furthermore, through optimizing medium and organically combining with a fermentation control process, the yield of trans-Aconitic acid is effectively improved. The submerged liquid fermentation process in a 20 t fermenter realizes industrial production of trans-Aconitic acid. Based on the submerged liquid fermentation process of the present disclosure, the industrial production of trans-Aconitic acid on a hundred-ton scale or larger can be designed with very high economic and social values.
5. The present disclosure also provides a method for preparing high-purity aconitate. During the preparation of aconitate, pigment impurities in the aconitic acid raw material obtained from fermentation are removed. On the basis of ensuring smooth progress of the aconitate reaction, the purity of the aconitate can be effectively improved, thereby solving the chromaticity problem of the esterified product of fermentation-derived aconitic acid. This method adopts first-step decolorization and second-step decolorization process to separately decolorize and remove fat-soluble pigments and water-soluble pigments in aconitic acid, so as to improve the impurity removal effect of aconitate and increase the purity of aconitate from 95% to greater than or equal to 99% to obtain high-purity aconitate. In the preparation method of high-purity aconitate provided in the present disclosure, the decolorization process using hydrogen peroxide and a decolorizing agent can achieve decolorization at low temperatures, thereby not only reducing energy consumption during the decolorization process, but also improving decolorization efficiency and

pigment removal rate. In the preparation method of high-purity aconitate provided by the present disclosure, the esterification reaction conditions of aconitic acid and alcohols, the type and dosage of catalysts, the type and dosage of water-carrying agent, the type of alkaline washing solution, and the neutralization temperature are matched with each other, so as to improve the reaction conversion rate of aconitic acid. In combination with the first-step decolorization and second-step decolorization processes, the synthesized aconitate is characterized by high purity, low acid value, and low chromaticity. The aconitate obtained by this method has high purity and low chromaticity. When the aconitate is used as a plasticizer and added to plastic products, it will not affect the coloring of the plastic products themselves, and will improve the appearance quality and enhance the plasticizing effect on the plastic products.

6. The high cold-resistant PVC composite material provided by the present disclosure uses a single aconitate as a plasticizer. The aconitic acid in this plasticizer is a non-toxic, environmentally friendly biological acid derived from microbial fermentation. As the main structure, the single aconitate can improve the plasticizing efficiency of the plasticizer and effectively reduce the use of petrochemical raw materials. Moreover, the substituent of the single aconitate is an alkyl group having no more than 5 carbon atoms, which can endow the aconitate with high compatibility, enabling it to better mix with the material matrix and provide a better service performance for subsequent products. The macromolecular structure and benzene-like ring structure of the single aconitate both improve compatibility with PVC, achieving an efficient plasticizing effect on PVC. On the one hand, the processing difficulty of the material is reduced; on the other hand, the application fields and scope of the material are expanded. The high cold-resistant PVC composite material provided by the present disclosure uses a specific single aconitate plasticizer, and has better compatibility with PVC materials compared with traditional plasticizers. Through the coordinated use with specific cold-resistant modifiers, stabilizers, and lubricants in a scientific ratio, the stability, toughness, tensile resistance, and cold resistance of the composite material are effectively enhanced. Additionally, no obvious phase separation is observed in the composite material under magnification of a scanning electron microscope.

7. The high-strength and volatility-resistant composite material provided by the present disclosure uses a plasticizer, and the plasticizer has alkyl substituents containing 6 to 18 carbon atoms, which can effectively improve the mobility of the matrix molecular chains, and increase the plasticity of the material. Moreover, the long-chain alkyl groups endow the plasticizer with a higher boiling point, enabling it to fully contact the matrix material, making it more difficult to volatilize from the high-temperature environment, and improving the volatility resistance and migration resistance of the plasticizer in the base material. In the high-strength and volatility-resistant composite material provided by the present disclosure, the base material and the plasticizer are proportioned scientifically to give full play to the modification ability of the plasticizer on the matrix material. At the same time, the plasticizer and the inorganic filler have a good synergistic effect, so as to improve the mechanical properties and thermal stability of the composite material and prolong the service life of the composite material. The high-strength and volatility-resistant composite material provided by the present disclosure is added with organotin compounds and rare earth stearates, helping to improve the uniformity of the composite material system. In addition to endowing the material with good thermal decomposition resistance and mold release properties, organotin compounds and rare earth stearates further enhance the mechanical properties and stability of the composite material.

8. The mixed aconitates provided by the present disclosure has ester groups with different carbon chain lengths in its molecular structure. The mixed aconitates can not only balance the plasticizing effect, heat resistance, and durability of the product, but also balance the resistance of the product to extraction by water solvents and organic solvents. The mixed aconitates provided by the present disclosure contain both ester groups having 1 to 5 carbon atoms and ester groups having more than 6 carbon atoms. The ester groups having more than 6 carbon atoms can effectively increase the relative molecular weight of the aconitate, making it more difficult to separate from the high-temperature environment. At the same time, the ester groups having 1 to 5 carbon atoms have good compatibility with PVC molecular chains. By combining the two types of ester groups at the molecular level, the effect of having both good plasticizing performance and good safety performance is achieved. Therefore, this type of mixed aconitates can maintain higher plasticizing efficiency while effectively increasing the upper limit of the service temperature and prolonging the service life of the composite material. The mixed aconitates provided by the present disclosure use aconitic acid as the main structure. On the one hand, aconitic acid is biologically derived, environmentally friendly, safe, and non-toxic. On the other hand, as a tricarboxylic acid structure, aconitic acid has excellent product designability and can be esterified to form a triester structure. Compared with diester structures (phthalate esters), the triester structure has a larger steric hindrance volume, which can more effectively weaken the interactions between the main molecular chains of the material to achieve plasticization. Meanwhile, compared with other tricarboxylic acids, especially citric acid, the lack of hydroxyl groups in aconitic acid can avoid unnecessary chemical reactions or formation of hydrogen bonds, thereby improving the modification effect. The additional double bonds can increase the interaction force between the plasticizer and the material, endowing the material with special properties and stability.

9. The preparation method of the mixed aconitates provided by the present disclosure involves the catalytic synthesis of aconitic acid and alcohol compounds into mixed aconitates. This synthesis route has good atom economy, does not

produce impurities that are difficult to remove, and has a higher yield. Moreover, through performing neutralization, bleaching and decolorization, solvent removal, and adsorption and decolorization on the synthesized mixed solution containing aconitate, transparent mixed aconitates with high purity, low chromaticity and low acid value is obtained without affecting the yield of the mixed aconitate plasticizer. The treatment process is simple, efficient, and low in cost, which is conducive to large-scale industrial production and applications.

## BRIEF DESCRIPTION OF THE DRAWINGS

The drawings described herein are used to provide a further understanding of the present disclosure and form a part of the present disclosure. The schematic embodiments and descriptions of the present disclosure are used to explain the present disclosure and do not constitute an improper limitation of the present disclosure. In the accompanying drawings:

Fig. 1 is a statistical chart of synthesis amounts of cis-Aconitic acid and *trans-Aconitic* acid produced by shake flask fermentation of recombinant *Aspergillus niger* strains constructed with isomerase TbrA expression cassettes using different promoters.

Fig. 2 is a statistical chart of synthesis amounts of cis-Aconitic acid and *trans-Aconitic* acid produced by shake flask fermentation of recombinant *Aspergillus niger* strains constructed with isomerase Adil expression cassettes using different promoters.

Fig. 3 is a statistical chart of synthesis amounts of cis-Aconitic acid and trans-Aconitic acid produced by shake flask fermentation of recombinant *Aspergillus niger* strains constructed with pyrithiamine *ptrA* as a selection marker.

Fig. 4 shows the content of trans-Aconitic acid produced by shake flask fermentation of recombinant *Aspergillus niger* strains, taking the combination of aconitate isomerase TbrA driven by PglaA and aconitase ATEG_03325 driven by different promoters as an example, provided in the examples of the present disclosure.

Fig. 5 shows the content of trans-Aconitic acid produced by shake flask fermentation of recombinant *Aspergillus niger* strains, taking the combination of aconitate isomerase Adi1 driven by PgpdAn and aconitase ATEG_03325 driven by different promoters as an example, provided in the examples of the present disclosure.

Fig. 6 shows the genomic PCR verification results of the recombinant engineered strains constructed in the present disclosure for mediating the green fluorescent protein StayGold through different mitochondrial targeting signal peptides (M is the DNA molecular weight marker, PCR verification is performed using primers YZ-ku80-F/YZ-ku80-R, and C is the control strain At-Δ*ku80*).

Fig. 7 is a diagram showing mycelial fluorescence results observed under a laser scanning confocal microscope (LSCM) for characterizing the mitochondrial localization effects of the signal peptides MTSacoAt, MTScitAt, and MTSicdAn involved in the present disclosure, wherein the mycelia are fused with different mitochondrially targeted sequences (MTS).

Fig. 8 shows the genomic PCR verification results of transformants of strains expressing mitochondrially targeted aconitate isomerase involved in the present disclosure (M is the DNA molecular weight marker, PCR verification is performed using primers C-*cadA*-F/C-*cadA*-R, and wt is the control strain At-Δ*ku80*).

Fig. 9 shows the results of evaluating the yield and conversion rate of *trans-Aconitic* acid in *Aspergillus terreus* engineered strains by shake flask fermentation involved in the present disclosure.

Fig. 10 shows the results of evaluating the yield of trans-Aconitic acid in *MTSicdAn-tbrA Aspergillus niger* engineered strains by shake flask fermentation involved in the present disclosure.

Fig. 11 shows the results of evaluating the yield of *trans-Aconitic* acid in MTSicdAn-adi1 *Aspergillus niger* engineered strains by shake flask fermentation involved in the present disclosure.

Fig. 12 is a data chart of evaluating the yield of *trans-Aconitic* acid at 96 h for fermentation media with different components designed by orthogonal experiment involved in the present disclosure.

Fig. 13 is a data chart comparing the differential components of high-yield corn steep liquor powder A and low-yield corn steep liquor powder B for trans-Aconitic acid involved in the present disclosure (CSL is the abbreviation of corn steep liquor powder, and the triangular markers indicate the difference multiples of the content of each component).

Fig. 14 is a data chart showing the effects of batch addition of amino acids, organic acids, and vitamins on the yield based on the optimal medium, and a yield chart of the recombinant engineered strains involved in the present disclosure.

Fig. 15 is a data chart showing the effect of adding nutritional factors in a 30 L fermenter on the yield of trans-Aconitic acid produced from fermentation involved in the present disclosure.

Fig. 16 is a data chart comparing the yields of trans-Aconitic acid under different dissolved oxygen control parameters in 30 L trans-Aconitic acid fermenter involved in the present disclosure.

Fig. 17 is a data chart showing the effect of different fermentation control DO processes and sugar feeding processes on the yield of trans-Aconitic acid in a 20 t fermenter involved in the present disclosure.

Fig. 18 is a product diagram of tri-n-butyl aconitate involved in Comparative Example 1 of the present disclosure.
Fig. 19 is a product diagram of tri-n-butyl aconitate involved in Example 19 of the present disclosure.
Fig. 20 is a product diagram of tri-n-butyl citrate involved in Comparative Example 4 of the present disclosure.
Fig. 21 is a product diagram of tri-n-butyl citrate involved in Comparative Example 5 of the present disclosure.
Fig. 22 is a liquid chromatogram of tri-n-butyl aconitate involved in Comparative Example 1 of the present disclosure.
Fig. 23 is a liquid chromatogram of tri-n-butyl aconitate involved in Example 19 of the present disclosure.
Fig. 24 is an SEM image of a composite material of Example 37.
Fig. 25 is an SEM image of a composite material of Example 38.
Fig. 26 is an SEM image of a composite material of Example 39.
Fig. 27 is an SEM image of a composite material of Comparative Example 6.
Fig. 28 is an SEM image of a composite material of Comparative Example 7.
Fig. 29 is an SEM image of a composite material of Comparative Example 8.
Fig. 30 is an SEM image of a composite material of Comparative Example 9.

Strains preservation information:

*Aspergillus niger* MEFC1501 *(Aspergillus niger* MEFC1501) was preserved in China General Microbiological Culture Collection Center (CGMCC), with a strain preservation number of CGMCC NO.40614. *Aspergillus niger* MEFC1501 was preserved on April 27, 2023 in the Institute of Microbiology, Chinese Academy of Sciences, No. 3, Courtyard 1, Beichen West Road, Chaoyang District, Beijing.

## DETAILED DESCRIPTION OF THE EMBODIMENTS

Technical terms

Aconitate isomerase (AI): aconitate isomerase is an enzyme protein that can convert cis-Aconitic acid to *trans-Aconitic* acid.

Expression: the term "expression" includes any steps involved in the production of an enzyme or its mutant, including but not limited to transcription, post-transcriptional modification, translation, post-translational modification, and secretion.

Homology: homology refers to the degree of similarity between the nucleotide sequences of two nucleic acid molecules or the amino acid sequences of two protein molecules in molecular evolution research.

Recombination: in a broad sense, any genetic exchange process that causes changes in the genotype is called recombination.

One-step cloning: one-step cloning homologous recombination is a seamless cloning technology that utilizes the principle of homologous recombination.

Expression cassette: the expression cassette refers to a set of DNA sequences consisting of a promoter, a target gene, a reporter gene, etc., which can be expressed in specific tissues and easily detected.

Expression vector: the expression vector refers to a vector that adds expression elements (such as promoters, RBS, terminators, etc.) to a basic backbone of a cloning vector to enable the expression of a target gene.

Whole-cell catalyst: whole-cell biocatalysis refers to a process of using intact biological organisms (i.e., whole cells, tissues, or even individuals) as catalysts for chemical conversion. The intact biological organisms involved in this catalytic process are just the whole-cell catalysts.

Engineered strains: the engineered strains refers to bacterial cell strains and/or fungi cell strains in which foreign genes are efficiently expressed using genetic engineering methods.

Host cell: the term "host cell" refers to any cell type that is susceptible to transformation, transfection, transduction, etc., with a nucleic acid construct or expression vector containing the polynucleotide of the present disclosure. The term "host cell" encompasses any progeny of the parent cell that is not identical to the parent cell due to mutations occurring during replication.

For a clearer explanation of the overall concept of the present disclosure, a detailed description will be given below with reference to the accompanying drawings and examples. In the following description, numerous additional details are provided to facilitate a more thorough understanding of the present disclosure. However, it will be apparent to those skilled in the art that the present disclosure can be practiced without one or more of these details. In other examples, some technical features well known in the art are not described to avoid confusion with the present disclosure.

Unless otherwise specified, in the following implementation methods, the reagents or instruments used without indicating manufacturers are conventional commercial products that can be purchased from the market. For the examples where specific conditions are not indicated, the experiments are conducted according to conventional conditions or the conditions recommended by the manufacturers.

The plasmids, restriction enzymes, PCR enzymes, column-type DNA extraction kits, and DNA gel recovery kits used in the following examples are commercial products, and the specific operations are performed in accordance with the kit instructions.

Unless otherwise stated, the experimental methods, detection methods, and preparation methods disclosed in the present disclosure employ conventional molecular biology, biochemistry, chromatin structure and analysis, analytical chemistry, cell culture, recombinant DNA technology, and related conventional technologies in the art. For details, reference can be made to Molecular Cloning: A Laboratory Manual (Fourth Edition).

In the present disclosure, the Plasmid Mini Kit I (D6943-02) from OMEGA Company is used for plasmid extraction, the Cycle-Pure Kit (D6492-02) from OMEGA Company is used for DNA fragment recovery, and the Gel Extraction Kit (D2500-01) from OMEGA Company is used for gel recovery.

Medium PDA: 3.9 g $L^{-1}$ Potato Dextrose Agar (Difco™ Potato Dextrose Agar, BD, LOT: 1165825), prepared into plates after sterilization.

Liquid medium PDB: 2.4 g $L^{-1}$ Potato Dextrose Broth (Difco™ Potato Dextrose Broth, BD, LOT: 2095227).

Sporulation (slant) medium: 10 g $L^{-1}$ glucose, 2 g $L^{-1}$ $NaNO_3$, 0.2 g $L^{-1}$ $KH_2PO_4$, 20 mg $L^{-1}$ $FeSO_4$, 5 g $L^{-1}$ $MgSO_4$, 0.5 g $L^{-1}$ NaCl, 40 mg $L^{-1}$ $ZnSO_4$, 40 mg $L^{-1}$ $CuSO_4$ and 1.5% agar are sterilized at 115°C for 15 min and then dispensed into test tubes to prepare slants.

Regeneration selection medium plates PDA-SH: 3.9 g $L^{-1}$ Potato Dextrose Agar (Difco™ Potato Dextrose Agar, BD, LOT: 1165825) and 1.2 M sorbitol are sterilized and cooled to approximately 55°C, and then hygromycin B (Solarbio, Catalog No.: M419099) is added to a final concentration of 100 μg/mL to prepare plates.

Regeneration selection medium top layer PDBS: 2.4 g $L^{-1}$ Potato Dextrose Broth (Difco™ Potato Dextrose Broth, BD, LOT: 2095227), 1.2 M sorbitol and 0.5% agarose are sterilized to prepare top-layer agar.

Regeneration selection medium plates CD-SPt: 10 g $L^{-1}$ glucose, 3 g $L^{-1}$ $NaNO_3$, 2 g $L^{-1}$ KCl, 1 g $L^{-1}$ $KH_2PO_4$, 0.5 g $L^{-1}$ $MgSO_4$, 20 mg $L^{-1}$ $FeSO_4$, 1.5 g $L^{-1}$ agar, 1.2 M sorbitol and the balance of water are sterilized and cooled to approximately 55°C, pyrithiamine (Sigma, Catalog No.: P0256) is added to a final concentration of 100 μg/L to prepare plates.

Selection medium plates CD-Pt: 10 g $L^{-1}$ glucose, 3 g $L^{-1}$ $NaNO_3$, 2 g $L^{-1}$ KCl, 1 g $L^{-1}$ $KH_2PO_4$, 0.5 g $L^{-1}$ $MgSO_4$, 20 mg $L^{-1}$ $FeSO_4$, 1.5 g $L^{-1}$ agar and the balance of water are sterilized and cooled to approximately 55°C, pyrithiamine (Sigma, Catalog No.: P0256) is added to a final concentration of 100 μg/L to prepare plates.

Fermentation medium: 160 g $L^{-1}$ glucose, 2 g $L^{-1}$ $NH_4NO_3$, 0.2 g $L^{-1}$ $(NH_4)_2HPO_4$, 20 mg $L^{-1}$ $FeSO_4$, 0.4 g $L^{-1}$ $MgSO_4$, 40 mg $L^{-1}$ $ZnSO_4$, 40 mg $L^{-1}$ $CuSO_4$, the pH is 3.5.

Plasmid pSGF957 is obtained from Seoul National University and is recorded in Kim, J.G., Choi, Y.D., Chang, Y.J., Kim, S.U., Genetic transformation of Monascus purpureus DSM1379, Biotechnology Letters, 2003, 25, 1509-1514.

*Aspergillus niger* MEFC1501 is preserved in China General Microbiological Culture Collection Center (CGMCC) with a strain preservation number of CGMCC NO.40614. *Aspergillus niger* MEFC1501 was preserved on April 27, 2023 in the Institute of Microbiology, Chinese Academy of Sciences, No. 3, Courtyard 1, Beichen West Road, Chaoyang District, Beijing.

*Aspergillus niger* Co827 was purchased from China Industrial Microbial Culture Collection Management Center.

*Aspergillus niger* NRRL 27809, NRRL 31821, NRRL 41873, and NRRL 6276 were purchased from the ARS Culture Collection.

*Aspergillus phoenicis* ATCC 30164 was purchased from American Type Culture Collection.

*Aspergillus tubingensis* M368 was purchased from Shanghai Institute of Industrial Microbiology.

*Aspergillus terreus* CICC 40205 was purchased from China Industrial Microbial Culture Collection Management Center.

Plasmid pSGF957 (obtained from Seoul National University, and recorded in Kim, J.G., Choi, Y.D., Chang, Y.J., Kim, S.U., Genetic transformation of Monascus purpureus DSM1379, Biotechnology Letters, 2003, 25, 1509-1514).

Plasmid pAN52-4 (obtained from TNO Medical Biological Laboratory, and recorded in Punt P. J., Zegers N. D., Busscher M., Pouwels P. H., van den Hondel C. A., Journal of Biotechnology, 1991, 7, 19-33).

Plasmid pmWM23 is recorded in Patent ZL202010783221.6.

Plasmid pUC57-Kan was purchased from Genewiz Biotechnology Co., Ltd.

Determination methods for trans-Aconitic acid, citric acid, cis-Aconitic acid, etc.: the content and purity of various organic acids in the fermentation broth are detected by high-performance liquid chromatography (HPLC).

**Example** 1 Construction of recombinant *Aspergillus niger* heterologously expressing aconitate isomerase TbrA from *Bacillus thuringiensis*

1. Construction of an expression cassette for aconitate isomerase TbrA

The aconitate isomerase TbrA gene was artificially synthesized and codon-optimized for expression in *Asper-*

*gillus niger.* The optimized sequence is shown as SEQ ID NO. 3. This gene was cloned into the pUC57-Kan vector to construct the plasmid puc57-TbrA.

1) Construction of aconitate isomerase TbrA expression cassette using the PgpdAt promoter

PCR amplification was performed using fuPgpdAt-TbrA-F (5'-ctcatcaatcatcacaacatgaagatcccctgcttcg-3', SEQ ID NO. 21) and fuTbrA-TtrpC-R (5'-tcagtaacgttaagtggatccttaggggatgatcagctcg-3', SEQ ID NO. 22) as a primer pair and using the puc57-TbrA plasmid as a template. The amplified product was purified and recovered to obtain the TbrA gene fragment.

PCR amplification was performed using fuTbrA-TtrpC-F (5'-cgagctgatcatcccctaaggatccacttaacgttactga-3', SEQ ID NO. 23) and fuPgpdAt-TbrA-R (5'-cgaagcaggggatcttcatgttgtgatgattgatgag-3', SEQ ID NO. 24) as a primer pair and using the plasmid pmWM23 as a template. The amplified product was purified and recovered to obtain the PgpdAt-TtrpC fragment.

The above two fragments were subjected to one-step cloning. PCR amplification was performed using PgpdAt-F (5'-ttacactctgggaggatccaggtac-3', SEQ ID NO. 25) and TtrpC-R (5'-gtcgtgccagtcgactctaga-3', SEQ ID NO. 26) as a primer pair. The amplified product was purified and recovered to obtain the PgpdAt-TbrA-TtrpC expression cassette fragment.

2) Construction of aconitate isomerase TbrA expression cassette using the PgpdAn promoter

PCR amplification was performed using fuPgpdAn-TbrA-F (5'-ccgcttgagcagacatcaccatgaagatcccctgcttcgt-3', SEQ ID NO. 82) and fuTbrA-TtrpC-R (5'-tcagtaacgttaagtggatccttaggggatgatcagctcg-3', SEQ ID NO. 22) as a primer pair and using the puc57-TbrA plasmid as a template. The amplified product was purified and recovered to obtain the TbrA gene fragment.

PCR amplification was performed using fuTbrA-TtrpC-F (5'-cgagctgatcatcccctaaggatccacttaacgttactga-3', SEQ ID NO. 23) and fuPgpdAn-TbrA-R (5'-acgaagcaggggatcttcatggtgatgtctgctcaagcgg-3', SEQ ID NO. 27) as a primer pair and using the plasmid pAN52-4 as a template. The amplified product was purified and recovered to obtain the PgpdAn-TtrpC fragment. PgpdAn is the glyceraldehyde-3-phosphate dehydrogenase gene promoter from *Aspergillus nidulans,* and TtrpC is the tryptophan synthase terminator from *Aspergillus nidulans.*

The above two fragments were subjected to one-step cloning. PCR amplification was performed using PgpdAn-F (5'-ttgatcgagacctaatacagc-3', SEQ ID NO. 28) and TtrpC-R (5'-gtcgtgccagtcgactctaga-3', SEQ ID NO. 26) as a primer pair. The amplified product was purified and recovered to obtain the PgpdAn-TbrA-TtrpC expression cassette fragment.

3) Construction of an expression cassette of aconitate isomerase TbrA using PglaA promoter

PCR amplification was performed using fuPglaA-TbrA-F (5'-gcatcattacacctcagcaatgaagatcccctgcttcg-3', SEQ ID NO. 29) and fuTbrA-TtrpC-R (5'-tcagtaacgttaagtggatccttaggggatgatcagctcg-3', SEQ ID NO. 22) as a primer pair and using the puc57-TbrA plasmid as a template. The amplified product was purified and recovered to obtain the TbrA gene fragment.

PCR amplification was performed using PglaA-F (5'-ggattgcctgaacattgacattcgg-3', SEQ ID NO. 30) and PglaA-R (5'-tgctgaggtgtaatgatgctggggat-3', SEQ ID NO. 31) as a primer pair and using the genome of *Aspergillus niger* MEFC1501 as a template. The amplified product was purified and recovered to obtain the PglaA promoter fragment.

PCR amplification was performed using fuTbrA-TtrpC-F (5'-cgagctgatcatcccctaaggatccacttaacgttactga-3', SEQ ID NO. 23) and TtrpC-R (5'-gtcgtgccagtcgactctaga-3', SEQ ID NO. 26) as a primer pair and using the plasmid pAN52-4 as a template. The amplified product was purified and recovered to obtain the TtrpC fragment.

The above three fragments were subjected to one-step cloning. PCR amplification was performed using PglaA-F (5'-ggattgcctgaacattgacattcgg-3', SEQ ID NO. 30) and TtrpC-R (5'-gtcgtgccagtcgactctaga-3', SEQ ID NO. 26) as a primer pair. The amplified product was purified and recovered to obtain the PglaA-TbrA-TtrpC expression cassette fragment.

4) Construction of an expression cassette of aconitate isomerase TbrA using a PcadA promoter

PCR amplification was performed using fuPcadA-TbrA-F (5'-cctcttaaattgaccatgaatgaagatcccctgcttcg-3', SEQ ID NO. 32) and fuTbrA-TtrpC-R (5'-tcagtaacgttaagtggatccttaggggatgatcagctcg-3', SEQ ID NO. 22) as a primer pair and using the puc57-TbrA plasmid as a template. The amplified product was purified and recovered to obtain the TbrA gene fragment.

PCR amplification was performed using PcadA-F (5'-ctaccaacagtctcgcggtgaatag-3', SEQ ID NO. 33) and PcadA-R (5'-ggtcaatttaagaggacgatcttcg-3', SEQ ID NO. 34) as a primer pair and using the genome of *Aspergillus terreus* CICC 40205 as a template. The amplified product was purified and recovered to obtain the PcadA promoter fragment.

PCR amplification was performed using fuTbrA-TtrpC-F (5'-cgagctgatcatcccctaaggatccacttaacgttactga-3', SEQ

ID NO. 23) and TtrpC-R (5'-gtcgtgccagtcgactctaga-3', SEQ ID NO. 26) as a primer pair and using the plasmid pAN52-4 as a template. The amplified product was purified and recovered to obtain the TtrpC fragment.

The above three fragments were subjected to one-step cloning. PCR amplification was performed using PcadA-F (5'-ctaccaacagtctcgcggtgaatag-3', SEQ ID NO. 33) and TtrpC-R (5'-gtcgtgccagtcgactctaga-3', SEQ ID NO. 26) as a primer pair. The amplified product was purified and recovered to obtain the PcadA-TbrA-TtrpC expression cassette fragment.

5) Construction of an expression cassette of aconitate isomerase TbrA using a PmfsA promoter

PCR amplification was performed using fuPmfsA-TbrA-F (5'-accgacttctcatccatcttcaaaatgaagatcccctgcttcg-3', SEQ ID NO. 35) and fuTbrA-TtrpC-R (5'-tcagtaacgttaagtggatccttaggggatgatcagctcg-3', SEQ ID NO. 22) as a primer pair and using the puc57-TbrA plasmid as a template. The amplified product was purified and recovered to obtain the TbrA gene fragment.

PCR amplification was performed using PmfsA-F (5'-gtacagtggccatgaaatccaatc-3', SEQ ID NO. 36) and PmfsA-R (5'-tttgaagatggatgagaagtcggt-3', SEQ ID NO. 37) as a primer pair and using the genome of *Aspergillus niger* MEFC1501 as a template. The amplified product was purified and recovered to obtain the PmfsA promoter fragment.

PCR amplification was performed using fuTbrA-TtrpC-F (5'-cgagctgatcatcccctaaggatccacttaacgttactga-3', SEQ ID NO. 23) and TtrpC-R (5'-gtcgtgccagtcgactctaga-3', SEQ ID NO. 26) as a primer pair and using the plasmid pAN52-4 as a template. The amplified product was purified and recovered to obtain the TtrpC fragment.

The above three fragments were subjected to one-step cloning. PCR amplification was performed using PmfsA-F (5'-gtacagtggccatgaaatccaatc-3', SEQ ID NO. 36) and TtrpC-R (5'-gtcgtgccagtcgactctaga-3', SEQ ID NO. 26) as a primer pair. The amplified product was purified and recovered to obtain the PmfsA-TbrA-TtrpC expression cassette fragment.

6) Construction of an expression cassette of aconitate isomerase TbrA using a PacoA promoter

PCR amplification was performed using fuPacoA-TbrA-F (5'-cctcgcatagagagcttccatcatgaagatcccctgcttcg-3', SEQ ID NO. 38) and fuTbrA-TtrpC-R (5'-tcagtaacgttaagtggatccttaggggatgatcagctcg-3', SEQ ID NO. 22) as a primer pair and using the puc57-TbrA plasmid as a template. The amplified product was purified and recovered to obtain the *tbrA* gene fragment.

PCR amplification was performed using PacoA-F (5'-tggcaccggtccgcggga-3', SEQ ID NO. 39) and PacoA-R (5'-gatggaagctctctatgcgagg-3', SEQ ID NO. 40) as a primer pair and using the genome of *Aspergillus terreus* CICC 40205 as a template. The amplified product was purified and recovered to obtain the PacoA promoter fragment.

PCR amplification was performed using fuTbrA-TtrpC-F (5'-cgagctgatcatcccctaaggatccacttaacgttactga-3', SEQ ID NO. 23) and TtrpC-R (5'-gtcgtgccagtcgactctaga-3', SEQ ID NO. 26) as a primer pair and using the plasmid pAN52-4 as a template. The amplified product was purified and recovered to obtain the TtrpC fragment.

The above three fragments were subjected to one-step cloning. PCR amplification was performed using PacoA-F (5'-tggcaccggtccgcggga-3', SEQ ID NO. 39) and TtrpC-R (5'-gtcgtgccagtcgactctaga-3', SEQ ID NO. 26) as a primer pair. The amplified product was purified and recovered to obtain the PacoA-TbrA-TtrpC expression cassette fragment.

7) Construction of an expression cassette of aconitate isomerase TbrA using a PcitA promoter

PCR amplification was performed using fuPcitA-TbrA-F (5'-cttttttagactcttgttggattcaaaatgaagatcccctgcttcg-3', SEQ ID NO. 41) and fuTbrA-TtrpC-R (5'-tcagtaacgttaagtggatccttaggggatgatcagctcg-3', SEQ ID NO. 22) as a primer pair and using the puc57-TbrA plasmid as a template. The amplified product was purified and recovered to obtain the *tbrA* gene fragment.

PCR amplification was performed using PcitA-F (5'-caaccaaggaccgcgatg-3', SEQ ID NO. 42) and PcitA-R (5'-ttgaatccaacaagagtctaaaaaag-3', SEQ ID NO. 43) as a primer pair and using the genome of *Aspergillus terreus* CICC 40205 as a template. The amplified product was purified and recovered to obtain the PcitA promoter fragment.

PCR amplification was performed using fuTbrA-TtrpC-F (5'-cgagctgatcatcccctaaggatccacttaacgttactga-3', SEQ ID NO. 23) and TtrpC-R (5'-gtcgtgccagtcgactctaga-3', SEQ ID NO. 26) as a primer pair and using the plasmid pAN52-4 as a template. The amplified product was purified and recovered to obtain the TtrpC fragment.

The above three fragments were subjected to one-step cloning. PCR amplification was performed using PcitA-F (5'-caaccaaggaccgcgatg-3', SEQ ID NO. 42) and TtrpC-R (5'-gtcgtgccagtcgactctaga-3', SEQ ID NO. 26) as a primer pair. The amplified product was purified and recovered to obtain the PcitA-TbrA-TtrpC expression cassette fragment.

8) Construction of an expression cassette of aconitate isomerase TbrA using a PicdA promoter

PCR amplification was performed using fuPicdA-TbrA-F (5'-cgcaggccacgcttcactgtcgaaatgaagatcccctgcttcg-3', SEQ ID NO. 46) and fuTbrA-TtrpC-R (5'-tcagtaacgttaagtggatccttaggggatgatcagctcg-3', SEQ ID NO. 22) as a primer pair and using the puc57-TbrA plasmid as a template. The amplified product was purified and recovered to obtain the *tbrA* gene fragment.

PCR amplification was performed using PicdA-F (5'-ctttaacgttgcagatacagggatgcg-3', SEQ ID NO. 47) and PicdA-R (5'-ttcgacagtgaagcgtggcctgcg-3', SEQ ID NO. 48) as a primer pair and using the genome of *Aspergillus niger* MEFC1501 as a template. The amplified product was purified and recovered to obtain the PicdA promoter fragment.

PCR amplification was performed using fuTbrA-TtrpC-F (5'-cgagctgatcatcccctaaggatccacttaacgttactga-3', SEQ ID NO. 23) and TtrpC-R (5'-gtcgtgccagtcgactctaga-3', SEQ ID NO. 26) as a primer pair and using the plasmid pAN52-4 as a template. The amplified product was purified and recovered to obtain the TtrpC fragment.

The above three fragments were subjected to one-step cloning. PCR amplification was performed using PicdA-F (5'-ctttaacgttgcagatacagggatgcg-3', SEQ ID NO. 47) and TtrpC-R (5'-gtcgtgccagtcgactctaga-3', SEQ ID NO. 26) as a primer pair. The amplified product was purified and recovered to obtain the PicdA-TbrA-TtrpC expression cassette fragment.

9) Construction of an expression cassette of aconitate isomerase TbrA using a PgasA promoter

PCR amplification was performed using fuPgasA-TbrA-F (5'-gtcttctttcgttcacctcctcacatgaagatcccctgcttcg-3', SEQ ID NO. 49) and fuTbrA-TtrpC-R (5'-tcagtaacgttaagtggatccttaggggatgatcagctcg-3', SEQ ID NO. 22) as a primer pair and using the puc57-TbrA plasmid as a template. The amplified product was purified and recovered to obtain the *tbrA* gene fragment.

PCR amplification was performed using PgasA-F (5'-ctgctctctctctgctctctttct-3', SEQ ID NO. 50) and PgasA-R (5'-gtgaggaggtgaacgaaagaagac-3', SEQ ID NO. 51) as a primer pair and using the genome of *Aspergillus niger* MEFC1501 as a template. The amplified product was purified and recovered to obtain the PgasA promoter fragment.

PCR amplification was performed using fuTbrA-TtrpC-F (5'-cgagctgatcatcccctaaggatccacttaacgttactga-3', SEQ ID NO. 23) and TtrpC-R (5'-gtcgtgccagtcgactctaga-3', SEQ ID NO. 26) as a primer pair and using the plasmid pAN52-4 as a template. The amplified product was purified and recovered to obtain the TtrpC fragment.

The above three fragments were subjected to one-step cloning. PCR amplification was performed using PgasA-F (5'-ctgctctctctctgctctctttct-3', SEQ ID NO. 50) and TtrpC-R (5'-gtcgtgccagtcgactctaga-3', SEQ ID NO. 26) as a primer pair. The amplified product was purified and recovered to obtain the PgasA-TbrA-TtrpC expression cassette fragment.

2. Construction of an expression cassette for the hygromycin B selection marker gene *hph*

PCR amplification was performed using hph-F (5'-ttcgggatcgcaagcgtaaag-3', SEQ ID NO. 52) and hph-R (5'-caattatctttgcgaacccagg-3', SEQ ID NO. 53) as a primer pair and using the plasmid pSGF957 as a template to obtain the PtrpC-hph-TtrpC fragment. PtrpC is the tryptophan synthase gene promoter from *Aspergillus nidulans,* hph is the hygromycin phosphotransferase gene, and TtrpC is the tryptophan synthase terminator from *Aspergillus nidulans.* The amplified product was purified and recovered to obtain the hygromycin B-resistant hph gene expression cassette PtrpC-hph-TtrpC.

3. Transformation of *Aspergillus niger* protoplasts with aconitate isomerase TbrA expression cassette to obtain recombinant *Aspergillus niger* strains

1) Preparation of *Aspergillus niger* protoplasts

The spore suspension of *Aspergillus niger* MEFC1501 was inoculated into 50 mL of PDB liquid medium, with a spore concentration of approximately $10^7$ spores/mL, followed by incubation at 33°C under 200 rpm for 12 to 18 h. The grown mycelia were collected by filtration through a sterile single-layer 100-mesh nylon cloth, rinsed for three times with a sterile 0.6 M $MgSO_4$ solution, and pressed dry and then placed in a 50 mL sterile Erlenmeyer flask. 1 g of mycelia was weighed, 10 mL of enzyme solution was added, and the mixture was treated at 30°C under 120 rpm for 1 to 2 h. The components of the enzyme solution include 1% cellulase (Sigma, Catalog No.: C1184), 1% lysing enzyme (Sigma, Catalog No.: L1412), 1% snailase (Sangon Biotech, Catalog No.: SB0870), and 0.6 M $MgSO_4$. The enzyme solution was sterilized by filtration using a 0.22 μm sterile filter.

The above mixture after enzymatic digestion was filtered through a 300-mesh nylon cloth, and the filtrate was collected. Protoplasts were collected by centrifugation at 4°C, washed once with 1.0 M pre-cooled sorbitol solution, then washed once with pre-cooled STC (1.0 M sorbitol, 50 mM Tris·HCl-pH 8.0, 50 mM $CaCl_2$). Finally, the protoplasts were

resuspended in 150 μL of pre-cooled STC, and the concentration of protoplasts was adjusted to $5 \times 10^7$ protoplasts/mL using STC to obtain the protoplast suspension.

2) Co-transformation of *Aspergillus niger* protoplasts with aconitate isomerase TbrA expression cassette and resistance gene *hph*

Approximately 5 μg of aconitate isomerase TbrA expression cassette DNA and 1 μg of hygromycin B-resistant *hph* gene expression cassette DNA were added to the above protoplast suspension, followed by the addition of 50 μL of PSTC (40% PEG4000, 50 mM Tris-HCl pH 8.0, 50 mM $CaCl_2$). The mixture was gently mixed and placed on ice for 30 min. 1.5 mL of PSTC was added and mixed uniformly, and then left at room temperature for 20 min. Then it was mixed with the upper layer agar and poured onto the regeneration selection medium plates PDA-SH, followed by incubation at 30°C in the dark for 3 to 4 days.

Transformants were transferred from the plates to the selection plates PDA-H (4 g of potato dextrose agar medium was weighed and dissolved in 100 mL of distilled water, and then sterilized and cooled to approximately 55°C, hygromycin was added to a final concentration of 100 μg/mL to prepare the plates); the mixture was incubated at 30°C for 3 to 5 days to obtain transformants.

In a preparation process of the above recombinant *Aspergillus niger* strains containing the aconitate isomerase TbrA expression cassette, the aconitate isomerase TbrA expression cassette and the hygromycin B-resistant *hph* gene expression cassette can also be linked into a single expression cassette, which is then used to transform protoplasts. This does not affect the screening of positive transformants and the identification of genotypes.

3) Verification of genotype of recombinant *Aspergillus niger* strains

The prepared recombinant *Aspergillus niger* transformants were picked and inoculated onto PDA-H plates to culture spores. Then, the spores were respectively inoculated into liquid medium for culture, and mycelia were collected to extract the genome. PCR was performed using PgpdAt-F743 (5'-ttacactctgggaggatccaggtact-3', SEQ ID NO. 54) and TtrpC-R (5'-gtcgtgccagtcgactctaga-3', SEQ ID NO. 26) as a primer pair to amplify the PgpdAt-TbrA-TtrpC inserted into the genome. The PtrpC-hph-TtrpC fragment inserted into the recombinant strains were amplified using hph-F (5'-ttcgggat cgcaagcgtaaag-3', SEQ ID NO. 52) and hph-R (5'-caattatctttgcgaacccagg-3', SEQ ID NO. 53) as a primer pair. The PCR products were analyzed by 0.8% agarose gel electrophoresis. The genomic DNA template of the starting strain *Aspergillus niger* MEFC1501 was used as a negative control.

Similarly, PCR was performed using PglaA-F (5'-ggattgcctgaacattgacattcgg-3', SEQ ID NO. 30) and TtrpC-R (5'-gtcgtgccagtcgactctaga-3', SEQ ID NO. 26) as a primer pair to amplify the PglaA-TbrA-TtrpC inserted into the genome; PCR was performed using PgpdAn-F (5'-ttgatcgagacctaatacagc-3', SEQ ID NO. 28) and TtrpC-R (5'-gtcgtgccagtcgac tctaga-3', SEQ ID NO. 26) as a primer pair to amplify the PgpdAn-TbrA-TtrpC inserted into the genome; PCR was performed using PcadA-F (5'-ctaccaacagtctcgcggtgaatag-3', SEQ ID NO. 33) and TtrpC-R (5'-gtcgtgccagtcgactctaga-3', SEQ ID NO. 26) as a primer pair to amplify the PcadA-TbrA-TtrpC inserted into the genome; PCR was performed using PmfsA-F (5'-gtacagtggccatgaaatccaatc-3', SEQ ID NO. 36) and TtrpC-R (5'-gtcgtgccagtcgactctaga-3', SEQ ID NO. 26) as a primer pair to amplify the PmfsA-TbrA-TtrpC inserted into the genome; PCR was performed using PacoA-F (5'-tggc accggtccgcggga-3', SEQ ID NO. 39) and TtrpC-R (5'-gtcgtgccagtcgactctaga-3', SEQ ID NO. 26) as a primer pair to amplify the PacoA-TbrA-TtrpC inserted into the genome; PCR was performed using PcitA-F (5'-caaccaaggaccgcgatg-3', SEQ ID NO. 42) and TtrpC-R (5'-gtcgtgccagtcgactctaga-3', SEQ ID NO. 26) as a primer pair to amplify the PcitA-TbrA-TtrpC inserted into the genome; PCR was performed using PicdA-F (5'-ctttaacgttgcagatacagggatgcg-3', SEQ ID NO. 47) and TtrpC-R (5'-gtcgtgccagtcgactctaga-3', SEQ ID NO. 26) as a primer pair to amplify the PicdA-TbrA-TtrpC inserted into the genome; and PCR was performed using PgasA-F (5'-ctgctctctctctgctctctttct-3', SEQ ID NO. 50) and TtrpC-R (5'-gtcgt gccagtcgactctaga-3', SEQ ID NO. 26) as a primer pair to amplify the PgasA-TbrA-TtrpC inserted into the genome.

Transformants with correct genotype verification were transferred to PDA-H plates for subculture, with three passages in total. Then, the spores were collected respectively and appropriately diluted with physiological saline, and 100 μL of the diluted spore suspension was spread on the PDA-H plates to allow the growth of independent single colonies, which was referred to as single-spore isolation. Spores were taken from the single colonies for single-spore isolation again, and a total of four rounds of single-spore isolation and subculture were performed. The recombinant strains after single-spore isolation were subjected to genotype identification again for verification. Some recombinant strains with correct verification were selected for shake flask fermentation screening. The specific shake flask screening method and results are shown in Fig. 1.

**Example** 2 Construction of recombinant *Aspergillus niger* heterologously expressing aconitate isomerase Adil from *ustilago maydis*

1. Construction of an expression cassette of aconitate isomerase Adil

The aconitate isomerase Adi1 gene was artificially synthesized and codon-optimized for expression in *Aspergillus niger.* The optimized sequence is shown as SEQ ID NO. 4. This gene was cloned into the pUC57-Kan vector to construct the plasmid puc57-Adi1.

1) Construction of an expression cassette of aconitate isomerase Adi1 using a PgpdAt promoter

PCR amplification was performed using fuPgpdAt-Adi1-F (5'-ctcatcaatcatcacaacatgctgcaccccatcgacacca-3', SEQ ID NO. 55) and fuAdi1-TtrpC-R (5'-tcagtaacgttaagtggatccttaggacaggctacggtcg-3', SEQ ID NO. 56) as a primer pair and using the puc57-Adi1 plasmid as a template. The amplified product was purified and recovered to obtain the *Adil* gene fragment.

PCR amplification was performed using fuAdi1-TtrpC-F (5'-cgaccgtagcctgtcctaaggatccacttaacgttactga-3', SEQ ID NO. 57) and fuPgpdAt-Adi1-R (5'-tggtgtcgatggggtgcagcatgttgtgatgattgatgag-3', SEQ ID NO. 58) as a primer pair and using the plasmid pmWM23 as a template. The amplified product was purified and recovered to obtain the PgpdAt-TtrpC fragment.

The above two fragments were subjected to one-step cloning. PCR amplification was performed using PgpdAt-F (5'-ttacactctgggaggatccaggtac-3', SEQ ID NO. 25) and TtrpC-R (5'-gtcgtgccagtcgactctaga-3', SEQ ID NO. 26) as a primer pair. The amplified product was purified and recovered to obtain the PgpdAt-Adil-TtrpC expression cassette fragment.

2) Construction of an expression cassette of aconitate isomerase Adil using a PgpdAn promoter

PCR amplification was performed using fuPgpdAn-Adi1-F (5'-ccgcttgagcagacatcaccatgctgcaccccatcgacacca t-3', SEQ ID NO. 59) and fuAdi1-TtrpC-R (5'-tcagtaacgttaagtggatccttaggacaggctacggtcg-3', SEQ ID NO. 56) as a primer pair and using the puc57-Adi1 plasmid as a template. The amplified product was purified and recovered to obtain the *Adil* gene fragment.

PCR amplification was performed using fuAdi1-TtrpC-F (5'-cgaccgtagcctgtcctaaggatccacttaacgttactga-3', SEQ ID NO. 57) and fuPgpdAn-Adi1-R (5'-tggtgtcgatggggtgcagcatggtgatgtctgctcaagcgg-3', SEQ ID NO. 60) as a primer pair and using the plasmid pAN52-4 as a template. The amplified product was purified and recovered to obtain the PgpdAn-TtrpC fragment. PgpdAn is the glyceraldehyde-3-phosphate dehydrogenase gene promoter from *Aspergillus nidulans,* and TtrpC is the tryptophan synthase terminator from *Aspergillus nidulans.*

The above two fragments were subjected to one-step cloning. PCR amplification was performed using PgpdAn-F (5'-ttgatcgagacctaatacagc-3', SEQ ID NO. 28) and TtrpC-R (5'-gtcgtgccagtcgactctaga-3', SEQ ID NO. 26) as a primer pair. The amplified product was purified and recovered to obtain the PgpdAn-Adil-TtrpC expression cassette fragment.

3) Construction of an expression cassette of aconitate isomerase Adil using a PglaA promoter

PCR amplification was performed using fuPglaA-Adi1-F (5'-gcatcattacacctcagcaatgctgcaccccatcgacacca-3', SEQ ID NO. 61) and fuAdi1-TtrpC-R (5'-tcagtaacgttaagtggatccttaggacaggctacggtcg-3', SEQ ID NO. 56) as a primer pair and using the puc57-Adi1 plasmid as a template. The amplified product was purified and recovered to obtain the *Adil* gene fragment.

PCR amplification was performed using PglaA-F (5'-ggattgcctgaacattgacattcgg-3', SEQ ID NO. 30) and PglaA-R (5'-tgctgaggtgtaatgatgctggggat-3', SEQ ID NO. 31) as a primer pair and using the genome of *Aspergillus niger* MEFC1501 as a template. The amplified product was purified and recovered to obtain the PglaA promoter fragment.

PCR amplification was performed using fuAdi1-TtrpC-F (5'-cgaccgtagcctgtcctaaggatccacttaacgttactga-3', SEQ ID NO. 57) and TtrpC-R (5'-gtcgtgccagtcgactctaga-3', SEQ ID NO. 26) as a primer pair and using the plasmid pAN52-4 as a template. The amplified product was purified and recovered to obtain the TtrpC fragment.

The above three fragments were subjected to one-step cloning. PCR amplification was performed using PglaA-F (5'-ggattgcctgaacattgacattcgg-3', SEQ ID NO. 30) and TtrpC-R (5'-gtcgtgccagtcgactctaga-3', SEQ ID NO. 26) as a primer pair. The amplified product was purified and recovered to obtain the PglaA-Adil-TtrpC expression cassette fragment.

4) Construction of an expression cassette of aconitate isomerase Adil using a PcadA promoter

PCR amplification was performed using fuPcadA-Adi1-F (5'-cctcttaaattgaccatgaatgctgcaccccatcgacacca-3', SEQ ID NO. 62) and fuAdil-TtrpC-R (5'-tcagtaacgttaagtggatccttaggacaggctacggtcg-3', SEQ ID NO. 56) as a primer pair and using the puc57-Adi1 plasmid as a template. The amplified product was purified and recovered to obtain the *Adil* gene fragment.

PCR amplification was performed using PcadA-F (5'-ctaccaacagtctcgcggtgaatag-3', SEQ ID NO. 33) and

PcadA-R (5'-ggtcaatttaagaggacgatcttcg-3', SEQ ID NO. 34) as a primer pair and using the genome of *Aspergillus terreus* CICC 40205 as a template. The amplified product was purified and recovered to obtain the PcadA promoter fragment.

PCR amplification was performed using fuAdi1-TtrpC-F (5'-cgaccgtagectgtectaaggatccacttaacgttactga-3', SEQ ID NO. 57) and TtrpC-R (5'-gtcgtgccagtcgactctaga-3', SEQ ID NO. 26) as a primer pair and using the plasmid pAN52-4 as a template. The amplified product was purified and recovered to obtain the TtrpC fragment.

The above three fragments were subjected to one-step cloning. PCR amplification was performed using PcadA-F (5'-ctaccaacagtctcgcggtgaatag-3', SEQ ID NO. 33) and TtrpC-R (5'-gtcgtgccagtcgactctaga-3', SEQ ID NO. 26) as a primer pair. The amplified product was purified and recovered to obtain the PcadA-Adi1-TtrpC expression cassette fragment.

5) Construction of an expression cassette of aconitate isomerase Adil using a PmfsA promoter

PCR amplification was performed using fuPmfsA-Adi1-F (5'-accgacttctcatccatcttcaaaatgctgcaccccatcgacacc a-3', SEQ ID NO. 63) and fuAdil-TtrpC-R (5'-tcagtaacgttaagtggatccttaggacaggctacggtcg-3', SEQ ID NO. 56) as a primer pair and using the puc57-Adi1 plasmid as a template. The amplified product was purified and recovered to obtain the Adi1 gene fragment.

PCR amplification was performed using PmfsA-F (5'-gtacagtggccatgaaatccaatc-3', SEQ ID NO. 36) and PmfsA-R (5'-tttgaagatggatgagaagtcggt-3', SEQ ID NO. 37) as a primer pair and using the genome of *Aspergillus niger* MEFC1501 as a template. The amplified product was purified and recovered to obtain the PmfsA promoter fragment.

PCR amplification was performed using fuAdi1-TtrpC-F (5'-cgaccgtagcctgtcctaaggatccacttaacgttactga-3', SEQ ID NO. 57) and TtrpC-R (5'-gtcgtgccagtcgactctaga-3', SEQ ID NO. 26) as a primer pair and using the plasmid pAN52-4 as a template. The amplified product was purified and recovered to obtain the TtrpC fragment.

The above three fragments were subjected to one-step cloning. PCR amplification was performed using PmfsA-F (5'-gtacagtggccatgaaatccaatc-3', SEQ ID NO. 36) and TtrpC-R (5'-gtcgtgccagtcgactctaga-3', SEQ ID NO. 26) as a primer pair. The amplified product was purified and recovered to obtain the PmfsA-Adil-TtrpC expression cassette fragment.

6) Construction of an expression cassette of aconitate isomerase Adil using a PacoA promoter

PCR amplification was performed using fuPacoA-Adi1-F (5'-cctcgcatagagagcttccatcatgctgcaccccatcgacacc a-3', SEQ ID NO. 64) and fuAdil-TtrpC-R (5'-tcagtaacgttaagtggatccttaggacaggctacggtcg-3', SEQ ID NO. 56) as a primer pair and using the puc57-Adi1 plasmid as a template. The amplified product was purified and recovered to obtain the *Adil* gene fragment.

PCR amplification was performed using PacoA-F (5'-tggcaccggtccgcggga-3', SEQ ID NO. 39) and PacoA-R (5'-gatggaagctctctatgcgagg-3', SEQ ID NO. 40) as a primer pair and using the genome of *Aspergillus terreus* CICC 40205 as a template. The amplified product was purified and recovered to obtain the PacoA promoter fragment.

PCR amplification was performed using fuAdi1-TtrpC-F (5'-cgaccgtagcctgtcctaaggatccacttaacgttactga-3', SEQ ID NO. 57) and TtrpC-R (5'-gtcgtgccagtcgactctaga-3', SEQ ID NO. 26) as a primer pair and using the plasmid pAN52-4 as a template. The amplified product was purified and recovered to obtain the TtrpC fragment.

The above three fragments were subjected to one-step cloning. PCR amplification was performed using PacoA-F (5'-tggcaccggtccgcggga-3', SEQ ID NO. 39) and TtrpC-R (5'-gtcgtgccagtcgactctaga-3', SEQ ID NO. 26) as a primer pair. The amplified product was purified and recovered to obtain the PacoA-Adil-TtrpC expression cassette fragment.

7) Construction of an expression cassette of aconitate isomerase Adil using a PcitA promoter

PCR amplification was performed using fuPcitA-Adi1-F (5'-cttttttagactcttgttggattcaaaatgctgcaccccatcgacacc a-3', SEQ ID NO. 65) and fuAdil-TtrpC-R (5'-tcagtaacgttaagtggatccttaggacaggctacggtcg-3', SEQ ID NO. 56) as a primer pair and using the puc57-Adi1 plasmid as a template. The amplified product was purified and recovered to obtain the Adil gene fragment. PCR amplification was performed using PcitA-F (5'-caaccaaggaccgcgatg-3', SEQ ID NO. 42) and PcitA-R (5'-ttgaatccaacaagagtctaaaaaag-3', SEQ ID NO. 43) as a primer pair and using the genome of *Aspergillus terreus* CICC 40205 as a template. The amplified product was purified and recovered to obtain the PcitA promoter fragment.

PCR amplification was performed using fuAdi1-TtrpC-F (5'-cgaccgtagcctgtcctaaggatccacttaacgttactga-3', SEQ ID NO. 57) and TtrpC-R (5'-gtcgtgccagtcgactctaga-3', SEQ ID NO. 26) as a primer pair and using the plasmid pAN52-4 as a template. The amplified product was purified and recovered to obtain the TtrpC fragment.

The above three fragments were subjected to one-step cloning. PCR amplification was performed using PcitA-F (5'-caaccaaggaccgcgatg-3', SEQ ID NO. 42) and TtrpC-R (5'-gtcgtgccagtcgactctaga-3', SEQ ID NO. 26) as a primer pair. The amplified product was purified and recovered to obtain the PcitA-Adil-TtrpC expression cassette fragment.

8) Construction of an expression cassette of aconitate isomerase Adil using a PicdA promoter

PCR amplification was performed using fuPicdA-Adi1-F (5'-cgcaggccacgcttcactgtcgaaatgctgcaccccatcgacacca-3', SEQ ID NO. 66) and fuAdil-TtrpC-R (5'-tcagtaacgttaagtggatccttaggacaggctacggtcg-3', SEQ ID NO. 56) as a primer pair and using the puc57-Adi1 plasmid as a template. The amplified product was purified and recovered to obtain the *Adi1* gene fragment.

PCR amplification was performed using PicdA-F (5'-ctttaacgttgcagatacagggatgcg-3', SEQ ID NO. 47) and PicdA-R (5'-ttcgacagtgaagcgtggcctgcg-3', SEQ ID NO. 48) as a primer pair and using the genome of *Aspergillus niger* MEFC1501 as a template. The amplified product was purified and recovered to obtain the PicdA promoter fragment.

PCR amplification was performed using fuAdi1-TtrpC-F (5'-cgaccgtagcctgtcctaaggatccacttaacgttactga-3', SEQ ID NO. 57) and TtrpC-R (5'-gtcgtgccagtcgactctaga-3', SEQ ID NO. 26) as a primer pair and using the plasmid pAN52-4 as a template. The amplified product was purified and recovered to obtain the TtrpC fragment.

The above three fragments were subjected to one-step cloning. PCR amplification was performed using PicdA-F (5'-ctttaacgttgcagatacagggatgcg-3', SEQ ID NO. 47) and TtrpC-R (5'-gtcgtgccagtcgactctaga-3', SEQ ID NO. 26) as a primer pair. The amplified product was purified and recovered to obtain the PicdA-Adi1-TtrpC expression cassette fragment.

9) Construction of an expression cassette of aconitate isomerase Adil using a PgasA promoter

PCR amplification was performed using fuPgasA-Adi1-F (5'-gtcttctttcgttcacctcctcacatgctgcaccccatcgacacca-3', SEQ ID NO. 67) and fuAdil-TtrpC-R (5'-tcagtaacgttaagtggatccttaggacaggctacggtcg-3', SEQ ID NO. 56) as a primer pair and using the puc57-Adi1 plasmid as a template. The amplified product was purified and recovered to obtain the *Adi1* gene fragment.

PCR amplification was performed using PgasA-F (5'-ctgctctctctctgctctctttct-3', SEQ ID NO. 50) and PgasA-R (5'-gtgaggaggtgaacgaaagaagac-3', SEQ ID NO. 51) as a primer pair and using the genome of *Aspergillus niger* MEFC1501 as a template. The amplified product was purified and recovered to obtain the PgasA promoter fragment.

PCR amplification was performed using fuAdi1-TtrpC-F (5'-cgaccgtagcctgtcctaaggatccacttaacgttactga-3', SEQ ID NO. 57) and TtrpC-R (5'-gtcgtgccagtcgactctaga-3', SEQ ID NO. 26) as a primer pair and using the plasmid pAN52-4 as a template. The amplified product was purified and recovered to obtain the TtrpC fragment.

The above three fragments were subjected to one-step cloning. PCR amplification was performed using PgasA-F (5'-ctgctctctctctgctctctttct-3', SEQ ID NO. 50) and TtrpC-R (5'-gtcgtgccagtcgactctaga-3', SEQ ID NO. 26) as a primer pair. The amplified product was purified and recovered to obtain the PgasA-Adil-TtrpC expression cassette fragment.

2. Construction of hygromycin B selection marker gene *hph* expression cassette

The same as what is described in Example 1.

3. Transformation of *Aspergillus niger* protoplasts with aconitate isomerase Adil expression cassette to obtain recombinant *Aspergillus niger* strains

1) Preparation of *Aspergillus niger* protoplasts

The same as what is described in Example 1.

2) Co-transformation of *Aspergillus niger* protoplasts with aconitate isomerase Adil expression cassette and resistance gene *hph*

Approximately 5 μg of aconitate isomerase Adil expression cassette DNA and 1 μg of hygromycin B-resistant *hph* gene expression cassette DNA were added to the above protoplast suspension, followed by the addition of 50 μL of PSTC (40% PEG4000, 50 mM Tris-HCl pH 8.0, 50 mM $CaCl_2$). The mixture was gently mixed and placed on ice for 30 min. 1.5 mL of PSTC was added and mixed uniformly, and then left at room temperature for 20 min. Then it was mixed with the upper layer agar and poured onto the regeneration selection medium plates PDA-SH, followed by incubation at 30°C in the dark for 3 to 4 days.

Transformants were transferred from the plates to the selection plates PDA-H (4 g of potato dextrose agar medium was weighed and dissolved in 100 mL of distilled water, and then sterilized and cooled to approximately 55°C, hygromycin was added to a final concentration of 100 μg/mL to prepare the plates); the mixture was incubated at 30°C for 3 to 5 days to obtain transformants.

In a preparation process of the above recombinant *Aspergillus niger* strains containing the aconitate isomerase

Adil expression cassette, the aconitate isomerase Adil expression cassette and the hygromycin B-resistant *hph* gene expression cassette can also be linked into a single expression cassette, which is then used to transform protoplasts. This does not affect the screening of positive transformants and the identification of genotypes.

3) Verification of genotype of recombinant *Aspergillus niger* strains

The recombinant *Aspergillus niger* transformants obtained above were picked and inoculated onto PDA-H plates to culture spores. Then, the spores were respectively inoculated into liquid medium for culture, and mycelia were collected to extract the genome. PCR was performed using PgpdAt-F743 (5'-ttacactctgggaggatccaggtact-3', SEQ ID NO. 54) and TtrpC-R (5'-gtcgtgccagtcgactctaga-3', SEQ ID NO. 26) as a primer pair to amplify the PgpdAt-Adil-TtrpC inserted into the genome. The PtrpC-hph-TtrpC fragment inserted into the recombinant strains was amplified using hph-F (5'-ttcgggatc gcaagcgtaaag-3', SEQ ID NO. 52) and hph-R (5'-caattatctttgcgaacccagg-3', SEQ ID NO. 53) as a primer pair. The PCR products were analyzed by 0.8% agarose gel electrophoresis. The genomic DNA template of the starting strains *Aspergillus niger* MEFC1501 was used as a negative control.

Similarly, PCR was performed using PglaA-F (5'-ggattgcctgaacattgacattcgg-3', SEQ ID NO. 30) and TtrpC-R (5'-gtcgtgccagtcgactctaga-3', SEQ ID NO. 26) as a primer pair to amplify the PglaA-Adil-TtrpC inserted into the genome; PCR was performed using PgpdAn-F (5'-ttgatcgagacctaatacagc-3', SEQ ID NO. 28) and TtrpC-R (5'-gtcgtgccagtcgactctaga-3', SEQ ID NO. 26) as a primer pair to amplify the PgpdAn-Adil-TtrpC inserted into the genome; PCR was performed using PcadA-F (5'-ctaccaacagtctcgcggtgaatag-3', SEQ ID NO. 33) and TtrpC-R (5'-gtcgtgccagtcgactctaga-3', SEQ ID NO. 26) as a primer pair to amplify the PcadA-Adil-TtrpC inserted into the genome; PCR was performed using PmfsA-F (5'-gt acagtggccatgaaatccaatc-3', SEQ ID NO. 36) and TtrpC-R (5'-gtcgtgccagtcgactctaga-3', SEQ ID NO. 26) as a primer pair to amplify the PmfsA-Adil-TtrpC inserted into the genome; PCR was performed using PacoA-F (5'-tggcaccggtccgcgg ga-3', SEQ ID NO. 39) and TtrpC-R (5'-gtcgtgccagtcgactctaga-3', SEQ ID NO. 26) as a primer pair to amplify the PacoA-Adil-TtrpC inserted into the genome; PCR was performed using PcitA-F (5'-caaccaaggaccgcgatg-3', SEQ ID NO. 42) and TtrpC-R (5'-gtcgtgccagtcgactctaga-3', SEQ ID NO. 26) as a primer pair to amplify the PcitA-Adil-TtrpC inserted into the genome; PCR was performed using PicdA-F (5'-ctttaacgttgcagatacagggatgcg-3', SEQ ID NO. 47) and TtrpC-R (5'-gtc gtgccagtcgactctaga-3', SEQ ID NO. 26) as a primer pair to amplify the PicdA-Adi1-TtrpC inserted into the genome; PCR was performed using PgasA-F (5'-ctgctctctctctgctctctttct-3', SEQ ID NO. 50) and TtrpC-R (5'-gtcgtgccagtcgactctaga-3', SEQ ID NO. 26) as a primer pair to amplify the PgasA-Adi1-TtrpC inserted into the genome.

Transformants with correct genotype verification were transferred to PDA-H plates for subculture, with three passages in total. Then, the spores were collected respectively and appropriately diluted with physiological saline, and 100 μL of the diluted spore suspension was spread on PDA-H plates to allow the growth of independent single colonies, which was referred to as single-spore isolation. Spores were taken from the single colonies for single-spore isolation again, and a total of four rounds of single-spore isolation and subculture were performed. The recombinant strains after single-spore isolation were subjected to genotype identification again for verification. Some recombinant strains with correct verification were selected for shake flask fermentation screening. The specific shake flask screening method and results are shown in Fig. 2 in Example 4.

**Example 3** Construction of recombinant *Aspergillus niger* strains using pyrithiamine ptrA as a selection marker

Example 2 was repeated, and the difference lies in that in this example, the selection marker gene used in the recombinant *Aspergillus niger* strains was the pyrithiamine selection marker *ptrA* gene. The specific method is described as follows:

1. For the construction of expression cassettes of aconitate isomerase Adil with different promoters, please refer to Example 2.
2. Construction of recombinant *Aspergillus niger* strains using pyrithiamine resistance gene *ptrA* as a selection marker

    1) Construction of an expression cassette of pyrithiamine resistance gene *ptrA*

PCR amplification was performed using ptrA-F (5'-gggcaattgattacgggatc-3', SEQ ID NO. 68) and ptrA-R (5'-atggggtgacgatgagccgc-3', SEQ ID NO. 69) as a primer pair and using the plasmid pmWM23 as a template. The amplified product was purified and recovered to obtain the expression cassette of pyrithiamine resistance gene *ptrA.*

Protoplasts of *Aspergillus niger* MEFC1501 were prepared with reference to the preparation method in Example 1. Approximately 5 μg of aconitate isomerase Adil expression cassette DNA and 1 μg of the selection marker ptrA fragment were added to the protoplast suspension, followed by the addition of 50 μL of PSTC (40% PEG4000, 50 mM Tris-HCl pH 8.0, 50 mM $CaCl_2$). The mixture was gently mixed and placed on ice for 30 min. 1.5 mL of PSTC was added and mixed uniformly, and then left at room temperature for 20 min. Then it was mixed with the upper layer agar and poured onto the

regeneration selection medium plates CD-SPt, followed by incubation at 33°C in the dark for 3 to 4 days. Transformants were transferred from the plates to the selection plates CD-Pt and incubated at 33°C for 3 to 5 days to obtain transformants.

The prepared recombinant *Aspergillus terreus* transformants were picked and inoculated onto CD-Pt plates to culture spores. Then, the spores were respectively inoculated into PDB liquid medium for culture, and mycelia were collected to extract the genome. PCR was performed using PgpdAt-F743 (5'-ttacactctgggaggatccaggtact-3', SEQ ID NO. 54) and TtrpC-R (5'-gtcgtgccagtcgactctaga-3', SEQ ID NO. 26) as a primer pair to amplify the PgpdAt-Adil-TtrpC inserted into the genome. The ptrA fragment inserted into the recombinant strains were amplified using ptrA-F (5'-gggcaattgatt acgggatc-3', SEQ ID NO. 68) and ptrA-R (5'-atggggtgacgatgagccgc-3', SEQ ID NO. 69) as a primer pair. The PCR products were analyzed by 0.8% agarose gel electrophoresis. The genomic DNA template of the starting strains *Aspergillus niger* MEFC1501 was used as a negative control.

Similarly, PCR was performed using PglaA-F (5'-ggattgcctgaacattgacattcgg-3', SEQ ID NO. 30) and TtrpC-R (5'-gtcgtgccagtcgactctaga-3', SEQ ID NO. 26) as a primer pair to amplify the PglaA-Adil-TtrpC inserted into the genome; PCR was performed using PgpdAn-F (5'-ttgatcgagacctaatacagc-3', SEQ ID NO. 28) and TtrpC-R (5'-gtcgtgccagtcgactctaga-3', SEQ ID NO. 26) as a primer pair to amplify the PgpdAn-Adil-TtrpC inserted into the genome; PCR was performed using PcadA-F (5'-ctaccaacagtctcgcggtgaatag-3', SEQ ID NO. 33) and TtrpC-R (5'-gtcgtgccagtcgactctaga-3', SEQ ID NO. 26) as a primer pair to amplify the PcadA-Adil-TtrpC inserted into the genome; PCR was performed using PmfsA-F (5'-gt acagtggccatgaaatccaatc-3', SEQ ID NO. 36) and TtrpC-R (5'-gtcgtgccagtcgactctaga-3', SEQ ID NO. 26) as a primer pair to amplify the PmfsA-Adil-TtrpC inserted into the genome; PCR was performed using PacoA-F (5'-tggcaccggtccgcgg ga-3', SEQ ID NO. 39) and TtrpC-R (5'-gtcgtgccagtcgactctaga-3', SEQ ID NO. 26) as a primer pair to amplify the PacoA-Adil-TtrpC inserted into the genome; PCR was performed using PcitA-F (5'-caaccaaggaccgcgatg-3', SEQ ID NO. 42) and TtrpC-R (5'-gtcgtgccagtcgactctaga-3', SEQ ID NO. 26) as a primer pair to amplify the PcitA-Adil-TtrpC inserted into the genome; PCR was performed using PicdA-F (5'-ctttaacgttgcagatacagggatgcg-3', SEQ ID NO. 47) and TtrpC-R (5'-gtc gtgccagtcgactctaga-3', SEQ ID NO. 26) as a primer pair to amplify the PicdA-Adi1-TtrpC inserted into the genome; and PCR was performed using PgasA-F (5'-ctgctctctctctgctctctttct-3', SEQ ID NO. 50) and TtrpC-R (5'-gtcgtgccagtcgactct aga-3', SEQ ID NO. 26) as a primer pair to amplify the PgasA-Adi1-TtrpC inserted into the genome.

For the verification, isolation and purification of positive transformants, please refer to Example 1 or Example 2. The specific shake flask screening method and results are shown in Fig. 3 in Example 4.

**Example 4** Fermentation of recombinant *Aspergillus niger* to produce *trans*-Aconitic acid

1. Shake flask screening of recombinant *Aspergillus niger* strains for producing *trans*-Aconitic acid

The stable recombinant *Aspergillus niger* strains obtained after subculture of the recombinant strains from Examples 1, 2 and 3 were respectively inoculated into *Aspergillus niger* sporulation medium and incubated at 32°C for 6 days to obtain mature spores. Then, the mature spores of each strain were respectively inoculated into trans-Aconitic acid fermentation medium, with three parallel flasks for each strain. Fermentation was performed at 34°C under 220 rpm for 135 h. Mycelia in the fermentation broth were removed by filtration, and the fermentation supernatant was appropriately diluted for high-performance liquid chromatography (HPLC) analysis.

2. Analysis of content of aconitic acid in fermentation broth

The diluted fermentation supernatants of the recombinant strains and the starting strains *Aspergillus niger* MEFC1501 were subjected to high-performance liquid chromatography analysis. Standard curves were prepared using standard samples of *cis*-Aconitic acid and *trans*-Aconitic acid at different concentrations to analyze and compare the content and purity of cis-Aconitic acid and *trans*-Aconitic acid in the fermentation broth. The chromatographic conditions are as follows: chromatographic column: Aminex HPX-87H Organic Acid Analysis Column, 300 mm×7.8 mm (Bio-rad, Cat No. 1250140); mobile phase: 5 mmol/L sulfuric acid; flow rate: 0.5 mL/min; column temperature: 30°C; detection temperature: 30°C; UV detector (210 nm).

The production of *trans*-Aconitic acid by fermentation of the recombinant strains obtained in Examples 1, 2 and 3 is shown in Figs. 1 to 3.

The analysis results from Figs. 1 to 3 show that at the shake flask fermentation level, the contents of *cis*-Aconitic acid and *trans*-Aconitic acid in the fermentation broth of the recombinant strains are significantly higher than those of the wild-type strains. The effects of the two isomerases TbrA and Adil are not significantly different, but the gene expression driven by different promoters shows significant differences, and the content of *trans*-Aconitic acid can reach 34 g/L. There are no significant differences between different terminators such as TtrpC or Tpgk, and there are no significant differences between different selection markers such as hygromycin or pyrithiamine. Similar results are obtained with different transformation methods, such as co-transformation with the selection marker or constructing the expression cassette of the target gene and the expression cassette of the selection marker together for transformation.

In addition to *Aspergillus niger,* recombinant *Aspergillus terreus* engineered strains obtained by heterologously expressing aconitate isomerase TbrA from *Bacillus thuringiensis* or obtained by heterologously expressing aconitate isomerase Adil from *Ustilago maydis* in *Aspergillus terreus* can all produce trans-Aconitic acid. Especially after further knocking out the *cis*-aconitate decarboxylase CadA in *Aspergillus terreus* to block the synthesis pathway of itaconic acid, the yield of trans-Aconitic acid reaches 15.34 g/L to 24.65 g/L (recorded in patents CN112029671B and CN112011469B). This indicates that overexpression of aconitate isomerase can achieve the synthesis of *trans*-Aconitic acid in different Aspergillus species.

**Example 5** Construction of recombinant *Aspergillus niger* heterologously expressing aconitase Aco from *Aspergillus terreus* and heterologously expressing aconitate isomerase TbrA from *Bacillus thuringiensis*

1. Construction of an expression cassette of aconitase gene ATEG_03325

1) Construction of an expression cassette of aconitase gene ATEG_03325 using a PgpdAt promoter

PCR amplification was performed using fuPgpdAt-03325-F (5'-aacaactcatcaatcatcacaacatgatctccacccgccttg cgcgcat-3', SEQ ID NO. 70) and fu03325-TtrpC-R (5'-ttcagtaacgttaagtggatccttagttgctagcagccttgcgggc-3', SEQ ID NO. 71) as a primer pair and using the genome of *Aspergillus terreus* CICC 40205 as a template. The amplified product was purified and recovered to obtain the ATEG_03325 gene fragment.

PCR amplification was performed using fu03325-TtrpC-F (5'-gcccgcaaggctgctagcaactaaggatccacttaacgttact gaa-3', SEQ ID NO. 44) and fuPgpdAt-03325-R (5'-atgcgcgcaaggcgggtggagatcatgttgtgatgattgatgagttgtt-3', SEQ ID NO. 72) as a primer pair and using the plasmid pmWM23 as a template. The amplified product was purified and recovered to obtain the PgpdAt-TtrpC fragment. PgpdAt is the glyceraldehyde-3-phosphate dehydrogenase gene promoter from *Aspergillus terreus,* and TtrpC is the tryptophan synthase terminator from *Aspergillus nidulans.*

The above two fragments were subjected to one-step cloning. PCR amplification was performed using PgpdAt-F (5'-ttacactctgggaggatccaggtac-3', SEQ ID NO. 25) and TtrpC-R (5'-attacctctaaacaagtgtac-3', SEQ ID NO. 45) as a primer pair. The amplified product was purified and recovered to obtain the PgpdAt-ATEG_03325-TtrpC expression cassette fragment.

2) Construction of an expression cassette of aconitase gene ATEG_03325 using a PgpdAn promoter

PCR amplification was performed using fuPgpdAn-03325-F (5'-ccgcttgagcagacatcaccatgatctccacccgccttgcg cgcat-3', SEQ ID NO. 73) and fu03325-TtrpC-R (5'-ttcagtaacgttaagtggatccttagttgctagcagccttgcgggc-3', SEQ ID NO. 71) as a primer pair and using the genome of *Aspergillus terreus* CICC 40205 as a template. The amplified product was purified and recovered to obtain the ATEG_03325 gene fragment.

PCR amplification was performed using fu03325-TtrpC-F (5'-gcccgcaaggctgctagcaactaaggatccacttaacgttact gaa-3', SEQ ID NO. 44) and fuPgpdAn-03325-R (5'-atgcgcgcaaggcgggtggagatcatggtgatgtctgctcaagcgg-3', SEQ ID NO. 74) as a primer pair and using the plasmid pAN52-4 as a template. The amplified product was purified and recovered to obtain the PgpdAn-TtrpC fragment. PgpdAn is the glyceraldehyde-3-phosphate dehydrogenase gene promoter from *Aspergillus nidulans,* and TtrpC is the tryptophan synthase terminator from *Aspergillus nidulans.*

The above two fragments were subjected to one-step cloning. PCR amplification was performed using PgpdAn-F (5'-ttgatcgagacctaatacagc-3', SEQ ID NO. 28) and TtrpC-R (5'-attacctctaaacaagtgtac-3', SEQ ID NO. 45) as a primer pair. The amplified product was purified and recovered to obtain the PgpdAn-ATEG_03325-TtrpC expression cassette fragment.

3) Construction of an expression cassette of aconitase gene ATEG_03325 using a PglaA promoter

PCR amplification was performed using fuPglaA-03325-F (5'-cagcatcattacacctcagcaatgatctccacccgccttgcgc gcat-3', SEQ ID NO. 75) and fu03325-TtrpC-R (5'-ttcagtaacgttaagtggatccttagttgctagcagccttgcgggc-3', SEQ ID NO. 71) as a primer pair and using the genome of *Aspergillus terreus* CICC 40205 as a template. The amplified product was purified and recovered to obtain the ATEG_03325 gene fragment.

PCR amplification was performed using PglaA-F (5'-ggattgcctgaacattgacattcgg-3', SEQ ID NO. 30) and PglaA-R (5'-tgctgaggtgtaatgatgctggggat-3', SEQ ID NO. 31) as a primer pair and using the genome of *Aspergillus niger* MEFC1501 as a template. The amplified product was purified and recovered to obtain the PglaA promoter fragment.

PCR amplification was performed using fu03325-TtrpC-F (5'-gcccgcaaggctgctagcaactaaggatccacttaacgttact gaa-3', SEQ ID NO. 44) and TtrpC-R (5'-attacctctaaacaagtgtac-3', SEQ ID NO. 45) as a primer pair and using the plasmid pAN52-4 as a template. The amplified product was purified and recovered to obtain the TtrpC fragment.

The above three fragments were subjected to one-step cloning. PCR amplification was performed using PglaA-F

(5'-ggattgcctgaacattgacattcgg-3', SEQ ID NO. 30) and TtrpC-R (5'-attacctctaaacaagtgtac-3', SEQ ID NO. 45) as a primer pair. The amplified product was purified and recovered to obtain the PglaA-ATEG_03325-TtrpC expression cassette fragment.

4) Construction of an expression cassette of aconitase gene ATEG_03325 using a PcadA promoter

PCR amplification was performed using fuPcadA-03325-F (5'-cctcttaaattgaccatgaatgatctccacccgccttgcgcgca t-3', SEQ ID NO. 76) and fu03325-TtrpC-R (5'-ttcagtaacgttaagtggatccttagttgctagcagccttgcgggc-3', SEQ ID NO. 71) as a primer pair and using the genome of *Aspergillus terreus* CICC 40205 as a template. The amplified product was purified and recovered to obtain the ATEG_03325 gene fragment.
PCR amplification was performed using PcadA-F (5'-ctaccaacagtctcgcggtgaatag-3', SEQ ID NO. 33) and PcadA-R (5'-ggtcaatttaagaggacgatcttcg-3', SEQ ID NO. 34) as a primer pair and using the genome of *Aspergillus terreus* CICC 40205 as a template. The amplified product was purified and recovered to obtain the PcadA promoter fragment.
PCR amplification was performed using fu03325-TtrpC-F (5'-gcccgcaaggctgctagcaactaaggatccacttaacgttact gaa-3', SEQ ID NO. 44) and TtrpC-R (5'-attacctctaaacaagtgtac-3', SEQ ID NO. 45) as a primer pair and using the plasmid pAN52-4 as a template. The amplified product was purified and recovered to obtain the TtrpC fragment.
The above three fragments were subjected to one-step cloning. PCR amplification was performed using PcadA-F (5'-ctaccaacagtctcgcggtgaatag-3', SEQ ID NO. 33) and TtrpC-R (5'-attacctctaaacaagtgtac-3', SEQ ID NO. 45) as a primer pair. The amplified product was purified and recovered to obtain the PcadA-ATEG_03325-TtrpC expression cassette fragment.

5) Construction of an expression cassette of aconitase gene ATEG_03325 using a PmfsA promoter

PCR amplification was performed using fuPmfsA-03325-F "(5'-accgacttctcatccatcttcaaaatgatctccacccgccttgc gcgcat-3, SEQ ID NO. 77) and fu03325-TtrpC-R (5'-ttcagtaacgttaagtggatccttagttgctagcagccttgcgggc-3', SEQ ID NO. 71) as a primer pair and using the genome of *Aspergillus terreus* CICC 40205 as a template. The amplified product was purified and recovered to obtain the ATEG_03325 gene fragment.
PCR amplification was performed using PmfsA-F (5'-gtacagtggccatgaaatccaatc-3', SEQ ID NO. 36) and PmfsA-R (5'-tttgaagatggatgagaagtcggt-3', SEQ ID NO. 37) as a primer pair and using the genome of *Aspergillus niger* MEFC1501 as a template. The amplified product was purified and recovered to obtain the PmfsA promoter fragment.
PCR amplification was performed using fu03325-TtrpC-F (5'-gcccgcaaggctgctagcaactaaggatccacttaacgttact gaa-3', SEQ ID NO. 44) and TtrpC-R (5'-attacctctaaacaagtgtac-3', SEQ ID NO. 45) as a primer pair and using the plasmid pAN52-4 as a template. The amplified product was purified and recovered to obtain the TtrpC fragment.
The above three fragments were subjected to one-step cloning. PCR amplification was performed using PmfsA-F (5'-gtacagtggccatgaaatccaatc-3', SEQ ID NO. 36) and TtrpC-R (5'-attacctctaaacaagtgtac-3', SEQ ID NO. 45) as a primer pair. The amplified product was purified and recovered to obtain the PmfsA-ATEG_03325-TtrpC expression cassette fragment.

6) Construction of an expression cassette of aconitase gene ATEG_03325 using a PacoA promoter

PCR amplification was performed using fuPacoA-03325-F (5'-cctcgcatagagagcttccatcatgatctccacccgccttgcg cgcat-3', SEQ ID NO. 78) and fu03325-TtrpC-R (5'-ttcagtaacgttaagtggatccttagttgctagcagccttgcgggc-3', SEQ ID NO. 71) as a primer pair and using the genome of *Aspergillus terreus* CICC 40205 as a template. The amplified product was purified and recovered to obtain the ATEG_03325 gene fragment.
PCR amplification was performed using PacoA-F (5'-tggcaccggtccgcggga-3', SEQ ID NO. 39) and PacoA-R (5'-gatggaagctctctatgcgagg-3', SEQ ID NO. 40) as a primer pair and using the genome of *Aspergillus terreus* CICC 40205 as a template. The amplified product was purified and recovered to obtain the PacoA promoter fragment.
PCR amplification was performed using fu03325-TtrpC-F (5'-gcccgcaaggctgctagcaactaaggatccacttaacgttact gaa-3', SEQ ID NO. 44) and TtrpC-R (5'-attacctctaaacaagtgtac-3', SEQ ID NO. 45) as a primer pair and using the plasmid pAN52-4 as a template. The amplified product was purified and recovered to obtain the TtrpC fragment.
The above three fragments were subjected to one-step cloning. PCR amplification was performed using PacoA-F (5'-tggcaccggtccgcggga-3', SEQ ID NO. 39) and TtrpC-R (5'-attacctctaaacaagtgtac-3', SEQ ID NO. 45) as a primer pair. The amplified product was purified and recovered to obtain the PacoA-ATEG_03325-TtrpC expression cassette fragment.

7) Construction of an expression cassette of aconitase gene ATEG_03325 using a PcitA promoter

PCR amplification was performed using fuPcitA-03325-F (5'-cttttttagactcttgttggattcaaaatgatctccacccgccttgcg cgcat-3', SEQ ID NO. 79) and fu03325-TtrpC-R (5'-ttcagtaacgttaagtggatccttagttgctagcagccttgcgggc-3', SEQ ID NO. 71)

as a primer pair and using the genome of *Aspergillus terreus* CICC 40205 as a template. The amplified product was purified and recovered to obtain the ATEG_03325 gene fragment.

PCR amplification was performed using PcitA-F (5'-caaccaaggaccgcgatg-3', SEQ ID NO. 42) and PcitA-R (5'-ttgaatccaacaagagtctaaaaaag-3', SEQ ID NO. 43) as a primer pair and using the genome of *Aspergillus terreus* CICC 40205 as a template. The amplified product was purified and recovered to obtain the PcitA promoter fragment.

PCR amplification was performed using fu03325-TtrpC-F (5'-gcccgcaaggctgctagcaactaaggatccacttaacgttact gaa-3', SEQ ID NO. 44) and TtrpC-R (5'-attacctctaaacaagtgtac-3', SEQ ID NO. 45) as a primer pair and using the plasmid pAN52-4 as a template. The amplified product was purified and recovered to obtain the TtrpC fragment.

The above three fragments were subjected to one-step cloning. PCR amplification was performed using PcitA-F (5'-caaccaaggaccgcgatg-3', SEQ ID NO. 42) and TtrpC-R (5'-attacctctaaacaagtgtac-3', SEQ ID NO. 45) as a primer pair. The amplified product was purified and recovered to obtain the PcitA-ATEG_03325-TtrpC expression cassette fragment.

8) Construction of an expression cassette of aconitase gene ATEG_03325 using a PicdA promoter

PCR amplification was performed using fuPicdA-03325-F (5'-cgcaggccacgcttcactgtcgaaatgatctccacccgccttg cgcgcat-3', SEQ ID NO. 80) and fu03325-TtrpC-R (5'-ttcagtaacgttaagtggatccttagttgctagcagccttgcgggc-3', SEQ ID NO. 71) as a primer pair and using the genome of *Aspergillus terreus* CICC 40205 as a template. The amplified product was purified and recovered to obtain the ATEG_03325 gene fragment.

PCR amplification was performed using PicdA-F (5'-ctttaacgttgcagatacagggatgcg-3', SEQ ID NO. 47) and PicdA-R (5'-ttcgacagtgaagcgtggcctgcg-3', SEQ ID NO. 48) as a primer pair and using the genome of *Aspergillus niger* MEFC1501 as a template. The amplified product was purified and recovered to obtain the PicdA promoter fragment.

PCR amplification was performed using fu03325-TtrpC-F (5'-gcccgcaaggctgctagcaactaaggatccacttaacgttact gaa-3', SEQ ID NO. 44) and TtrpC-R (5'-attacctctaaacaagtgtac-3', SEQ ID NO. 45) as a primer pair and using the plasmid pAN52-4 as a template. The amplified product was purified and recovered to obtain the TtrpC fragment.

The above three fragments were subjected to one-step cloning. PCR amplification was performed using PicdA-F (5'-ctttaacgttgcagatacagggatgcg-3', SEQ ID NO. 47) and TtrpC-R (5'-attacctctaaacaagtgtac-3', SEQ ID NO. 45) as a primer pair. The amplified product was purified and recovered to obtain the PicdA-ATEG_03325-TtrpC expression cassette fragment.

9) Construction of an expression cassette of aconitase gene ATEG_03325 using a PgasA promoter

PCR amplification was performed using fuPgasA-03325-F (5'-gtcttctttcgttcacctcctcacatgatctccacccgccttgcgc gcat-3', SEQ ID NO. 81) and fu03325-TtrpC-R (5'-ttcagtaacgttaagtggatccttagttgctagcagccttgcgggc-3', SEQ ID NO. 71) as a primer pair and using the genome of *Aspergillus terreus* CICC 40205 as a template. The amplified product was purified and recovered to obtain the ATEG_03325 gene fragment.

PCR amplification was performed using PgasA-F (5'-ctgctctctctctgctctctttct-3', SEQ ID NO. 50) and PgasA-R (5'-gtgaggaggtgaacgaaagaagac-3', SEQ ID NO. 51) as a primer pair and using the genome of *Aspergillus niger* MEFC1501 as a template. The amplified product was purified and recovered to obtain the PgasA promoter fragment.

PCR amplification was performed using fu03325-TtrpC-F (5'-gcccgcaaggctgctagcaactaaggatccacttaacgttact gaa-3', SEQ ID NO. 44) and TtrpC-R (5'-attacctctaaacaagtgtac-3', SEQ ID NO. 45) as a primer pair and using the plasmid pAN52-4 as a template. The amplified product was purified and recovered to obtain the TtrpC fragment.

The above three fragments were subjected to one-step cloning. PCR amplification was performed using PgasA-F (5'-ctgctctctctctgctctctttct-3', SEQ ID NO. 50) and TtrpC-R (5'-attacctctaaacaagtgtac-3', SEQ ID NO. 45) as a primer pair. The amplified product was purified and recovered to obtain the PgasA-ATEG_03325-TtrpC expression cassette fragment.

2. Construction of targeting element for aconitate isomerase TbrA

The aconitate isomerase TbrA gene was artificially synthesized and codon-optimized for expression in *Aspergillus niger.* The aconitate isomerase TbrA gene was cloned into the pUC57-Kan vector to construct the plasmid puc57-TbrA.

1) Construction of an expression cassette of aconitate isomerase TbrA using a PgpdAt promoter

PCR amplification was performed using fuPgpdAt-TbrA-F (5'-ctcatcaatcatcacaacatgaagatcccctgcttcg-3', SEQ ID NO. 21) and fuTbrA-TtrpC-R (5'-tcagtaacgttaagtggatccttaggggatgatcagctcg-3', SEQ ID NO. 22) as a primer pair and using the puc57-TbrA plasmid as a template. The amplified product was purified and recovered to obtain the *tbrA* gene fragment.

PCR amplification was performed using fuTbrA-TtrpC-F (5'-cgagctgatcatcccctaaggatccacttaacgttactga-3', SEQ

ID NO. 23) and fuPgpdAt-TbrA-R (5'-cgaagcaggggatcttcatgttgtgatgattgatgag-3', SEQ ID NO. 24) as a primer pair and using the plasmid pmWM23 as a template. The amplified product was purified and recovered to obtain the PgpdAt-TtrpC fragment.

The above two fragments were subjected to one-step cloning. PCR amplification was performed using PgpdAt-F (5'-ttacactctgggaggatccaggtac-3', SEQ ID NO. 25) and TtrpC-R (5'-attacctctaaacaagtgtac-3', SEQ ID NO. 45) as a primer pair. The amplified product was purified and recovered to obtain the PgpdAt-TbrA-TtrpC expression cassette fragment.

2) Construction of an expression cassette of aconitate isomerase TbrA using a PgpdAn promoter

PCR amplification was performed using fuPgpdAn-TbrA-F (5'-ccgcttgagcagacatcaccatgaagatcccctgcttcgt-3', SEQ ID NO. 82) and fuTbrA-TtrpC-R (5'-tcagtaacgttaagtggatccttaggggatgatcagctcg-3', SEQ ID NO. 22) as a primer pair and using the puc57-TbrA plasmid as a template. The amplified product was purified and recovered to obtain the *tbrA* gene fragment.

PCR amplification was performed using fuTbrA-TtrpC-F (5'-cgagctgatcatcccctaaggatccacttaacgttactga-3', SEQ ID NO. 23) and fuPgpdAn-TbrA-R (5'-acgaagcaggggatcttcatggtgatgtctgctcaagcgg-3', SEQ ID NO. 27) as a primer pair and using the plasmid pAN52-4 as a template. The amplified product was purified and recovered to obtain the PgpdAn-TtrpC fragment. PgpdAn is the glyceraldehyde-3-phosphate dehydrogenase gene promoter from *Aspergillus nidulans,* and TtrpC is the tryptophan synthase terminator from *Aspergillus nidulans.*

The above two fragments were subjected to one-step cloning. PCR amplification was performed using PgpdAn-F (5'-ttgatcgagacctaatacagc-3', SEQ ID NO. 28) and TtrpC-R (5'-attacctctaaacaagtgtac-3', SEQ ID NO. 45) as a primer pair. The amplified product was purified and recovered to obtain the PgpdAn-TbrA-TtrpC expression cassette fragment.

3) Construction of an expression cassette of aconitate isomerase TbrA using a PglaA promoter

PCR amplification was performed using fuPglaA-TbrA-F (5'-gcatcattacacctcagcaatgaagatcccctgcttcg-3', SEQ ID NO. 29) and fuTbrA-TtrpC-R (5'-tcagtaacgttaagtggatccttaggggatgatcagctcg-3', SEQ ID NO. 22) as a primer pair and using the puc57-TbrA plasmid as a template. The amplified product was purified and recovered to obtain the *tbrA* gene fragment.

PCR amplification was performed using PglaA-F (5'-ggattgcctgaacattgacattcgg-3', SEQ ID NO. 30) and PglaA-R (5'-tgctgaggtgtaatgatgctggggat-3', SEQ ID NO. 31) as a primer pair and using the genome of *Aspergillus niger* MEFC1501 as a template. The amplified product was purified and recovered to obtain the PglaA promoter fragment.

PCR amplification was performed using fuTbrA-TtrpC-F (5'-cgagctgatcatcccctaaggatccacttaacgttactga-3', SEQ ID NO. 23) and TtrpC-R (5'-attacctctaaacaagtgtac-3', SEQ ID NO. 45) as a primer pair and using the plasmid pAN52-4 as a template. The amplified product was purified and recovered to obtain the TtrpC fragment.

The above three fragments were subjected to one-step cloning. PCR amplification was performed using PglaA-F (5'-ggattgcctgaacattgacattcgg-3', SEQ ID NO. 30) and TtrpC-R (5'-attacctctaaacaagtgtac-3', SEQ ID NO. 45) as a primer pair. The amplified product was purified and recovered to obtain the PglaA-TbrA-TtrpC expression cassette fragment.

4) Construction of an expression cassette of aconitate isomerase TbrA using a PcadA promoter

PCR amplification was performed using fuPcadA-TbrA-F (5'-cctcttaaattgaccatgaatgaagatcccctgcttcg-3', SEQ ID NO. 32) and fuTbrA-TtrpC-R (5'-tcagtaacgttaagtggatccttaggggatgatcagctcg-3', SEQ ID NO. 22) as a primer pair and using the puc57-TbrA plasmid as a template. The amplified product was purified and recovered to obtain the *tbrA* gene fragment.

PCR amplification was performed using PcadA-F (5'-ctaccaacagtctcgcggtgaatag-3', SEQ ID NO. 33) and PcadA-R (5'-tcatggtcaatttaagagg-3', SEQ ID NO. 83) as a primer pair and using the genome of *Aspergillus terreus* CICC 40205 as a template. The amplified product was purified and recovered to obtain the PcadA promoter fragment.

PCR amplification was performed using fuTbrA-TtrpC-F (5'-cgagctgatcatcccctaaggatccacttaacgttactga-3', SEQ ID NO. 23) and TtrpC-R (5'-attacctctaaacaagtgtac-3', SEQ ID NO. 45) as a primer pair and using the plasmid pAN52-4 as a template. The amplified product was purified and recovered to obtain the TtrpC fragment.

The above three fragments were subjected to one-step cloning. PCR amplification was performed using PcadA-F (5'-ctaccaacagtctcgcggtgaatag-3', SEQ ID NO. 33) and TtrpC-R (5'-attacctctaaacaagtgtac-3', SEQ ID NO. 45) as a primer pair. The amplified product was purified and recovered to obtain the PcadA-TbrA-TtrpC expression cassette fragment.

5) Construction of an expression cassette of aconitate isomerase TbrA using a PmfsA promoter

PCR amplification was performed using fuPmfsA-TbrA-F (5'-accgacttctcatccatcttcaaaatgaagatcccctgcttcg-3',

SEQ ID NO. 35) and fuTbrA-TtrpC-R (5'-tcagtaacgttaagtggatccttaggggatgatcagctcg-3', SEQ ID NO. 22) as a primer pair and using the puc57-TbrA plasmid as a template. The amplified product was purified and recovered to obtain the *tbrA* gene fragment.

PCR amplification was performed using PmfsA-F (5'-gtacagtggccatgaaatccaatc-3', SEQ ID NO. 36) and PmfsA-R (5'-tttgaagatggatgagaagtcggt-3', SEQ ID NO. 37) as a primer pair and using the genome of *Aspergillus niger* MEFC1501 as a template. The amplified product was purified and recovered to obtain the PmfsA promoter fragment. PCR amplification was performed using fuTbrA-TtrpC-F (5'-cgagctgatcatcccctaaggatccacttaacgttactga-3', SEQ ID NO. 23) and TtrpC-R (5'-attacctctaaacaagtgtac-3', SEQ ID NO. 45) as a primer pair and using the plasmid pAN52-4 as a template. The amplified product was purified and recovered to obtain the TtrpC fragment.

The above three fragments were subjected to one-step cloning. PCR amplification was performed using PmfsA-F (5'-gtacagtggccatgaaatccaatc-3', SEQ ID NO. 36) and TtrpC-R (5'-attacctctaaacaagtgtac-3', SEQ ID NO. 45) as a primer pair. The amplified product was purified and recovered to obtain the PmfsA-TbrA-TtrpC expression cassette fragment.

6) Construction of an expression cassette of aconitate isomerase TbrA using a PacoA promoter

PCR amplification was performed using fuPacoA-TbrA-F (5'-cctcgcatagagagcttccatcatgaagatcccctgcttcg-3', SEQ ID NO. 38) and fuTbrA-TtrpC-R (5'-tcagtaacgttaagtggatccttaggggatgatcagctcg-3', SEQ ID NO. 22) as a primer pair and using the puc57-TbrA plasmid as a template. The amplified product was purified and recovered to obtain the *tbrA* gene fragment.

PCR amplification was performed using PacoA-F (5'-tggcaccggtccgcggga-3', SEQ ID NO. 39) and PacoA-R (5'-gatggaagctctctatgcgagg-3', SEQ ID NO. 40) as a primer pair and using the genome of *Aspergillus terreus* CICC 40205 as a template. The amplified product was purified and recovered to obtain the PacoA promoter fragment.

PCR amplification was performed using fuTbrA-TtrpC-F (5'-cgagctgatcatcccctaaggatccacttaacgttactga-3', SEQ ID NO. 23) and TtrpC-R (5'-attacctctaaacaagtgtac-3', SEQ ID NO. 45) as a primer pair and using the plasmid pAN52-4 as a template. The amplified product was purified and recovered to obtain the TtrpC fragment.

The above three fragments were subjected to one-step cloning. PCR amplification was performed using PacoA-F (5'-tggcaccggtccgcggga-3', SEQ ID NO. 39) and TtrpC-R (5'-attacctctaaacaagtgtac-3', SEQ ID NO. 45) as a primer pair. The amplified product was purified and recovered to obtain the PacoA-TbrA-TtrpC expression cassette fragment.

7) Construction of an expression cassette of aconitate isomerase TbrA using a PcitA promoter

PCR amplification was performed using fuPcitA-TbrA-F (5'-cttttttagactcttgttggattcaaaatgaagatcccctgcttcg-3', SEQ ID NO. 41) and fuTbrA-TtrpC-R (5'-tcagtaacgttaagtggatccttaggggatgatcagctcg-3', SEQ ID NO. 22) as a primer pair and using the puc57-TbrA plasmid as a template. The amplified product was purified and recovered to obtain the *tbrA* gene fragment.

PCR amplification was performed using PcitA-F (5'-caaccaaggaccgcgatg-3', SEQ ID NO. 42) and PcitA-R (5'-ttgaatccaacaagagtctaaaaaag-3', SEQ ID NO. 43) as a primer pair and using the genome of *Aspergillus terreus* CICC 40205 as a template. The amplified product was purified and recovered to obtain the PcitA promoter fragment.

PCR amplification was performed using fuTbrA-TtrpC-F (5'-cgagctgatcatcccctaaggatccacttaacgttactga-3', SEQ ID NO. 23) and TtrpC-R (5'-attacctctaaacaagtgtac-3', SEQ ID NO. 45) as a primer pair and using the plasmid pAN52-4 as a template. The amplified product was purified and recovered to obtain the TtrpC fragment.

The above three fragments were subjected to one-step cloning. PCR amplification was performed using PcitA-F (5'-caaccaaggaccgcgatg-3', SEQ ID NO. 42) and TtrpC-R (5'-attacctctaaacaagtgtac-3', SEQ ID NO. 45) as a primer pair. The amplified product was purified and recovered to obtain the PcitA-TbrA-TtrpC expression cassette fragment.

8) Construction of an expression cassette of aconitate isomerase TbrA using a PicdA promoter

PCR amplification was performed using fuPicdA-TbrA-F (5'-cgcaggccacgcttcactgtcgaaatgaagatcccctgcttcg-3', SEQ ID NO. 46) and fuTbrA-TtrpC-R (5'-tcagtaacgttaagtggatccttaggggatgatcagctcg-3', SEQ ID NO. 22) as a primer pair and using the puc57-TbrA plasmid as a template. The amplified product was purified and recovered to obtain the *tbrA* gene fragment.

PCR amplification was performed using PicdA-F (5'-ctttaacgttgcagatacagggatgcg-3', SEQ ID NO. 47) and PicdA-R (5'-ttcgacagtgaagcgtggcctgcg-3', SEQ ID NO. 48) as a primer pair and using the genome of *Aspergillus niger* MEFC1501 as a template. The amplified product was purified and recovered to obtain the PicdA promoter fragment.

PCR amplification was performed using fuTbrA-TtrpC-F (5'-cgagctgatcatcccctaaggatccacttaacgttactga-3', SEQ ID NO. 23) and TtrpC-R (5'-attacctctaaacaagtgtac-3', SEQ ID NO. 45) as a primer pair and using the plasmid pAN52-4 as a template. The amplified product was purified and recovered to obtain the TtrpC fragment.

The above three fragments were subjected to one-step cloning. PCR amplification was performed using PicdA-F (5'-ctttaacgttgcagatacagggatgcg-3', SEQ ID NO. 47) and TtrpC-R (5'-attacctctaaacaagtgtac-3', SEQ ID NO. 45) as a primer pair. The amplified product was purified and recovered to obtain the PicdA-TbrA-TtrpC expression cassette fragment.

9) Construction of an expression cassette of aconitate isomerase TbrA using a PgasA promoter

PCR amplification was performed using fuPgasA-TbrA-F (5'-gtcttctttcgttcacctcctcacatgaagatcccctgcttcg-3', SEQ ID NO. 49) and fuTbrA-TtrpC-R (5'-tcagtaacgttaagtggatccttaggggatgatcagctcg-3', SEQ ID NO. 22) as a primer pair and using the puc57-TbrA plasmid as a template. The amplified product was purified and recovered to obtain the *tbrA* gene fragment.

PCR amplification was performed using PgasA-F (5'-ctgctctctctctgctctctttct-3', SEQ ID NO. 50) and PgasA-R (5'-gtgaggaggtgaacgaaagaagac-3', SEQ ID NO. 51) as a primer pair and using the genome of *Aspergillus niger* MEFC1501 as a template. The amplified product was purified and recovered to obtain the PgasA promoter fragment.

PCR amplification was performed using fuTbrA-TtrpC-F (5'-cgagctgatcatcccctaaggatccacttaacgttactga-3', SEQ ID NO. 23) and TtrpC-R (5'-attacctctaaacaagtgtac-3', SEQ ID NO. 45) as a primer pair and using the plasmid pAN52-4 as a template. The amplified product was purified and recovered to obtain the TtrpC fragment.

The above three fragments were subjected to one-step cloning. PCR amplification was performed using PgasA-F (5'-ctgctctctctctgctctctttct-3', SEQ ID NO. 50) and TtrpC-R (5'-attacctctaaacaagtgtac-3', SEQ ID NO. 45) as a primer pair. The amplified product was purified and recovered to obtain the PgasA-TbrA-TtrpC expression cassette fragment.

3. Construction of an expression cassette of hygromycin B selection marker gene *hph*

PCR amplification was performed using hph-F (5'-ttcgggatcgcaagcgtaaag-3', SEQ ID NO. 52) and hph-R (5'-caattatctttgcgaacccagg-3', SEQ ID NO. 53) as a primer pair and using the plasmid pSGF957 as a template, to obtain the PtrpC-hph-TtrpC fragment. PtrpC is the tryptophan synthase gene promoter from *Aspergillus nidulans,* hph is the hygromycin phosphotransferase gene, and TtrpC is the tryptophan synthase terminator from *Aspergillus nidulans.* The amplified product was purified and recovered to obtain the hygromycin B-resistant *hph* gene expression cassette PtrpC-hph-TtrpC.

4. Co-transformation of *Aspergillus niger* protoplasts with aconitase gene ATEG_03325 expression cassette and aconitate isomerase TbrA expression cassette to obtain recombinant *Aspergillus niger* strains

1) Preparation of *Aspergillus niger* protoplasts

The spore suspension of *Aspergillus niger* MEFC1501 was inoculated into 50 mL of PDB liquid medium, with a spore concentration of approximately $10^7$ spores/mL, followed by incubation at 33°C under 200 rpm for 12 to 18 h. The grown mycelia were collected by filtration through a sterile single-layer 100-mesh nylon cloth, rinsed for three times with a sterile 0.6 M $MgSO_4$ solution, and pressed dry and then placed in a 50 mL sterile Erlenmeyer flask. 1 g of mycelia was weighed, 10 mL of enzyme solution was added, and the mixture was treated at 30°C under 120 rpm for 1 to 2 h. The components of the enzyme solution include 1% cellulase (Sigma, Catalog No.: C1184), 1% lysing enzyme (Sigma, Catalog No.: L1412), 1% snailase (Sangon Biotech, Catalog No.: SB0870), and 0.6 M $MgSO_4$. The enzyme solution was sterilized by filtration through a 0.22 $\mu$m sterile filter.

The above mixture after enzymatic digestion was filtered through a 300-mesh nylon cloth, and the filtrate was collected. Protoplasts were collected by centrifugation at 4°C, washed once with 1.0 M pre-cooled sorbitol solution, then washed once with pre-cooled STC (1.0 M sorbitol, 50 mM Tris·HCl-pH 8.0, 50 mM $CaCl_2$). Finally, the protoplasts were resuspended in 150 $\mu$L of pre-cooled STC, and the concentration of protoplasts was adjusted to $5\times10^7$ protoplasts/mL using STC to obtain the protoplast suspension.

2) Co-transformation of *Aspergillus niger* protoplasts with aconitase gene ATEG_03325 expression cassette, aconitate isomerase TbrA expression cassette and resistance gene *hph*

Approximately 5 $\mu$g of aconitase gene ATEG_03325 expression cassette DNA, approximately 5 $\mu$g of aconitate isomerase TbrA expression cassette DNA, and 1 $\mu$g of hygromycin B-resistant *hph* gene expression cassette DNA were added to the above protoplast suspension, followed by the addition of 50 $\mu$L of PSTC (40% PEG4000, 50 mM Tris-HCl pH 8.0, 50 mM $CaCl_2$). The mixture was gently mixed and placed on ice for 30 min. 1.5 mL of PSTC was added and mixed uniformly, and then left at room temperature for 20 min. Then it was mixed with the upper layer agar and poured onto the regeneration selection medium plates PDA-SH, followed by incubation at 30°C in the dark for 3 to 4 days.

Transformants were transferred from the plates to the selection plates PDA-H (4 g of potato dextrose agar medium was weighed and dissolved in 100 mL of distilled water, and then sterilized and cooled to approximately 55°C, hygromycin was added to a final concentration of 100 $\mu$g/mL to prepare the plates); the mixture was incubated at 30°C for 3 to 5 days to obtain transformants.

In a preparation process of the above recombinant *Aspergillus niger* strains containing the ATEG_03325 expression cassette and TbrA expression cassette driven by various promoters, the ATEG_03325 expression cassette and the hygromycin B-resistant hph gene expression cassette can also be linked into a single expression cassette, or the TbrA expression cassette and the hygromycin B-resistant hph gene expression cassette can be linked into a single expression cassette, which is then used to transform protoplasts. This does not affect the screening of positive transformants and the identification of genotypes.

3) Verification of genotype of recombinant *Aspergillus niger* strains

The recombinant *Aspergillus niger* transformants obtained above were picked and inoculated onto PDA-H plates to culture spores. Then, the spores were respectively inoculated into liquid medium for culture, and mycelia were collected to extract the genome. PCR was performed using PgpdAt-F743 (5'-ttacactctgggaggatccaggtact-3', SEQ ID NO. 54) and TtrpC-R (5'-attacctctaaacaagtgtac-3', SEQ ID NO. 45) as a primer pair to amplify the PgpdAt-ATEG_03325-TtrpC element and/or PgpdAt-TbrA-TtrpC inserted into the genome; PCR was performed using PglaA-F (5'-ggattgcctgaacattgacattc gg-3', SEQ ID NO. 30) and TtrpC-R (5'-attacctctaaacaagtgtac-3', SEQ ID NO. 45) as a primer pair to amplify the PglaA-ATEG_03325-TtrpC element and/or PglaA-TbrA-TtrpC inserted into the genome; PCR was performed using PgpdAn-F (5'-ttgatcgagacctaatacagc-3', SEQ ID NO. 28) and TtrpC-R (5'-attacctctaaacaagtgtac-3', SEQ ID NO. 45) as a primer pair to amplify the PgpdAn-ATEG_03325-TtrpC element and/or PgpdAn-TbrA-TtrpC inserted into the genome; PCR was performed using PcadA-F (5'-ctaccaacagtctcgcggtgaatag-3', SEQ ID NO. 33) and TtrpC-R (5'-attacctctaaacaagtgtac-3', SEQ ID NO. 45) as a primer pair to amplify the PcadA-ATEG_03325-TtrpC element and/or PcadA-TbrA-TtrpC inserted into the genome; PCR was performed using PmfsA-F (5'-gtacagtggccatgaaatccaatc-3', SEQ ID NO. 36) and TtrpC-R (5'-attacctctaaacaagtgtac-3', SEQ ID NO. 45) as a primer pair to amplify the PmfsA-ATEG_03325-TtrpC element and/or PmfsA-TbrA-TtrpC inserted into the genome; PCR was performed using PacoA-F (5'-tggcaccggtccgcggga-3', SEQ ID NO. 39) and TtrpC-R (5'-attacctctaaacaagtgtac-3', SEQ ID NO. 45) as a primer pair to amplify the PacoA-ATEG_03325-TtrpC element and/or PacoA-TbrA-TtrpC inserted into the genome; PCR was performed using PcitA-F (5'-caaccaagg accgcgatg-3', SEQ ID NO. 42) and TtrpC-R (5'-attacctctaaacaagtgtac-3', SEQ ID NO. 45) as a primer pair to amplify the PcitA-ATEG_03325-TtrpC element and/or PcitA-TbrA-TtrpC inserted into the genome; PCR was performed using PicdA-F (5'-ctttaacgttgcagatacagggatgcg-3', SEQ ID NO. 47) and TtrpC-R (5'-attacctctaaacaagtgtac-3', SEQ ID NO. 45) as a primer pair to amplify the PicdA-ATEG_03325-TtrpC element and/or PicdA-TbrA-TtrpC inserted into the genome; PCR was performed using PgasA-F (5'-ctgctctctctctgctctctttct-3', SEQ ID NO. 50) and TtrpC-R (5'-attacctctaaacaagtgtac-3', SEQ ID NO. 45) as a primer pair to amplify the PgasA-ATEG_03325-TtrpC element and/or PgasA-TbrA-TtrpC inserted into the genome.

The PtrpC-hph-TtrpC fragment inserted into the recombinant strains was amplified using hph-F (5'-ttcgggatc gcaagcgtaaag-3', SEQ ID NO. 52) and hph-R (5'-caattatctttgcgaacccagg-3', SEQ ID NO. 53) as a primer pair. The PCR products were analyzed by 0.8% agarose gel electrophoresis.

The genomic DNA template of the starting strains *Aspergillus niger* MEFC1501 was used as a negative control.

Transformants with correct genotype verification were transferred to PDA-H plates for subculture, with three passages in total. Then, the spores were collected respectively and appropriately diluted with physiological saline, and 100 $\mu$L of the diluted spore suspension was spread on PDA-H plates to allow the growth of independent single colonies, which was referred to as single-spore isolation. Spores were taken from the single colonies for single-spore isolation again, and a total of four rounds of single-spore isolation and subculture were performed. The recombinant strains after single-spore isolation were subjected to genotype identification again for verification. Some recombinant strains with correct verification were selected for shake flask fermentation screening.

**Example 6** Construction of recombinant *Aspergillus niger* heterologously expressing aconitase Aco and aconitate isomerase Adi1

1. For the construction of an expression cassette of aconitase gene ATEG_03325, please refer to Example 5.
2. Construction of an expression cassette of aconitate isomerase Adil

The aconitate isomerase Adil gene was artificially synthesized and codon-optimized for expression in *Aspergillus niger.* It was cloned into the pUC57-Kan vector to construct the plasmid puc57-Adi1.

1) Construction of an expression cassette of aconitate isomerase Adil using a PgpdAt promoter

PCR amplification was performed using fuPgpdAt-Adi1-F (5'-ctcatcaatcatcacaacatgctgcaccccatcgacacca-3', SEQ ID NO. 55) and fuAdil-TtrpC-R (5'-tcagtaacgttaagtggatccttaggacaggctacggtcg-3', SEQ ID NO. 56) as a primer pair and using the puc57-Adi1 plasmid as a template. The amplified product was purified and recovered to obtain the Adil gene fragment.

PCR amplification was performed using fuAdi1-TtrpC-F (5'-cgaccgtagectgtectaaggatccacttaacgttactga-3', SEQ ID NO. 57) and fuPgpdAt-Adi1-R (5'-tggtgtcgatggggtgcagcatgttgtgatgattgatgag-3', SEQ ID NO. 58) as a primer pair and using the plasmid pmWM23 as a template. The amplified product was purified and recovered to obtain the PgpdAt-TtrpC fragment.

The above two fragments were subjected to one-step cloning. PCR amplification was performed using PgpdAt-F (5'-ttacactctgggaggatccaggtac-3', SEQ ID NO. 25) and TtrpC-R (5'-attacctctaaacaagtgtac-3', SEQ ID NO. 45) as a primer pair. The amplified product was purified and recovered to obtain the PgpdAt-Adil-TtrpC expression cassette fragment.

2) Construction of an expression cassette of aconitate isomerase Adil using a PgpdAn promoter

PCR amplification was performed using fuPgpdAn-Adi1-F (5'-ccgcttgagcagacatcaccatgctgcaccccatcgacacca t-3', SEQ ID NO. 59) and fuAdil-TtrpC-R (5'-tcagtaacgttaagtggatccttaggacaggctacggtcg-3', SEQ ID NO. 56) as a primer pair and using the puc57-Adi1 plasmid as a template. The amplified product was purified and recovered to obtain the Adi1 gene fragment.

PCR amplification was performed using fuAdi1-TtrpC-F (5'-cgaccgtagcctgtcctaaggatccacttaacgttactga-3', SEQ ID NO. 57) and fuPgpdAn-Adi1-R (5'-tggtgtcgatggggtgcagcatggtgatgtctgctcaagcgg-3', SEQ ID NO. 60) as a primer pair and using the plasmid pAN52-4 as a template. The amplified product was purified and recovered to obtain the PgpdAn-TtrpC fragment. PgpdAn is the glyceraldehyde-3-phosphate dehydrogenase gene promoter from *Aspergillus nidulans,* and TtrpC is the tryptophan synthase terminator from *Aspergillus nidulans.*

The above two fragments were subjected to one-step cloning. PCR amplification was performed using PgpdAn-F (5'-ttgatcgagacctaatacagc-3', SEQ ID NO. 28) and TtrpC-R (5'-attacctctaaacaagtgtac-3', SEQ ID NO. 45) as a primer pair. The amplified product was purified and recovered to obtain the PgpdAn-Adil-TtrpC expression cassette fragment.

3) Construction of an expression cassette of aconitate isomerase Adil using a PglaA promoter

PCR amplification was performed using fuPglaA-Adi1-F (5'-gcatcattacacctcagcaatgctgcaccccatcgacacca-3', SEQ ID NO. 61) and fuAdil-TtrpC-R (5'-tcagtaacgttaagtggatccttaggacaggctacggtcg-3', SEQ ID NO. 56) as a primer pair and using the puc57-Adi1 plasmid as a template. The amplified product was purified and recovered to obtain the Adil gene fragment.

PCR amplification was performed using PglaA-F (5'-ggattgcctgaacattgacattcgg-3', SEQ ID NO. 30) and PglaA-R (5'-tgctgaggtgtaatgatgctggggat-3', SEQ ID NO. 31) as a primer pair and using the genome of *Aspergillus niger* MEFC1501 as a template. The amplified product was purified and recovered to obtain the PglaA promoter fragment.

PCR amplification was performed using fuAdi1-TtrpC-F (5'-cgaccgtagectgtectaaggatccacttaacgttactga-3', SEQ ID NO. 57) and TtrpC-R (5'-attacctctaaacaagtgtac-3', SEQ ID NO. 45) as a primer pair and using the plasmid pAN52-4 as a template. The amplified product was purified and recovered to obtain the TtrpC fragment.

The above three fragments were subjected to one-step cloning. PCR amplification was performed using PglaA-F (5'-ggattgcctgaacattgacattcgg-3', SEQ ID NO. 30) and TtrpC-R (5'-attacctctaaacaagtgtac-3', SEQ ID NO. 45) as a primer pair. The amplified product was purified and recovered to obtain the PglaA-Adil-TtrpC expression cassette fragment.

4) Construction of an expression cassette of aconitate isomerase Adil using a PcadA promoter

PCR amplification was performed using fuPcadA-Adi1-F (5'-cctcttaaattgaccatgaatgctgcaccccatcgacacca-3', SEQ ID NO. 62) and fuAdil-TtrpC-R (5'-tcagtaacgttaagtggatccttaggacaggctacggtcg-3', SEQ ID NO. 56) as a primer pair and using the puc57-Adi1 plasmid as a template. The amplified product was purified and recovered to obtain the Adil gene fragment.

PCR amplification was performed using PcadA-F (5'-ctaccaacagtctcgcggtgaatag-3', SEQ ID NO. 33) and PcadA-R (5'-tcatggtcaatttaagagg-3', SEQ ID NO. 83) as a primer pair and using the genome of *Aspergillus terreus* CICC 40205 as a template. The amplified product was purified and recovered to obtain the PcadA promoter fragment.

PCR amplification was performed using fuAdi1-TtrpC-F (5'-cgaccgtagcctgtcctaaggatccacttaacgttactga-3', SEQ ID NO. 57) and TtrpC-R (5'-attacctctaaacaagtgtac-3', SEQ ID NO. 45) as a primer pair and using the plasmid pAN52-4 as a template. The amplified product was purified and recovered to obtain the TtrpC fragment.

The above three fragments were subjected to one-step cloning. PCR amplification was performed using PcadA-F (5'-ctaccaacagtctcgcggtgaatag-3', SEQ ID NO. 33) and TtrpC-R (5'-attacctctaaacaagtgtac-3', SEQ ID NO. 45) as a primer pair. The amplified product was purified and recovered to obtain the PcadA-Adil-TtrpC expression cassette fragment.

5) Construction of an expression cassette of aconitate isomerase Adil using a PmfsA promoter

PCR amplification was performed using fuPmfsA-Adi1-F (5'-accgacttctcatccatcttcaaaatgctgcaccccatcgacacc a-3', SEQ ID NO. 63) and fuAdil-TtrpC-R (5'-tcagtaacgttaagtggatccttaggacaggctacggtcg-3', SEQ ID NO. 56) as a primer pair and using the puc57-Adi1 plasmid as a template. The amplified product was purified and recovered to obtain the Adil gene fragment.

PCR amplification was performed using PmfsA-F (5'-gtacagtggccatgaaatccaatc-3', SEQ ID NO. 36) and PmfsA-R (5'-tttgaagatggatgagaagtcggt-3', SEQ ID NO. 37) as a primer pair and using the genome of *Aspergillus niger* MEFC1501 as a template. The amplified product was purified and recovered to obtain the PmfsA promoter fragment.

PCR amplification was performed using fuAdi1-TtrpC-F (5'-cgaccgtagcctgtcctaaggatccacttaacgttactga-3', SEQ ID NO. 57) and TtrpC-R (5'-attacctctaaacaagtgtac-3', SEQ ID NO. 45) as a primer pair and using the plasmid pAN52-4 as a template. The amplified product was purified and recovered to obtain the TtrpC fragment.

The above three fragments were subjected to one-step cloning. PCR amplification was performed using PmfsA-F (5'-gtacagtggccatgaaatccaatc-3', SEQ ID NO. 36) and TtrpC-R (5'-attacctctaaacaagtgtac-3', SEQ ID NO. 45) as a primer pair. The amplified product was purified and recovered to obtain the PmfsA-Adil-TtrpC expression cassette fragment.

6) Construction of an expression cassette of aconitate isomerase Adil using a PacoA promoter

PCR amplification was performed using fuPacoA-Adi1-F (5'-cctcgcatagagagcttccatcatgctgcaccccatcgacacc a-3', SEQ ID NO. 64) and fuAdil-TtrpC-R (5'-tcagtaacgttaagtggatccttaggacaggctacggtcg-3', SEQ ID NO. 56) as a primer pair and using the puc57-Adi1 plasmid as a template. The amplified product was purified and recovered to obtain the Adil gene fragment.

PCR amplification was performed using PacoA-F (5'-tggcaccggtccgcggga-3', SEQ ID NO. 39) and PacoA-R (5'-gatggaagctctctatgcgagg-3', SEQ ID NO. 40) as a primer pair and using the genome of *Aspergillus terreus* CICC 40205 as a template. The amplified product was purified and recovered to obtain the PacoA promoter fragment.

PCR amplification was performed using fuAdi1-TtrpC-F (5'-cgaccgtagcctgtcctaaggatccacttaacgttactga-3', SEQ ID NO. 57) and TtrpC-R (5'-attacctctaaacaagtgtac-3', SEQ ID NO. 45) as a primer pair and using the plasmid pAN52-4 as a template. The amplified product was purified and recovered to obtain the TtrpC fragment.

The above three fragments were subjected to one-step cloning. PCR amplification was performed using PacoA-F (5'-tggcaccggtccgcggga-3', SEQ ID NO. 39) and TtrpC-R (5'-attacctctaaacaagtgtac-3', SEQ ID NO. 45) as a primer pair. The amplified product was purified and recovered to obtain the PacoA-Adil-TtrpC expression cassette fragment.

7) Construction of an expression cassette of aconitate isomerase Adil using a PcitA promoter

PCR amplification was performed using fuPcitA-Adi1-F (5'-ctttttagactcttgttggattcaaaatgctgcaccccatcgacacc a-3', SEQ ID NO. 65) and fuAdil-TtrpC-R (5'-tcagtaacgttaagtggatccttaggacaggctacggtcg-3', SEQ ID NO. 56) as a primer pair and using the puc57-Adi1 plasmid as a template. The amplified product was purified and recovered to obtain the Adil gene fragment.

PCR amplification was performed using PcitA-F (5'-caaccaaggaccgcgatg-3', SEQ ID NO. 42) and PcitA-R (5'-ttgaatccaacaagagtctaaaaaag-3', SEQ ID NO. 43) as a primer pair and using the genome of *Aspergillus terreus* CICC 40205 as a template. The amplified product was purified and recovered to obtain the PcitA promoter fragment.

PCR amplification was performed using fuAdi1-TtrpC-F (5'-cgaccgtagcctgtcctaaggatccacttaacgttactga-3', SEQ ID NO. 57) and TtrpC-R (5'-attacctctaaacaagtgtac-3', SEQ ID NO. 45) as a primer pair and using the plasmid pAN52-4 as a template. The amplified product was purified and recovered to obtain the TtrpC fragment.

The above three fragments were subjected to one-step cloning. PCR amplification was performed using PcitA-F (5'-caaccaaggaccgcgatg-3', SEQ ID NO. 42) and TtrpC-R (5'-attacctctaaacaagtgtac-3', SEQ ID NO. 45) as a primer pair. The amplified product was purified and recovered to obtain the PcitA-Adil-TtrpC expression cassette fragment.

8) Construction of an expression cassette of aconitate isomerase Adil using a PicdA promoter

PCR amplification was performed using fuPicdA-Adi1-F (5'-cgcaggccacgcttcactgtcgaaatgctgcaccccatcgacac ca-3', SEQ ID NO. 66) and fuAdil-TtrpC-R (5'-tcagtaacgttaagtggatccttaggacaggctacggtcg-3', SEQ ID NO. 56) as a primer pair and using the puc57-Adi1 plasmid as a template. The amplified product was purified and recovered to obtain the Adi1

gene fragment.

PCR amplification was performed using PicdA-F (5'-ctttaacgttgcagatacagggatgcg-3', SEQ ID NO. 47) and PicdA-R (5'-ttcgacagtgaagcgtggcctgcg-3', SEQ ID NO. 48) as a primer pair and using the genome of *Aspergillus niger* MEFC1501 as a template. The amplified product was purified and recovered to obtain the PicdA promoter fragment.

PCR amplification was performed using fuAdi1-TtrpC-F (5'-cgaccgtagcctgtcctaaggatccacttaacgttactga-3', SEQ ID NO. 57) and TtrpC-R (5'-attacctctaaacaagtgtac-3', SEQ ID NO. 45) as a primer pair and using the plasmid pAN52-4 as a template. The amplified product was purified and recovered to obtain the TtrpC fragment.

The above three fragments were subjected to one-step cloning. PCR amplification was performed using PicdA-F (5'-ctttaacgttgcagatacagggatgcg-3', SEQ ID NO. 47) and TtrpC-R (5'-attacctctaaacaagtgtac-3', SEQ ID NO. 45) as a primer pair. The amplified product was purified and recovered to obtain the PicdA-Adi1-TtrpC expression cassette fragment.

9) Construction of an expression cassette of aconitate isomerase Adil using a PgasA promoter

PCR amplification was performed using fuPgasA-Adi1-F (5'-gtcttctttcgttcacctcctcacatgctgcaccccatcgacacca-3', SEQ ID NO. 67) and fuAdil-TtrpC-R (5'-tcagtaacgttaagtggatccttaggacaggctacggtcg-3', SEQ ID NO. 56) as a primer pair and using the puc57-Adi1 plasmid as a template. The amplified product was purified and recovered to obtain the Adil gene fragment.

PCR amplification was performed using PgasA-F (5'-ctgctctctctctgctctctttct-3', SEQ ID NO. 50) and PgasA-R (5'-gtgaggaggtgaacgaaagaagac-3', SEQ ID NO. 51) as a primer pair and using the genome of *Aspergillus niger* MEFC1501 as a template. The amplified product was purified and recovered to obtain the PgasA promoter fragment.

PCR amplification was performed using fuAdi1-TtrpC-F (5'-cgaccgtagcctgtcctaaggatccacttaacgttactga-3', SEQ ID NO. 57) and TtrpC-R (5'-attacctctaaacaagtgtac-3', SEQ ID NO. 45) as a primer pair and using the plasmid pAN52-4 as a template. The amplified product was purified and recovered to obtain the TtrpC fragment.

The above three fragments were subjected to one-step cloning. PCR amplification was performed using PgasA-F (5'-ctgctctctctctgctctctttct-3', SEQ ID NO. 50) and TtrpC-R (5'-attacctctaaacaagtgtac-3', SEQ ID NO. 45) as a primer pair. The amplified product was purified and recovered to obtain the PgasA-Adil-TtrpC expression cassette fragment.

3. For the construction of hygromycin B selection marker gene hph expression cassette, please refer to Example 5.

4. Co-transformation of *Aspergillus niger* protoplasts with aconitase gene ATEG_03325 expression cassette and aconitate isomerase Adil expression cassette to obtain recombinant *Aspergillus niger* strains

1) For the preparation of *Aspergillus niger* protoplasts, please refer to Example 5.

2) Co-transformation of *Aspergillus niger* protoplasts with aconitase gene ATEG_03325 expression cassette, aconitate isomerase Adil expression cassette and resistance gene *hph*

Approximately 5 μg of aconitase gene ATEG_03325 expression cassette DNA, approximately 5 μg of aconitate isomerase Adil expression cassette DNA, and 1 μg of hygromycin B-resistant hph gene expression cassette DNA were added to the above protoplast suspension, followed by the addition of 50 μL of PSTC (40% PEG4000, 50 mM Tris-HCl pH 8.0, 50 mM $CaCl_2$). The mixture was gently mixed and placed on ice for 30 min. 1.5 mL of PSTC was added and mixed uniformly, and then left at room temperature for 20 min. Then it was mixed with the upper layer agar and poured onto the regeneration selection medium plates PDA-SH, followed by incubation at 30°C in the dark for 3 to 4 days.

Transformants were transferred from the plates to the selection plates PDA-H (4 g of potato dextrose agar medium was weighed and dissolved in 100 mL of distilled water, and then sterilized and cooled to approximately 55°C, hygromycin was added to a final concentration of 100 μg/mL to prepare the plates); the mixture was incubated at 30°C for 3 to 5 days to obtain transformants.

In a preparation process of the above recombinant *Aspergillus niger* strains containing ATEG_03325 expression cassettes and Adil expression cassettes driven by various promoters, the ATEG_03325 expression cassette and the hygromycin B resistance hph gene expression cassette can also be linked into a single expression cassette, or the Adil expression cassette and the hygromycin B resistance hph gene expression cassette can also be linked into a single expression cassette, followed by transformation of protoplasts. This does not affect the screening of positive transformants and the identification of genotypes.

3) Verification of genotype of recombinant *Aspergillus niger* strains

The recombinant *Aspergillus niger* transformants obtained above were picked and inoculated onto PDA-H plates to culture spores. Then, the spores were respectively inoculated into liquid medium for culture, and mycelia were collected to extract the genome. PCR was performed using PgpdAt-F743 (5'-ttacactctgggaggatccaggtact-3', SEQ ID NO. 54) and

TtrpC-R (5'-attacctctaaacaagtgtac-3', SEQ ID NO. 45) as a primer pair to amplify the PgpdAt-ATEG_03325-TtrpC element and/or PgpdAt-Adil-TtrpC inserted into the genome; PCR was performed using PglaA-F (5'-ggattgcctgaacattgacattcgg-3', SEQ ID NO. 30) and TtrpC-R (5'-attacctctaaacaagtgtac-3', SEQ ID NO. 45) as a primer pair to amplify the PglaA-ATEG_03325-TtrpC element and/or PglaA-Adil-TtrpC inserted into the genome; PCR was performed using PgpdAn-F (5'-ttgatcgagacctaatacagc-3', SEQ ID NO. 28) and TtrpC-R (5'-attacctctaaacaagtgtac-3', SEQ ID NO. 45) as a primer pair to amplify the PgpdAn-ATEG_03325-TtrpC element and/or PgpdAn-Adil-TtrpC inserted into the genome; PCR was performed using PcadA-F (5'-ctaccaacagtctcgcggtgaatag-3', SEQ ID NO. 33) and TtrpC-R (5'-attacctctaaacaagtgtac-3', SEQ ID NO. 45) as a primer pair to amplify the PcadA-ATEG_03325-TtrpC element and/or PcadA-Adil-TtrpC inserted into the genome; PCR was performed using PmfsA-F (5'-gtacagtggccatgaaatccaatc-3', SEQ ID NO. 36) and TtrpC-R (5'-attacctctaaacaagtgtac-3', SEQ ID NO. 45) as a primer pair to amplify the PmfsA-ATEG_03325-TtrpC element and/or PmfsA-Adil-TtrpC inserted into the genome; PCR was performed using PacoA-F (5'-tggcaccggtccgcggga-3', SEQ ID NO. 39) and TtrpC-R (5'-attacctctaaacaagtgtac-3', SEQ ID NO. 45) as a primer pair to amplify the PacoA-ATEG_03325-TtrpC element and/or PacoA-Adil-TtrpC inserted into the genome; PCR was performed using PcitA-F (5'-caaccaaggaccgcg atg-3', SEQ ID NO. 42) and TtrpC-R (5'-attacctctaaacaagtgtac-3', SEQ ID NO. 45) as a primer pair to amplify the PcitA-ATEG_03325-TtrpC element and/or PcitA-Adil-TtrpC inserted into the genome; PCR was performed using PicdA-F (5'-ctttaacgttgcagatacagggatgcg-3', SEQ ID NO. 47) and TtrpC-R (5'-attacctctaaacaagtgtac-3', SEQ ID NO. 45) as a primer pair to amplify the PicdA-ATEG_03325-TtrpC element and/or PicdA-Adi1-TtrpC inserted into the genome; and PCR was performed using PgasA-F (5'-ctgctctctctctgctctctttct-3', SEQ ID NO. 50) and TtrpC-R (5'-attacctctaaacaagtgtac-3', SEQ ID NO. 45) as a primer pair to amplify the PgasA-ATEG_03325-TtrpC element and/or PgasA-Adil-TtrpC inserted into the genome.

The PtrpC-hph-TtrpC fragment inserted into the recombinant strains was amplified using hph-F (5'- ttcgggat cgcaagcgtaaag -3', SEQ ID NO.52) and hph-R (5'-caattatctttgcgaacccagg-3', SEQ ID NO.53) as a primer pair, and the PCR products were analyzed by 0.8% agarose gel electrophoresis. The genomic DNA template of the starting strains *Aspergillus niger* MEFC1501 was used as a negative control.

Transformants with correct genotype verification were transferred to PDA-H plates for subculture, with three passages in total. Then, the spores were collected respectively and appropriately diluted with physiological saline, and 100 μL of the diluted spore suspension was spread on PDA-H plates to allow the growth of independent single colonies, which was referred to as single-spore isolation. Spores were taken from the single colonies for single-spore isolation again, and a total of four rounds of single-spore isolation and subculture were performed. The recombinant strains after single-spore isolation were subjected to genotype identification again for verification. Some recombinant strains with correct verification were selected for shake flask fermentation screening.

**Example 7** Construction of recombinant *Aspergillus niger* strains using pyrithiamine ptrA as a selection marker

Example 6 was repeated, and the difference from Example 6 lies in that in this example, the selection marker gene used in the recombinant *Aspergillus niger* strains was the pyrithiamine selection marker *ptrA* gene. The specific method is described as follows:

1. For the construction of expression cassettes of aconitase ATEG_03325 with different promoters, please refer to Example 5.
2. For the construction of expression cassettes of aconitate isomerase Adil with different promoters, please refer to Example 6.
3. Construction of recombinant *Aspergillus niger* strains using pyrithiamine resistance gene *ptrA* as a selection marker

1) Construction of pyrithiamine resistance gene *ptrA* expression cassette

PCR amplification was performed using ptrA-F (5'-gggcaattgattacgggatc-3', SEQ ID NO.68) and ptrA-R (5'-atggggtgacgatgagccgc-3', SEQ ID NO.69) as a primer pair and using the plasmid pmWM23 as a template. The amplified product was purified and recovered to obtain the pyrithiamine resistance gene *ptrA* expression cassette.

Protoplasts of *Aspergillus niger* MEFC1501 were prepared with reference to the preparation method in Example 5. Approximately 5 μg of aconitase gene ATEG_03325 expression cassette DNA, approximately 5 μg of aconitate isomerase Adil expression cassette DNA, and 1 μg of selection marker ptrA fragment were added to the protoplast suspension, followed by the addition of 50 μL of PSTC (40% PEG4000, 50 mM Tris-HCl pH8.0, 50 mM $CaCl_2$). The mixture was gently mixed and placed on ice for 30 min. 1.5 mL of PSTC was added and mixed uniformly, and then left at room temperature for 20 min. Then it was mixed with the upper layer agar and poured onto the regeneration selection medium plates CD-SPt, followed by incubation at 33°C in the dark for 3 to 4 days. Transformants were transferred from the plates to the selection plates CD-Pt and incubated at 33°C for 3 to 5 days to obtain transformants.

The prepared recombinant *Aspergillus niger* transformants were picked and inoculated onto CD-Pt plates to

culture spores. Then, the spores were respectively inoculated into PDB liquid medium for culture, and mycelia were collected to extract the genome. The PmfsA-ATEG_03325-TtrpC element or PgpdAn-Adil-TtrpC inserted into the genome was amplified. The ptrA fragment inserted into the recombinant strains was amplified using ptrA-F (5'-gggcaattgattac gggatc-3', SEQ ID NO.68) and ptrA-R (5'-atggggtgacgatgagccgc-3', SEQ ID NO.69) as a primer pair, and the PCR product was analyzed by 0.8% agarose gel electrophoresis.

The genomic DNA template of the starting strains *Aspergillus niger* MEFC1501 was used as a negative control.

For the verification, isolation and purification of positive transformants, please refer to Example 5 and Example 6.

**Example 8** Fermentation of recombinant *Aspergillus niger* for producing *trans*-Aconitic acid

1. Shake flask screening of recombinant *Aspergillus niger* strains for producing *trans*-Aconitic acid

The stable recombinant *Aspergillus niger* strains obtained after subculture of the recombinant strains from Examples 5, 6, and 7 were respectively inoculated into *Aspergillus niger* sporulation medium and incubated at 32°C for 6 days to obtain mature spores. Then, the mature spores of each strain were respectively inoculated into *trans*-Aconitic acid fermentation medium, with three parallel flasks for each strain. Fermentation was performed at 34°C under 220 rpm. Mycelia in the fermentation broth were removed by filtration, and the fermentation supernatant was appropriately diluted for high-performance liquid chromatography (HPLC) analysis.

2. Analysis of content and purity of *trans*-Aconitic acid in fermentation broth

The diluted fermentation supernatants of the recombinant strains and the starting strains *Aspergillus niger* MEFC1501 were subjected to high-performance liquid chromatography analysis. Standard curves were prepared using standard samples of *trans*-Aconitic acid at different concentrations to analyze and compare the content and purity of *trans*-Aconitic acid in the fermentation broth. The chromatographic conditions are as follows: chromatographic column: Aminex HPX-87H Organic Acid Analysis Column, 300 mm × 7.8 mm (Bio-rad, Cat No.1250140); mobile phase: 5 mmol/L sulfuric acid; flow rate: 0.5 mL/min; column temperature: 30°C; detection temperature: 30°C; UV detector (210 nm).

The results obtained are shown in Figs. 4 and 5.

The results show that at the shake flask fermentation level, the contents of cis-Aconitic acid and *trans*-Aconitic acid in the fermentation broth of the recombinant strains are significantly higher than those of the wild-type strains. The effects of the two isomerases TbrA and Adil are not significantly different, but the gene expression driven by different promoters shows significant differences, and the highest content of *trans*-Aconitic acid can reach 51.11 g/L. There are no significant differences between different terminators such as TtrpC or Tpgk, and there are no significant differences between different selection markers such as hygromycin or pyrithiamine. Similar results are obtained with different transformation methods, such as co-transformation with the selection marker or constructing the expression cassette of the target gene and the expression cassette of the selection marker together for transformation.

**Example 9** Characterization of mitochondrial localization effects of signal peptides MTSacoAt, MTScitAt, and MTSicdAn

1) Prediction of mitochondrial targeting signal peptides of *acoA* and *citA* genes from *Aspergillus terreus* and *icdA* gene from *Aspergillus niger*

The MITOPROT 11 program (M.G. Claros et al., 1996) was used to predict the amino acid sequences of *acoAt* (Genbank accession number: EAU36599.1), *citAt* (Genbank accession number: EAU31163.1), and *icdAn* (Genbank accession number: BAA19073.1) genes respectively. The probabilities of their localization to mitochondria are 90.54%, 95.91%, and 99.55% respectively. Therefore, it is confirmed that the signal peptides contained therein are likely targeting sequences targeted to mitochondria. The finally obtained three mitochondrial targeting signal peptide sequences are shown as follows.

MTSacoAt (SEQ ID NO.7):
MISTRLARMGALAPKSRLLLGTRGLATVADSP
MTScitAt (SEQ ID NO.8):
MAASLRLGTSALRSTTLAGKPVVQSAAFNGLRCY
MTSicdAn (SEQ ID NO.9):

MSSVRFSSALARRSFAVASPPLSAPLSSSARRFLSSSSSISSSSSSVSTRSPRSLTSAS SLLSSRTASARWTGLSSLNLTQSRT

2) Construction of fluorescent protein gene expression cassette fused with mitochondrial signal peptide

Although three mitochondrial targeting signal peptides are obtained through prediction, their mitochondrial localization ability still needs to be verified through experiments. The inventors use the fluorescent protein gene as the target protein to be localized for mitochondrial localization tests, which facilitates verification through fluorescence detection.

KU80: this site is a DNA repair-related protein, with a Genbank accession number of EAU32303.1.

StayGold: a high-green fluorescent protein with a Genbank accession number of LC601652. The coding nucleotide fragment of StayGold optimized and synthesized according to the codon preference of Aspergillus is shown as SEQ ID NO. 139.

Primers are designed and synthesized according to the gene sequence information, and the specific primer sequences are shown below.

PCR amplification was performed using plasmid pXH106 (recorded in CN110527637A, entitled *"Aspergillus terreus* Strains for Aconitic Acid and Construction Method and Applications Thereof", and publicly available from Qingdao Institute of Bioenergy and Bioprocess Technology, Chinese Academy of Sciences) as a template and using DNA polymerase (Nanjing Vazyme Medical Technology Co., Ltd., 2×Phanta Max Master Mix). Primers pXH106-X-F/pXH106-X-R can be used to amplify a plasmid backbone fragment of approximately 6.6 kb, which includes the downstream homology arm D-ku80 of the *ku80* gene, the pUC57 plasmid backbone fragment (containing the ampicillin resistance gene *amp*), the upstream homology arm U-ku80 of the *ku80* gene, and the pXH106 plasmid backbone fragment of the orotidine-5'-phosphate decarboxylase gene *pyrG*.

PCR amplification was sequentially performed using the genomic DNA of *Aspergillus terreus* At-△ku80 (*Aspergillus terreus* At-*Δku80* is an engineered strain constructed by knocking out the *ku80* gene in *Aspergillus terreus* CICC40205, and for details, please refer to CN104894165A entitled "Method and Application for Improving Application Efficiency of Gene Targeting Technology in *Aspergillus terreus*") as a template and using primers PgpdAt-F (PyrG)/PgpdAt-R, PgpdAt-F (PyrG)/PgpdAt-R (MTScitAt), PgpdAt-F (PyrG)/PgpdAt-R (MTSacoAt), and PgpdAt-F (PyrG)/PgpdAt-R (MTSicdAt) to obtain the constitutive promoter PgpdAt of *Aspergillus terreus* containing the downstream partial sequence, i.e., PgpdAt, PgpdAt (MTScitAt), PgpdAt (MTSacoAt), and PgpdAt (MTSicdAn) fragments.

The mitochondrial targeting signal peptides MTS were sequentially amplified using the genomic DNA of *Aspergillus terreus* At-△*ku80* as a template and using primers MTScitAt-F/MTScitAt-R and MTSacoAt/MTSacoAt-R, to obtain MTScitAt and MTSacoAt fragments. The mitochondrial targeting signal peptides MTS were amplified using the genomic DNA of *Aspergillus niger* MEFC1501 (preserved in the China General Microbiological Culture Collection Center, the strain preservation number: CGMCC NO.40614, preservation time: April 27, 2023, and preservation location: Institute of Microbiology, Chinese Academy of Sciences, No. 3, Courtyard 1, Beichen West Road, Chaoyang District, Beijing) as a template and using primers MTSicdAn-F/MTSicdAn-R, to obtain the MTSicdAt fragment.

PCR amplification was sequentially performed using pUC-staygold-Kan (a plasmid carrying the StayGold gene synthesized by Genewiz, which can be synthesized by the public according to the nucleotide sequence of the StayGold gene) as a template and using StayGold (PgpdAt)-F/StayGold-R (Ku80-D), StayGold (MTScitAt)-F/StayGold-R (Ku80-D), StayGold (MTSacoAt)-F/StayGold-R (Ku80-D), and StayGold (MTSicdAn)-F/StayGold-R (Ku80-D), to obtain green fluorescent protein StayGold fragments containing the upstream partial sequence, i.e., StayGold, StayGold (MTScitAt), StayGold (MTSacoAt), and StayGold (MTSicdAn) fragments.

All the PCR products were detected by 0.8% agarose gel electrophoresis, followed by gel cutting, recovery, and purification. The "pXH106 plasmid backbone fragment, PgpdAt fragment, and StayGold fragment", "pXH106 plasmid backbone fragment, PgpdAt (MTScitAt) fragment, MTScitAt, and StayGold (MTScitAt) fragment", "pXH106 plasmid backbone fragment, PgpdAt (MTSacoAt) fragment, MTSacoAt, and StayGold (MTSacoAt) fragment", and "pXH106 plasmid backbone fragment, PgpdAt (MTSicdAt) fragment, MTSicdAt, and StayGold (MTSicdAt) fragment" were fused respectively using the one-step cloning kit (purchased from Nanjing Vazyme Medical Technology Co., Ltd., ClonExpress Ultra One Step Cloning Kit), to obtain plasmids pXH106-PgdpAt-StayGold, pXH106-PgdpAt-MTScitAt-StayGold, pXH106-PgdpAt-MTSacoAt-StayGold, and pXH106-PgdpAt-MTSicdAt-StayGold. Through a series of steps such as *Escherichia coli* transformation, expansion culture, and plasmid extraction, plasmids with a higher concentration were obtained. Amplification was performed using D-ku80-F/U-ku80-R as the primer, to obtain gene targeting elements ku80::PgpdAt-staygold-pyrG, ku80::PgpdAt-MTScitAt-staygold-pyrG, ku80::PgpdAt-MTSacoAt-staygold-pyrG, and ku80::PgpdAt-MTSicdAn-staygold-pyrG with a size of approximately 5.5 kb. The gene targeting elements constructed above can be used to construct engineered strains that expresses a fluorescent protein gene targeted to mitochondria by the PgpdAt promoter at the *ku80* site.

3) Construction of *Aspergillus terreus* engineered strains heterologously expressing fluorescent protein targeted to mitochondria

At-Δ*ku80*-Δ*pyrG* is an uracil auxotrophic engineered strains obtained by knocking out the *pyrG* gene on the basis of the At-Δ*ku80* strains (for details, please refer to Patent ZL201510275491.5 entitled "Method and Application for Improving Application Efficiency of Gene Targeting Technology in *Aspergillus terreus*"). The spores of the engineered strains At-Δ*ku80*-Δ*pyrG* were inoculated into 55 mL of IPM-FU (IPM which is added with 5 mM 5-fluoroorotic acid and 10 mM uridine) liquid medium (the preparation method of IPM is as follows: 100 g/L glucose, 2 g/L $NH_4NO_3$, 0.2 g/L $(NH_4)_2HPO_4$, 20 mg/L $FeSO_4$, 0.4 g/L $MgSO_4$, 40 mg/L $ZnSO_4$, and 40 mg/L $CuSO_4$ were adjusted with sulfuric acid to a pH of 3.5, and then were sterilized at 115°C for 30 min to obtain the product), such that the spore concentration was approximately $10^7$ spores/mL and the spores were incubated at 220 rpm and 37°C for 36 h. The grown mycelia were collected by filtration through a sterile single-layer 500-mesh nylon cloth, rinsed for three times with a sterile 0.6 M $MgSO_4$ solution, and pressed dry and then placed in a 50 mL sterile Erlenmeyer flask. An appropriate amount of enzymatic hydrolysis solution was added according to the weight of the mycelia (10 mL of enzymatic hydrolysis solution was added per 1 g of mycelia), and was treated at 30°C under 130 rpm for 1 to 3 h. The above enzymatic hydrolysis mixture was filtered through 300-mesh nylon cloth or lens tissue, and the filtrate was collected. The filtrate was centrifuged at 4°C under 4000 rpm to collect protoplasts. Protoplasts were washed once with pre-cooled 1.2 M sorbitol solution, and then washed once with pre-cooled STC (STC composition: 1.2 M sorbitol, 50 mM Tris-HCl (pH=8.0), 50 mM $CaCl_2$). Finally, the protoplasts were resuspended in pre-cooled STC, and the concentration of protoplasts was adjusted to $5\times10^7$ protoplasts/mL using STC to obtain the protoplast suspension. 10 μL of DNA fragments (approximately 5 μg) of the targeting elements ku80::PgpdAt-staygold-pyrG, ku80::PgpdAt-MTScitAt-staygold-pyrG, ku80::PgpdAt-MTSacoAt-staygold-pyrG, and ku80::PgpdAt-MTSicdAn-staygold-pyrG were added to 150 μL of this protoplast suspension. Then 50 μL of ice-cold PSTC was added (PSTC composition: 40% PEG4000, 1.2 M sorbitol, 50 mM Tris-HCl (pH=8.0), 50 mM $CaCl_2$). The mixture was gently mixed and placed on ice for 30 min. 1 mL of room-temperature PSTC was added and mixed uniformly, and then left at room temperature for 20 min. Then it was mixed with 30 mL of PDBS top agar and poured onto 10 PDAS plates for regeneration selection culture, and incubated at 30°C in the dark for 5 to 7 days.

Transformants with good growth status were picked from the transformation selection plates and transferred to PDA plates, and then incubated at 30°C for 5 days for subculture and purification. The spores of the stably subcultured transformants were inoculated into IPM liquid medium, and incubated at 30°C under 200 rpm for 48 h; the mycelia were collected to extract genomic DNA, and PCR verification was performed using primers YZ-ku80-F/YZ-ku80-R and using the genome of the At-*Δku80-ΔpyrG* strains as a control. Positive transformants can amplify a band of approximately 3.0 to 3.5 kb, while the control can amplify a band of approximately 2.0 kb. Five positive transformants were selected for single-spore isolation and purification, three single spores were verified for each transformant, and genomic PCR verification was performed again using primers YZ-ku80-F/YZ-ku80-R. The results are shown in Fig. 6. Homozygous transformants integrating green fluorescent protein StayGold elements mediated by different mitochondrial targeting signal peptides at the *ku80* site were successfully obtained, i.e., genetic engineered strains used to evaluate the mitochondrial localization effect of signal peptides are labeled as At-Δ*ku80*::PgpdAt-staygold, At-Δ*ku80*::PgpdAt-MTScitAt-staygold, At-*Δku80*::PgpdAt-MTSacoAt-staygold, and At-Δ*ku80*::PgpdAt-MTSicdAt-staygold, respectively.

4) Evaluation of mitochondrial localization effect of signal peptides by fluorescent co-localization

Positive transformant strains of At-Δ*ku80*::PgpdAt-MTScitAt-staygold, At-Δ*ku80*::PgpdAt-MTSacoAt-staygold, and At-Δ*ku80*::PgpdAt-MTSicdAn-staygold were selected respectively, and the positive transformant strains were respectively inoculated into IPM medium respectively using At-Δku80::PgpdAt-staygold as the control strains, with an inoculum size of approximately $5\times10^5$ spores/mL as the final concentration, and the mixture was incubated at 37°C under 220 rpm for 24 h. 1 mL of bacterial solution was taken and washed twice with sterile water, the bacterial cells were collected by centrifugation, the supernatant was removed, and the cells were resuspended with 300 nM MitoTracker® Red CM-H2XRos reagent (Invitrogen, Cat. no. M7513) preheated at 37°C, and the cells were then incubated at 37°C in the dark for 30 min. After staining, bacterial cells were collected by centrifugation, and then resuspended with fresh culture medium preheated at 37°C. The mycelial fluorescence co-localization results were observed under a confocal microscope (FluoView FV1000).

The mitochondrial localization results are shown in Fig. 7. In Fig. 7, the wild-type strains (WT) without the introduction of StayGold fluorescent protein is used as a blank control, and the StayGold fluorescent protein without an MTS sequence is expressed in the cytoplasm (cStayGold) as a negative control. The mStayGOLD fused with the n-terminus MTS sequence of *citAt, acoAt, and icdAn* genes is expressed in mitochondria as an experimental group. Meanwhile, to show the locations of mitochondria, the mycelia are stained with mitoTracker Red. By observing the results in Fig. 7, it can be seen that the At-Δ*ku80*::PgpdAt-staygold strains without the signal peptide sequence causes the entire mycelium to be filled with green fluorescence. In contrast, when any one of the signal peptides MTScitAt, MTSacoAt, and

MTSicdAn is combined with Staygold, the green fluorescence shows a punctate distribution in the mycelium. At the same time, when MitoTracker, a reagent specifically used for mitochondrial staining, is used for co-localization with Staygold, it is found that the green fluorescence excited by the At-Δ*ku80*::*PgpdAt*-MTScitAt-staygold, At-Δ*ku80*::*PgpdAt*-MTSacoAt-staygold, and At-Δkuc80::PgpdAt-MTSicdAn-staygold strains fused with signal peptides highly overlaps with the location of mitochondria stained by the MitoTracker reagent. Based on the above results, it can be concluded that three signal peptides MTScitAt, MTSacoAt, and MTSicdAn that can be targeted to mitochondria are successfully obtained.

The inventors obtained three signal peptides with mitochondrial targeting function. However, in a process of constructing *trans*-Aconitic acid-producing strains, it is also necessary to determine whether the enzymes related to the production of *trans*-Aconitic acid can be successfully introduced, and whether the successfully introduced enzymes related to *trans*-Aconitic acid can function normally. Therefore, the inventors continued to conduct the construction and verification of recombinant *Aspergillus terreus* strains with mitochondrially targeted aconitate isomerase.

**Example 10** Construction of recombinant *Aspergillus terreus* strains with mitochondrially targeted aconitate isomerase

1) Construction of targeting elements for knocking out *cadA* and overexpressing mitochondrially targeted aconitate isomerase *adi1*/*tbrA* genes

The amino acid sequence of aconitate isomerase Adil from *Ustilago maydis* (as shown as SEQ ID NO. 2, and for details, please refer to CN 112011469A entitled "Recombinant *Aspergillus terreus* Strains with High Yield of *trans*-Aconitic Acid and Construction Method and Applications Thereof'):

The amino acid sequence of aconitate isomerase TbrA from *Bacillus thuringiensis* (as shown as SEQ ID NO. 1, and for details, please refer to CN 112029671A entitled "Recombinant *Aspergillus terreus* Strains with High Yield of *trans*-Aconitic Acid and Preparation Method and Applications Thereof'):

Primers are designed and synthesized according to the gene sequence information, and the specific primer sequences are shown below.

Amplification can be performed using the genomic DNA of *Aspergillus terreus* At-△*ku80* (*Aspergillus terreus* At-Δ*ku80* is an engineered strain generated by knocking out the *ku80* gene in *Aspergillus terreus* CICC40205, and for details, please refer to Patent CN104894I6SA entitled "Method and Application for Improving Application Efficiency of Gene Targeting Technology in *Aspergillus terreus*") as a template and using primers U-cadA-F/U-cadA-R (MTScitAt) and U-cadA-F/U-cadA-R (MTSacoAt), to obtain upstream homology arm U-cadA (MTScitAt) and U-cadA (MTSacoAt) fragments of the *cadA* gene with a size of approximately 1.2 kb, and amplification can be performed using primers D-cadA-F (pyrG)/D-cadA-R, to obtain the downstream homology arm D-cadA fragment of the *cadA* gene with a size of approximately 1.3 kb. The nucleotide sequence of U-cadA is shown as SEQ ID NO. 137, and the nucleotide sequence of D-cadA is shown as SEQ ID NO. 138.

The mitochondrial targeting signal peptides MTS were amplified sequentially using the genomic DNA of *Aspergillus terreus* At-*△ku80* as a template and using primers MTScitAt-F/MTScitAt-R and MTSacoAt/MTSacoAt-R, to obtain MTScitAt and MTSacoAt fragments.

Aconitate isomerase adil was amplified sequentially using the plasmid containing the synthesized *adil* gene as a template and using primers adil-F (MTScitAt)/adi1-R and adil-F (MTSacoAt)/adi1-R, to obtain adil (MTScitAt) and adil (MTSacoAt) fragments. Aconitate isomerase tbrA was amplified sequentially using the plasmid containing the synthesized *tbrA* gene as a template and using primers tbrA-F (MTScitAt)/tbrA-R and tbrA-F (MTSacoAt)/tbrA-R, to obtain tbrA (MTScitAt) and tbrA (MTSacoAt) fragments.

The terminator TtrpC was amplified sequentially using plasmid pXH2-1 as a template and using primers TtrpC-F (adi1)/TtrpC-R and TtrpC-F (tbrA)/TtrpC-R, to obtain TtrpC (adil) and TtrpC (tbrA) fragments.

The nucleotide sequence of TtrpC is shown as SEQ ID NO. 19.

The orotidine-5'-decarboxylase gene pyrG was amplified using plasmid pXH-106 as a template and using primers pyrG-F (TtrpC)/pyrG-R, to obtain the pyrG fragment.

The nucleotide sequence of PyrG is shown as SEQ ID NO. 20.

All the PCR products were detected by 0.8% agarose gel electrophoresis, followed by gel cutting, recovery, and purification. The "U-cadA (MTScitAt) fragment, MTScitAt fragment, adil (MTScitAt) fragment, TtrpC (adil) fragment, pyrG fragment, and D-cadA fragment", the "U-cadA (MTScitAt) fragment, MTScitAt fragment, tbrA (MTScitAt) fragment, TtrpC (tbrA) fragment, pyrG fragment, and D-cadA fragment", the "U-cadA (MTSacoAt) fragment, MTSacoAt fragment, adil (MTSacoAt) fragment, TtrpC (adil) fragment, pyrG fragment, and D-cadA fragment", and the "U-cadA (MTSacoAt) fragment, MTSacoAt fragment, tbrA (MTSacoAt) fragment, TtrpC (tbrA) fragment, pyrG fragment, and D-cadA fragment" were fused by fusion PCR. Amplification was performed using the product of this fusion PCR as a template and using primers C-cadA-F/C-cadA-R, to obtain Δ*cadA*::MTScitAt-adi1, Δ*cadA*::MTScitAt-tbrA, Δ*cadA*::MTSacoAt-adi1, and Δ*cadA*::MTSacoAt-tbrA gene targeting elements with a size of approximately 6.1 kb, which can be used for site-specific

overexpression of the mitochondrially targeted aconitate isomerase *adi1*/*tbrA* genes driven by the P*cadA* promoter at the *cadA* site.

2) Construction of *Aspergillus terreus* engineered strains heterologously expressing mitochondrially targeted aconitate isomerase adi1/tbrA at the *cadA* site

For details of the specific operation of transforming genes into the strains, please refer to "3) Construction of *Aspergillus terreus* engineered strains heterologously expressing fluorescent protein targeted to mitochondria" in Example 9.

Transformants with good growth status were picked from the transformation selection plates and transferred to PDA plates, and then incubated at 30°C for 5 days for subculture and purification. The spores of the stably subcultured transformants were inoculated into IPM liquid medium, and incubated at 30°C under 200 rpm for 48 h; the mycelia were collected to extract genomic DNA, and PCR verification was performed using primers C-cadA-F/C-cadA-R. At the same time, the genome of the At-Δ*ku80*-Δ*pyrG* strain was used as a control. Positive transformants can amplify a band of approximately 6.2 kb, while the control can amplify a band of approximately 4.5 kb. Five positive transformants were selected for single-spore isolation and purification, three single spores were verified for each transformant, and genomic PCR verification was performed again using primers C-cadA-F/C-cadA-R. The results are shown in Fig. 8. Homozygous transformants heterologously expressing mitochondrially targeted aconitate isomerase *adi1*/*tbrA* at the *cadA* site were successfully obtained, i.e., genetic engineered strains with a higher yield of *trans-Aconitic* acid, which are labeled as At-Δ*cadA*::MTScitAt-*adi1, At-ΔcadA*::MTScitAt-*tbrA,* At-Δ*cadA*::MTSacoAt-*adi1,* and At-Δ*cadA*::MTSacoAt-*tbrA* respectively.

3) Shake flask analysis of *trans*-Aconitic acid production by *Aspergillus terreus* engineered strains overexpressing mitochondrially targeted aconitate isomerase *adi1*/*tbrA*

Three positive transformant strains of At-Δ*cadA*::MTScitAt-*adi1, At-ΔcadA*::MTScitAt-*tbrA,* At-Δ*cadA*::MTSacoAt-*adi1,* and At-Δ*cadA*::MTSacoAt-*tbrA* were selected respectively, and At-Δ*cadA* was used as a control strain, then they were inoculated into PDA medium respectively, and were incubated at 30°C for 7 days to obtain mature spores. The spores on the plates were inoculated into IPM medium (55 mL of medium in a 500 mL Erlenmeyer flask) respectively, with an inoculum size of approximately $5\times10^5$ spores/mL as the final concentration. Three shake flasks were set as parallels for each transformant, and fermentation was performed at 37°C under 220 rpm for 72 h. The supernatant was filtered through a 0.45 μm filter, and was diluted appropriately, and the content of organic acid was detected by high-performance liquid chromatography (HPLC). The chromatographic column is Bio-rad Aminex HPX-87H, 300 mm × 7.8 mm, the mobile phase is 5 mM sulfuric acid, the flow rate is 0.5 mL/min, the column temperature is 55°C, the detection temperature is 35°C, and the wavelength of the ultraviolet detector is 210 nm.

The results of fermentation yield are shown in Fig. 9. In Fig. 9, the left side shows the yield of aconitic acid, and the right side shows the proportion of trans-Aconitic acid in the aconitic acid. The *trans*-Aconitic acid yield of strain At-Δ*cadA*::MTSacoAt-*adi1* was 37.67 g/L, the conversion rate of the *trans*-Aconitic acid was 88.63%; the *trans*-Aconitic acid yield of *At-ΔcadA*::MTScitAt-*adi1* was 33.72 g/L, the conversion rate of the trans-Aconitic acid was 88.77%; the trans-Aconitic acid yield of At-Δ*cadA*::MTSacoAt-*tbrA* was 11.18 g/L, the conversion rate of *trans*-Aconitic acid was 68.43%; trans-Aconitic acid yield of At-Δ*cadA*::MTScitAt-*tbrA* was 7.16 g/L, the conversion rate of trans-Aconitic acid was 62.09%; the *trans-Aconitic* acid yield of the starting strains At-Δ*cadA* was 7.08 g/L, and the conversion rate of the *trans*-Aconitic acid was 43.78%. The Error bar in the figure represents the SEM of three parallel experiments (significance: $^*p \leq 0.05$; $^{**}p < 0.01$; $^{***}p < 0.001$).

Statistical comparative analysis of the fermentation yields of the engineered strains showed that after 72 hours of shake flask fermentation, the *trans-Aconitic* acid yields of the three strains At-Δ*cadA*::MTSacoAt-*adi1* (37.67 g/L), At-Δ*cadA::MTScitAt-adi1* (33.72 g/L), and At-Δ*cadA*::MTSacoAt-*tbrA* (11.18 g/L) are significantly higher than that of the starting strain At-Δ*cadA* (7.08 g/L). Meanwhile, although the *trans*-Aconitic acid yield of another strain *At*-Δ*cadA*::MTScitAt-*tbrA* (7.16 g/L) does not increase significantly compared with the starting strain *At-ΔcadA,* its *trans*-Aconitic acid conversion rate reaches 62.09%, which is much higher than 43.78% of the starting strain At-Δ*cadA.* This indicates that targeting aconitate isomerase to mitochondria can significantly increase the yield and conversion rate of *trans*-Aconitic acid.

The inventors speculate that due to the specificity of enzyme, the Adil strains (At-Δ*cadA*::*MTScitAt-adi1* and At-Δ*cadA*::*MTSacoAt-adi1*) targeted to mitochondria mediated by the two signal peptides MTScitAt and MTSacoAt are significantly higher than the TbrA strains (At-Δ*cadA*::MTScitA-*tbrA* and At-Δ*cadA*::MTSacoA-*tbrA*). Meanwhile, the *trans*-Aconitic acid yields of these two strains are also significantly higher than the *trans*-Aconitic acid yields of 24.65 g/L (168 h) (202010866757.4) and 15.34 g/L (72 h) (202010866728.8) in other patents. The strains with Adil isomerase targeted to mitochondria by the MTSacoAt signal peptide is the best, which can significantly increase the yield and conversion rate of

*trans*-Aconitic acid.

Based on the above experimental verification results, compared with the *trans*-Aconitic acid yield and conversion rate of the starting strains *At-ΔcadA,* it can be confirmed that targeting aconitate isomerase to mitochondria can significantly increase the yield and conversion rate of *trans*-Aconitic acid. The improved strategy made by the inventors based on the previously constructed high-yield *trans*-Aconitic acid-producing strains can be successfully implemented and has achieved very significant progressive effects.

**Example 11** Construction of recombinant *Aspergillus niger* strains with mitochondrially targeted aconitate isomerase

To further verify the broad applicability of the above strategy, namely, the constructing concept of targeting *trans*-Aconitic acid-related enzymes to the mitochondria by introducing mitochondrial targeting signal peptides, the inventors subsequently modified the *Aspergillus niger* strains based on the above construction strategy.

1) Construction of a targeting element for overexpressing the aconitate isomerase *tbrA gene*/*adi1* gene containing the mitochondrial targeting signal peptide MTSicdAn

The recombinant *Aspergillus niger* includes a promoter that drives the expression of aconitate isomerase. The promoter is a PicdA promoter, and the sequence of the P*icdA* promoter is shown as SEQ ID NO. 15. However, the selection of the promoter herein is not limited to the P*icdA* promoter; other promoters commonly used in the art can also be adopted, such as a P*gpdAt* promoter whose nucleotide sequence is shown as SEQ ID NO. 11, *a* P*gpdAn* promoter whose nucleotide sequence is shown as SEQ ID NO. 12, a P*acoA* promoter whose nucleotide sequence is shown as SEQ ID NO. 13, a P*citA* promoter whose nucleotide sequence is shown as SEQ ID NO. 14, a P*glaA* promoter whose nucleotide sequence is shown as SEQ ID NO. 10, a P*cadA* promoter whose nucleotide sequence is shown as SEQ ID NO. 16, a P*mfsA* promoter whose nucleotide sequence is shown as SEQ ID NO. 17, and a P*gasA* promoter whose nucleotide sequence is shown as SEQ ID NO. 18.

The recombinant *Aspergillus niger* also includes a terminator for terminating the expression of aconitate isomerase. The terminator is a TtrpC terminator, and the sequence of the TtrpC terminator is shown as SEQ ID NO. 19. It should be noted that the selection of the terminator herein is not limited to this; those skilled in the art can select other terminators according to conventional practices, such as a T*pgk* terminator whose sequence is shown as SEQ ID NO. 20.

Primers were designed and synthesized based on the gene sequence information, and the specific primer sequences are provided below.

The PicdAn promoter (PicdA is a general term for promoters of the same gene, PicdAn is the promoter obtained from *Aspergillus niger,* and PicdAt is the corresponding promoter obtained from *Aspergillus terreus;* for other promoters, those skilled in the art can understand their expression meanings according to common general knowledge in the art or the above numbering rules) was amplified using the genome of *Aspergillus niger* MEFC1501 as a template and using primers PicdAn-F/PicdAn-R, to obtain the PicdAn fragment.

Similarly, the mitochondrial targeting signal peptide was amplified using the genome of *Aspergillus niger* MEFC1501 as a template and using primers MTSicdAn (PicdAn)-F/MTSicdAn (tbrA)-R, to obtain the MTSicdAn fragment.

tbrA was amplified using plasmid puc57-tbrA as a template and using primers tbrA-F/tbrA-R, to obtain the *tbrA* fragment.

adil was amplified using plasmid puc57-adi1 as a template and using primers adi1-F/adi1-R, to obtain the *adi1* fragment.

The terminator T*trpC* was amplified using plasmid pXH2-1 as a template and using primers TtrpC-F (TbrA)/TtrpC-R, to obtain the TtrpC (tbrA) fragment.

All the PCR products were detected by 0.8% agarose gel electrophoresis, followed by gel cutting, recovery, and purification. The "PicdAn fragment, MTSicdAn fragment, tbrA fragment, and TtrpC (tbrA) fragment" were fused by fusion PCR. The PicdA-MTSicdA-tbrA-TtrpC gene targeting element was amplified using the product of this fusion PCR as a template and using primers PicdAn-F/TtrpC-R, which can be used for subsequent construction of strains with random insertion and overexpression of aconitate isomerase *tbrA* containing the mitochondrial targeting signal peptide MTSicdAn.

The "PicdAn fragment, MTSicdAn fragment, adil fragment, and TtrpC (tbrA) fragment" were fused by fusion PCR. The PicdA-MTSicdA-adi1-TtrpC gene targeting element was amplified using the product of this fusion PCR as a template and using primers PicdAn-F/TtrpC-R, which can be used for subsequent construction of strains with random insertion and overexpression of aconitate isomerase *adil* containing the mitochondrial targeting signal peptide MTSicdAn.

PCR was performed using the plasmid pSGF957 (obtained from Seoul National University, and recorded in Kim, J.G., Choi, Y.D., Chang, Y.J., Kim, S.U., Genetic transformation of Monascus purpureus DSM1379, Biotechnology Letters, 2003, 25, 1509-1514) as a template, to amplify the PtrpC-hph-TtrpC fragment. PtrpC is the promoter of the tryptophan synthase gene from *Aspergillus nidulans, hph* is the hygromycin phosphotransferase gene, and T*trpC* is the terminator of the tryptophan synthase gene from *Aspergillus nidulans.* The PCR product was detected by 0.8% agarose gel electrophoresis, followed by gel cutting, recovery, and purification to obtain the hygromycin B resistance *hph* gene expression cassette PtrpC-hph-TtrpC, which can be used for the construction of strains with random insertion and overexpression of aconitate isomerase *tbrA*/*adi1* containing the mitochondrial targeting signal peptide MTSicdAn.

2) Construction of *Aspergillus niger* engineered strains with random insertion and overexpression of aconitate isomerase *tbrA*/*adi1* fused with mitochondrial targeting signal peptide MTSicdAn

For the specific operation of transforming genes into the strains, please refer to the construction methods of *Aspergillus niger* engineered strains in Examples 1 to 6.

The recombinant *Aspergillus niger* transformants overexpressing aconitate isomerase *tbrA* fused with the mitochondrial targeting signal peptide MTSicdAn obtained above were picked and inoculated onto PDA-H plates to culture spores. Then, the spores were respectively inoculated into liquid medium for culture, and mycelia were collected to extract the genome. PCR verification was performed using PicdA-F/TtrpC-R as primers, while the genome of *Aspergillus niger* MEFC1501 was used as a control. Positive transformants can amplify a band of approximately 3.0 kb, while no target band was amplified in the control. The transformants with correct genotype verification were transferred to PDA-H plates for subculture, with three passages in total. Then, the spores were collected and appropriately diluted with physiological saline, and 100 μL of the diluted spores was spread on PDA-H plates to allow the growth of independent single colonies, which was referred to as single-spore isolation. Spores were taken from the single colonies for single-spore isolation again, and a total of four rounds of single-spore isolation and subculture were performed. The recombinant strains after single-spore isolation were subjected to genotype identification again for verification, and the genetic engineered strains with high yield of *trans*-Aconitic acid was successfully obtained and labeled as An::*PicdA*-MTSicdA-*tbrA*.

The recombinant *Aspergillus niger* transformants overexpressing aconitate isomerase *adil* fused with the mitochondrial targeting signal peptide MTSicdAn obtained above were picked and inoculated onto PDA-H plates to culture spores. Then, the spores were respectively inoculated into liquid medium for culture, and mycelia were collected to extract the genome. PCR verification was performed using PicdA-F/TtrpC-R as primers and using the genome of *Aspergillus niger* MEFC1501 as a control. Positive transformants can amplify a band of approximately 3.2 kb, while no target band was amplified in the control. The transformants with correct genotype verification were transferred to PDA-H plates for subculture, with three passages in total. Then, the spores were collected and appropriately diluted with physiological saline, and 100 μL of the diluted spores was spread on PDA-H plates to allow the growth of independent single colonies, which was referred to as single-spore isolation. Spores were taken from the single colonies for single-spore isolation again, and a total of four rounds of single-spore isolation and subculture were performed. The recombinant strains after single-spore isolation were subjected to genotype identification again for verification, and the genetic engineered strains with high yield of *trans*-Aconitic acid was successfully obtained and labeled as An::PicdA-MTSicdA-*adi1*.

3) Shake flask analysis of *trans*-Aconitic acid production by *Aspergillus niger* engineered strains overexpressing mitochondrially targeted aconitate isomerase *tbrA*

The An::PicdA-MTSicdA-*tbrA* engineered strains and the starting strains *Aspergillus niger* MEFC1501 were selected and respectively inoculated into *Aspergillus niger* sporulation medium, and incubated at 32°C for 6 days to obtain mature spores. Then, the mature spores of each strain were respectively inoculated into the *trans*-Aconitic acid fermentation medium IPM, with an inoculum size of approximately $5\times10^7$ spores/mL as the final concentration. Three shake flasks were set as parallels for each transformant, and fermentation was performed at 34°C under 220 rpm for 170 h. The supernatant was filtered through a 0.45 μm filter, and was diluted appropriately, and the content of organic acid was detected by high-performance liquid chromatography (HPLC). The chromatographic column is Bio-rad Aminex HPX-87H (300 mm$\times$7.8 mm), the mobile phase is 5 mM sulfuric acid, the flow rate is 0.5 mL/min, the column temperature is 55°C, the detection temperature is 35°C, and the wavelength of the ultraviolet detector is 210 nm.

The results of *trans*-Aconitic acid yield are shown in Fig. 10. In Fig. 10, the white part represents *cis*-Aconitic acid, the left diagonal pattern represents *trans*-Aconitic acid, WT is the control strains MEFC1501, and MTSicdAn-tbrA is the *Aspergillus niger* engineered strains containing aconitate isomerase *tbrA* fused with the mitochondrial targeting signal peptide MTSicdAn. The Error bar in the figure represents the SEM of three parallel experiments (significance: *$p \leq 0.05$; **$p < 0.01$; ***$p < 0.001$). As can be seen from the results in Fig. 10, the *trans*-Aconitic acid yield of the strains An::PicdA-MTSicdA-*tbrA* (17.19 g/L) is much higher than that of the starting strains MEFC1501 (1.02 g/L), with an increase of 1685%. The inventors also used different transformation methods, such as co-transformation with the selection marker or constructing the expression cassette of the target gene and the expression cassette of the selection marker together for transformation, and similar results were obtained. It can be seen that targeting the isomerase tbrA to mitochondria can significantly increase the trans-Aconitic acid yield of *Aspergillus niger.*

4) Shake flask analysis of *trans*-Aconitic acid production by *Aspergillus niger* engineered strains overexpressing mitochondrially targeted aconitate isomerase *adil*

The An::PicdA-MTSicdA-*adi1* engineered strains and the starting strains *Aspergillus niger* MEFC1501 were selected and respectively inoculated into *Aspergillus niger* sporulation medium, and incubated at 32°C for 6 days to obtain mature spores. Then, the mature spores of each strain were respectively inoculated into the *trans*-Aconitic acid fermentation medium IPM, with an inoculum size of approximately $5\times10^7$ spores/mL as the final concentration. Three shake flasks were set as parallels for each transformant, and fermentation was performed at 34°C under 220 rpm for 170 h. The supernatant was filtered through a 0.45 $\mu$m filter, and was diluted appropriately, and the content of organic acid was detected by high-performance liquid chromatography (HPLC). The chromatographic column is Bio-rad Aminex HPX-87H (300 mm$\times$7.8 mm), the mobile phase is 5 mM sulfuric acid, the flow rate is 0.5 mL/min, the column temperature is 55°C, the detection temperature is 35°C, and the wavelength of the ultraviolet detector is 210 nm.

The results of *trans*-Aconitic acid yield are shown in Fig. 11. In Fig. 11, the white part represents *cis*-Aconitic acid, the left diagonal pattern represents *trans*-Aconitic acid, WT is the control strains MEFC1501, and MTSicdAn-adi1 is the *Aspergillus niger* engineered strains containing aconitate isomerase *tbrA* fused with the mitochondrial targeting signal peptide MTSicdAn. The Error bar in the figure represents the SEM of three parallel experiments (significance: *$p\leq0.05$; **$p < 0.01$; ***$p < 0.001$). The *trans*-Aconitic acid yield of the strains An::PicdA-MTSicdA-*adi1* (25.42 g/L) is 25 times higher than that of the starting strains MEFC1501. The inventors also used different transformation methods, such as co-transformation with the selection marker or constructing the expression cassette of the target gene and the expression cassette of the selection marker together for transformation, and similar results were obtained. It can be seen that targeting the isomerase *adil* to mitochondria can significantly increase the *trans*-Aconitic acid yield in *Aspergillus niger.*

Based on the above examples and verification results, it can be concluded that the mitochondrial targeting signal peptides MTSacoAt, MTScitAt, and MTSicdAn can accurately localize the target protein to mitochondria. Moreover, mitochondrial targeting signal peptides can be indeed used to localize aconitate isomerase to the mitochondria of *Aspergillus terreus* and *Aspergillus niger.* Based on the above research results, it is reasonably expected by those skilled in the art that the strategy of targeting aconitate isomerase to mitochondria using mitochondrial targeting signal peptides can successfully affect the metabolic pathway of *trans*-Aconitic acid and significantly increase the yield and conversion rate of *trans*-Aconitic acid in *Aspergillus terreus* and *Aspergillus niger.*

Primers used in the specific embodiments of the present disclosure are listed as follows:

pXH106-X-F: 5'-cccggagttaggtagataga-3' (SEQ ID NO.84);
pXH106-X-R: 5'-gcatcaaatcgtcgtaccgca-3' (SEQ ID NO.85);
PgpdAt-F (PyrG): 5'-gcggtacgacgatttgatgcttacactctgggaggatccagg-3' (SEQ ID NO.86);
PgpdAt-R: 5'-gttgtgatgattgatgagtt-3' (SEQ ID NO.87);
PgpdAt-R (MTScitAt): 5'-tctgagagaggcagccatgttgtgatgattgatgagttgttgg-3' (SEQ ID NO.88);
PgpdAt-R (MTSacoAt): 5'-gcaaggcgggtggagatcatgttgtgatgattgatgagtt-3' (SEQ ID NO.89);
PgpdAt-R (MTSicdAn): 5'-ctgaaccggacagacgacatgttgtgatgattgatgagtt-3' (SEQ ID NO.90);
MTScitAt-F: 5'-atggctgcctctctcagac-3' (SEQ ID NO.91);
MTScitAt-R: 5'-gtagcagcgcaagccattga-3' (SEQ ID NO.92);
MTSacoAt-F: 5'-atgatctccacccgccttg-3' (SEQ ID NO.93);
MTSacoAt-R: 5'-ataggggaatcagcaacggtg-3' (SEQ ID NO.94);
MTSicdAn-F: 5'-atgtcgtctgtccggttcag-3' (SEQ ID NO.95);
MTSicdAn-R: 5'-ggtgcgggattgagtaaggt-3' (SEQ ID NO.96);
StayGold (PgpdAt)-F:
5'-ctcatcaatcatcacaacatggcctccaccccccttcaagt-3' (SEQ ID NO.97);
StayGold (MTScitAt)-F:
5'-aatggcttgcgctgctacatggcctccacccccttc-3' (SEQ ID NO.98);
StayGold (MTSacoAt)-F:
5'-accgttgctgattcccctatggcctccacccccttc-3' (SEQ ID NO.99);
StayGold (MTSicdAn)-F:
5'-accttactcaatcccgcaccatggcctccaccccccttcaa-3' (SEQ ID NO.100);
StayGold-R (Ku80-D):
5'-attctatctacctaactccgggttacaggtgggcctccagg-3' (SEQ ID NO.101);
D-ku80-F: 5'-gggtttctagaagtcacatcage-3' (SEQ ID NO.102);
U-ku80-R: 5'-atcaccgaccctacgctgtg-3' (SEQ ID NO.103);
YZ-ku80-F: 5'-atataccactccacactaaccg-3' (SEQ ID NO.104);
YZ-ku80-F: 5'-gccatagagttgctcttgatc-3' (SEQ ID NO.105);
U-cadA-F: 5'-gcgataaatgttgaacgaggc-3' (SEQ ID NO.106);

U-cadA-R (MTScitAt):
5’-agtctgagagaggcagccatggtcaatttaataggacaattttc-3’ (SEQ ID NO.107);
U-cadA-R (MTSacoAt):
5’-aggcgggtggagatcatggtcaatttaataggacaattttc-3’ (SEQ ID NO.108);
MTScitAt-F: 5’-atggctgcctctctcagact-3’ (SEQ ID NO.109);
MTScitAt-R: 5’-gtagcagcgcaagccattga-3’ (SEQ ID NO.110);
MTSacoAt-F: 5’-atgatctccacccgccttg-3’ (SEQ ID NO.111);
MTSacoAt-R: 5’-ataggggaatcagcaacggtg-3’ (SEQ ID NO.112);
adil-F (MTScitAt):
5’-ttcaatggcttgcgctgctacatgctgcaccccatcgacac-3’ (SEQ ID NO.113);
adil-F (MTSacoAt):
5’ -gccaccgttgctgattcccctatgctgcaccccatcgacac-3’ (SEQ ID NO.114);
adi1-R: 5’-ttaggacaggctacggtcgctag-3’ (SEQ ID NO.52);
tbrA-F (MTScitAt):
5’-caatggcttgcgctgctacatgaagatcccctgcttcgtc-3’ (SEQ ID NO.115);
tbrA-F (MTSacoAt):
5’-aattgtcctattaaattgaccatgatctccacccgccttg-3’ (SEQ ID NO.116);
tbrA-R: 5’-ttaggggatgatcagctcgc-3’ (SEQ ID NO.117);
TtrpC-F (adil): 5’-gcgaccgtagcctgtcctaaggatccacttaacgttactg-3’ (SEQ ID NO.118);
TtrpC-F (tbrA):
5’-gcgagctgatcatcccctaaggatccacttaacgttactg-3’ (SEQ ID NO. 119);
TtrpC-R: 5’-aagaaggttacctctaaacaag-3’ (SEQ ID NO.120);
pyrG-F (TtrpC): 5’-cttgtttagaggtaaccttctttaagggagatggtgattgaactag-3’ (SEQ ID NO.121);
pyrG-R: 5’-gcatcaaatcgtcgtaccgca-3’ (SEQ ID NO.122);
D-cadA-F (pyrG):
5’-tgcggtacgacgatttgatgcatgggaagcgatatggaaac-3’ (SEQ ID NO. 123);
D-cadA-R: 5’-cattgcagggaagtatatgcttc-3’ (SEQ ID NO.124);
C-cadA-F: 5’-tgtggttcctaccaaggtggc-3’ (SEQ ID NO.125);
C-cadA-R: 5’-cgactatagctggattgatcac-3’ (SEQ ID NO.126);
PicdAn-F:
5’-CTTTAACGTTGCAGATACAGGGATGCG-3’ (SEQ ID NO.47);
PicdAn-R: 5’-TTCGACAGTGAAGCGTGGCC-3’ (SEQ ID NO.127);
MTSicdAn (PicdAn)-F:
5’-GGCCACGCTTCACTGTCGAAATGTCGTCTGTCCGGTTCAG-3’ (SEQ ID NO.128);
MTSicdAn (tbrA)-R:
5’-gacgaagcaggggatcttcatGGTGCGGGATTGAGGAAGGTTC-3’ (SEQ ID NO.129);
tbrA-F: 5’-atgaagatcccctgcttcgtc-3’ (SEQ ID NO.130);
tbrA-R: 5’-ttaggggatgatcagctcgcc-3’ (SEQ ID NO.131);
TtrpC-F (TbrA):
5’-ggcgagctgatcatcccctaaggatccacttaacgttactgaaatcatc-3’ (SEQ ID NO.132);
TtrpC-R: 5’-GAATCAGAGCTCCATCATGTCTTG-3’ (SEQ ID NO.133);
hph-F: 5’-ttcgggatcgcaagcgtaaag-3’ (SEQ ID NO.52);
hph-R: 5’-caattatctttgcgaacccagg-3’ (SEQ ID NO.53);
MTSicdAn (adi1)-R: 5’-gtgtcgatggggtgcagcatggtgcgggattgaggaaggt-3’ (SEQ ID NO. 134);
adil-F: 5’-atgctgcaccccatcgacac-3’ (SEQ ID NO.135);
adi1-R: 5’-ttaggacaggctacggtcgc-3’ (SEQ ID NO.136);

**Example 12** Optimization of fermentation medium for TAA production

In the fermentation process for submerged liquid fermentation of *trans*-Aconitic acid provided by the present disclosure, fermentation strains include, but are not limited to, the *Aspergillus terreus* strains with defective cadA cis-Aconitic acid decarboxylase function, which are protected by the authorized patents CN110527637B, CN112011468B, CN112011469B, CN112011470B, and CN112029671B of the inventors; or the *Aspergillus terreus* strains and *Aspergillus niger* strains constructed in Examples 1 to 11 above.

Optionally, *Aspergillus terreus* strains can be either the one with P450 being knocked out after the loss of function of cis-aconitate decarboxylase encoded by the *cadA* gene, or the one with introduced aconitate isomerase. It can be selected from the genetically engineered strains such as At-ΔcadA::adi1 and At-Δ*cadA*::tbrA obtained by modification based on *Aspergillus terreus* CICC40205, *Aspergillus terreus* NRRL1960, *Aspergillus terreus* DSM23081, *Aspergillus*

*terreus* TN484, and *Aspergillus terreus* TN484-M1 as described in the above patents.

Specifically, the process includes the following steps:

(1) Seed streaking culture: the spores were gently scraped with an inoculating loop and were inoculated into PDA medium or IPM (organic acid solid medium IPM: 10 g/L glucose, 3 g/L ammonium sulfate, 1 g/L diammonium hydrogen phosphate, 10 mg/L ferrous sulfate, 5 g/L magnesium sulfate, wheat bran, 16 g/L agar strip), the mixture was sterilized at 115°C for 30 min, and cultured in a 25°C incubator for 5 to 6 days.

(2) Preparation of seed: the wheat bran and the distilled water were mixed at a ratio of 1:1 and were sterilized at 121°C to prepare the wheat bran medium for later use; the streaked seeds were used to elute spores with 10 mL of sterile water and were inoculated into the wheat bran medium, and incubated at 25°C for 5 days to obtain a seed culture for later use, ensuring that the final spore count reaches an order of magnitude of $1\times10^{12}$ spores/g.

(3) Fermentation of primary seeds:

1T fermenter was loaded with 300 L of feed liquid and fermented at 37°C for 14 to 16 h. The medium includes the following components: 100 g/L glucose, 3 g/L ammonium sulfate, 2 g/L corn steep liquor powder, 100 mL defoamer, 0.2 g/L diammonium hydrogen phosphate, 20 mg/L ferrous sulfate, 0.4 g/L magnesium sulfate, 40 mg/L zinc sulfate, 40 mg/L copper sulfate, and 0.5 g/L sodium chloride. The pH was adjusted to 4.2 with sulfuric acid.

(4) Fermentation of secondary seeds:

4T fermenter was loaded with 1.5T of feed liquid and fermented at 37°C for 9 to 12 h. The medium includes the following components: 40 g/L glucose, 3 g/L ammonium sulfate, 1 g/L corn steep liquor powder, 0.5 L defoamer, 0.2 g/L diammonium hydrogen phosphate, 20 mg/L ferrous sulfate, 0.4 g/L magnesium sulfate, 40 mg/L zinc sulfate, 40 mg/L copper sulfate, and 0.5 g/L sodium chloride. The pH was adjusted to 5 to 6 with sulfuric acid. The pressure in the fermenter was 0.1 to 0.12 MPa.

(5) Fermentation of products:

20T fermenter was loaded with 13.6 T of feed liquid and fermented at 37°C. The medium includes the following components: 120 g/L glucose, 3 g/L ammonium sulfate, 1 g/L corn steep liquor powder, 8 L defoamer (no adjustment of the pH value was required), 0.2 g/L diammonium hydrogen phosphate, 10 mg/L ferrous sulfate, 40 mg/L zinc sulfate, 40 mg/L copper sulfate, 0.5 g/L sodium chloride, and 0.4 g/L magnesium sulfate. The pressure in the fermenter was 0.08 to 0.1 MPa, the agitation speed was 90 rpm, the aeration rate was 90 to 180 $m^3$/h, and the aeration ratio was 0.11 to 0.22 vvm.

The above fermentation medium for *Aspergillus terreus* producing itaconic acid was used as the basal medium for producing *trans*-Aconitic acid (authorized patent No. CN110527637B). On the basis of this medium, a gradient test was performed on five factors (glucose, ammonium sulfate, corn steep liquor powder, ferrous sulfate, and diammonium hydrogen phosphate). After determining the influencing factors of each single component, an orthogonal experiment with 4 levels was further performed due to the interaction effects between each factor. The L16=4×5 experimental combinations designed by Minitab software are shown in Table 1. For each medium code, 3 parallel experiments were performed to evaluate the TAA yield and sugar-acid conversion rate at 96 h. The results of the single-component analysis showed that increasing the addition amount of corn steep liquor powder or ferrous sulfate could significantly affect the yield of aconitic acid. The results of the further horizontal orthogonal experiments are shown in Fig. 12. Corn steep liquor powder (increased to 2-fold) and diammonium hydrogen phosphate (increased to 6-fold) were added simultaneously, resulting in a 39% increase in yield compared to the basal medium, with the yield reaching 30 g/L (Medium No. 11). The contents of corn steep liquor powder (increased to 2-fold) and ferrous sulfate (increased to 6-fold) were simultaneously increased, and 150 g/L high-concentration initial glucose was used, the yield of *trans*-Aconitic acid was increased by 49% compared with the original basal medium, reaching 32 g/L (Medium No. 15).

Table 1 Orthogonally Designed Fermentation Media with Different Components of Nutritional Factors for Aconitic Acid-Producing *Aspergillus terreus* Strains (L16=4×5)

| No. of medium | Monohydrate glucose | Ammoniu m sulfate | Corn steep liquor powder | Ferrous sulfate | Diammonium hydrogen phosphate | Magnesium sulfate |
|---|---|---|---|---|---|---|
| 0 | 120 | 3 | 1 | 0.01 | 0.01 | 0.4 |
| 1 | 80 | 2 | 1 | 0.01 | 0.01 | 0.4 |
| 2 | 80 | 3 | 2 | 0.02 | 0.02 | 0.4 |

(continued)

| No. of medium | Monohydrate glucose | Ammoniu m sulfate | Corn steep liquor powder | Ferrous sulfate | Diammonium hydrogen phosphate | Magnesium sulfate |
|---|---|---|---|---|---|---|
| 3 | 80 | 4 | 3 | 0.04 | 0.04 | 0.4 |
| 4 | 80 | 5 | 4 | 0.06 | 0.06 | 0.4 |
| 5 | 100 | 2 | 2 | 0.04 | 0.06 | 0.4 |
| 6 | 100 | 3 | 1 | 0.06 | 0.04 | 0.4 |
| 7 | 100 | 4 | 4 | 0.01 | 0.02 | 0.4 |
| 8 | 100 | 5 | 3 | 0.02 | 0.01 | 0.4 |
| 9 | 120 | 2 | 3 | 0.06 | 0.02 | 0.4 |
| 10 | 120 | 3 | 4 | 0.04 | 0.01 | 0.4 |
| 11 | 120 | 4 | 1 | 0.02 | 0.06 | 0.4 |
| 12 | 120 | 5 | 2 | 0.01 | 0.04 | 0.4 |
| 13 | 150 | 2 | 4 | 0.02 | 0.04 | 0.4 |
| 14 | 150 | 3 | 3 | 0.01 | 0.06 | 0.4 |
| 15 | 150 | 4 | 2 | 0.06 | 0.01 | 0.4 |
| 16 | 150 | 5 | 1 | 0.04 | 0.02 | 0.4 |

**Example 13** Effects of adding nutritional factors such as biotin-vitamin B7, L-leucine, L-tryptophan, and L-methionine on TAA yield

Shake flask fermentation experiments in a shaker demonstrated significant differences in corn steep liquor powder. Therefore, the amino acid and vitamin components in high-yield and low-yield corn steep liquor powder were analyzed and identified, and their contents were determined (see Fig. 13).

1) Effect on TAA yield of *Aspergillus terreus* engineered strains

Additional supplementation experiments were performed on differential amino acids and vitamins between high-yield and low-yield corn steep liquor powders. Based on the high-yield corn steep liquor powder medium, fermentation verification was performed by adding differential amino acid components and vitamin components separately. Each group was set up with three parallel replicates. After 96 hours of shake flask fermentation, the supernatant of the fermentation broth was collected for quantitative analysis using HPLC detection. The results showed that the addition of certain amino acid components could increase the yield of *trans*-Aconitic acid (see Figs. 14A and 14B), and there was a dose effect. For example: the addition of 0.125 g/L L-leucine could increase the yield of *trans*-Aconitic acid by 42%; the addition of 0.11 g/L biotin-vitamin B7 could increase the yield of *trans*-Aconitic acid by 17.5%; the addition of 0.115 g/L L-methionine could increase the yield of *trans*-Aconitic acid by 86.5%; the addition of 0.2 g/L L-tryptophan could increase the yield of *trans*-Aconitic acid by 20.7%; and the addition of 0.099 g/L L-tryptophan could increase the yield of *trans*-Aconitic acid by 46.2%. In addition, the additionally added lactic acid, L-glycine, L-isoleucine, L-glutamine, L-proline, and L-lysine at certain doses resulted in varying degrees of decrease in the yield of *trans*-Aconitic acid.

L-tryptophan and L-methionine, which significantly increased the yield of *trans*-Aconitic acid, were selected and added to the optimal medium in Example 12 at final concentrations of 0.099 g/L and 0.115 g/L respectively for verification in a 30 L fermenter. Meanwhile, the medium without added amino acids was used as a control to compare the fermentation yields. The beneficial fermentation control process in Example 12 was adopted: the seeds were 5.2 g of wheat bran seeds; the primary shake flasks were four 1 L shake flasks containing 250 mL of medium, and the medium was incubated in a shaker at 230 rpm for 15 h; the secondary seed fermenter had a liquid volume of 10 L, and the seeds were incubated with an aeration ratio of 1 vvm under an agitation speed of 300 rpm for 10 h. The primary and secondary seed media were: 150 g/L glucose monohydrate, 4 g/L ammonium sulfate, 2 g/L corn steep liquor powder, 0.4 g/L magnesium sulfate heptahydrate, 0.06 g/L ferrous sulfate heptahydrate, 0.01 g/L diammonium hydrogen phosphate, 40 mg/L zinc sulfate, 40 mg/L copper sulfate, and 0.5 g/L sodium chloride; no L-Trp or L-Met was added to the primary and secondary media. The tertiary fermentation medium was the same as the secondary seed medium, but L-Trp or L-Met was added. The control

parameters of the 30 L fermenter were as follows: 300 to 480 rpm, dissolved oxygen (DO) level maintained at 25% to 29%, aeration rate at 20 L/min; DO control was linked during agitation. The control parameters of the 100 L fermenter were as follows: 250 to 280 rpm, DO controlled from 25 to 30% during agitation linkage, and aeration rate controlled f 60 to 34 L/min. The results are shown in Fig. 15. The yield of *trans*-Aconitic acid was 26 g/L without adding amino acid; after adding L-tryptophan, the yield of *trans*-Aconitic acid reached 41.95 g/L at 60 h; and after L-methionine was added, the final fermentation yield of *trans*-Aconitic acid reached 65 g/L, both showing significant increases. This confirms that nutritional factors such as L-tryptophan and L-methionine can significantly increase the yield of *trans*-Aconitic acid in a 30 L small fermenter.

2) Effect on TAA yield of *Aspergillus niger* engineered strains

Nutritional factor optimization experiments were performed on the *trans*-Aconitic acid-producing *Aspergillus niger* engineered strains constructed in Example 1. Amino acid components were added for fermentation verification using the high-yield corn steep liquor powder medium as the basis. For the shake flask experiment, please refer to the fermentation conditions in Examples 1 to 8, except that nutritional factors were added to the *Aspergillus niger* fermentation medium, wherein the addition amount of L-methionine was 0.12 g/L. The control medium was the *Aspergillus niger* fermentation medium without any added nutritional factors, and three parallel replicates were set for each group. The results showed that the addition of L-methionine significantly increased the yields of both wild-type *Aspergillus niger* and recombinant engineered strains, and the yield of the recombinant engineered strains reached 31.76 g/L after L-methionine was added (see Fig. 14C). This confirms that nutritional factors can also significantly increase the yield of *trans*-Aconitic acid in *Aspergillus niger.*

Fermentation was performed in a 30 L fermenter using the medium added with L-methionine. The control parameters were as follows: 300 to 480 rpm, DO level maintained at 25 to 29%, and aeration rate controlled at 20 L/min. The TAA yield reached 60 g/L after 132 h of fermentation.

**Example 14** Verification of optimal fermentation dissolved oxygen (DO) control process in 30 L to 100 L fermenters

Basic process and medium: the primary seed fermenter was loaded with 70% feed liquid and fermented at 37°C for 14 to 16 h. The medium includes the following components: 40 g/L glucose, 3 g/L ammonium sulfate, 0.1% defoamer, 2 g/L corn steep liquor powder, 0.2 g/L diammonium hydrogen phosphate, 20 mg/L ferrous sulfate, 0.4 g/L magnesium sulfate, 40 mg/L zinc sulfate, 40 mg/L copper sulfate, and 0.5 g/L sodium chloride. The pH was adjusted to 4.2 with sulfuric acid, and sterilized at 115 to 120°C for 15 to 20 min for later use. One or two hours before inoculation, the wheat bran seeds were suspended with sterile distilled water, shaken at 100 rpm in a shaker after suspension, the final concentration of the inoculum was $1\times10^7$ spores, the fermenter pressure was 0.12 MPa, the agitation speed was 30 Hz, and the aeration ratio was 1.4 vvm. The standard references for subculture transfer were mainly based on a time range of 13 to 15 h and a solid biomass proportion of 4% before transfer.

The secondary seed fermenter was loaded with 70% feed liquid and fermented at 37°C for 9 to 12 h. The medium includes the following components: 100 g/L glucose, 3 g/L ammonium sulfate, 2 g/L corn steep liquor powder, 0.1% defoamer, 0.2 g/L diammonium hydrogen phosphate, 20 mg/L ferrous sulfate, 0.4 g/L magnesium sulfate, 40 mg/L zinc sulfate, 40 mg/L copper sulfate, and 0.5 g/L sodium chloride. The pH was adjusted to 5 to 6 with sulfuric acid. The pressure of the secondary fermenter was 0.12 MPa, the agitation speed was 120 rpm, and the aeration ratio was 0.3 to 0.35 vvm. The proportion of solid biomass before transfer was 9%.

The tertiary fermenter was loaded with 80% feed liquid for fermentation. The medium includes the following components: 120 g/L glucose, 4 g/L ammonium sulfate, 0.1% defoamer, 2 g/L corn steep liquor powder, 0.01 g/L diammonium hydrogen phosphate, 60 mg/L ferrous sulfate, 0.4 g/L magnesium sulfate, 40 mg/L zinc sulfate, 40 mg/L copper sulfate, and 0.5 g/L sodium chloride. The pressure of the fermenter was 0.08 to 0.1 MPa, the agitation speed was 90 rpm, and the optimal aeration ratio was 0.19 to 0.22 vvm. After seed transfer, acid production has reached 7 to 8 g/L. The change of residual sugar was monitored at any time; if the residual sugar was lower than 15 g/L, glucose was supplemented in time to maintain fermentation until the yield of the main product TAA no longer increased. After fermentation, sterilization at 100°C for 30 min or sterilization at 121°C for 10 min could achieve the highest conversion ratio of the main product.

In view of poor fermentation data of the aconitic acid-producing *Aspergillus terreus* strains (authorized patent numbers: CN112011468B; CN112011469B; CN112011470B; CN112029671B) in the 30 L small fermenter, it was predicted that the fundamental reason was that the level of dissolved oxygen (DO) control was not optimal. Different dissolved oxygen (DO) control levels (25%, 45%, 65%) were used to evaluate the *trans*-Aconitic acid yield of the *trans*-Aconitic acid-producing strains. A careful analysis showed that in 30 to 100 L small fermenters, the factor affecting DO control is mainly the agitation speed. The evaluation of the effect of different dissolved oxygen (DO) levels, which are controlled by varying agitation speeds, on the yield of *trans*-Aconitic acid was performed using the basal medium for

*trans*-Aconitic acid production.

As shown in Fig. 16, when the DO level was maintained at 25% in the 30 L fermenter for the *trans*-Aconitic acid-producing strains, the acid production reached 27 g/L at 72 h; when the DO was 45%, the acid production reached 19 g/L at 72 h; when the DO was 65%, the acid production reached 26 g/L at 72 h. The agitation speed was linked during DO control. When the sugar-acid conversion rates at 72 h was compared, it can be observed that the sugar consumption is significantly reduced when the DO levels are 25% and 65%. The yield of produced acid was the highest when the DO level was maintained at 25%, and the acid production rate was 0.375 $g/L^{-1}\ h^{-1}$, which was 94.2% higher than the optimal acid production rate of the first-generation strains (0.194 $g/L^{-1}\ h^{-1}$). From the fermentation data of the 30 L fermenter, when the DO level was maintained at 25%, a yield of 30 g/L could be achieved at 96 h. The comprehensive total yield was significantly higher than the yield of 40 g/L of the first-generation strains at 250 h. The fermentation cycle for the same yield was shortened by 48.5%; the agitating power requirement was reduced to 2/5 of the original (the agitation speed was reduced from 450 rpm to 180 rpm). Not only the fermentation cycle was significantly shortened, but also the fermentation power cost was significantly reduced.

**Example 15** Optimization of fermentation process in 20T fermenter - process of DO level maintained at 25% - Batch 1

The primary seed 1T fermenter was loaded with 300 L of feed liquid and fermented at 37°C for 14 to 16 h. The medium includes the following components: 100 g/L glucose, 4 g/L ammonium sulfate, 1 g/L corn steep liquor powder, 100 mL defoamer, 0.01 g/L diammonium hydrogen phosphate, 60 mg/L ferrous sulfate, 0.4 g/L magnesium sulfate, 40 mg/L zinc sulfate, 40 mg/L copper sulfate, 0.5 g/L sodium chloride, and 0.05-0.46 g/L nutritional factors (the nutritional factors screened in Example 13). The pH was adjusted to 4.2 with sulfuric acid. The mixture was sterilized at 115 to 120°C for 15 to 20 min for later use. The inoculum size was $160\times10^{12}$ spores, the fermenter pressure was 0.12 MPa, the agitation speed was 160 rpm, the aeration rate was 26 $m^3/h$, and the aeration ratio was 1.4 vvm.

The secondary seed 4T fermenter was loaded with 1.5T of feed liquid and fermented at 37°C for 9 to 12 h. The medium includes the following components: 100 g/L glucose, 4 g/L ammonium sulfate, 1 g/L corn steep liquor powder, 0.5 L defoamer, 0.01 g/L diammonium hydrogen phosphate, 60 mg/L ferrous sulfate, 0.4 g/L magnesium sulfate, 40 mg/L zinc sulfate, 40 mg/L copper sulfate, 0.5 g/L sodium chloride, and 0.05-0.46 g/L nutritional factors. The pH was adjusted to 4.2 with sulfuric acid. The pressure of the secondary fermenter was 0.12 MPa, the agitation speed was 120 rpm, and the aeration ratio was 0.3 to 0.35 vvm. The proportion of solid biomass before transfer was 9%.

The tertiary fermentation 20T fermenter was loaded with 13.6 T of feed liquid and fermented at 37°C. The medium includes the following components: 100 g/L glucose, 3 g/L ammonium sulfate, 1 g/L corn steep liquor powder, 8 L defoamer (no pH adjustment was required), 0.2 g/L diammonium hydrogen phosphate, 20 mg/L ferrous sulfate, 0.4 g/L magnesium sulfate, 40 mg/L zinc sulfate, 40 mg/L copper sulfate, 0.5 g/L sodium chloride, and 0.05-0.46 g/L nutritional factors. After seed transfer, the pH became approximately 3.5 after inoculation. The pressure of the fermenter was 0.12 to 0.1 MPa, the aeration rate was 180 $m^3/h$, the optimal aeration ratio was 0.14 to 0.16 vvm, the initial agitation speed was 110 rpm, and the DO level was maintained at 25 to 30% during agitation.

The change of residual sugar was monitored at any time, and fed-batch fermentation was performed once according to the change of residual sugar. If the residual sugar was lower than 30 g/L, glucose was supplemented in time to maintain fermentation until the yield of the main product TAA no longer increased, then fermentation was terminated.

**Example 16** Optimization of fermenter fermentation process in 20T fermenter-optimization of aeration rate and agitation speed - Batch 2

The primary seed 1T fermenter was loaded with 300 L of feed liquid and fermented at 37°C for 14 to 16 h. The medium includes the following components: 100 g/L glucose, 4 g/L ammonium sulfate, 2 g/L corn steep liquor powder, 100 mL defoamer, 0.01 g/L diammonium hydrogen phosphate, 60 mg/L ferrous sulfate, 0.4 g/L magnesium sulfate, 40 mg/L zinc sulfate, 40 mg/L copper sulfate, 0.5 g/L sodium chloride, and 0.05-0.46 g/L nutritional factors. The pH was adjusted to 4.2 with sulfuric acid. The mixture was sterilized at 115 to 120°C for 15 to 20 min for later use. The inoculum size was $1.6\times10^{14}$ spores, the fermenter pressure was 0.12 MPa, the agitation speed was 160 rpm, the aeration rate was 26 $m^3/h$, and the aeration ratio was 1.4 vvm.

The secondary seed 4T fermenter was loaded with 1.5T of feed liquid and fermented at 37°C for 9 to 12 h. The medium includes the following components: 100 g/L glucose, 4 g/L ammonium sulfate, 2 g/L corn steep liquor powder, 0.5 L defoamer, 0.01 g/L diammonium hydrogen phosphate, 60 mg/L ferrous sulfate, 0.4 g/L magnesium sulfate, 40 mg/L zinc sulfate, 40 mg/L copper sulfate, 0.5 g/L sodium chloride, and 0.05-0.46 g/L nutritional factors. The pH was adjusted to 4.2 with sulfuric acid. The pressure of the secondary fermenter was 0.12 MPa, the agitation speed was 120 rpm, and the aeration ratio was 0.3 to 0.35 vvm. The proportion of solid biomass before transfer was 9%.

The tertiary fermentation 20T fermenter was loaded with 13.6 T of feed liquid and fermented at 37°C. The medium includes the following components: 150 g/L glucose, 4 g/L ammonium sulfate, 2 g/L corn steep liquor powder, 8 L

defoamer, 0.01 g/L diammonium hydrogen phosphate, 60 mg/L ferrous sulfate, 0.4 g/L magnesium sulfate, 40 mg/L zinc sulfate, 40 mg/L copper sulfate, 0.5 g/L sodium chloride, and 0.05-0.46 g/L nutritional factors (no pH adjustment was required). After seed transfer, the pH became approximately 3.5 after inoculation. The pressure of the fermenter was 0.12 to 0.1 MPa, the aeration rate was 180 $m^3$/h, the optimal aeration ratio was 0.11 to 0.13 vvm, the initial agitation speed was 60 rpm, and the DO level was maintained at 25 to 30% during agitation.

The change of residual sugar was monitored at any time, and fed-batch fermentation was performed once according to the change of residual sugar. If the residual sugar was lower than 30 g/L, glucose was supplemented in time to maintain fermentation until the yield of the main product TAA no longer increased, then fermentation was terminated.

**Example 17** Optimization of fermenter fermentation process in 20T fermenter - optimization of feeding time - Batch 3

The primary seed 1T fermenter was loaded with 300 L of feed liquid and fermented at 37°C for 14 to 16 h. The medium includes the following components: 100 g/L glucose, 4 g/L ammonium sulfate, 2 g/L corn steep liquor powder, 100 mL defoamer, 0.01 g/L diammonium hydrogen phosphate, 60 mg/L ferrous sulfate, 0.4 g/L magnesium sulfate, 40 mg/L zinc sulfate, 40 mg/L copper sulfate, 0.5 g/L sodium chloride, and 0.05-0.46 g/L nutritional factors. The pH was adjusted to 4.2 with sulfuric acid. The mixture was sterilized at 115 to 120°C for 15 to 20 min for later use. The inoculum size was $1.6\times10^{14}$ spores, the fermenter pressure was 0.12 MPa, the agitation speed was 160 rpm, the aeration rate was 26 $m^3$/h, and the aeration ratio was 1.4 vvm.

The secondary seed 4T fermenter was loaded with 1.5T of feed liquid and fermented at 37°C for 9 to 12 h. The medium includes the following components: 100 g/L glucose, 4 g/L ammonium sulfate, 2 g/L corn steep liquor powder, 0.5 L defoamer, 0.01 g/L diammonium hydrogen phosphate, 60 mg/L ferrous sulfate, 0.4 g/L magnesium sulfate, 40 mg/L zinc sulfate, 40 mg/L copper sulfate, 0.5 g/L sodium chloride, and 0.05-0.46 g/L nutritional factors. The pH was adjusted to 4.2 with sulfuric acid. The pressure of the secondary fermenter was 0.12 MPa, the agitation speed was 120 rpm, and the aeration ratio was 0.3 to 0.35 vvm. The proportion of solid biomass before transfer was 9%.

The tertiary fermentation 20T fermenter was loaded with 13.6 T of feed liquid and fermented at 37°C. The medium includes the following components: 150 g/L glucose, 4 g/L ammonium sulfate, 2 g/L corn steep liquor powder, 8 L defoamer, 0.01 g/L diammonium hydrogen phosphate, 60 mg/L ferrous sulfate, 0.4 g/L magnesium sulfate, 40 mg/L zinc sulfate, 40 mg/L copper sulfate, 0.5 g/L sodium chloride, and 0.05-0.46 g/L nutritional factors (no pH adjustment was required). After seed transfer, the pH became approximately 3.5 after inoculation. The pressure of the fermenter was 0.08 to 0.1 MPa, the aeration rate was 180 $m^3$/h, the optimal aeration ratio was 0.11 to 0.13 vvm, the initial agitation speed was 60 rpm, and the DO level was maintained at 25 to 30% during agitation.

Fed-batch fermentation was performed once according to the change of residual sugar. If the residual sugar was lower than 80 g/L, glucose was supplemented in time to maintain fermentation until the yield of the main product TAA no longer increased, then fermentation was terminated.

**Example 18** Fermentation process in 20T fermenter - first-generation strains (unoptimized medium and process)

The primary seed 1T fermenter was loaded with 300 L of feed liquid and fermented at 37°C for 14 to 16 h. The medium includes the following components: 100 g/L glucose, 4 g/L ammonium sulfate, 1 g/L corn steep liquor powder, 100 mL defoamer, 0.01 g/L diammonium hydrogen phosphate, 60 mg/L ferrous sulfate, 0.4 g/L magnesium sulfate, 40 mg/L zinc sulfate, 40 mg/L copper sulfate, and 0.5 g/L sodium chloride. The pH was adjusted to 4.2 with sulfuric acid. The mixture was sterilized at 115 to 120°C for 15 to 20 min for later use. One or two hours before inoculation, wheat bran seeds were suspended with sterile distilled water, shaken at 100 rpm in a shaker after suspension, manually shaken during inoculation, and quickly poured into a flame inoculation port. The inoculum size was $1.6\times10^{14}$ spores, the fermenter pressure was 0.12 MPa, the agitation speed was 160 rpm, the aeration rate was 26 $m^3$/h, and the aeration ratio was 1.4 vvm.

The secondary seed 4T fermenter was loaded with 1.5T of feed liquid and fermented at 37°C for 9 to 12 h. The medium includes the following components: 100 g/L glucose, 4 g/L ammonium sulfate, 1 g/L corn steep liquor powder, 0.5 L defoamer, 0.01 g/L diammonium hydrogen phosphate, 60 mg/L ferrous sulfate, 0.4 g/L magnesium sulfate, 40 mg/L zinc sulfate, 40 mg/L copper sulfate, and 0.5 g/L sodium chloride. The pH was adjusted to 4.2 with sulfuric acid. The pressure of the secondary fermenter was 0.12 MPa, the agitation speed was 120 rpm, and the aeration ratio was 0.3 to 0.35 vvm. The proportion of solid biomass before transfer was 9%.

The tertiary fermentation 20T fermenter was loaded with 13.6 T of feed liquid and fermented at 37°C. The medium includes the following components: 100 g/L glucose, 3 g/L ammonium sulfate, 1 g/L corn steep liquor powder, and 8 L defoamer (no pH adjustment was required). Since the pH would drop below 4.0 when the secondary seeds were transferred, other components include: 0.2 g/L diammonium hydrogen phosphate, 10 mg/L ferrous sulfate, 0.4 g/L magnesium sulfate, 40 mg/L zinc sulfate, 40 mg/L copper sulfate, and 0.5 g/L sodium chloride. The pressure of the fermenter was 0.12 to 0.1 MPa, and the optimal aeration ratio was 0.14 to 0.16 vvm with an aeration rate of 180 $m^3$/h. After seed transfer, the pH became approximately 3.5 after inoculation, the agitation speed was 90 rpm, and there was no DO

control or agitation speed adjustment throughout the whole process.

The change of residual sugar was monitored at any time, and fed-batch fermentation was performed once according to the change of residual sugar. If the residual sugar was lower than 30 g/L, glucose was supplemented in time to maintain fermentation until the yield of the main product TAA no longer increased, then fermentation was terminated.

Comprehensive Analysis:

By comparing different aeration rates and different sugar feeding strategies in Examples 15, 16, and 17 (corresponding to Batches 1, 2, and 3 of the 20T fermenter), the optimal medium and fermentation process for production of aconitic acid in the 20T fermenter at the industrial pilot scale can be obtained.

Firstly, Example 16 and Example 15 are compared in terms of the process of controlling different DO through initial agitation, and results show that the yield of Example 16 reaches 52 g/L at 70 h, which is significantly higher than the yield of 42 g/L achieved by the high aeration process in Example 15.

Secondly, in Fig. 17, Example 17 and Example 16 are compared under the identical 20T fermentation process. A comparison shows that in Example 16, when the residual sugar is below 30 g/L at 72 h, sugar feeding fails to achieve the acid yield in the later stage (70 to 100 h). In contrast, in Example 17, when the glucose concentration reaches 80 g/L, sugar feeding effectively increases the acid yield in the later stage, resulting in a yield of 60 g/L, which is 15% higher than that of Example 16. The feeding process is further improved by advancing the sugar feeding time to 40 h, and it can be clearly seen that an increase in the sugar-acid conversion rate after 50 h is achieved in Example 17.

In addition, three batches of 20T fermentation (Examples 15 to 17) all use the optimized medium supplemented with nutritional factors, while the first-generation strains in the 20T fermentation (Example 18) use the original unoptimized fermentation process and medium, with a maximum *trans*-Aconitic acid yield of only 42 g/L. Industrial-scale trials in 20T fermenters have demonstrated that optimizing the medium with effective nutritional molecules screened in shake flasks can significantly increase the TAA yield in the fermentation production scale.

Example 19

This example provides a method for preparing pure tributyl aconitate, including the following steps:

(1) mixing 30 g of aconitic acid (the aconitic acid can be prepared by the above method (Examples 1 to 18)) and 51 g of n-butanol, and agitating to dissolve; after the aconitic acid is completely dissolved, adding 2.7 g of sodium bisulfate catalyst (with a purity of 99%), and performing esterification reaction at 125°C under a rotational speed of 300 rpm for 6 h, wherein during the reaction, water generated by esterification was separated in a timely manner, and after the reaction was completed, a solution containing aconitate was obtained; adding an equal volume of alkaline washing solution to the solution containing aconitate for neutralization reaction, repeating for three times, then taking out the upper organic phase, wherein the mass fraction of the solute in the alkaline washing solution is 5%, and the solute is $Na_2CO_3$, the neutralization temperature is 40°C, and the neutralization time is 20 min;

(2) adding hydrogen peroxide with a concentration of 35% and accounting for 3% of the weight of the upper organic phase to the upper organic phase, and performing first-step decolorization at 30°C for 15 min, then washing with clean water to remove hydrogen peroxide, residual alkaline washing solution, and salts generated by the alkaline washing reaction, performing phase separation to take the upper organic phase, and removing solvent at 70°C under a vacuum degree of 50 mbar until no distillate is evaporated, to obtain the crude aconitate product; and

(3) adding activated carbon with an iodine value of 810 and a methylene blue value of 12 to the crude aconitate product, and performing second-step decolorization at 30°C for 30 min, wherein the weight ratio of activated carbon to the crude aconitate product is 1:7; and then filtering to obtain a pure tributyl aconitate product, wherein the appearance diagram of the product is shown in Fig. 19, and the liquid chromatogram is shown in Fig. 23; the detection wavelength is 230 nm. For specific detection information, please refer to Table 2; Peak 6 is cis-tributyl aconitate, and Peak 8 is trans-tributyl aconitate; and the sum of the two peaks shows that the purity of the product is 99.818%, which is rounded to 99.8%.

Table 2

| Characteristic peak | Retention time | Area | Height | Area percentage(%) |
|---|---|---|---|---|
| 1 | 2.898 | 1632 | 367 | 0.015 |
| 2 | 3.209 | 3346 | 582 | 0.030 |
| 3 | 4.282 | 2300 | 288 | 0.021 |

(continued)

| Characteristic peak | Retention time | Area | Height | Area percentage(%) |
|---|---|---|---|---|
| 4 | 5.375 | 4709 | 673 | 0.043 |
| 5 | 6.114 | 1551 | 228 | 0.014 |
| 6 | 7.547 | 125317 | 14261 | 1.134 |
| 7 | 9.273 | 4619 | 449 | 0.042 |
| 8 | 11.021 | 10901651 | 880085 | 98.684 |
| 9 | 13.226 | 1933 | 130 | 0.017 |

Example 20

This example provides a method for preparing pure tri(2-ethylhexyl) aconitate, including the following steps:

(1) mixing 30 g of aconitic acid (the aconitic acid can be prepared by the above method), 112 g of 2-ethylhexanol, and 50 g of toluene as a water-carrying agent, and agitating to dissolve; after the aconitic acid is completely dissolved, adding 15 g of p-toluenesulfonic acid monohydrate catalyst (with a purity of 99%), and performing esterification reaction at 145°C under a rotational speed of 1200 rpm for 8 h, wherein during the reaction, water generated by esterification is separated in a timely manner; after the reaction is completed, a solution containing aconitate is obtained; adding an equal volume of alkaline washing solution to the solution containing aconitate for neutralization reaction, repeating for three times, and then taking out the upper organic phase, wherein the mass fraction of the solute in the alkaline washing solution is 5%, the solute is $Na_2CO_3$, the neutralization temperature is 40°C, and the neutralization time is 20 min.

(2) adding hydrogen peroxide with a concentration of 40% and accounting for 1% of the weight of the upper organic phase to the upper organic phase, and performing first-step decolorization at 50°C for 20 min, then washing with clean water to remove hydrogen peroxide, residual alkaline washing solution, and salts generated by the alkaline washing reaction, performing phase separation to take the upper organic phase, and removing solvent at 90°C under a vacuum degree of 10 mbar until no distillate is evaporated, to obtain the crude aconitate product; and

(3) adding activated carbon with an iodine value of 810 and a methylene blue value of 12 to the crude aconitate product, and performing second-step decolorization at 50°C for 40 min, wherein the weight ratio of activated carbon to the crude aconitate product is 1:9; and after filtration, a pure tri(2-ethylhexyl) aconitate product is obtained.

Example 21

This example provides a method for preparing pure triethyl aconitate, including the following steps:

(1) mixing 30 g of aconitic acid (the aconitic acid can be prepared by the above method), 71.4 g of ethanol, and 50 g of toluene as a water-carrying agent, and agitating to dissolve; after the aconitic acid is completely dissolved, adding 15 g of p-toluenesulfonic acid monohydrate catalyst (with a purity of 99%), and performing esterification reaction at 75°C under a rotational speed of 100 rpm for 2 h, wherein during the reaction, water generated by esterification is separated in a timely manner; after the reaction is completed, a solution containing aconitate is obtained; adding an equal volume of alkaline washing solution to the solution containing aconitate for neutralization reaction, repeating for three times, and then taking out the upper organic phase, wherein the mass fraction of the solute in the alkaline washing solution is 25%, the solute is NaOH, the neutralization temperature is 20°C, and the neutralization time is 30 min.

(2) adding hydrogen peroxide with a concentration of 33% and accounting for 5% of the weight of the upper organic phase to the upper organic phase, and performing first-step decolorization at 70°C for 30 min, then washing with clean water to remove hydrogen peroxide, residual alkaline washing solution, and salts generated by the alkaline washing reaction, performing phase separation to take the upper organic phase, and removing solvent at 40°C under a vacuum degree of 100 mbar until no distillate is evaporated, to obtain the crude aconitate product; and

(3) adding diatomite with a particle size of 200 mesh to the crude aconitate product, and performing second-step decolorization at 50°C for 120 min, wherein the weight ratio of diatomite to the crude aconitate product is 1:2; and filtering to obtain a pure tri(2-ethylhexyl) aconitate product.

Example 22

This example provides a method for preparing pure mixed aconitate, including the following steps:

(1) mixing 30 g of aconitic acid (the aconitic acid can be prepared by the above method), 25.5 g of n-butanol, and 22.4 g of 2-ethylhexanol, and agitating to dissolve; after the aconitic acid is completely dissolved, adding 2.7 g of sodium bisulfate catalyst (with a purity of 99%), and performing esterification reaction at 125°C under a rotational speed of 600 rpm for 6 h, wherein during the reaction, water generated by esterification is separated in a timely manner; and after the reaction is completed, a solution containing aconitate is obtained; adding an equal volume of alkaline washing solution to the solution containing aconitate for neutralization reaction, repeating for three times, and then taking out the upper organic phase, wherein the mass fraction of the solute in the alkaline washing solution is 5%, the solute is $NaHCO_3$, the neutralization temperature is 70°C, and the neutralization time is 15 min;
(2) adding hydrogen peroxide with a concentration of 35% and accounting for 3% of the weight of the upper organic phase to the upper organic phase, and performing first-step decolorization at 30°C for 15 min, then washing with clean water to remove hydrogen peroxide, residual alkaline washing solution, and salts generated by the alkaline washing reaction, performing phase separation to take the upper layer, and removing solvent at 100°C under a vacuum degree of 0.01 mbar until no distillate is evaporated, to obtain the crude aconitate product; and
(3) adding kaolin with a particle size of 300 mesh to the crude aconitate product, and performing second-step decolorization at 30°C for 10 min, wherein the weight ratio of kaolin to the crude aconitate product is 1:100; and filtering to obtain a pure mixed aconitate product.

Example 23

This example differs from Example 19 in that the concentration of hydrogen peroxide in step (2) is 30%, and the amounts of other substances and the steps are the same as those in Example 19.

Example 24

This example differs from Example 19 in that the concentration of hydrogen peroxide in step (2) is 45%, and the amounts of other substances and the steps are the same as those in Example 19.

Example 25

This example differs from Example 19 in that the weight of hydrogen peroxide in step (2) accounts for 8% of the weight of the upper organic phase, and the amounts of other substances and the steps are the same as those in Example 19.

Example 26

This example differs from Example 19 in that the weight of hydrogen peroxide in step (2) accounts for 0.5% of the weight of the upper organic phase, and the amounts of other substances and the steps are the same as those in Example 19.

Example 27

This example differs from Example 19 in that the decolorization temperature of hydrogen peroxide in step (2) is 80°C, and the amounts of other substances and the steps are the same as those in Example 19.

Example 28

This example differs from Example 19 in that the decolorization time of hydrogen peroxide in step (2) is 10 min, and the amounts of other substances and the steps are the same as those in Example 19.

Example 29

This example differs from Example 19 in that the weight ratio of activated carbon to the crude aconitate product in step (3) is 1:120, and the amounts of other substances and the steps are the same as those in Example 19.

Example 30

This example differs from Example 19 in that the decolorization temperature of activated carbon in step (3) is 80°C, and the amounts of other substances and the steps are the same as those in Example 19.

Example 31

This example differs from Example 19 in that the decolorization temperature of activated carbon in step (3) is 10°C, and the amounts of other substances and the steps are the same as those in Example 19.

Example 32

This example differs from Example 19 in that the decolorization time of activated carbon in step (3) is 5 min, and the amounts of other substances and the steps are the same as those in Example 19.

Example 33

This example differs from Example 19 in that the activated carbon used in step (3) has an iodine value of 700 and a methylene blue value of 10, and the amounts of other substances and the steps are the same as those in Example 19.

Example 34

This example differs from Example 19 in that in step (3), activated carbon is replaced with basic aluminum oxide having a particle size of 200 mesh, a specific surface area of 90 $m^2/g$, and an average pore diameter of 15 nm, and the amounts of other substances and the steps are the same as those in Example 19.

Example 35

This example differs from Example 19 in that the esterification reaction of raw materials in step (1) is performed at 200°C under a rotational speed of 300 rpm for 6 h, and the amounts of other substances and the steps are the same as those in Example 19.

Example 36

This example differs from Example 19 in that the neutralization temperature in step (1) is 80°C, and the amounts of other substances and the steps are the same as those in Example 19.

Comparative Example 1

This comparative example provides a method for preparing pure tributyl aconitate, including the following steps:

(1) mixing 30 g of aconitic acid (the aconitic acid can be prepared by the above method) and 51 g of n-butanol, and agitating to dissolve; after the aconitic acid is completely dissolved, adding 2.7 g of sodium bisulfate catalyst (with a purity of 99%), and performing esterification reaction at 125°C under a rotational speed of 300 rpm for 6 h, wherein during the reaction, water generated by esterification is separated in a timely manner, and after the reaction, a solution containing aconitate is obtained; adding an equal volume of alkaline washing solution to the aconitate-containing solution for neutralization reaction, repeating for three times, and then taking out the upper organic phase, wherein the mass fraction of the solute in the alkaline washing solution is 5%, the solute is $Na_2CO_3$, the neutralization temperature is 40°C, and the neutralization time is 20 min.

(2) removing solvent at 70°C under a vacuum degree of 50 mbar until no distillate is evaporated, so as to obtain the finished tributyl aconitate product. The appearance diagram of the product is shown in Fig. 18, and the liquid chromatogram is shown in Fig. 22, the detection wavelength is 230 nm. For specific detection information, please refer to Table 3, wherein Peak 16 is cis-tributyl aconitate and Peak 20 is trans-tributyl aconitate; and the sum of the two peaks shows that the purity of the product is 93.854%, which is rounded to 93.9%.

Table 3

| Characteristic peak | Retention time | Area | Height | Area percentage (%) |
|---|---|---|---|---|
| 1 | 0.155 | 2339 | 85 | 0.017 |
| 2 | 2.827 | 12665 | 449 | 0.092 |
| 3 | 3.068 | 4603 | 518 | 0.034 |
| 4 | 3.255 | 6118 | 734 | 0.045 |
| 5 | 3.434 | 24166 | 2430 | 0.176 |
| 6 | 3.769 | 207607 | 27079 | 1.514 |
| 7 | 3.944 | 116713 | 17615 | 0.851 |
| 8 | 4.417 | 245521 | 24341 | 1.791 |
| 9 | 4.675 | 127202 | 17374 | 0.928 |
| 10 | 5.145 | 18181 | 1637 | 0.133 |
| 11 | 5.438 | 17063 | 1547 | 0.124 |
| 12 | 5.786 | 4463 | 341 | 0.033 |
| 13 | 6.046 | 1020 | 114 | 0.007 |
| 14 | 6.668 | 4327 | 540 | 0.032 |
| 15 | 6.876 | 21662 | 1506 | 0.158 |
| 16 | 7.790 | 274719 | 25590 | 2.004 |
| 17 | 8.309 | 1357 | 111 | 0.010 |
| 18 | 8.637 | 3105 | 159 | 0.023 |
| 19 | 9.149 | 3712 | 191 | 0.027 |
| 20 | 11.322 | 12591176 | 858662 | 91.850 |
| 21 | 12.779 | 10440 | 595 | 0.076 |
| 22 | 13.595 | 10299 | 528 | 0.075 |

Comparative Example 2

This comparative example provides a method for preparing pure tributyl aconitate, including the following steps:

(1) this step is the same as that in Example 19;
(2) adding activated carbon with an iodine value of 810 and a methylene blue value of 12 to the upper organic phase, wherein the weight ratio of activated carbon to the upper organic phase is 1:7; performing first-step decolorization at 30°C for 30 min, then filtering and washing with clean water to remove residual alkaline washing solution and salts generated by the alkaline washing reaction; and removing solvent at 70°C under a vacuum degree of 50 mbar until no distillate is evaporated, so as to obtain the crude aconitate product; and
(3) adding hydrogen peroxide with a concentration of 35% and accounting for 3% of the weight of the crude aconitate product to the crude aconitate product, performing second-step decolorization at 30°C for 15 min, then washing with clean water to remove hydrogen peroxide, and removing solvent at 70°C under a vacuum degree of 50 mbar until no distillate is evaporated, so as to obtain the finished tributyl aconitate product.

Comparative Example 3

This comparative example provides a method for preparing pure tributyl aconitate, including the following steps:

(1) this step is the same as that in Example 19; and
(2) adding hydrogen peroxide with a concentration of 35% and accounting for 3% of the weight of the upper organic phase and activated carbon with an iodine value of 810 and a methylene blue value of 12 to the upper organic phase, wherein the weight ratio of activated carbon to the upper organic phase is 1:7; performing decolorization at 30°C for 30

min, and then filtering and washing with clean water to remove hydrogen peroxide, residual alkaline washing solution, and salts generated by the alkaline washing reaction, and removing solvent at 70°C under a vacuum degree of 50 mbar until no distillate is evaporated, so as to obtain the finished tributyl aconitate product.

Comparative Example 4

(1) 33 g of citric acid and 51 g of n-butanol were mixed and agitated to dissolve, wherein after the citric acid was completely dissolved, 2.7 g of sodium bisulfate catalyst (with a purity of 99%) was added, and esterification reaction was performed at 125°C under a rotational speed of 300 rpm for 8 h, wherein during the reaction, water generated by esterification was separated in a timely manner; and after the reaction, a solution containing citrate was obtained; an equal volume of alkaline washing solution was added to the citrate-containing solution for neutralization reaction, after repeating for three times, the upper organic phase was taken out, wherein the mass fraction of the solute in the alkaline washing solution is 5%, the solute is $Na_2CO_3$, the neutralization temperature is 40°C, and the neutralization time is 20 min; and
(2) solvent was removed at 70°C under a vacuum degree of 50 mbar until no distillate was evaporated, so as to obtain the finished tributyl citrate product, wherein the appearance diagram of the product is shown in Fig. 20.

Comparative Example 5

This comparative example differs from Comparative Example 4 in that in step (2), hydrogen peroxide with a concentration of 35% and accounting for 3% of the weight of the upper organic phase was added to the upper organic phase, and activated carbon with an iodine value of 810 and a methylene blue value of 12 was also added at the same time, wherein the weight ratio of activated carbon to the upper organic phase is 1:7. The mixture was decolorized at 30°C for 30 min, and then filtered and washed with clean water to remove hydrogen peroxide, residual alkaline washing solution, and salts generated by the alkaline washing reaction. Devolatilization at 70°C under a vacuum degree of 50 mbar was performed until no distillate is evaporated, so as to obtain the finished tributyl citrate product. The appearance diagram of the product is shown in Fig. 21.

Test Example 1

The aconitates and citrates prepared in the above examples and comparative examples were tested for acid value, purity, chromaticity, yield, triester conversion rate, and water content. The specific test results are shown in Table 4, and the test methods are as follows:

**1. Acid value:** 50 mL of ethanol was taken, 0.25 mL of phenolphthalein indicator solution was added, and two portions were prepared for later use. The mixture was neutralized with 0.02 $mol \cdot L^{-1}$ potassium hydroxide aqueous solution until a faint pink color appears, and the consumed volume Vo was recorded. 1 g of the sample (accurate to 0.01 g) was weighed using a ground-neck conical flask, then one portion of the above neutralized solution was added. After the sample was completely dissolved, the sample was titrated with 0.02 $mol \cdot L^{-1}$ potassium hydroxide-ethanol standard titration solution (the titration should be completed within 30 seconds) until a faint pink color appeared and persisted for 5 seconds without fading, which was considered as an end point. The consumed volume V was recorded.

$$\text{Calculation formula of acid value: } X=[(V-V_0)\times c\times M]/m;$$

where X is the acid value of the sample (calculated as KOH), in milligrams per gram ($mg \cdot g^{-1}$); V is the volume of the potassium hydroxide-ethanol standard titration solution consumed by the sample, in milliliters (mL); Vo is the volume of the potassium hydroxide-ethanol standard titration solution consumed in the blank test, in milliliters (mL); c is the exact concentration of the potassium hydroxide-ethanol standard titration solution, in moles per liter ($mol \cdot L^{-1}$); m is the mass of the sample, in grams (g); M is the molar mass of potassium hydroxide, in grams per mole ($g \cdot mol^{-1}$) [M=56.11].
**2. Purity:** a high-performance liquid chromatograph (HPLC) was used, and the sensitivity and stability of the detector should comply with the provisions of GB/T 16631-2008. Chromatographic column: C18 column; injector: 10 $\mu$L micro-syringe; solvent: acetonitrile (HPLC grade); acquisition time: 15 min; mobile phase: 80% acetonitrile (HPLC grade) and 20% deionized water; injection volume: 1 $\mu$L; wavelength: 230 nm.

Test: the instrument was adjusted according to the above provisions. After the baseline was stable, 0.2 μL of the sample was injected using a micro-syringe, and the integrator or chromatographic workstation was started at the same time. The instrument automatically gave the area percentage of each component.

Calculation formula of purity: purity=(integral area of aconitate/total integral area)×100%

**3. Chromaticity:** preparation of 500# platinum-cobalt colorimetric standard stock solution: 1.2450 g of potassium chloroplatinate and 1.0000 g of cobalt chloride were weighed, with an accuracy of 0.0002 g, the mixture was dissolved in 100 mL of hydrochloric acid, and then diluted to 1000 mL with distilled water. This solution is just the 500# colorimetric standard comparison solution.

Preparation of colorimetric standards below 500#: different amounts of the 500# stock solution was prepared, and diluted with distilled water to the volume of a colorimetric tube to prepare colorimetric standard solutions of any number. The calculation formula is as follows:

$$V=(N\times V_1)/500$$

In the formula, V is the volume of the 500# standard stock solution taken to prepare the N# colorimetric standard solution, in mL; $V_1$ is the volume of the colorimetric tube used, in mL; and N is the number of the colorimetric standard solution to be prepared.

Determination: after the sample was uniformly mixed, it was injected into a colorimetric tube and was observed with the naked eye; the sample should be a transparent oily liquid without turbidity and obvious mechanical impurities. Color comparison determination was performed at 25°C.

**4. Yield:** the yield is used to characterize the efficiency of the reaction synthesis route.

Yield calculation formula: yield=(mass of the finally obtained product/theoretical mass of aconitate) × 100%.

**5. Triester conversion rate:** the characteristic peak of aconitate (around 6.8) was analyzed through a nuclear magnetic spectrum. Triester conversion rate calculation formula:

triester conversion rate = (integral area of aconitic acid triester/total area of aconitic acid and aconitate (monoester, diester, triester) in this region)×100%.

**6. Water content:** a Karl Fischer moisture meter is used to test the water content (retained to two decimal places).

7. Residual amounts of fat-soluble pigments and water-soluble pigments:

a high-performance liquid chromatograph (HPLC) was used, and the instrument sensitivity and stability of the detector should comply with the provisions of GB/T 16631-2008. Chromatographic column: C18 column; injector: 10 μL micro-syringe; solvent: acetonitrile (HPLC grade); acquisition time: 15 min; mobile phase: 80% acetonitrile (HPLC grade) and 20% deionized water; injection volume: 1 μL; wavelength: 230 nm.

Test: the instrument was adjusted according to the above provisions. After the baseline was stable, 0.2 μL of the sample was injected using a micro-syringe, and the integrator or chromatographic workstation was started at the same time. A pigment concentration-integral area standard curve was established by testing the integral areas of pigments with different concentrations. When the pigment content was measured, the corresponding pigment integral area was automatically given by the instrument, and the pigment concentration was obtained by comparing with the standard curve. The ratio of the pigment concentration to the sample concentration is the pigment content.

Table 4

| Sample No. | Acid value (KOH/g ) | Purity (%) | Chromaticity (Pt-Co) | Yield (%) | Triester conversion rate (%) | Water content (%) | Residual amounts of fat-soluble pigments (ppm) | Residual amounts of water-soluble pigments (ppm) |
|---|---|---|---|---|---|---|---|---|
| Example 19 | 0.05 | 99.8 | 30 | 95.3 | >99% | 0.03 | 431 | 405 |

(continued)

| Sample No. | Acid value (KOH/g ) | Purity (%) | Chromaticity (Pt-Co) | Yield (%) | Triester conversion rate (%) | Water content (%) | Residual amounts of fat-soluble pigments (ppm) | Residual amounts of water-soluble pigments (ppm) |
|---|---|---|---|---|---|---|---|---|
| Example 20 | 0.05 | 99.4 | 30 | 94.6 | >99% | 0.05 | 634 | 610 |
| Example 21 | 0.05 | 99.6 | 30 | 96.1 | >99% | 0.02 | 385 | 328 |
| Example 22 | 0.05 | 99.1 | 40 | 94.7 | >99% | 0.04 | 715 | 681 |
| Example 23 | 0.05 | 97.6 | 60 | 94.1 | >99% | 0.03 | 1356 | 842 |
| Example 24 | 0.05 | 97.8 | 60 | 93.4 | >99% | 0.02 | 1386 | 420 |
| Example 25 | 0.05 | 96.2 | 50 | 92.3 | >99% | 0.03 | 865 | 454 |
| Example 26 | 0.05 | 96.6 | 70 | 94.5 | >99% | 0.04 | 2015 | 465 |
| Example 27 | 0.05 | 96.3 | 200 | 94.8 | >99% | 0.05 | 4650 | 415 |
| Example 28 | 0.05 | 98.8 | 40 | 95.2 | >99% | 0.04 | 1658 | 408 |
| Example 29 | 0.05 | 98.2 | 60 | 95.1 | >99% | 0.03 | 435 | 1302 |
| Example 30 | 0.05 | 97.4 | 65 | 94.3 | >99% | 0.04 | 463 | 1501 |
| Example 31 | 0.05 | 96.6 | 60 | 95.1 | >99% | 0.03 | 427 | 1715 |
| Example 32 | 0.05 | 97.1 | 60 | 95.4 | >99% | 0.02 | 382 | 1325 |
| Example 33 | 0.05 | 94.6 | 100 | 94.7 | >99% | 0.04 | 395 | 4064 |
| Example 34 | 0.05 | 99.3 | 50 | 94.8 | >99% | 0.03 | 428 | 864 |
| Example 35 | 0.05 | 89.8 | 300 | 86.4 | 92.7% | 0.03 | 3475 | 5348 |
| Example 36 | 0.05 | 97.2 | 60 | 82.6 | >99% | 0.04 | 836 | 1368 |
| Comparative Example 1 | 0.05 | 93.9 | >500 | 95.2 | >99% | 0.03 | 16424 | 13861 |
| Comparative Example 2 | 0.05 | 96.6 | 120 | 94.6 | >99% | 0.05 | 2406 | 2248 |
| Comparative Example 3 | 0.05 | 96.3 | 150 | 92.2 | >99% | 0.04 | 2864 | 2698 |
| Comparative Example 4 | 0.05 | 97.2 | 150 | 95.8 | >99% | 0.04 | 3054 | 2156 |
| Comparative Example 5 | 0.05 | 98.4 | 50 | 94.9 | >99% | 0.03 | 852 | 653 |

Combined with the data in Table 4, the comparison between Examples 23 to 26 and Example 19 shows that the concentration and dosage of hydrogen peroxide have a significant impact on the final chromaticity of the product, and they need to be within a reasonable range to achieve the condition that the chromaticity is lower than 50. The analysis shows that an excessive concentration or dosage of hydrogen peroxide will increase the chromaticity of the product, and more hydrogen peroxide will participate in the hydrolysis of aconitate. Although the reaction rate is very slow, the yield will be reduced. A low concentration or dosage of hydrogen peroxide will result in incomplete bleaching of the product pigments, failing to meet the pigment requirements.

The comparison between Examples 27 to 28 and Example 19 indicates that the higher the decolorization temperature of hydrogen peroxide, the more hydrogen peroxide will decompose, leading to a poor oxidation effect on fat-soluble pigments. As a result, the amount of residual fat-soluble pigments increases, thereby leading to an increase in chromaticity and a decrease in purity of aconitate. At the same time, the hydrolysis of aconitate by hydrogen peroxide is promoted, such that the yield of aconitate is decreased; shortened decolorization time of hydrogen peroxide will affect the damage effect of hydrogen peroxide on the main structures of fat-soluble pigments, further leading to an increase in

chromaticity and a decrease in purity of aconitate.

The comparison between Example 29 and Example 19 shows that when the dosage of activated carbon is reduced, the adsorption capacity for water-soluble pigments decreases. Therefore, the amount of residual water-soluble pigments increases, resulting in an increase in chromaticity and a decrease in purity of aconitate.

The comparison between Examples 30 to 31 and Example 19 shows that the decolorization temperature of the decolorizing agent affects the decolorization effect on water-soluble pigments. The higher the temperature, the more intense the Brownian motion of molecules, and the more difficult it is for the decolorizing agent to adsorb pigments, which instead leads to a decrease in the removal rate of water-soluble pigment impurities. The lower the temperature, the lower the adsorption capacity of the decolorizing agent for water-soluble pigments, and the higher the viscosity of aconitate, which is not conducive to uniform dispersion of the decolorizing agent, and will also lead to a decrease in the removal rate of water-soluble pigments, and further lead to a decrease in purity and an increase in chromaticity of aconitate.

The comparison between Example 32 and Example 19 shows that the shorter the decolorization time of the decolorizing agent, the worse the decolorization effect on water-soluble pigments, thereby leading to an increase in chromaticity and a decrease in purity of aconitate.

The comparison between Examples 23 to 34 and Example 19 shows that the type of decolorizing agent and the parameters of activated carbon influence the removal effect of water-soluble pigments, and further influence the purity and chromaticity of the aconitate product.

The comparison between Example 35 and Example 19 indicates that an increase in esterification temperature leads to the caramelization reaction of polysaccharides. Under the same decolorization conditions, the contents of the two types of pigments in the final aconitate increase, the product chromaticity increases and the purity decreases. Moreover, the monosaccharides produced by the decomposition of polysaccharides will compete with alcohols for the esterification reaction with aconitic acid, resulting in a decrease in the yield and triester conversion rate of aconitate.

The comparison between Example 36 and Example 19 shows that an increase in neutralization temperature causes hydrolysis of the product in an alkaline environment, then the purity of the obtained product is insufficient. The decrease in purity increases the relative content of pigments in the product, which further affects the one-step decolorization process and second-step decolorization process, then the amounts of residual fat-soluble pigments and water-soluble pigments are increased, the chromaticity of aconitate is increased, and the purity is decreased. Additionally, the loss due to high-temperature hydrolysis leads to a decrease in the yield of aconitate.

The comparison between Comparative Examples 1 to 3 and Example 19 shows that the aconitate obtained without decolorization treatment has higher contents of water-soluble pigments and fat-soluble pigments, the chromaticity of the product is more than 500, and the purity decreases. When water-soluble pigments are removed first and then fat-soluble pigments were removed, or when the two types of pigments are removed simultaneously, not only preparation and treatment processes will be increased, but also the removal effect of the two types of pigments will be influenced, thereby resulting in an increase of chromaticity and a decrease of purity of aconitate. Moreover, when fat-soluble pigments and water-soluble pigments are removed simultaneously, activated carbon will agglomerate in the water phase and distribute in the organic phase, resulting in poor decolorization effects of both types of pigments in the product, and the yield will decrease due to the aggregated activated carbon.

The comparison between Fig. 18-4, Comparative Example 4, and Comparative Example 5 shows that citrate itself has a low pigment content, so it is less difficult to decolorize, unlike aconitate which is difficult to decolorize. Although the chromaticity of citrate decreases before and after decolorization, the range of chromaticity decrease after decolorization is much smaller than that of the product in the present disclosure.

Test Example 2

The aconitates and tributyl citrate prepared in the above examples and comparative examples were added as plasticizers to PVC resin to obtain PVC plastics. The PVC plastics include 100 parts of PVC resin, 35 parts of plasticizer, 2 parts of oxidized polyethylene wax, and 5 parts of octyltin maleate. The above raw materials were mixed and prepared using the same mixing process to obtain finished PVC products. The performance tests were performed on different plasticizers and the obtained PVC finished products in the above examples and comparative examples. The results are shown in Table 5, and the test methods are as follows:

**1. Hardness:** samples were prepared and tested in accordance with GB/T 531-2008 *Indentation Hardness Test Method and Shore Hardness Tester Method.* Three points were selected for each group of samples, each point was tested once, and a median value was taken.

**2. Tensile modulus at 100%, elongation at break, elastic modulus:** in accordance with GB/T 1040-2006 *Determination of Tensile Properties of Plastics,* tests were performed at a tensile speed of 50 mm/min and a temperature of 25°C.

**3. Compatibility:** tests were performed using a water value method. Since water molecules are polar, the compatibility between the plasticizer and water can characterize the compatibility between the plasticizer and polar materials (PVC, PLA, and nitrile rubber). When a certain plasticizer has good compatibility with water, it has good compatibility with PVC, and vice versa. In the experiment, 2.5 g of plasticizer and 25 g of acetone were added to a beaker, titrated with a burette filled with deionized water, and agitated while titrating until turbidity appears, and the volume of deionized water consumed was recorded.

**4. Glass transition temperature ($T_g$):** the glass transition temperature was tested using a dynamic mechanical analyzer. Test conditions: nitrogen was taken as the carrier gas, at a test temperature range of -40 to 80°C, a heating rate of 3°C/min, and a frequency of 1 Hz.

Table 5

| Sample No. | Hardness/Shore D | Tensile modulus at 100% (MPa) | Elongation at break (%) | Elastic modulus (MPa) | Compatibility (mL) | $T_g$ (°C) |
|---|---|---|---|---|---|---|
| Example 19 | 41 | 14.6 | 260.1 | 74.6 | 16.7 | 27.6 |
| Example 20 | 54 | 19.7 | 181.6 | 301.6 | 10.7 | 42.5 |
| Example 21 | 43 | 15.8 | 243.6 | 135.5 | 18.4 | 28.2 |
| Example 22 | 46 | 17.9 | 222.1 | 198.6 | 14.7 | 34.7 |
| Example 23 | 43 | 15.6 | 239.4 | 128.7 | 13.1 | 28.4 |
| Example 24 | 43 | 15.7 | 242.7 | 131.0 | 13.1 | 28.6 |
| Example 25 | 45 | 17.2 | 226.5 | 156.4 | 12.7 | 28.2 |
| Example 26 | 45 | 17.5 | 234.1 | 178.3 | 12.2 | 28.8 |
| Example 27 | 45 | 17.3 | 230.3 | 166.7 | 12.4 | 28.5 |
| Example 28 | 42 | 15.2 | 248.6 | 88.5 | 14.4 | 28.6 |
| Example 29 | 42 | 15.4 | 251.6 | 92.6 | 14.9 | 28.8 |
| Example 30 | 43 | 15.8 | 241.7 | 134.8 | 13.2 | 28.9 |
| Example 31 | 45 | 17.2 | 231.5 | 157.6 | 12.5 | 29.6 |
| Example 32 | 43 | 15.6 | 241.2 | 133.4 | 13.1 | 28.7 |
| Example 33 | 47 | 18.2 | 223.7 | 204.5 | 11.3 | 30.8 |
| Example 34 | 41 | 14.7 | 258.1 | 77.9 | 16.1 | 27.7 |
| Example 35 | 55 | 19.8 | 173.4 | 348.6 | 8.1 | 38.4 |
| Example 36 | 43 | 15.9 | 243.4 | 137.4 | 13.4 | 29.7 |
| Comparative Example 1 | 46 | 17.9 | 223.2 | 192.2 | 10.8 | 30.7 |
| Comparative Example 2 | 45 | 17.4 | 231.4 | 187.3 | 12.5 | 28.4 |
| Comparative Example 3 | 45 | 17.4 | 233.6 | 186.7 | 12.5 | 28.7 |
| Comparative Example 4 | 51 | 18.1 | 201.3 | 274.8 | 13.9 | 39.7 |
| Comparative Example 5 | 48 | 17.6 | 213.4 | 226.4 | 14.3 | 37.8 |

It can be seen from the contents in Table 5 that the purity of aconitate has a significant impact on its performance as a plasticizer. The lower the purity of aconitate, the higher the hardness, $T_g$, tensile modulus at 100%, and elastic modulus of the composite material, and the worse the elongation at break and compatibility. Especially through a comparison before and after decolorization, the hardness decreases by 12% and the toughness decreases by more than 14%. Therefore, decolorization can not only improve the chromaticity quality of the product itself, but also the purification effect after decolorization can enhance the plasticizing performance of aconitate on PVC.

Example 37

This example relates to a high cold-resistant PVC composite material and its preparation method. The composite includes the following components in parts by weight: 100 parts of PVC resin, 50 parts of triethyl aconitate, 5 parts of CPE, 4 parts of heat stabilizer, 1 part of UV-327 light stabilizer, and 1 part of ethylene bis-stearamide synthetic wax lubricant. The heat stabilizer is calcium stearate and zinc stearate in a weight ratio of 2:1. The triethyl aconitate has an acid value of 0.03 mgKOH/g, a water content of 0.05%, and a purity of 97.5%. The chlorine content of CPE is 30%. The degree of polymerization of PVC resin is 1251 to 1370, the polydispersity index is 4, and the value of K is 71. The structural formula of triethyl aconitate is as follows:

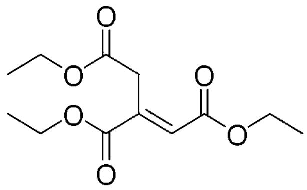

.

The preparation method of this composite material includes the following steps:

S1: mixing triethyl aconitate, PVC resin, stabilizer, and lubricant to obtain a premix;
S2: adding the premix to a high-speed mixer, mixing at 60°C for 40 min, then adding to two-roll open mixing mill, and mixing at 155°C for 20 min to obtain a mixed compound; and
S3: placing the mixed compound in a plate vulcanizer with a press gauge pressure of 10 MPa, and vulcanizing at 165°C for 15 min to obtain the product, wherein the SEM image of this composite material is shown in Fig. 24.

Example 38

This example differs from Example 37 in that tripropyl aconitate is used. The structural formula of tripropyl aconitate is as follows:

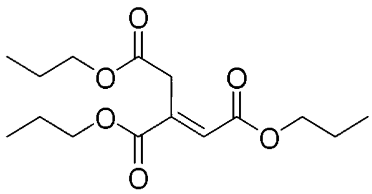

;

the tripropyl aconitate has an acid value of 0.07 mgKOH/g, a water content of 0.06%, and a purity of 98%. The other components and preparation method are the same as those in Example 37. The SEM image of this composite material is shown in Fig. 25.

Example 39

This example differs from Example 37 in that tributyl aconitate is used. The structural formula of tributyl aconitate is as follows:

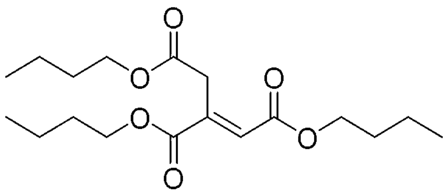

;

the tributyl aconitate has an acid value of 0.005 mgKOH/g, a water content of 0.007%, and a purity of 99%. The other components and preparation method are the same as those in Example 37. The SEM image of this composite material is shown in Fig. 26.

Example 40

This example differs from Example 37 in that trimethyl aconitate is used. The structural formula of trimethyl aconitate is as follows:

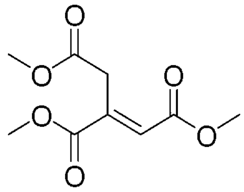

;

the trimethyl aconitate has an acid value of 0.02 mgKOH/g, a water content of 0.03%, and a purity of 98%. The other components and preparation method are the same as those in Example 37.

Example 41

This example differs from Example 37 in that tripentyl aconitate is used. The structural formula of tripentyl aconitate is as follows:

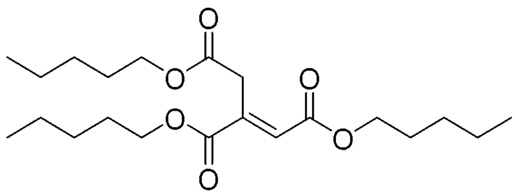

;

the tripentyl aconitate has an acid value of 0.07 mgKOH/g, a water content of 0.07%, and a purity of 97.5%. The other components and preparation method are the same as those in Example 37.

Example 42

This example differs from Example 37 in that triethyl aconitate and tributyl aconitate in a weight ratio of 1:1 is used. The parameters of the triethyl aconitate and tributyl aconitate are the same as those in Example 37 and Example 39. The other components and preparation method are the same as those in Example 37.

Example 43

This example differs from Example 37 in that the triethyl aconitate has an acid value of 1.5 mgKOH/g. The other components and preparation method are the same as those in Example 37.

Example 44

This example differs from Example 37 in that the water content of triethyl aconitate is 2%. The other components and preparation method are the same as those in Example 37.

Example 45

This example differs from Example 37 in that the purity of triethyl aconitate is 90%. The other components and preparation method are the same as those in Example 37.

Example 46

This example differs from Example 37 in that the degree of polymerization of PVC is 1900 to 1950. The other components and preparation method are the same as those in Example 37.

Example 47

This example differs from Example 37 in that the degree of polymerization of PVC is 450 to 500. The other components and preparation method are the same as those in Example 37.

Example 48

This example differs from Example 37 in that the polydispersity index of PVC is 5.5. The other components and preparation method are the same as those in Example 37.

Example 49

This example differs from Example 37 in that the polydispersity index of PVC is 2.5. The other components and preparation method are the same as those in Example 37.

Example 50

This example differs from Example 37 in that 1 part of CPE is used, and the chlorine content of CPE is 20%. The other components and preparation method are the same as those in Example 37.

Example 51

This example differs from Example 37 in that 10 parts of EVA are used as a cold-resistant modifier, and the content of VA in EVA is 30%. The other components and preparation method are the same as those in Example 37.

Example 52

This example differs from Example 37 in that oxidized polyethylene wax is used as the lubricant. The other components and preparation method are the same as those in Example 37.

Example 53

This example differs from Example 37 in that 2 parts of heat stabilizer and 3 parts of UV-327 light stabilizer are used. The composition of the heat stabilizer and the other components and preparation method are the same as those in Example 37.

Example 54

This example differs from Example 37 in that the heat stabilizer is calcium stearate and zinc stearate in a weight ratio of 8:1. The other components and preparation method are the same as those in Example 37.

Comparative Example 6

This comparative example differs from Example 37 in that dioctyl phthalate is used to replace triethyl aconitate. The other components and preparation method are the same as those in Example 37. The SEM image of this composite material is shown in Fig. 27.

Comparative Example 7

This comparative example differs from Example 37 in that tri-n-butyl acetylcitrate is used to replace triethyl aconitate. The other components and preparation method are the same as those in Example 37. The SEM image of this composite material is shown in Fig. 28.

Comparative Example 8

This comparative example differs from Example 37 in that dibutyl phthalate is used to replace triethyl aconitate. The other components and preparation method are the same as those in Example 37. The SEM image of this composite material is shown in Fig. 29.

Comparative Example 9

This comparative example differs from Example 37 in that dioctyl terephthalate is used to replace triethyl aconitate. The other components and preparation method are the same as those in Example 37. The SEM image of this composite material is shown in Fig. 30.

Comparative Example 10

This comparative example differs from Example 37 in that epoxidized soybean oil is used to replace triethyl aconitate. The other components and preparation method are the same as those in Example 37.

Comparative Example 11

This comparative example differs from Example 37 in that trihexyl aconitate is used to replace triethyl aconitate. The structural formula of trihexyl aconitate is as follows:

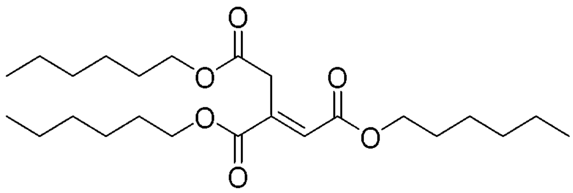
,

the other components and preparation method are the same as those in Example 37.

Comparative Example 12

This comparative example differs from Example 37 in that dibutyl mono-isooctyl aconitate is used to replace triethyl aconitate. The structural formula of dibutyl mono-isooctyl aconitate is as follows:

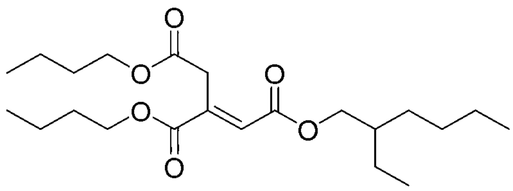
,

the other components and preparation method are the same as those in Example 37.

Comparative Example 13

This comparative example differs from Example 37 in that no CPE is contained. The other components and preparation method are the same as those in Example 37.

Comparative Example 14

This comparative example differs from Example 37 in that 4 parts of triethyl aconitate are used. The other components and preparation method are the same as those in Example 37.

Test 3

Performance tests were performed on the composite materials prepared in the above examples and comparative examples. The test methods are the same as those in Test 2, and the results are shown in Tables 6 and 7.

Table 6

| Sample No. | Hardness/Shore A | Tensile modulus at 100% (MPa) | Elongation at break (%) | Elastic modulus (MPa) | Compatibility (mL) |
|---|---|---|---|---|---|
| Example 37 | 60 | 11.1 | 757.5 | 268.5 | 18.41 |
| Example 38 | 57 | 10.3 | 868.8 | 226.7 | 17.25 |
| Example 39 | 42 | 8.2 | 1103.5 | 130.5 | 16.75 |
| Example 40 | 61 | 12.7 | 648.8 | 326.2 | 19.75 |
| Example 41 | 62 | 11.2 | 863.5 | 263.5 | 14.86 |
| Example 42 | 51 | 9.7 | 869.7 | 212.1 | 17.71 |
| Example 43 | 63 | 13.6 | 655.3 | 288.2 | 17.43 |
| Example 44 | 62 | 13.3 | 667.9 | 307.7 | 16.72 |
| Example 45 | 66 | 14.7 | 629.1 | 340.5 | 17.65 |
| Example 46 | 67 | 15.2 | 408.8 | 642.5 | 18.41 |
| Example 47 | 55 | 9.6 | 926.2 | 174.4 | 18.41 |
| Example 48 | 63 | 13.4 | 642.9 | 292.3 | 18.41 |
| Example 49 | 63 | 13.6 | 646.3 | 293.8 | 18.41 |
| Example 50 | 65 | 14.4 | 703.8 | 301.4 | 18.41 |
| Example 51 | 63 | 13.4 | 726.3 | 283.1 | 18.41 |
| Example 52 | 60 | 10.4 | 655.3 | 318.7 | 18.41 |
| Example 53 | 60 | 10.5 | 649.8 | 317.8 | 18.41 |
| Example 54 | 62 | 13.2 | 639.4 | 342.1 | 18.41 |
| Comparative Example 6 | 78 | 15.1 | 504.3 | 577.1 | 7.1 |
| Comparative Example 7 | 75 | 13.4 | 543.7 | 492.2 | 14.35 |
| Comparative Example 8 | 74 | 13.2 | 562.4 | 466.4 | 13.5 |
| Comparative Example 9 | 79 | 16.3 | 455.8 | 601.5 | 7.4 |
| Comparative Example 10 | 88 | 18.2 | 373.6 | 845.0 | 4.6 |
| Comparative Example 11 | 64 | 12.1 | 698.9 | 359.3 | 13.39 |
| Comparative Example 12 | 53 | 10.7 | 841.2 | 233.2 | 15.2 |
| Comparative Example 13 | 55 | 9.6 | 927.2 | 175.8 | 18.41 |
| Comparative Example 14 | 84 | 17.6 | 403.4 | 798.1 | 18.41 |

Table 7

| Sample No. | $T_g$ (°C) | -5°C Hardness/Shore D | After conversion-5°C Hardness/Shore A | -5°C Elastic modulus (MPa) |
|---|---|---|---|---|
| Example 37 | 23.3 | 57 | 107 | 1131.3 |
| Example 38 | 15.3 | 54 | 104 | 1078.1 |
| Example 39 | 5.6 | 39 | 89 | 1031.1 |
| Example 40 | 27.5 | 58 | 108 | 1232.8 |
| Example 41 | 18.6 | 59 | 109 | 1160.1 |
| Example 42 | 13.7 | 48 | 98 | 1087.7 |
| Example 43 | 24.1 | 62 | 112 | 1144.1 |

(continued)

| Sample No. | $T_g$ (°C) | -5°C Hardness/Shore D | After conversion-5°C Hardness/Shore A | -5°C Elastic modulus (MPa) |
|---|---|---|---|---|
| Example 44 | 25.7 | 64 | 114 | 1162.4 |
| Example 45 | 28.3 | 66 | 116 | 1192.7 |
| Example 46 | 28.6 | 67 | 117 | 1446.8 |
| Example 47 | 24.7 | 56 | 106 | 1049.9 |
| Example 48 | 25.1 | 65 | 115 | 1151.4 |
| Example 49 | 25.3 | 63 | 113 | 1147.7 |
| Example 50 | 29.1 | 68 | 118 | 1497.4 |
| Example 51 | 23.8 | 60 | 110 | 1182.1 |
| Example 52 | 23.4 | 57 | 107 | 1172.7 |
| Example 53 | 23.3 | 57 | 107 | 1171.2 |
| Example 54 | 23.9 | 61 | 111 | 1194.5 |
| Comparative Example 6 | 43.3 | 83 | 133 | 1392.9 |
| Comparative Example 7 | 30.6 | 78 | 128 | 1321.2 |
| Comparative Example 8 | 32.6 | 76 | 126 | 1297.4 |
| Comparative Example 9 | 40.3 | 84 | 134 | 1414.5 |
| Comparative Example 10 | 51.4 | 93 | 143 | 1618.1 |
| Comparative Example 11 | 28.7 | 63 | 113 | 1208 |
| Comparative Example 12 | 18.1 | 53 | 103 | 1232.5 |
| Comparative Example 13 | 28.1 | 59 | 109 | 1196.1 |
| Comparative Example 14 | 32.4 | 89 | 139 | 1329.4 |

It can be seen from the contents in Tables 6 and 7 that the short-chain aconitate has good compatibility with the material and can maintain good plasticizing effect at low temperatures. When the indicators of aconitic acid fail to meet the patent requirements, its plasticizing performance will be significantly reduced especially at low temperatures. In addition, compared with composite materials plasticized by other commonly used commercial plasticizers, the PVC materials formed by compounding with the aconitate used in the present disclosure have obvious advantages in compatibility and low-temperature performance.

The comparison between Example 43 and Example 37 shows that the higher the acid value of the single aconitate, the more acidic substances will be generated during the processing of the composite material, so as to corrode and degrade the composite material. As a result, the hardness, tensile modulus at 100%, and elastic modulus of the composite material increase, while the elongation at break, compatibility, and cold resistance decrease.

The comparison between Example 44 and Example 37 shows that an increase in the water content of the single aconitate will reduce the plasticizing effect of the single aconitate on the PVC resin and its synergistic effect with the cold-resistant modifier. This leads to an increase in the hardness, tensile modulus at 100%, and elastic modulus of the composite material, but a decrease in the elongation at break and cold resistance.

The comparison between Example 45 and Example 37 shows that a decrease in the purity of the single aconitate leads to a higher content of impurities introduced into the composite material. Therefore, the contact between the single aconitate and PVC as well as the cold-resistant modifier is reduced, which lowers the plasticizing performance of the composite material and decreases the cold resistance.

The comparison between Examples 46 to 47 and Example 37 shows that the higher the degree of polymerization of PVC, the higher the hardness, tensile modulus at 100%, and elastic modulus of the composite material, the lower the elongation at break, and the worse the cold resistance. A higher degree of polymerization leads to more entanglement between molecular chains and reduced mobility of molecular chains, and results in less modification of the PVC material by the plasticizer and cold-resistant modifier. However, the compatibility with the single aconitate remains unchanged. Therefore, when the degree of polymerization of PVC is in a range of 650 to 1800, PVC can react to cross-link with the single aconitate, cold-resistant modifier, lubricant, and stabilizer components to form a stable cross-linked network

structure, thereby endowing the composite material with better toughness, plasticity, tensile resistance, and cold resistance.

The comparison between Examples 49 to 50 and Example 37 shows that when the polydispersity index of the PVC resin is in a range of 3 to 5, PVC resin materials with different molecular weights can contact the single aconitate, and the synergistic effect between the single aconitate and the cold-resistant modifier is improved, to effectively enhance the toughness and cold resistance of the composite material.

The comparison between Examples 50 to 51, Comparative Examples 13 to 14, and Example 37 indicates that when no cold-resistant modifier is added, the relative proportion of the plasticizer increases. Although the plasticizing performance improves, the cold resistance of the composite material decreases. The type, parameters, and dosage of the cold-resistant modifier can affect the plasticizing effect of the single aconitate on the composite material and change the cold resistance of the composite material. The dosages of the cold-resistant modifier and the single aconitate can cooperate with each other to achieve a technical effect of 1+1>2.

The comparison between Examples 52 to 54 and Example 37 shows that the type and ratio of the lubricant and heat stabilizer both affect the plasticizing effect of the single aconitate on the composite material, and also affect the synergistic effect of the cold-resistant modifier and the single aconitate in improving the cold resistance of the composite material.

The comparison between Comparative Examples 6 to 12 and Example 37 shows that other types of plasticizers have poor synergistic effects with cold-resistant modifiers. An increase in the number of carbon atoms in R of the single aconitate will reduce the synergistic effect between the single aconitate and the cold-resistant modifier, and also reduce the compatibility between the single aconitate and PVC and its plasticizing effect on the composite material.

Example 55

Unless otherwise specified, the aconitic acid used in the mixed aconitates in the following embodiments of the present disclosure is the *trans*-Aconitic acid prepared by the fermentation method in the above examples.

Mixed aconitate 1#

The preparation method of mixed aconitate 1# includes the following steps:

S1: mixing aconitic acid, n-butanol, and isooctanol at a molar ratio of 1:6:3, adding sodium bisulfate catalyst, and performing esterification reaction at 125°C under a rotational speed of 300 rpm for 6 h to obtain a mixed solution containing aconitate, wherein the weight ratio of sodium bisulfate to aconitic acid is 1:10;
S2: neutralizing the mixed solution containing aconitate with an equal volume of $Na_2CO_3$ alkaline solution at 40°C for 20 min, wherein the mass fraction of the solute in the alkaline solution is 5%; after repeating for three times, performing phase separation to obtain an organic phase A; then using hydrogen peroxide with a concentration of 35% to bleach and decolorize the organic phase A at 30°C for 15 min, wherein the weight of hydrogen peroxide accounts for 3% of the weight of the organic phase A; and after washing with water, performing phase separation to obtain an organic phase B;
S3: performing solvent removal on the organic phase B at 70°C under a vacuum degree of 50 mbar until no distillate is evaporated, so as to obtain a purified product; and
S4: adding activated carbon decolorizing agent with an iodine value of 810 and a methylene blue value of 12 to the purified product, and performing adsorption decolorization at 30°C for 30 min, wherein the weight ratio of activated carbon to the purified product is 1:7; and filtering to obtain mixed aconitate 1#.

Mixed aconitate 2#

The preparation method of mixed aconitate 2# differs from the preparation method of mixed aconitate 1# in that in step S1, aconitic acid, n-butanol, and isooctanol at a molar ratio of 1:3:6 are mixed for the esterification reaction. The other steps and the amounts of substances are the same as those of mixed aconitate 1#, and the mixed aconitate 2# is obtained.

Mixed aconitate 3#

The preparation method of mixed aconitate 3# includes the following steps:

S1: mixing aconitic acid, ethanol, and benzyl alcohol at a molar ratio of 1:4:2, adding ferric sulfate catalyst, and performing esterification reaction at 145°C under a rotational speed of 100 rpm for 8 h to obtain a mixed solution containing aconitate, wherein the weight ratio of ferric sulfate to aconitic acid is 1:2;

S2: neutralizing the mixed solution containing aconitate with an equal volume of NaOH alkaline solution at 20°C for 30 min, wherein the mass fraction of the solute in the alkaline solution is 15%; after repeating for three times, performing phase separation to obtain an organic phase A; then using hydrogen peroxide with a concentration of 33% to bleach and decolorize the organic phase A at 20°C for 30 min, wherein the weight of hydrogen peroxide accounts for 5% of the weight of organic phase A; and after washing with water, performing phase separation to obtain an organic phase B;
S3: performing solvent removal on the organic phase B at 40°C under a vacuum degree of 100 mbar until no distillate is evaporated, so as to obtain a purified product; and
S4: adding activated carbon decolorizing agent with an iodine value of 810 and a methylene blue value of 12 to the purified product, and performing adsorption decolorization at 70°C for 10 min, wherein the weight ratio of activated carbon to the purified product is 1:2; and filtering to obtain the mixed aconitate 3#.

Mixed aconitate 4#

The preparation method of mixed aconitate 4# differs from the preparation method of mixed aconitate 3# in that in step S1, aconitic acid, ethanol, and benzyl alcohol at a molar ratio of 1:2:4 are mixed for the esterification reaction. The other steps and the amounts of substances are the same as those of mixed aconitate 3#, and the mixed aconitate 4# is obtained.

Mixed aconitate 5#

The preparation method of mixed aconitate 5# includes the following steps:

S1: mixing aconitic acid, isooctanol, and chloroethanol at a molar ratio of 1:2:1, adding p-toluenesulfonic acid monohydrate catalyst and toluene water-carrying agent, and performing esterification reaction at 75°C under a rotational speed of 1200 rpm for 2 h to obtain a mixed solution containing aconitate, wherein the weight ratio of p-toluenesulfonic acid monohydrate to aconitic acid is 1:100, and the molar ratio of toluene water-carrying agent to aconitic acid is 5:1;
S2: neutralizing the mixed solution containing aconitate with an equal volume of $NaHCO_3$ alkaline solution at 70°C for 15 min, wherein the mass fraction of the solute in the alkaline solution is 25%.; after repeating for three times, performing phase separation to obtain an organic phase A; then using hydrogen peroxide with a concentration of 40% to bleach and decolorize the organic phase A at 70°C for 15 min, wherein the weight of hydrogen peroxide accounts for 1% of the weight of organic phase A; and after washing with water, performing phase separation to obtain an organic phase B;
S3: performing solvent removal on organic phase B at 100°C under a vacuum degree of 0.01 mbar until no distillate is evaporated, so as to obtain a purified product; and
S4: adding activated carbon decolorizing agent with an iodine value of 810 and a methylene blue value of 12 to the purified product, and performing adsorption decolorization at 20°C for 120 min, wherein the weight ratio of activated carbon to the purified product is 1:100; and filtering to obtain the mixed aconitate 5#.

Mixed aconitate 6#

The preparation method of mixed aconitate 6# differs from the preparation method of mixed aconitate 5# in that in step S1, aconitic acid, isooctanol, and chloroethanol at a molar ratio of 1:1:2 are mixed for the esterification reaction. The other steps and the amounts of substances are the same as those of mixed aconitate 5#, and the mixed aconitate 6# is obtained.

Mixed aconitate 7#

The preparation method of mixed aconitate 7# differs from the preparation method of mixed aconitate 1# in that in step S1, aconitic acid, n-butanol, isooctanol, and 1-chloro-2-propanol at a molar ratio of 1:3:3:3 are mixed for the esterification reaction. The other steps and the amounts of substances are the same as those of mixed aconitate 1#, and the mixed aconitate 7# is obtained.

Mixed aconitate 8#

The preparation method of mixed aconitate 8# differs from the preparation method of mixed aconitate 1# in that in step S1, aconitic acid, n-butanol, isooctanol, and phenethyl alcohol at a molar ratio of 1:3:3:3 are mixed for the esterification reaction. The other steps and the amounts of substances are the same as those of mixed aconitate 1#,

and the mixed aconitate 8# is obtained.

Mixed aconitate 9#

The preparation method of mixed aconitate 9# differs from the preparation method of mixed aconitate 1# in that in step S1, aconitic acid, benzyl alcohol, and 3-chloro-1-propanol at a molar ratio of 1:3:6 are mixed for the esterification reaction. The other steps and the amounts of substances are the same as those of mixed aconitate 1#, and the mixed aconitate 9# is obtained.

Mixed aconitate 10#

The preparation method of mixed aconitate 10# differs from the preparation method of mixed aconitate 3# in that in step S1, aconitic acid, ethanol, and chloroethanol at a molar ratio of 1:2:4 are mixed for the esterification reaction. The other steps and the amounts of substances are the same as those of mixed aconitate 3#, and the mixed aconitate 10# is obtained.

Mixed aconitate 11#

The preparation method of mixed aconitate 11# differs from the preparation method of mixed aconitate 5# in that in step S1, aconitic acid, isooctanol, and phenethyl alcohol at a molar ratio of 1:1:2 are mixed for the esterification reaction. The other steps and the amounts of substances are the same as those of mixed aconitate 5#, and the mixed aconitate 11# is obtained.

Mixed aconitate 12#

The preparation method of mixed aconitate 12# differs from the preparation method of mixed aconitate 1# in that the concentration of hydrogen peroxide in step S2 is 30%. The other steps and the amounts of substances are the same as those of mixed aconitate 1#, and the mixed aconitate 12# is obtained.

Mixed aconitate 13#

The preparation method of mixed aconitate 13# differs from the preparation method of mixed aconitate 1# in that the concentration of hydrogen peroxide in step S2 is 45%. The other steps and the amounts of substances are the same as those of mixed aconitate 1#, and the mixed aconitate 13# is obtained.

Mixed aconitate 14#

The preparation method of mixed aconitate 14# differs from the preparation method of mixed aconitate 1# in that the weight of hydrogen peroxide in step S2 accounts for 8% of the weight of organic phase A. The other steps and the amounts of substances are the same as those of mixed aconitate 1#, and the mixed aconitate 14# is obtained.

Mixed aconitate 15#

The preparation method of mixed aconitate 15# differs from the preparation method of mixed aconitate 1# in that the weight of hydrogen peroxide in step S2 accounts for 0.5% of the weight of organic phase A. The other steps and the amounts of substances are the same as those of mixed aconitate 1#, and the mixed aconitate 15# is obtained.

Mixed aconitate 16#

The preparation method of mixed aconitate 16# differs from the preparation method of mixed aconitate 1# in that the decolorization temperature of hydrogen peroxide in step S2 is 80°C. The other steps and the amounts of substances are the same as those of mixed aconitate 1#, and the mixed aconitate 16# is obtained.

Mixed aconitate 17#

The preparation method of mixed aconitate 17# differs from the preparation method of mixed aconitate 1# in that the decolorization time of hydrogen peroxide in step S2 is 10 min. The other steps and the amounts of substances are the same as those of mixed aconitate 1#, and the mixed aconitate 17# is obtained.

Mixed aconitate 18#

The preparation method of mixed aconitate 18# differs from the preparation method of mixed aconitate 1# in that the weight ratio of activated carbon to the purified product in step S4 is 1:120. The other steps and the amounts of substances are the same as those of mixed aconitate 1#, and the mixed aconitate 18# is obtained.

Mixed aconitate 19#

The preparation method of mixed aconitate 19# differs from the preparation method of mixed aconitate 1# in that the decolorization temperature of activated carbon in step S4 is 80°C. The other steps and the amounts of substances are the same as those of mixed aconitate 1#, and the mixed aconitate 19# is obtained.

Mixed aconitate 20#

The preparation method of mixed aconitate 20# differs from the preparation method of mixed aconitate 1# in that the decolorization temperature of activated carbon in step S4 is 10°C. The other steps and the amounts of substances are the same as those of mixed aconitate 1#, and the mixed aconitate 20# is obtained.

Mixed aconitate 21#

The preparation method of mixed aconitate 21# differs from the preparation method of mixed aconitate 1# in that the decolorization time of activated carbon in step S4 is 5 min. The other steps and the amounts of substances are the same as those of mixed aconitate 1#, and the mixed aconitate 21# is obtained.

Mixed aconitate 22#

The preparation method of mixed aconitate 22# differs from the preparation method of mixed aconitate 1# in that the activated carbon in step S4 has an iodine value of 700 and a methylene blue value of 10. The other steps and the amounts of substances are the same as those of mixed aconitate 1#, and the mixed aconitate 22# is obtained.

Mixed aconitate 23#

The preparation method of mixed aconitate 23# differs from the preparation method of mixed aconitate 1# in that in step S4, activated carbon is replaced with basic aluminum oxide having a particle size of 200 mesh, a specific surface area of 90 $m^2/g$, and an average pore diameter of 15 nm. The other steps and the amounts of substances are the same as those of mixed aconitate 1#, and the mixed aconitate 23# is obtained.

Mixed aconitate 24#

The preparation method of mixed aconitate 24# differs from the preparation method of mixed aconitate 1# in that in step S1, the esterification reaction is performed at 200°C under a rotational speed of 300 rpm for 6 h to obtain a mixed solution containing aconitate. The other steps and the amounts of substances are the same as those of mixed aconitate 1#, and the mixed aconitate 24# is obtained.

Mixed aconitate 25#

The preparation method of mixed aconitate 25# differs from the preparation method of mixed aconitate 1# in that in step S2, neutralization is performed with an alkaline solution at 80°C. The other steps and the amounts of substances are the same as those of mixed aconitate 1#, and the mixed aconitate 25# is obtained.

Comparative mixed aconitate D1#

The preparation method of this comparative mixed aconitate D1# includes the following steps:

S1: mixing aconitic acid, n-butanol, and isooctanol at a molar ratio of 1:6:3, adding sodium bisulfate catalyst, and performing esterification reaction at 125°C under a rotational speed of 300 rpm for 6 h to obtain a mixed solution containing aconitate, wherein the weight ratio of sodium bisulfate to aconitic acid is 1:10;
S2: neutralizing the mixed solution containing aconitate with an equal volume of $Na_2CO_3$ alkaline solution at 40°C for

20 min, wherein the mass fraction of the solute in the alkaline solution is 5%, and after repeating for three times, performing phase separation to obtain an organic phase; and

S3: performing solvent removal on the organic phase at 70°C under a vacuum degree of 50 mbar until no distillate is evaporated, so as to obtain comparative mixed aconitate D1#.

Comparative mixed aconitate D2#

The preparation method of this comparative mixed aconitate D2# includes the following steps:

S1: this step is the same as that in Example 55;

S2: neutralizing the mixed solution containing aconitate with an equal volume of $Na_2CO_3$ alkaline solution at 40°C for 20 min, wherein the mass fraction of the solute in the alkaline solution is 5%; after repeating for three times, performing phase separation to obtain an organic phase A; then adding an activated carbon decolorizing agent with an iodine value of 810 and a methylene blue value of 12 to the organic phase A, and performing adsorption decolorization at 30°C for 30 min, wherein the weight ratio of activated carbon to organic phase A is 1:7; then after filtering and washing with water, performing phase separation to obtain an organic phase B;

S3: performing solvent removal on organic phase B at 70°C under a vacuum degree of 50 mbar until no distillate is evaporated, so as to obtain a purified product; and

S4: adding hydrogen peroxide with a concentration of 35% to the purified product, and performing bleaching and decolorization on organic phase A at 30°C for 15 min, wherein the weight of hydrogen peroxide accounts for 3% of the weight of organic phase A; then washing with clean water to remove hydrogen peroxide, and performing solvent removal at 70°C under a vacuum degree of 50 mbar until no distillate is evaporated, and the comparative mixed aconitate D2# is obtained.

Comparative mixed aconitate D3#

The preparation method of this comparative mixed aconitate D3# includes the following steps:

(1) this step is the same as that in Example 55;

(2) neutralizing the mixed solution containing aconitate with an equal volume of $Na_2CO_3$ alkaline solution at 40°C for 20 min, wherein the mass fraction of the solute in the alkaline solution is 5%; after repeating for three times, performing phase separation to obtain an organic phase A; adding 35% hydrogen peroxide (accounting for 3% of the weight of organic phase A) and activated carbon (with an iodine value of 810 and a methylene blue value of 12) to the organic phase A, wherein the weight ratio of activated carbon to organic phase A is 1:7; performing decolorization at 30°C for 30 min, then after filtering and washing with water, performing phase separation to obtain an organic phase B; and

S3: performing solvent removal on organic phase B at 70°C under a vacuum degree of 50 mbar until no distillate is evaporated, so as to obtain comparative mixed aconitate D3#.

Test Example 4

The mixed aconitates prepared in the above Example 55 were tested for acid value, purity, chromaticity, yield, triester conversion rate and water content. The specific test results are shown in Table 8, and the test method is the same as that in Test 1:

Table 8

| Sample No. | Acid value (KOH/g) | Purity (%) | Chromaticity (Pt-Co) | Yield (%) | Triester conversion rate (%) | Water content (%) | Residual amounts of fat-soluble pigments (ppm) | Residual amounts of water-soluble pigments (ppm) |
|---|---|---|---|---|---|---|---|---|
| Mixed aconitate 1# | 0.04 | 99 | 30 | 96.4 | >99 | 0.02 | 423 | 461 |
| Mixed aconitate 2# | 0.03 | 99 | 50 | 95.6 | >99 | 0.02 | 458 | 412 |

(continued)

| Sample No. | Acid value (KOH/g) | Purity (%) | Chromaticity (Pt-Co) | Yield (%) | Triester conversion rate (%) | Water content (%) | Residual amounts of fat-soluble pigments (ppm) | Residual amounts of water-soluble pigments (ppm) |
|---|---|---|---|---|---|---|---|---|
| Mixed aconitate 3# | 0.04 | 99 | 40 | 96.3 | >99 | 0.04 | 415 | 471 |
| Mixed aconitate 4# | 0.05 | 99 | 30 | 97.1 | >99 | 0.03 | 465 | 499 |
| Mixed aconitate 5# | 0.03 | 99 | 40 | 96.7 | >99 | 0.02 | 486 | 415 |
| Mixed aconitate 6# | 0.04 | 99 | 30 | 97.2 | >99 | 0.03 | 442 | 438 |
| Mixed aconitate 7# | 0.05 | 99 | 50 | 96.7 | >99 | 0.02 | 471 | 477 |
| Mixed aconitate 8# | 0.04 | 99 | 30 | 97.4 | >99 | 0.03 | 462 | 483 |
| Mixed aconitate 9# | 0.03 | 99 | 40 | 95.9 | >99 | 0.04 | 427 | 457 |
| Mixed aconitate 10# | 0.04 | 99 | 30 | 96.2 | >99 | 0.02 | 487 | 432 |
| Mixed aconitate 11# | 0.03 | 99 | 30 | 95.7 | >99 | 0.03 | 432 | 471 |
| Mixed aconitate 12# | 0.02 | 96 | 60 | 95.2 | >99 | 0.03 | 1315 | 462 |
| Mixed aconitate 13# | 0.04 | 95 | 60 | 92.6 | >99 | 0.02 | 1342 | 482 |
| Mixed aconitate 14# | 0.04 | 96 | 50 | 91.7 | >99 | 0.03 | 813 | 448 |
| Mixed aconitate 15# | 0.03 | 97 | 70 | 96.5 | >99 | 0.04 | 2155 | 485 |
| Mixed aconitate 16# | 0.04 | 95 | 200 | 93.2 | >99 | 0.05 | 4667 | 435 |
| Mixed aconitate 17# | 0.03 | 97 | 40 | 95.8 | >99 | 0.04 | 1678 | 454 |
| Mixed aconitate 18# | 0.02 | 96 | 60 | 95.6 | >99 | 0.03 | 482 | 1272 |
| Mixed aconitate 19# | 0.03 | 97 | 65 | 95.7 | >99 | 0.04 | 443 | 1431 |
| Mixed aconitate 20# | 0.04 | 95 | 60 | 95.9 | >99 | 0.03 | 461 | 1742 |
| Mixed aconitate 21# | 0.03 | 96 | 60 | 96.1 | >99 | 0.02 | 428 | 1235 |
| Mixed aconitate 22# | 0.04 | 95 | 100 | 95.9 | >99 | 0.04 | 445 | 4134 |
| Mixed aconitate 23# | 0.03 | 99 | 50 | 96.1 | >99 | 0.03 | 460 | 844 |

(continued)

| Sample No. | Acid value (KOH/g) | Purity (%) | Chromaticity (Pt-Co) | Yield (%) | Triester conversion rate (%) | Water content (%) | Residual amounts of fat-soluble pigments (ppm) | Residual amounts of water-soluble pigments (ppm) |
|---|---|---|---|---|---|---|---|---|
| Mixed aconitate 24# | 0.02 | 94 | 300 | 89.2 | 94.7 | 0.03 | 3615 | 5338 |
| Mixed aconitate 25# | 0.03 | 96 | 60 | 87.6 | >99 | 0.04 | 886 | 1264 |
| Comparative mixed aconitate D1# | 0.05 | 94 | >500 | 93.3 | >99 | 0.03 | 17214 | 14367 |
| Comparative mixed aconitateD2# | 0.26 | 97 | 120 | 95.8 | >99 | 0.04 | 2542 | 2421 |
| Comparative mixed aconitate D3# | 0.11 | 98 | 90 | 93.7 | >99 | 0.03 | 2877 | 2610 |

It can be seen from the contents in Table 8 that the mixed aconitates prepared by the preparation method of the present disclosure can remove the pigment impurities carried by aconitic acid itself during the preparation process, reduce the contents of fat-soluble pigments and water-soluble pigments, and improve the purity and yield of aconitates.

The comparison between mixed aconitates 12# to 15# and mixed aconitate 1# shows that the concentration and dosage of hydrogen peroxide can affect the removal effect of fat-soluble pigments. If the concentration of hydrogen peroxide is too low or the dosage is too small, the pigment bleaching of the product will be incomplete, and the pigment will not meet the requirements; if the concentration of hydrogen peroxide is too high or the dosage is too large, the chromaticity of the product will increase, and more hydrogen peroxide will participate in the hydrolysis of aconitate. Although the reaction rate is very slow, the yield will be reduced.

The comparison between mixed aconitates 16# to 17# and mixed aconitate 1# shows that the higher the decolorization temperature of hydrogen peroxide, the more hydrogen peroxide will decompose, leading to a poor oxidation effect on fat-soluble pigments. As a result, the amount of residual fat-soluble pigments increases, thereby leading to an increase in chromaticity and a decrease in purity of aconitate. At the same time, the hydrolysis of aconitate by hydrogen peroxide is promoted, such that the yield of aconitate is decreased; shortened decolorization time of hydrogen peroxide will affect the damage effect of hydrogen peroxide on the main structures of fat-soluble pigments, further leading to an increase in chromaticity and a decrease in purity of aconitate.

The comparison between mixed aconitate 18# and mixed aconitate 1# shows that when the dosage of activated carbon is reduced, the adsorption capacity for water-soluble pigments decreases. Therefore, the amount of residual water-soluble pigments increases, resulting in an increase in chromaticity and a decrease in purity of aconitate.

The comparison between mixed aconitates 19# to 20# and mixed aconitate 1# shows that the decolorization temperature of the decolorizing agent affects the decolorization effect on water-soluble pigments. The higher the temperature, the more intense the Brownian motion of molecules, and the more difficult it is for the decolorizing agent to adsorb pigments, which instead leads to a decrease in the removal rate of water-soluble pigment impurities. The lower the temperature, the lower the adsorption capacity of the decolorizing agent for water-soluble pigments, and the higher the viscosity of aconitate, which is not conducive to uniform dispersion of the decolorizing agent, and will also lead to a decrease in the removal rate of water-soluble pigments, and further lead to a decrease in purity and an increase in chromaticity of aconitate.

The comparison between mixed aconitate 21# and mixed aconitate 1# shows that the shorter the decolorization time of the decolorizing agent, the worse the decolorization effect on water-soluble pigments, thereby leading to an increase in chromaticity and a decrease in purity of aconitate.

The comparison between mixed aconitates 22# to 23# and mixed aconitate 1# shows that the type of decolorizing agent and the parameters of activated carbon can influence the removal effect of water-soluble pigments, and further influence the purity and chromaticity of the aconitate product.

The comparison between mixed aconitate 24# and mixed aconitate 1# shows that an increase in esterification

temperature leads to the caramelization reaction of polysaccharides. Under the same decolorization conditions, the contents of both types of pigments in the final aconitate increase, the product chromaticity increases and the purity decreases. Moreover, the monosaccharides produced by the decomposition of polysaccharides will compete with alcohols for the esterification reaction with aconitic acid, resulting in a decrease in the yield and triester conversion rate of aconitate.

The comparison between mixed aconitate 25# and mixed aconitate 1# shows that an increase in neutralization temperature causes hydrolysis of the product in an alkaline environment, then the purity of the obtained product is insufficient. The decrease in purity increases the relative content of pigments in the product, which further affects the one-step decolorization process and second-step decolorization process. The amounts of residual fat-soluble pigments and water-soluble pigments are increased, the chromaticity of aconitate is increased, and the purity is decreased. Additionally, the loss due to high-temperature hydrolysis leads to a decrease in the yield of aconitate.

The comparison between comparative mixed aconitates D1 to D3# and mixed aconitate 1# shows that the mixed aconitates obtained without decolorization treatment have high contents of water-soluble pigments and fat-soluble pigments, the chromaticity of the product is more than 500, and the purity decreases. When water-soluble pigments are removed first and then fat-soluble pigments are removed, or when the two types of pigments are removed simultaneously, not only the preparation and treatment processes will be increased, but also the removal effect of the two types of pigments will be affected, resulting in an increase in chromaticity and a decrease in purity of aconitate. Moreover, when fat-soluble pigments and water-soluble pigments are removed simultaneously, activated carbon will agglomerate in the water phase and distribute in the organic phase, the decolorization effect of both types of pigments in the product is poor, and the yield will decrease due to the aggregated activated carbon.

Test 5

This example relates to a composite material containing mixed aconitates and its preparation method. The composite material includes: 100 parts of PVC resin (with a K value of 60 and a polydispersity index of 4), 30 parts of plasticizer, 2 parts of oxidized polyethylene wax, and 5 parts of octyltin maleate. The above raw materials were mixed in a high-speed mixer at 60°C for 40 min, then mixed in two-roll open mixing mill at 155°C for 15 min, and finally placed in a plate vulcanizer for vulcanization at 165°C for 15 min to obtain the composite material.

The above plasticizer is the mixed aconitates prepared in Example 37, and composite materials are prepared according to the above weight ratio and preparation method respectively. At the same time, dioctyl phthalate, tributyl citrate, tributyl aconitate and triisooctyl aconitate are respectively selected as plasticizers to prepare composite materials according to the above weight ratio and preparation method. The performance tests of the above composite materials are performed, and the results are shown in Table 9 and Table 10. The test method is the same as that in Test 2 except for the following:

**1. Volatility loss rate:** tests were performed in accordance with HG/T 4458-2012 *Plastics - Determination of Plasticizer Loss by Activated Carbon Method* at a test temperature of 70°C for 24 h.

**2. Water extraction rate:** tests were performed in accordance with the standard ISO *175-2011 Test Method for Determination of Immersion Effects of Plastics in Liquid Chemicals* at room temperature for 72 h, with deionized water as the extraction solvent.

**3. Ethanol extraction rate:** tests were performed in accordance with the standard ISO *175-2011 Test Method for Determination of Immersion Effects of Plastics in Liquid Chemicals* at room temperature for 72 h, with ethanol as the extraction solvent.

**4. Petroleum ether extraction rate:** tests were performed in accordance with the standard ISO 175-2011 *Test Method for Determination of Immersion Effects of Plastics in Liquid Chemicals* at room temperature for 72 h, with petroleum ether as the extraction solvent.

**5. Migration rate:** tests were performed in accordance with HG/T 4458-2012 *Determination of Plasticizer Migration in Plastics* at a test temperature of 50°C for 48 h. The materials of absorbent sheets are polyethylene (PE) without additives and standard natural rubber (NR).

Table 9

| Types of plasticizers | Hardness/Shore D | Tensile modulus at 100% (MPa) | Elongation at break (%) | Elastic modulus (MPa) | Compatibility (mL) |
|---|---|---|---|---|---|
| Mixed aconitate 1# | 53 | 10.7 | 226.8 | 231.7 | 15.2 |
| Mixed aconitate 2# | 61 | 12.4 | 170.4 | 291.2 | 13.1 |
| Mixed aconitate 3# | 65 | 13.8 | 163.6 | 326.3 | 12.7 |
| Mixed aconitate 4# | 70 | 15.6 | 151.5 | 384.5 | 9.4 |
| Mixed aconitate 5# | 67 | 14.1 | 157.3 | 342.1 | 9.6 |
| Mixed aconitate 6# | 56 | 11.6 | 202.1 | 261.8 | 12.4 |
| Mixed aconitate 7# | 58 | 12.0 | 189.2 | 274.3 | 13.6 |
| Mixed aconitate 8# | 69 | 15.4 | 157.3 | 371.8 | 9.1 |
| Mixed aconitate 9# | 67 | 14.2 | 156.4 | 344.1 | 8.3 |
| Mixed aconitate 10# | 51 | 10.4 | 231.6 | 219.4 | 16.7 |
| Mixed aconitate 11# | 79 | 15.4 | 133.6 | 483.1 | 3.7 |
| Mixed aconitate 12# | 56 | 13.3 | 208.7 | 285.8 | 12.3 |
| Mixed aconitate 13# | 56 | 13.4 | 211.6 | 288.1 | 12.3 |
| Mixed aconitate 14# | 58 | 14.7 | 197.5 | 313.5 | 11.98 |
| Mixed aconitate 15# | 59 | 14.9 | 204.1 | 335.4 | 11.5 |
| Mixed aconitate 16# | 56 | 14.7 | 200.8 | 323.8 | 11.7 |
| Mixed aconitate 17# | 55 | 12.9 | 216.7 | 245.6 | 13.6 |
| Mixed aconitate 18# | 55 | 13.1 | 219.3 | 249.7 | 14.1 |
| Mixed aconitate 19# | 56 | 13.5 | 210.7 | 291.9 | 12.5 |
| Mixed aconitate 20# | 59 | 14.7 | 201.8 | 314.7 | 11.8 |
| Mixed aconitate 21# | 56 | 13.3 | 210.3 | 290.5 | 12.3 |
| Mixed aconitate 22# | 62 | 15.5 | 195.0 | 361.6 | 10.7 |
| Mixed aconitate 23# | 54 | 12.5 | 225.0 | 235 | 15.2 |
| Mixed aconitate 24# | 72 | 16.9 | 151.2 | 505.7 | 7.6 |
| Mixed aconitate 25# | 56 | 13.5 | 212.2 | 294.5 | 12.6 |
| Comparative mixed aconitate D1# | 61 | 15.3 | 194.6 | 349.3 | 10.2 |
| Comparative mixed aconitate D2# | 58 | 14.8 | 201.7 | 344.4 | 11.8 |
| Comparative mixed aconitate D3# | 59 | 14.8 | 203.6 | 343.8 | 11.8 |
| Dioctyl Phthalate | 78 | 15.1 | 134.9 | 487.3 | 7.1 |
| Tributyl citrate | 77 | 13.6 | 146.3 | 426.5 | 14.3 |
| Tributyl aconitate | 45 | 8.2 | 257.4 | 130.5 | 16.7 |
| Triisooctyl aconitate | 71 | 16.1 | 156.3 | 397.1 | 10.9 |

Table 10

| Types of plasticizers | $T_g$ (°C) | Volatility loss rate (%) | Water extraction rate (%) | Ethanol extraction rate (%) | Petroleum ether extraction rate (%) | Migration rate to PE (%) | Migration rate to NR (%) |
|---|---|---|---|---|---|---|---|
| Mixed aconitate 1# | 34.7 | 0.88 | 0.26 | 4.23 | 6.88 | 0.31 | 0.33 |
| Mixed aconitate 2# | 39.1 | 0.47 | 0.21 | 5.47 | 8.76 | 0.26 | 0.24 |
| Mixed aconitate 3# | 41.2 | 0.41 | 0.27 | 4.75 | 7.21 | 0.65 | 0.71 |
| Mixed aconitate 4# | 44.3 | 0.51 | 0.24 | 5.52 | 8.34 | 0.21 | 0.28 |
| Mixed aconitate 5# | 42.3 | 0.49 | 0.26 | 5.67 | 8.42 | 0.23 | 0.31 |
| Mixed aconitate 6# | 36.7 | 0.82 | 0.27 | 3.89 | 6.21 | 0.69 | 0.83 |
| Mixed aconitate 7# | 37.4 | 0.79 | 0.31 | 4.11 | 7.58 | 0.66 | 0.76 |
| Mixed aconitate 8# | 43.9 | 0.54 | 0.33 | 6.82 | 9.03 | 0.21 | 0.30 |
| Mixed aconitate 9# | 42.4 | 0.63 | 0.27 | 7.20 | 10.72 | 0.70 | 0.78 |
| Mixed aconitate 10# | 31.7 | 1.02 | 0.21 | 3.88 | 7.54 | 1.12 | 1.22 |
| Mixed aconitate 11# | 46.9 | 0.21 | 0.26 | 16.67 | 17.58 | 0.18 | 0.21 |
| Mixed aconitate 12# | 35.7 | 0.91 | 0.30 | 4.66 | 7.32 | 0.35 | 0.41 |
| Mixed aconitate 13# | 35.9 | 0.93 | 0.27 | 5.13 | 7.36 | 0.36 | 0.41 |
| Mixed aconitate 14# | 35.4 | 0.96 | 0.31 | 5.26 | 7.59 | 0.35 | 0.46 |
| Mixed aconitate 15# | 36.2 | 0.94 | 0.35 | 5.36 | 7.13 | 0.39 | 0.51 |
| Mixed aconitate 16# | 35.8 | 0.93 | 0.28 | 5.27 | 7.46 | 0.37 | 0.42 |
| Mixed aconitate 17# | 35.9 | 0.95 | 0.29 | 5.15 | 7.30 | 0.38 | 0.46 |
| Mixed aconitate 18# | 36.2 | 0.94 | 0.28 | 5.26 | 7.38 | 0.35 | 0.43 |
| Mixed aconitate 19# | 36.3 | 0.96 | 0.31 | 5.16 | 7.44 | 0.34 | 0.41 |
| Mixed aconitate 20# | 37.2 | 0.95 | 0.30 | 5.40 | 7.40 | 0.37 | 0.45 |
| Mixed aconitate 21# | 36.1 | 0.93 | 0.27 | 5.33 | 7.16 | 0.35 | 0.47 |
| Mixed aconitate 22# | 38.7 | 0.94 | 0.31 | 5.23 | 7.23 | 0.39 | 0.42 |

(continued)

| Types of plasticizers | $T_g$ (°C) | Volatility loss rate (%) | Water extraction rate (%) | Ethanol extraction rate (%) | Petroleum ether extraction rate (%) | Migration rate to PE (%) | Migration rate to NR (%) |
|---|---|---|---|---|---|---|---|
| Mixed aconitate 23# | 34.8 | 0.95 | 0.35 | 5.36 | 7.52 | 0.34 | 0.53 |
| Mixed aconitate 24# | 48.2 | 0.93 | 0.28 | 5.18 | 7.15 | 0.35 | 0.47 |
| Mixed aconitate 25# | 37.3 | 0.95 | 0.31 | 5.41 | 7.20 | 0.34 | 0.56 |
| Comparative mixed aconitate D1# | 38.5 | 0.96 | 0.29 | 5.38 | 7.31 | 0.37 | 0.43 |
| Comparative mixed aconitate D2# | 35.7 | 0.94 | 0.27 | 5.12 | 7.46 | 0.39 | 0.51 |
| Comparative mixed aconitate D3# | 36.1 | 0.98 | 0.28 | 5.23 | 7.88 | 0.38 | 0.33 |
| Dioctyl phthalate | 48.5 | 0.96 | 0.32 | 5.13 | 6.12 | 0.83 | 0.81 |
| Tributyl citrate | 38.3 | 3.87 | 1.16 | 4.96 | 8.91 | 2.12 | 2.71 |
| Tributyl aconitate | 28.1 | 1.08 | 0.36 | 3.71 | 6.03 | 1.26 | 1.31 |
| Triisooctyl aconitate | 43.5 | 0.38 | 0.14 | 14.52 | 16.44 | 0.13 | 0.18 |

It can be seen from the contents in Table 9 and Table 10 that mixed aconitates can better balance plasticizing performance and stability. Although they are not as effective in plasticization as short-chain esters (such as tributyl aconitate) and they are not as long-term stable as long-chain aconitates (such as triisooctyl aconitate), the products plasticized by mixed aconitates can adapt to more complex external environments, and can not only achieve effective plasticization but also have comprehensive stability in different environments. The structures of the above mixed aconitates can affect their plasticizing performance on the composite material and the stability of the mixed aconitates itself in the composite material. In addition, the content of water-soluble pigments, the content of fat-soluble pigments, purity and chromaticity in the above mixed aconitates can all affect the mechanical properties of the composite material, and also affect the volatility resistance, extraction resistance and migration resistance of the mixed aconitates. At the same time, the volatility loss rate is a heat resistance test performed at 70°C, and the result can directly characterize the heat resistance of the product. When triisooctyl aconitate is compared with dioctyl phthalate (octyl ester is an industrial abbreviation, and it is actually isooctyl ester), under the same octyl ester structure, triisooctyl aconitate has lower volatilization loss. When tributyl aconitate is compared with tributyl citrate, under the same butyl ester structure, tributyl aconitate also has lower volatilization loss. Therefore, aconitate can effectively improve the heat resistance of PVC composite materials.

Example 56

This example relates to a composite material and a preparation method thereof. The composite material includes the following components in parts by weight: 100 parts of non-cross-linked PVC resin, 50 parts of tri-(2-ethylhexyl) aconitate, 30 parts of talc powder, 2 parts of methyltin mercaptide, 1 part of lanthanum stearate, 1.5 parts of dimethyl dodecyl ammonium chloride and 3 parts of chlorinated paraffin. The particle size of the talc powder is 90 nm; the tri-(2-ethylhexyl) aconitate has an acid value of 0.03 mgKOH/g, a water content of 0.04% and a purity of 99%; the PVC has a weight-average molecular weight of 80,000 and a polydispersity index of 4.

The preparation method of the composite material includes the following steps:

S1: mixing the above non-cross-linked PVC resin, tri-(2-ethylhexyl) aconitate, methyltin mercaptide, lanthanum stearate, dimethyl dodecyl ammonium chloride and chlorinated paraffin to obtain a premix;

S2: adding the premix into a high-speed mixer, mixing at 60°C for 30 min, then adding into two-roll open mixing mill, and mixing at 155°C for 15 min to obtain a mixed compound; and

S3: placing the mixed compound in a plate vulcanizer with a gauge pressure of 10 MPa, and vulcanizing at 165°C for 15 min to obtain the composite material.

Example 57

This example relates to a composite material and a preparation method thereof. The composite material includes the following components in parts by weight: 100 parts of non-cross-linked PVC resin, 50 parts of n-octyl aconitate, 60 parts of barium sulfate, 5 parts of di-n-octyltin bis(2-ethylhexyl mercaptoacetate), 1 part of cerium stearate, 1.5 parts of stearyl trimethyl ammonium chloride and 3 parts of tricresyl phosphate. The particle size of barium sulfate is 100 nm; the n-octyl aconitate has an acid value of 0.05 mgKOH/g, a water content of 0.03% and a purity of 98%; the PVC has a weight-average molecular weight of 80,000 and a polydispersity index of 4. Its preparation method is the same as that in Example 56.

Example 58

This example relates to a composite material and a preparation method thereof. The composite material includes the following components in parts by weight: 100 parts of non-cross-linked PVC resin, 50 parts of n-hexyl aconitate, 10 parts of calcium carbonate, 1 part of di-n-butyltin maleate, 2 parts of lanthanum stearate, 1.5 parts of dodecyl trimethyl ammonium chloride and 3 parts of zinc borate. The particle size of calcium carbonate is 80 nm. The n-hexyl aconitate has an acid value of 0.03 mgKOH/g, a water content of 0.03% and a purity of 98%; the PVC has a weight-average molecular weight of 80,000 and a polydispersity index of 4. Its preparation method is the same as that in Example 56.

Example 59

This example differs from Example 56 in that n-dodecyl aconitate is used to replace tri-(2-ethylhexyl) aconitate. The n-dodecyl aconitate has an acid value of 0.03 mgKOH/g, a water content of 0.04% and a purity of 97.5%. The other components and preparation method are the same as those in Example 56.

Example 60

This example differs from Example 56 in that n-octadecyl aconitate is used to replace tri-(2-ethylhexyl) aconitate. The n-octadecyl aconitate has an acid value of 0.04 mgKOH/g, a water content of 0.05% and a purity of 98.5%. The other components and preparation method are the same as those in Example 56.

Example 61

This example differs from Example 56 in that dihexyl-monoisooctyl aconitate is used. In the dihexyl-monoisooctyl aconitate, two of the substituents $R_1$, $R_2$ and $R_3$ are hexyl groups, and one substituent is an isooctyl group. It has an acid value of 0.05 mgKOH/g, a water content of 0.03% and a purity of 98.5%. The other components and preparation method are the same as those in Example 56.

Example 62

This example differs from Example 56 in that tri-(2-ethylhexyl) aconitate and n-hexyl aconitate in a weight ratio of 1:1 are used. The parameters of the tri-(2-ethylhexyl) aconitate and n-hexyl aconitate are the same as those in Example 56 and Example 58. The other components and preparation method are the same as those in Example 56.

Example 63

This example differs from Example 56 in that the weight-average molecular weight of PVC is 120,000. The other components and preparation method are the same as those in Example 56.

Example 64

This example differs from Example 56 in that the weight-average molecular weight of PVC is 30,000. The other

components and preparation method are the same as those in Example 56.

Example 65

This example differs from Example 56 in that the polydispersity index of PVC is 5.5. The other components and preparation method are the same as those in Example 56.

Example 66

This example differs from Example 56 in that the polydispersity index of PVC is 2.5. The other components and preparation method are the same as those in Example 56.

Example 67

This example differs from Example 56 in that thermoplastic nitrile rubber is used to replace non-cross-linked PVC resin. The thermoplastic nitrile rubber has a molecular weight of 700,000. The other components and preparation method are the same as those in Example 56.

Example 68

This example differs from Example 56 in that the acid value of tri-(2-ethylhexyl) aconitate is 0.12 mgKOH/g. The other components and preparation method are the same as those in Example 56.

Example 69

This example differs from Example 56 in that the water content of tri-(2-ethylhexyl) aconitate is 4%. The other components and preparation method are the same as those in Example 56.

Example 70

This example differs from Example 56 in that the purity of tri-(2-ethylhexyl) aconitate is 95%. The other components and preparation method are the same as those in Example 56.

Example 71

This example differs from Example 56 in that 2.5 parts of methyltin mercaptide and 0.5 part of lanthanum stearate are used. The other components and preparation method are the same as those in Example 56.

Example 72

This example differs from Example 56 in that di-n-butyltin dodecanethiolate is used to replace methyltin mercaptide. The other components and preparation method are the same as those in Example 56.

Example 73

This example differs from Example 56 in that cerium stearate is used to replace lanthanum stearate. The other components and preparation method are the same as those in Example 56.

Example 74

This example differs from Example 56 in that dimethyl dodecyl ammonium chloride is not added. The other components and preparation method are the same as those in Example 56.

Example 75

This example differs from Example 56 in that chlorinated polyethylene is used to replace chlorinated paraffin. The other components and preparation method are the same as those in Example 56.

Example 76

This example differs from Example 56 in that 5 parts of tri-(2-ethylhexyl) aconitate are added. The other components and preparation method are the same as those in Example 56.

Example 77

This example differs from Example 56 in that the particle size of talc powder is 150 nm. The other components and preparation method are the same as those in Example 56.

Example 78

This example differs from Example 56 in that the particle size of talc powder is 20 nm. The other components and preparation method are the same as those in Example 56.

Comparative Example 14

This comparative example differs from Example 56 in that dioctyl phthalate is used to replace tri-(2-ethylhexyl) aconitate. The other components and preparation method are the same as those in Example 56.

Comparative Example 15

This comparative example differs from Example 56 in that dioctyl terephthalate is used to replace tri-(2-ethylhexyl) aconitate. The other components and preparation method are the same as those in Example 56.

Comparative Example 16

This comparative example differs from Example 56 in that dioctyl adipate is used to replace tri-(2-ethylhexyl) aconitate. The other components and preparation method are the same as those in Example 56.

Comparative Example 17

This comparative example differs from Example 56 in that tributyl citrate is used to replace tri-(2-ethylhexyl) aconitate. The other components and preparation method are the same as those in Example 56.

Comparative Example 18

This comparative example differs from Example 56 in that tripentyl aconitate is used to replace tri-(2-ethylhexyl) aconitate. The tripentyl aconitate has an acid value of 0.07 mgKOH/g, a water content of 0.07% and a purity of 97.5%. The other components and preparation method are the same as those in Example 56.

Comparative Example 19

This comparative example differs from Example 56 in that calcium stearate is used to replace lanthanum stearate. The other components and preparation method are the same as those in Example 56.

Comparative Example 20

This comparative example differs from Example 56 in that talc powder is not contained. The other components and preparation method are the same as those in Example 56.

Test Example 6

Performance tests are performed on the composite materials prepared in the above examples and comparative examples, and the results are shown in Table 11 and Table 12. The test method is the same as that in Test 5.

Table 11

| Sample No. | Hardness/Shore A | Tensile modulus at 100% (MPa) | Elongation at break (%) | Elastic modulus (MPa) | Compatibility (mL) |
|---|---|---|---|---|---|
| Example 56 | 71 | 16.1 | 469.5 | 398.7 | 10.75 |
| Example 57 | 72 | 15.9 | 475.8 | 391.0 | 10.81 |
| Example 58 | 66 | 13.8 | 581.5 | 317.2 | 13.36 |
| Example 59 | 89 | 17.2 | 375.6 | 702.7 | 8.93 |
| Example 60 | 98 | 18.9 | 233.5 | 981.3 | 4.62 |
| Example 61 | 68 | 14.6 | 544.1 | 344.4 | 12.49 |
| Example 62 | 69 | 14.8 | 533.5 | 347.9 | 11.83 |
| Example 63 | 80 | 17.6 | 338.2 | 673.6 | 10.75 |
| Example 64 | 73 | 15.8 | 412.3 | 302.1 | 10.75 |
| Example 65 | 73 | 15.2 | 416.4 | 351.4 | 10.75 |
| Example 66 | 72 | 16.7 | 379.5 | 433.1 | 10.75 |
| Example 67 | 61 | 12.7 | 645.4 | 233.1 | 10.75 |
| Example 68 | 73 | 16.5 | 451.7 | 417.4 | 10.21 |
| Example 69 | 74 | 16.7 | 427.5 | 431.7 | 8.26 |
| Example 70 | 74 | 16.8 | 438.9 | 425.1 | 10.66 |
| Example 71 | 71 | 16.4 | 457.1 | 408.9 | 10.75 |
| Example 72 | 71 | 16.2 | 462.5 | 402.7 | 10.75 |
| Example 73 | 71 | 16.1 | 461.3 | 397.6 | 10.75 |
| Example 74 | 71 | 16.1 | 479.6 | 407.1 | 10.75 |
| Example 75 | 71 | 16.1 | 461.5 | 398.2 | 10.75 |
| Example 76 | 93 | 16.7 | 127.9 | 894.4 | 10.75 |
| Example 77 | 71 | 15.4 | 345.1 | 362.4 | 10.75 |
| Example 78 | 71 | 15.7 | 402.8 | 324.8 | 10.75 |
| Comparative Example 14 | 82 | 18.7 | 568.4 | 487.3 | 7.1 |
| Comparative Example 15 | 87 | 19.4 | 526.1 | 507.9 | 7.4 |
| Comparative Example 16 | 76 | 17.1 | 651.7 | 406.3 | 12.53 |
| Comparative Example 17 | 77 | 17.6 | 627.3 | 415.6 | 14.35 |
| Comparative Example 18 | 62 | 11.2 | 863.5 | 263.5 | 14.86 |
| Comparative Example 19 | 73 | 16.4 | 429.5 | 412.7 | 10.75 |
| Comparative Example 20 | 66 | 12.9 | 524.7 | 286.3 | 10.75 |

Table 12

| Sample No. | $T_g$ (°C) | Volatility loss rate (%) | Migration rate to PE (%) | Migration rate to NR (%) |
|---|---|---|---|---|
| Example 56 | 44.5 | 0.38 | 0.13 | 0.18 |
| Example 57 | 44.7 | 0.41 | 0.12 | 0.14 |
| Example 58 | 30.7 | 0.74 | 0.28 | 0.26 |
| Example 59 | 57.2 | 0.21 | 0.09 | 0.11 |
| Example 60 | 62.3 | 0.17 | 0.04 | 0.07 |

(continued)

| Sample No. | $T_g$ (°C) | Volatility loss rate (%) | Migration rate to PE (%) | Migration rate to NR (%) |
|---|---|---|---|---|
| Example 61 | 36.7 | 0.61 | 0.21 | 0.19 |
| Example 62 | 39.8 | 0.58 | 0.22 | 0.21 |
| Example 63 | 45.7 | 0.82 | 0.18 | 0.25 |
| Example 64 | 45.2 | 0.83 | 0.22 | 0.29 |
| Example 65 | 44.2 | 0.84 | 0.21 | 0.24 |
| Example 66 | 44.7 | 0.83 | 0.18 | 0.19 |
| Example 67 | -35.3 | 0.79 | 0.12 | 0.24 |
| Example 68 | 45.1 | 0.82 | 0.22 | 0.24 |
| Example 69 | 45.2 | 1.43 | 1.25 | 1.36 |
| Example 70 | 45.0 | 2.32 | 1.93 | 2.02 |
| Example 71 | 44.6 | 0.81 | 0.15 | 0.18 |
| Example 72 | 44.5 | 0.41 | 0.14 | 0.17 |
| Example 73 | 44.5 | 0.39 | 0.13 | 0.20 |
| Example 74 | 44.6 | 0.51 | 0.14 | 0.22 |
| Example 75 | 44.6 | 0.42 | 0.12 | 0.19 |
| Example 76 | 57.9 | 0.28 | 0.06 | 0.09 |
| Example 77 | 44.5 | 0.43 | 0.21 | 0.28 |
| Example 78 | 44.5 | 0.42 | 0.24 | 0.31 |
| Comparative Example 14 | 51.4 | 0.96 | 0.83 | 0.81 |
| Comparative Example 15 | 52.1 | 1.07 | 0.94 | 0.87 |
| Comparative Example 16 | 41.6 | 5.76 | 7.24 | 8.12 |
| Comparative Example 17 | 43.3 | 3.87 | 2.12 | 2.71 |
| Comparative Example 18 | 42.7 | 0.91 | 0.54 | 0.47 |
| Comparative Example 19 | 44.5 | 0.81 | 0.17 | 0.22 |
| Comparative Example 20 | 43.9 | 0.67 | 0.26 | 0.39 |

The contents in Table 11 and Table 12 show that in this composite system, good synergistic effects are achieved between various additives, especially between aconitates and other additives. They do not play a role in only one single aspect; changes in the component ratio of the additives have a certain impact on the performance of the material. Compared with other plasticizers, long-chain aconitates have good volatility resistance and migration resistance.

The comparison between Examples 63 to 66 and Example 56 shows that the molecular weight and polydispersity index of PVC resin can affect the compatibility between the plasticizer and PVC resin, and also affect the synergistic effect between the plasticizer and inorganic filler, such that the toughness, volatility resistance and migration resistance of the composite material are decreased.

The comparison between Examples 68 to 70 and Example 56 shows that the acid value, water content and purity of the plasticizer can all affect the plasticizing effect of the plasticizer on PVC resin, and also affect the mobility of PVC molecular chains, thereby leading to poor dispersion of inorganic fillers in PVC molecular chains and a decrease in the mechanical properties of the composite material. The above acid value, water content and purity of the plasticizer can also affect the volatility resistance, migration resistance and thermal stability of the material.

The comparison between Examples 71 to 75 and Example 56 shows that the type of organotin compound, the type of rare earth stearate, the type of flame retardant, the weight ratio of organotin compound to rare earth stearate and the added parts of antistatic agent can all affect the synergistic effect between the above substances and inorganic fillers and plasticizers, and further affect the mechanical properties, volatility resistance and migration resistance of the composite material. The comparison between Comparative Example 6 and Example 1 shows that when calcium stearate is used to replace rare earth stearate, calcium stearate cannot play a synergistic effect with inorganic fillers and plasticizers, and all

the performances of the composite material decrease.

The comparison between Examples 76 to 78, Comparative Example 20 and Example 56 shows that the inorganic filler of the present disclosure can be uniformly dispersed in the composite material under the action of the plasticizer and plays a synergistic plasticizing effect with the plasticizer. The particle size of the inorganic filler can also affect its dispersion in the composite material and its synergistic effect with the plasticizer. When the particle size is less than 80 nm or greater than 100 nm, the reinforcing effect on the composite material is weakened. If the particle size is too small, agglomeration is prone to occur; if the particle size is too large, stress concentration is prone to occur, and cracks are likely to occur after long-term use, resulting in a decrease in all the performances of the composite material.

The comparison between Comparative Examples 14 to 18 and Example 56 shows that the use of other types of plasticizers has a poor effect on improving the mobility of PVC molecular chains, such that the dispersion of inorganic fillers in PVC molecular chains becomes poor, the synergistic effect between the plasticizer and inorganic filler is poor, and the plasticizing effect of the plasticizer on the composite material is reduced.

The above are only optional examples of the present disclosure and are not intended to limit the present disclosure. For those skilled in the art, various modifications and changes can be made to the present disclosure. Any modification, equivalent replacement, improvement and the like made within the spirit and principle of the present disclosure shall be included in the protection scope of the present disclosure.

## Claims

1. A preparation method of bio-based *trans*-Aconitic acid, wherein the method comprises:
   (1) preparing *trans*-Aconitic acid-producing engineered strains; (2) preparing seed; (3) performing product fermentation.

2. The preparation method according to claim 1, wherein the engineered strains express aconitate isomerases, and the aconitate isomerases include TbrA and/or Adi1.

3. The preparation method according to claim 2, wherein the amino acid sequence of the aconitate isomerase TbrA comprises amino acid sequence shown as SEQ ID NO. 1 or amino acid sequence having at least 60% homology with the amino acid sequence shown as SEQ ID NO. 1; and/or the amino acid sequence of the aconitate isomerase Adi1 comprises amino acid sequence shown as SEQ ID NO. 2 or amino acid sequence having at least 60% homology with the amino acid sequence shown as SEQ ID NO. 2.

4. The preparation method according to claim 2, wherein the engineered strains also express aconitase.

5. The preparation method according to claim 4, wherein the aconitase is Aco.

6. The preparation method according to claim 5, wherein the amino acid sequence of the aconitase Aco comprises amino acid sequence shown as SEQ ID NO. 5 or amino acid sequence having at least 60% homology with the amino acid sequence shown as SEQ ID NO. 5.

7. The preparation method according to claim 2, wherein at least one terminus of the aconitate isomerase is fused with a mitochondrial targeting signal peptide (MTS).

8. The preparation method according to claim 7, wherein the mitochondrial targeting signal peptide is one or more selected from MTSacoAt, MTScitAt and MTSicdAn.

9. The preparation method according to claim 8, wherein the amino acid sequence of the mitochondrial targeting signal peptide contains amino acid sequence shown as SEQ ID NO. 7, SEQ ID NO. 8 or SEQ ID NO. 9.

10. The preparation method according to claim 7, wherein the N-terminus of the aconitate isomerase is fused with the mitochondrial targeting signal peptide.

11. The preparation method according to any one of claims 1 to 10, wherein the engineered strains are selected from *Aspergillus terreus* or *Aspergillus niger;* the *Aspergillus niger* is one or more selected from *Aspergillus niger* MEFC1501, *Aspergillus niger* ATCC 1015, *Aspergillus niger* NRRL 41873, *Aspergillus niger* NRRL 27809, *Aspergillus niger* NRRL 31821, *Aspergillus niger* NRRL 6276, *Aspergillus niger* Co827, *Aspergillus niger* 5016, *Aspergillus niger* 3008, *Aspergillus niger* GCB75, *Aspergillus niger* ATCC 9142 and *Aspergillus niger* MTCC 282; and the

*Aspergillus terreus* is one or more selected from *Aspergillus terreus* CICC40205, *Aspergillus terreus* NRRL1960, *Aspergillus terreus* DSM23081, *Aspergillus terreus* TN484 and *Aspergillus terreus* TN484-M1.

**12.** The preparation method according to claim 11, wherein when the engineered strains are *Aspergillus terreus,* the cis-aconitate decarboxylase gene (*cadA*) is further knocked out.

**13.** The preparation method according to claim 1, wherein the dissolved oxygen (DO) level during the product fermentation process is maintained at 25% to 30%.

**14.** The preparation method according to claim 1, wherein sugar feeding is carried out between 30 and 60 h of the product fermentation process.

**15.** The preparation method according to claim 1, wherein in a DO control step during the product fermentation process, the aeration rate is controlled from 0.1 to 0.2 vvm; and the agitation speed is controlled from 50 to 150 rpm.

**16.** The preparation method according to claim 1, wherein a fermentation medium used in the product fermentation process contains a nutritional factor.

**17.** The preparation method according to claim 16, wherein the fermentation medium comprises: 120-150 g/L glucose, 2-4 g/L ammonium sulfate, 0.05-0.15% defoamer, 1-3 g/L corn steep liquor powder, 0.005-0.015 g/L diammonium hydrogen phosphate, 0.01-0.06 g/L ferrous sulfate, 0.2-0.4 g/L magnesium sulfate, 0.02-0.04 g/L zinc sulfate, 0.02-0.04 g/L copper sulfate, 0.3-0.6 g/L sodium chloride, and 0.05-0.5 g/L nutritional factor.

**18.** The preparation method according to claim 16, wherein the nutritional factor is one or more selected from L-tryptophan, L-methionine, L-leucine and biotin.

**19.** Engineered strains for producing *trans*-Aconitic acid, wherein the engineered strains express aconitate isomerases, and the aconitate isomerases comprise TbrA and/or Adi1.

**20.** A preparation method of high-purity aconitate, wherein the method comprises:

(1) reacting the bio-based *trans*-Aconitic acid prepared by the method according to any one of claims 1 to 18 with alcohols to obtain a solution containing aconitate, neutralizing the solution containing aconitate, and then performing liquid separation to collect an upper organic phase;
(2) adding hydrogen peroxide to the upper organic phase to perform first-step decolorization, followed by washing, performing liquid separation and removing solvent to obtain crude aconitate product; and
(3) adding decolorizing agent to the crude aconitate product for second-step decolorization, and then filtering to obtain the high-purity aconitate, wherein the decolorizing agent is selected from at least one of activated carbon, diatomite, kaolin, basic aluminum oxide and neutral aluminum oxide.

**21.** The method according to claim 20, wherein the concentration of the hydrogen peroxide is 33% to 40%, and the weight of the hydrogen peroxide accounts for 1% to 5% of the weight of the upper organic phase.

**22.** The method according to claim 20, wherein the first-step decolorization using hydrogen peroxide is performed at 20°C to 70°C for 15 to 30 min.

**23.** The method according to claim 20, wherein a weight ratio of the decolorizing agent to the crude aconitate product is 1:(2-100).

**24.** The method according to claim 20, wherein the second-step decolorization using the decolorizing agent is performed at 20°C to 70°C for 10 to 120 min.

**25.** The method according to claim 20, wherein the activated carbon has an iodine value of greater than 800 and a methylene blue value of greater than 10;

the particle size of diatomite is 200 to 300 mesh;
the particle size of kaolin is 200 to 300 mesh;
both particle sizes of basic aluminum oxide and neutral aluminum oxide are both 100 to 200 mesh, both specific

surface areas of basic aluminum oxide and neutral aluminum oxide are 60 to 90 $m^2/g$, and both average pore diameters of basic aluminum oxide and neutral aluminum oxide are 10 to 20 nm.

**26.** The method according to claim 20, wherein the reaction between the aconitic acid obtained by fermentation and alcohols is performed at 75°C to 145°C for 2 to 8 h.

**27.** A high-purity aconitate prepared by the method according to any one of claims 20 to 26.

**28.** The high-purity aconitate according to claim 27, wherein the purity of the high-purity aconitate is not less than 99%.

**29.** The high-purity aconitate according to claim 27, wherein the chromaticity of the high-purity aconitate is not higher than 50 Pt-Co.

**30.** The high-purity aconitate according to claim 27, wherein the content of fat-soluble pigments in the high-purity aconitate is less than 1000 ppm, and the content of water-soluble pigments is less than 1000 ppm.

**31.** The high-purity aconitate according to claim 27, wherein the structural formula of the high-purity aconitate is as follows:

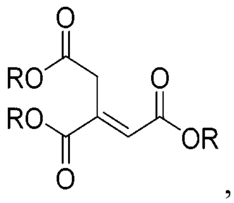

,

where R is an alkyl group.

**32.** The high-purity aconitate according to claim 31, wherein the structural formula of the high-purity aconitate is as follows:

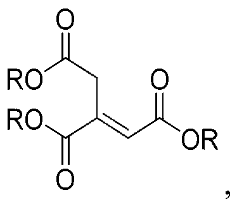

,

where R is an alkyl group having no more than 5 carbon atoms.

**33.** The high-purity aconitate according to claim 27, wherein the structural formula of the high-purity aconitate is as follows:

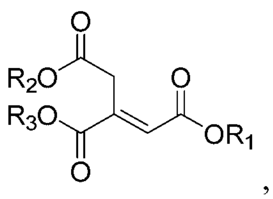

,

where $R_1$, $R_2$ and $R_3$ are each independently selected from one of alkyl groups having 1 to 5 carbon atoms, alkyl groups having more than 6 carbon atoms, alkyl groups having 1 to 5 carbon atoms and substituted by chlorine atoms or alkyl groups containing aromatic groups, and at least two of $R_1$, $R_2$ and $R_3$ are different.

**34.** The high-purity aconitate according to claim 27, wherein the structural formula of the high-purity aconitate is as follows:

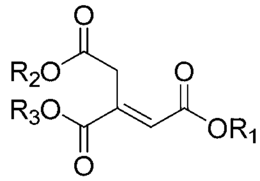

,

where $R_1$, $R_2$ and $R_3$ are independently selected from alkyl groups having 6 to 18 carbon atoms.

**35.** A composition containing the high-purity aconitate according to any one of claims 27 to 34.

**36.** The composition according to claim 35, comprising the following components in parts by weight: 100-120 parts of PVC resin, 5-90 parts of high-purity aconitate, 0.5-10 parts of lubricant, and 2-10 parts of stabilizer.

**37.** The composition according to claim 35, comprising the following components in parts by weight: 100 parts of PVC resin, 1-10 parts of cold-resistant modifier, 5-90 parts of high-purity aconitate, 0.5-10 parts of lubricant, and 3-20 parts of stabilizer.

**38.** The composition according to claim 35, comprising the following components in parts by weight: 100 parts of base material, 5-90 parts of high-purity aconitate, 10-60 parts of inorganic filler, 1-5 parts of organotin compound, 0.5-2 parts of rare earth stearate, 0-2 parts of antistatic agent, and 0.5-2 parts of flame retardant; the base material is selected from at least one of cross-linked PVC resin, non-cross-linked PVC resin, cross-linked nitrile rubber, and thermoplastic nitrile rubber.

**39.** A preparation method of the composition according to any one of claims 35 to 38, comprising the following steps:

S1: mixing the components of the composition to obtain a premix;
S2: adding the premix into a high-speed mixer, mixing at 30°C to 80°C for 10 to 60 min, then adding into two-roll open mixing mill, and mixing at 130°C to 190°C for 5 to 20 min to obtain a mixed compound; and
S3: placing the mixed compound in plate vulcanizer, and vulcanizing at 140°C to 190°C under a pressure of 10 to 15 MPa for 5 to 15 min to obtain the composition.

**40.** Use of the high-purity aconitate according to any one of claims 27 to 34 or the composition according to any one of claims 35 to 39 in improving cold resistance, heat resistance, volatility resistance, stability and plasticizing performance.

**41.** Use of the high-purity aconitate according to any one of claims 27 to 34 or the composition according to any one of claims 35 to 39 in household appliance housings, printer housings, charging gun housings, automobile taillights, PVC plastic films used in winter greenhouses, refrigerator door seals, snow boot soles, snowboards or blood storage bags.

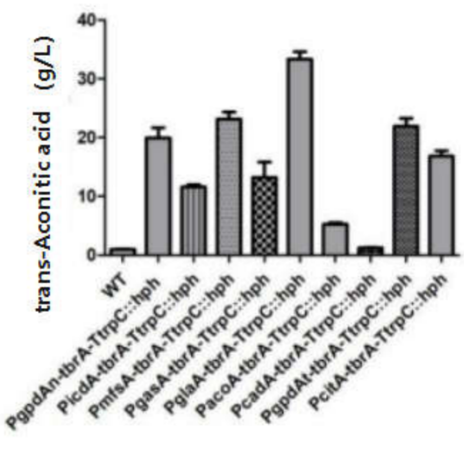


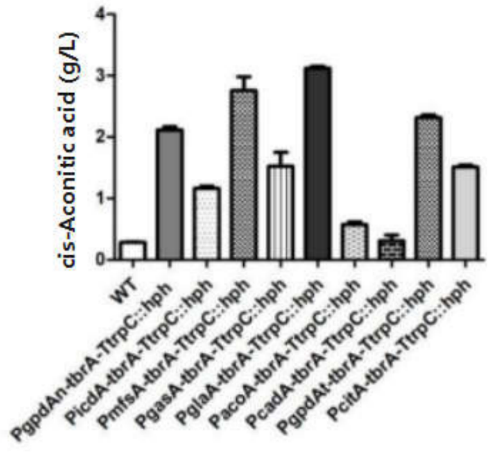


Fig. 1

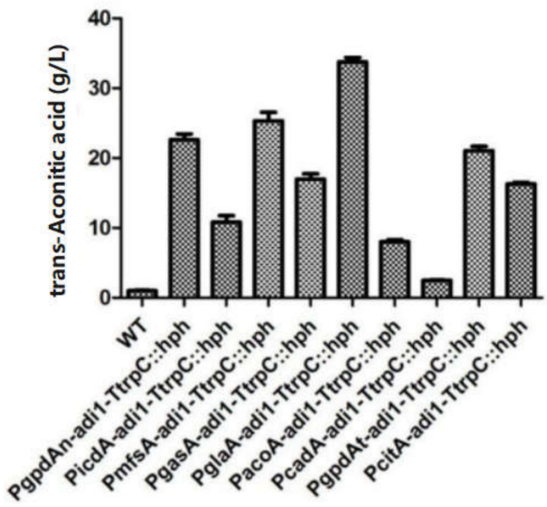


Fig. 2

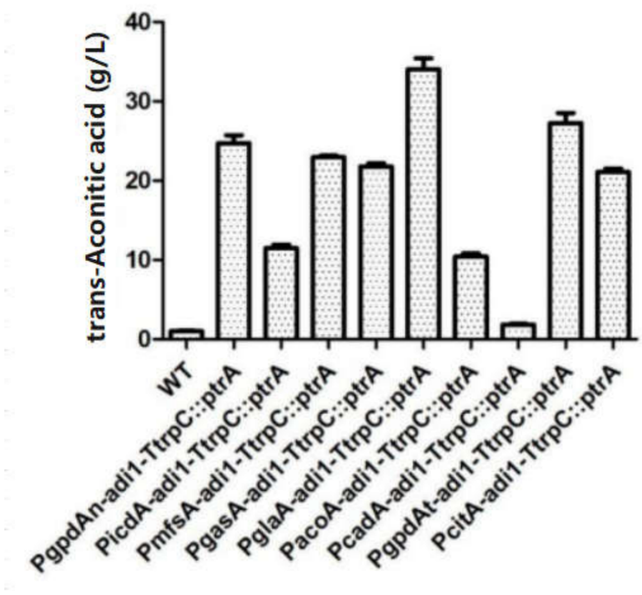


Fig. 3

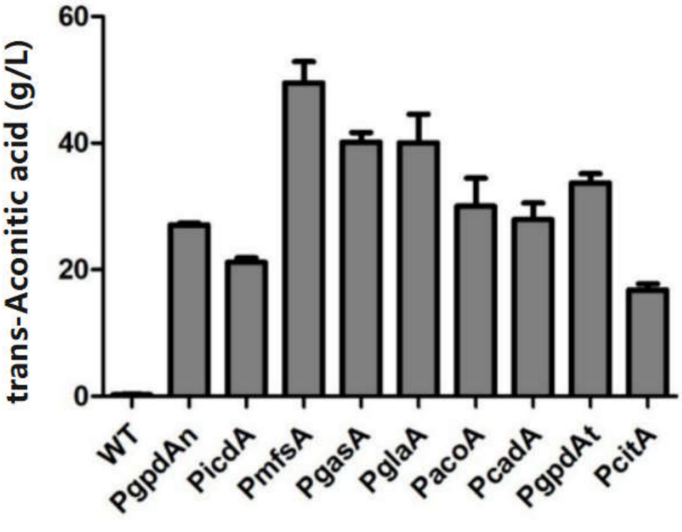


Fig. 4

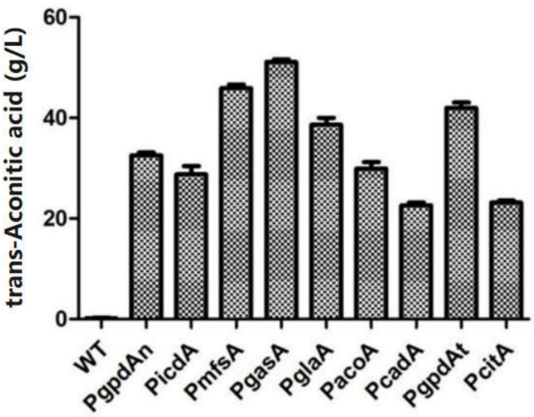


Fig. 5

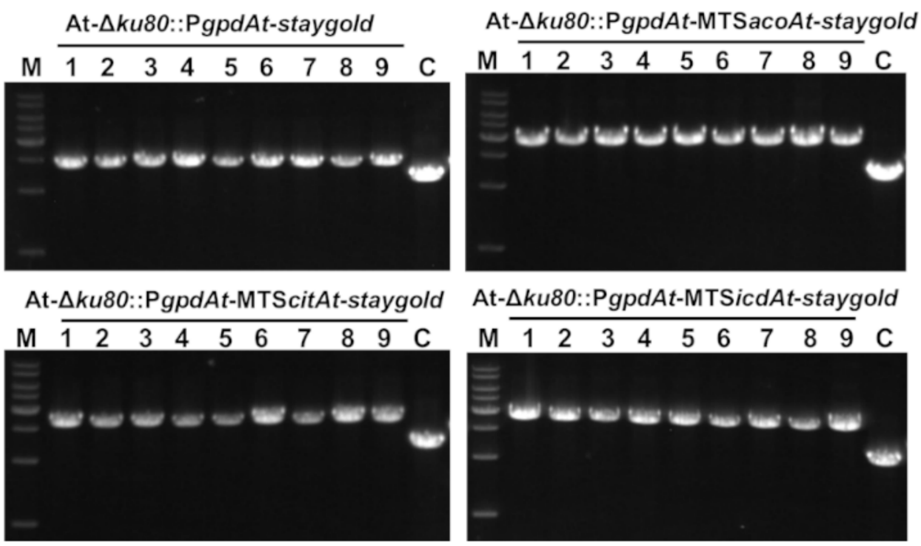


Fig. 6

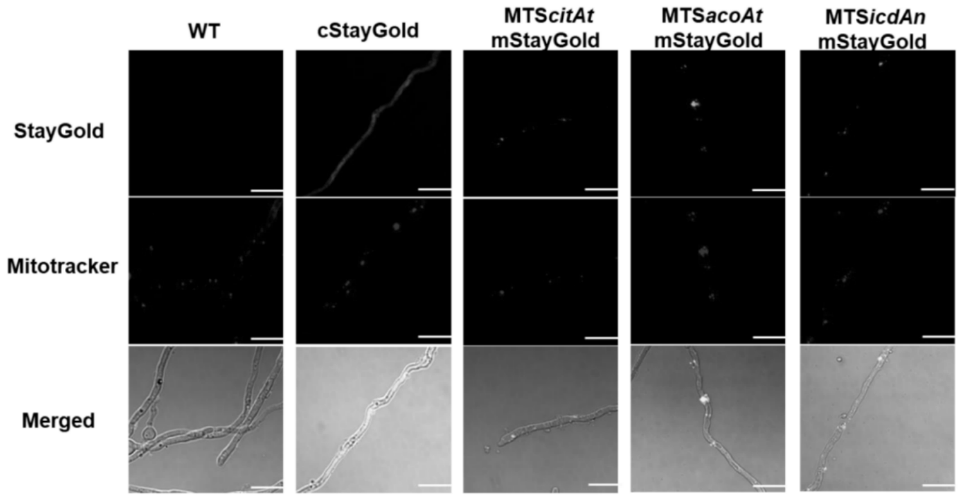
WT
cStayGold
MTScitAt mStayGold
MTSacoAt mStayGold
MTSicdAn mStayGold
StayGold
Mitotracker
Merged

Fig. 7

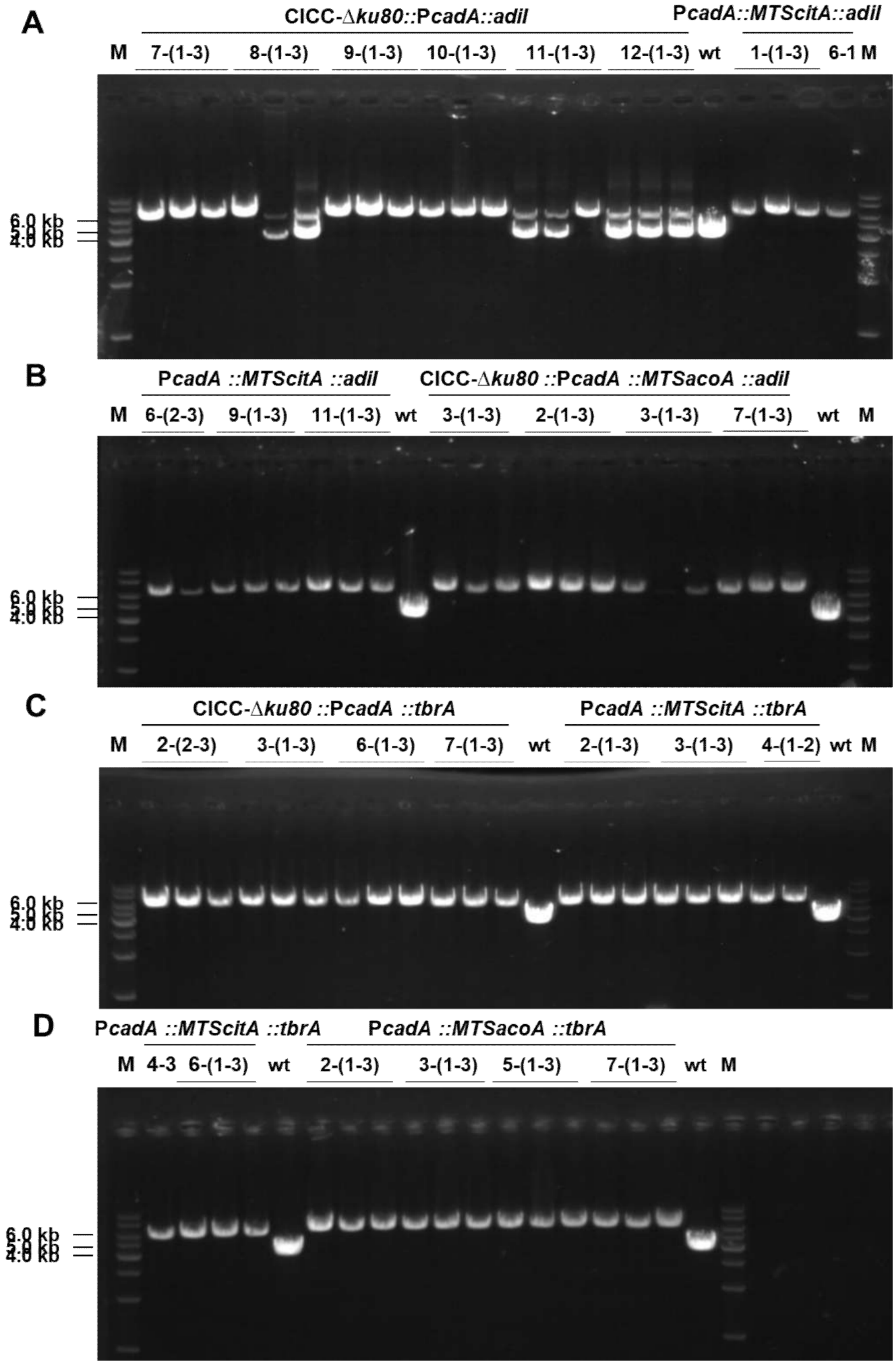
A
CICC-Δku80::PcadA::adiI
PcadA::MTScitA::adiI
M 7-(1-3) 8-(1-3) 9-(1-3) 10-(1-3) 11-(1-3) 12-(1-3) wt 1-(1-3) 6-1 M
6.0 kb
5.0 kb
4.0 kb
B
PcadA ::MTScitA ::adiI
CICC-Δku80 ::PcadA ::MTSacoA ::adiI
M 6-(2-3) 9-(1-3) 11-(1-3) wt 3-(1-3) 2-(1-3) 3-(1-3) 7-(1-3) wt M
6.0 kb
5.0 kb
4.0 kb
C
CICC-Δku80 ::PcadA ::tbrA
PcadA ::MTScitA ::tbrA
M 2-(2-3) 3-(1-3) 6-(1-3) 7-(1-3) wt 2-(1-3) 3-(1-3) 4-(1-2) wt M
6.0 kb
5.0 kb
4.0 kb
D
PcadA ::MTScitA ::tbrA
PcadA ::MTSacoA ::tbrA
M 4-3 6-(1-3) wt 2-(1-3) 3-(1-3) 5-(1-3) 7-(1-3) wt M
6.0 kb
5.0 kb
4.0 kb

Fig. 8

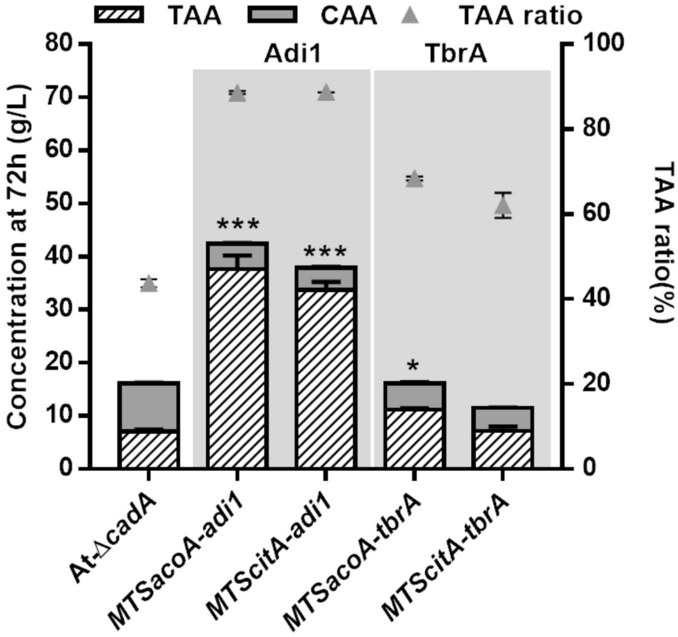
TAA
CAA
TAA ratio
Adi1
TbrA
Concentration at 72h (g/L)
TAA ratio(%)
80
70
60
50
40
30
20
10
0
100
80
60
40
20
0
***
***
*
At-ΔcadA
MTSacoA-adi1
MTScitA-adi1
MTSacoA-tbrA
MTScitA-tbrA

Fig. 9

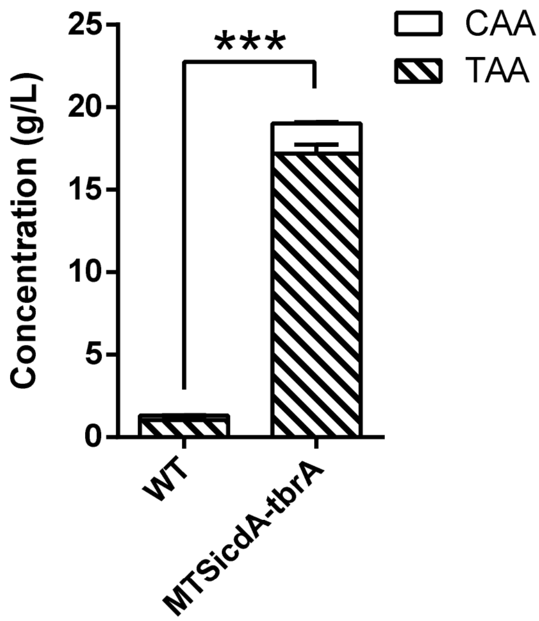
CAA
TAA
***
Concentration (g/L)
25
20
15
10
5
0
WT
MTSicdA-tbrA

Fig. 10

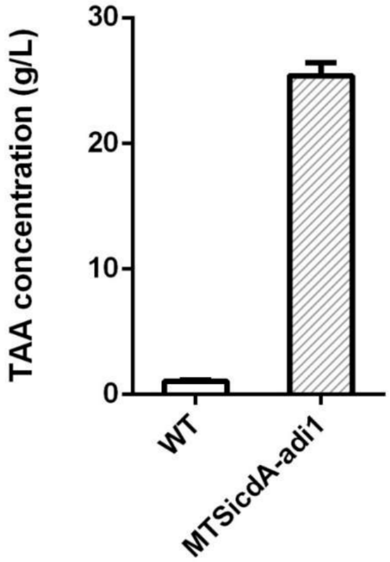
TAA concentration (g/L)
30
20
10
0
WT
MTSicdA-adi1

Fig. 11

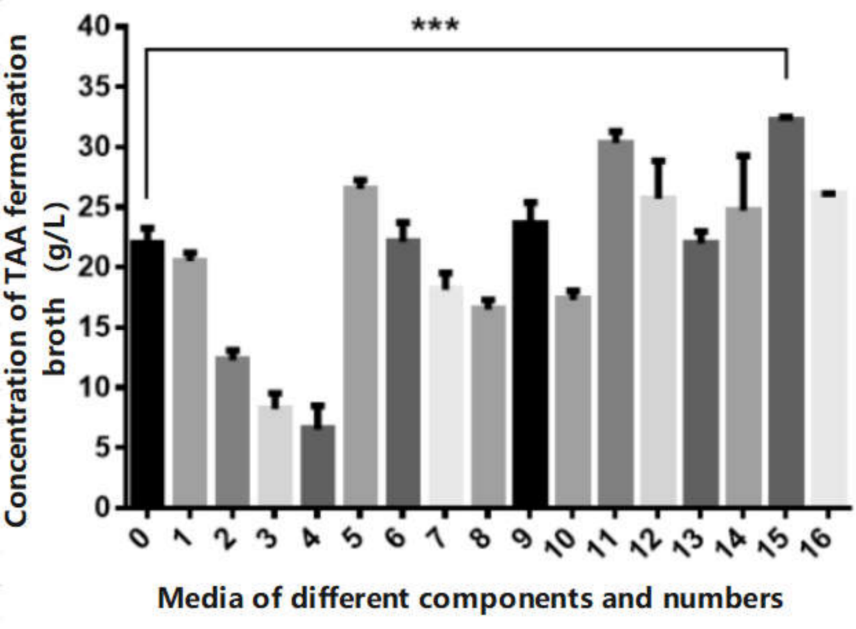
***
Concentration of TAA fermentation broth (g/L)
40
35
30
25
20
15
10
5
0
0
1
2
3
4
5
6
7
8
9
10
11
12
13
14
15
16
Media of different components and numbers

Fig. 12

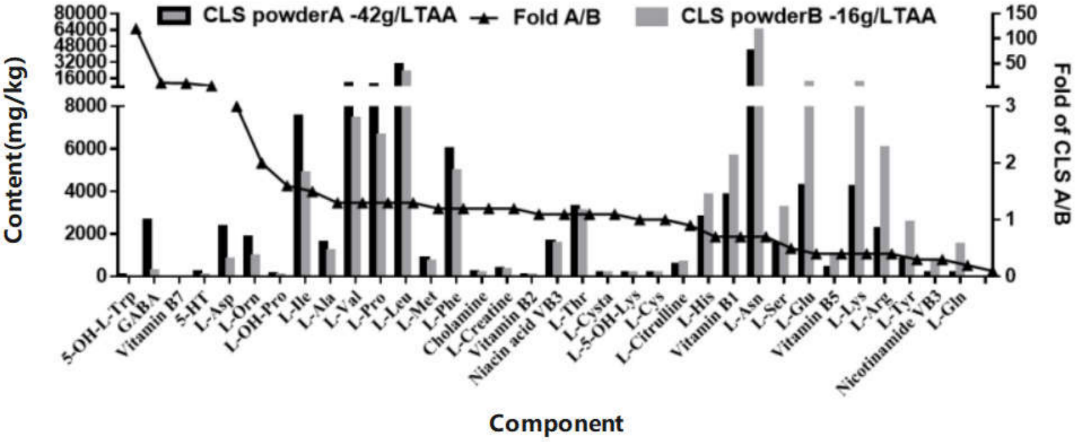
CLS powderA -42g/LTAA
Fold A/B
CLS powderB -16g/LTAA
Content(mg/kg)
Fold of CLS A/B
Component
5-OH-L-Trp
GABA
Vitamin B7
5-HT
L-Asp
L-Orn
L-OH-Pro
L-Ile
L-Ala
L-Val
L-Pro
L-Leu
L-Met
L-Phe
Cholamine
L-Creatine
Vitamin B2
Niacin acid VB3
L-Thr
L-Cysta
L-5-OH-Lys
L-Cys
L-Citrulline
L-His
Vitamin B1
L-Asn
L-Ser
L-Glu
Vitamin B5
L-Lys
L-Arg
L-Tyr
Nicotinamide VB3
L-Gln

Fig. 13

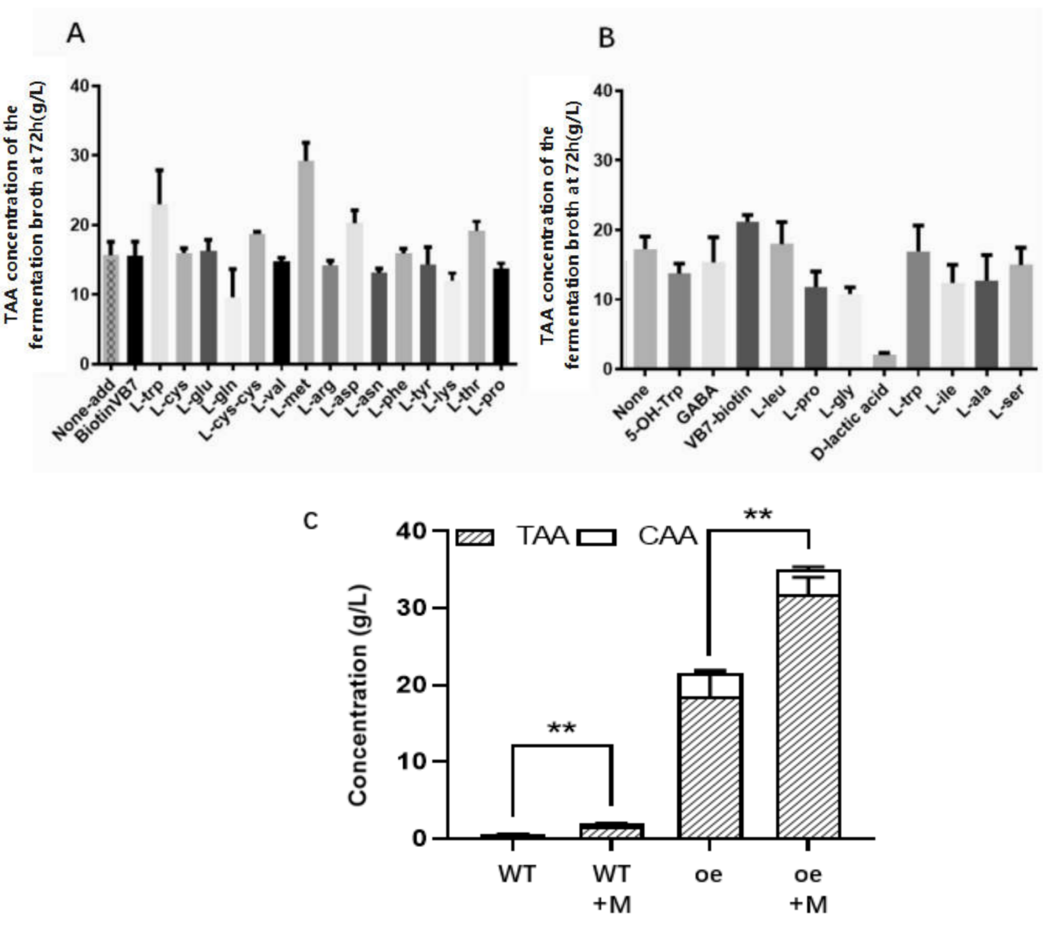
A
TAA concentration of the fermentation broth at 72h(g/L)
None-add
BiotinVB7
L-trp
L-cys
L-glu
L-gln
L-cys-cys
L-val
L-met
L-arg
L-asp
L-asn
L-phe
L-tyr
L-lys
L-thr
L-pro
B
TAA concentration of the fermentation broth at 72h(g/L)
None
5-OH-Trp
GABA
VB7-biotin
L-leu
L-pro
L-gly
D-lactic acid
L-trp
L-ile
L-ala
L-ser
C
TAA
CAA
Concentration (g/L)
**
**
WT
WT +M
oe
oe +M

Fig. 14

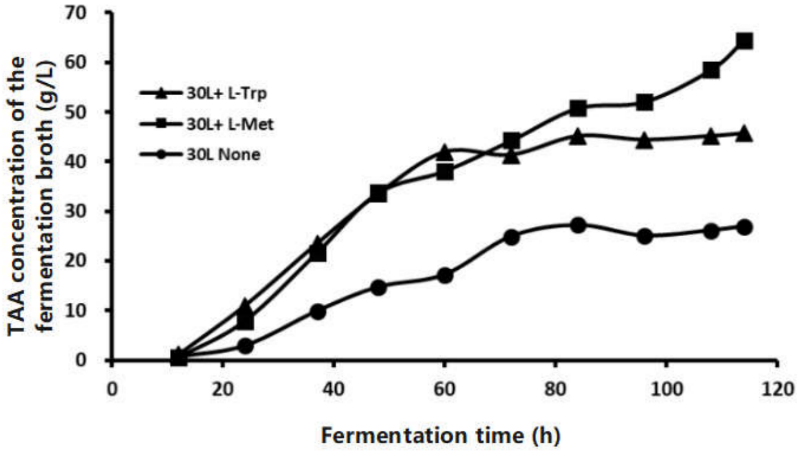


Fig. 15

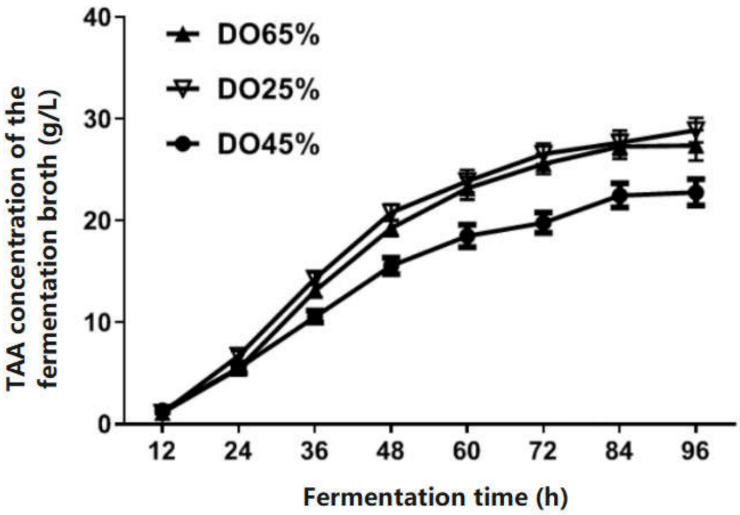


Fig. 16

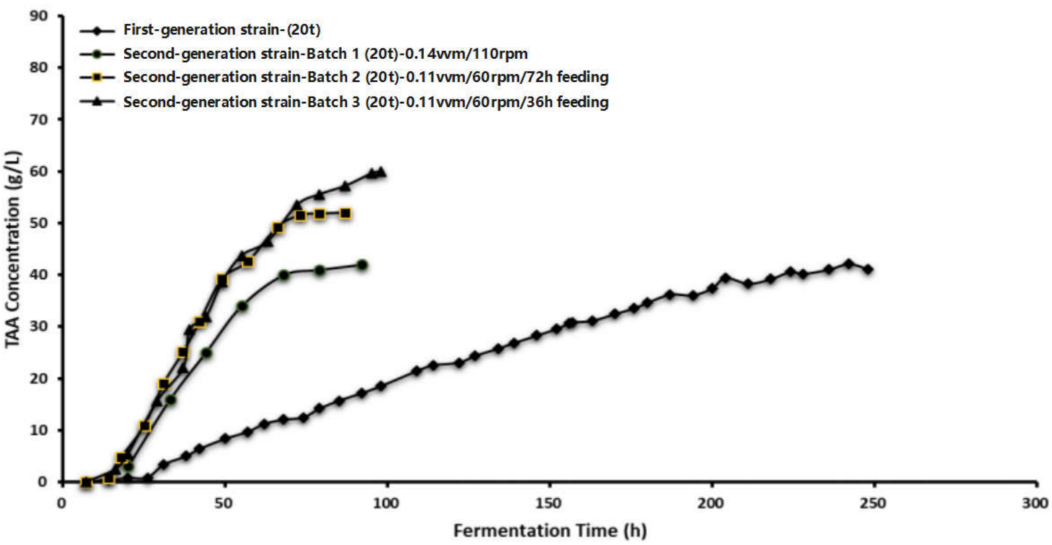


Fig. 17

Fig. 18]

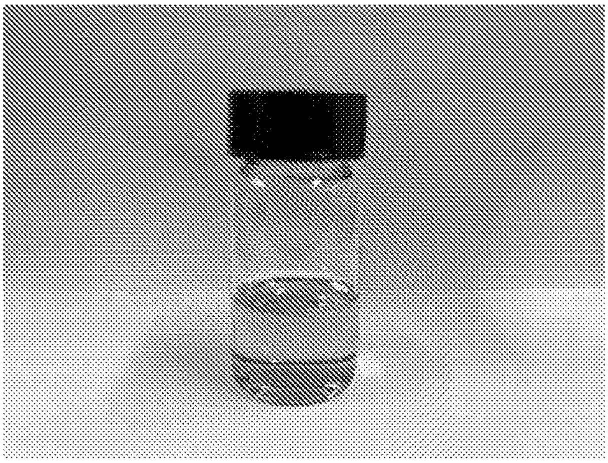

Fig. 19]

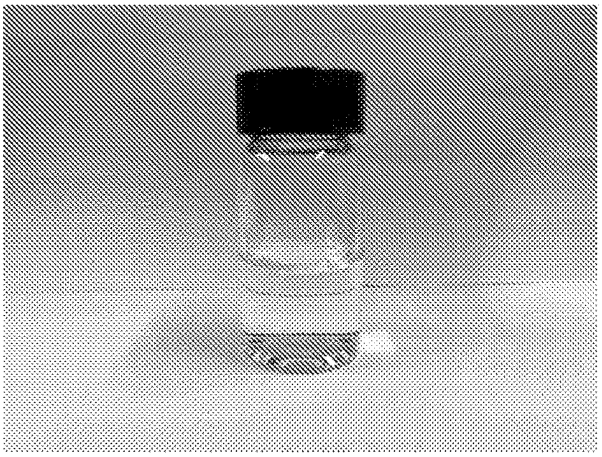

Fig. 20]

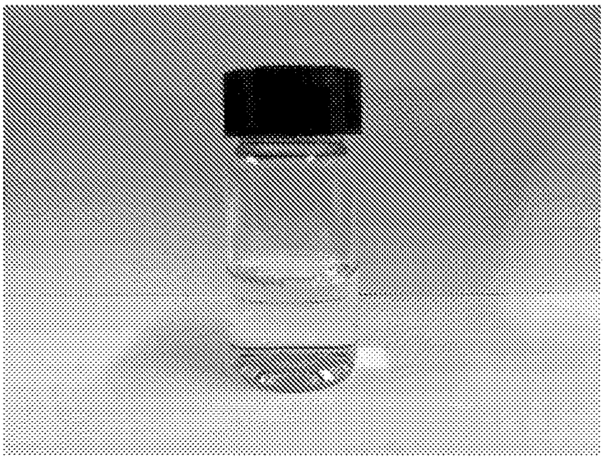

Fig. 21]

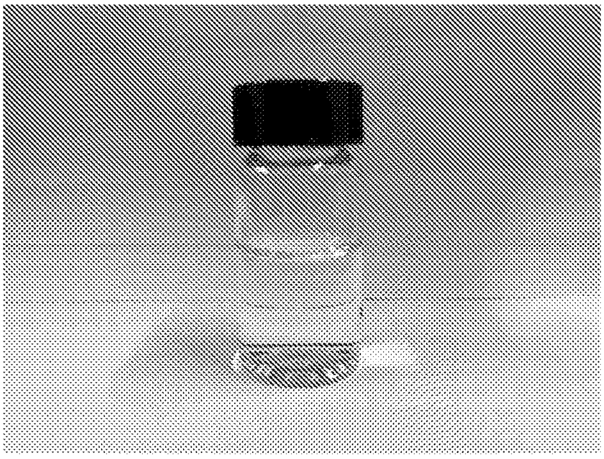

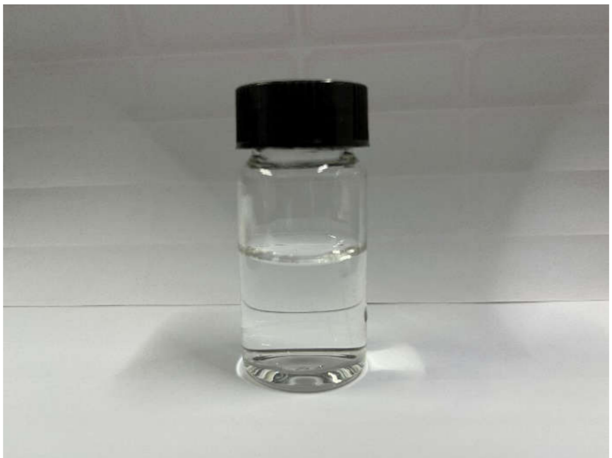

Fig. 21

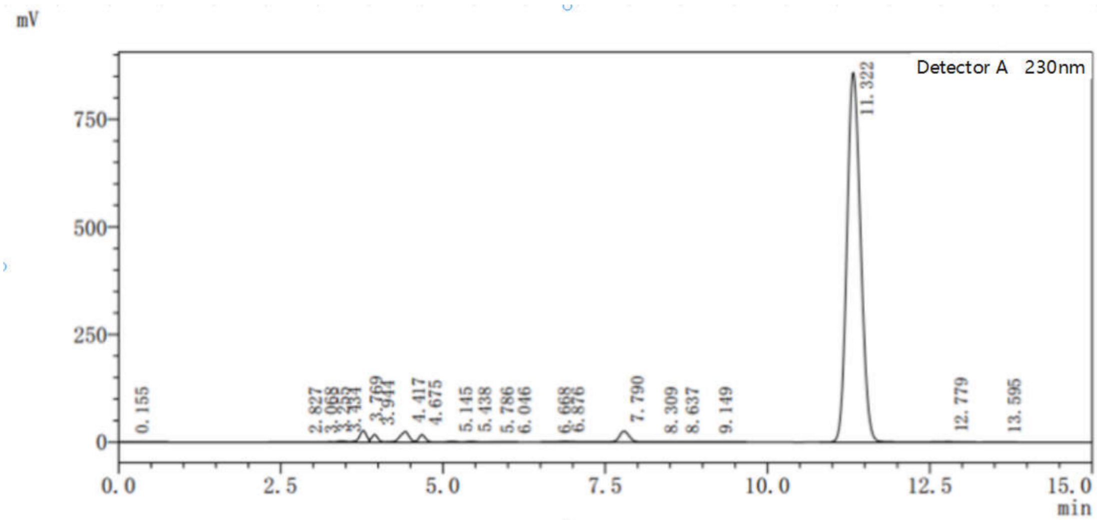


Fig. 22

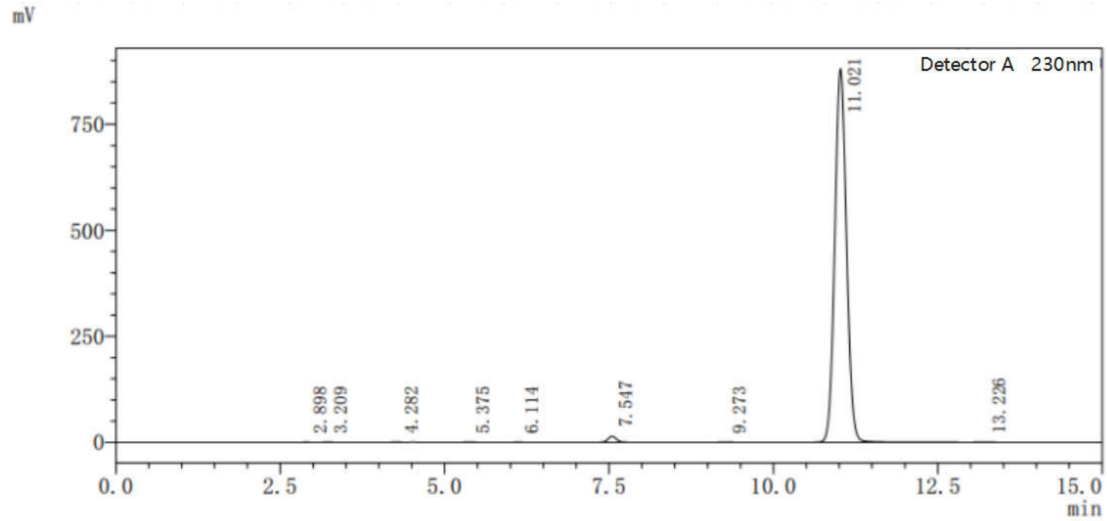


Fig. 23

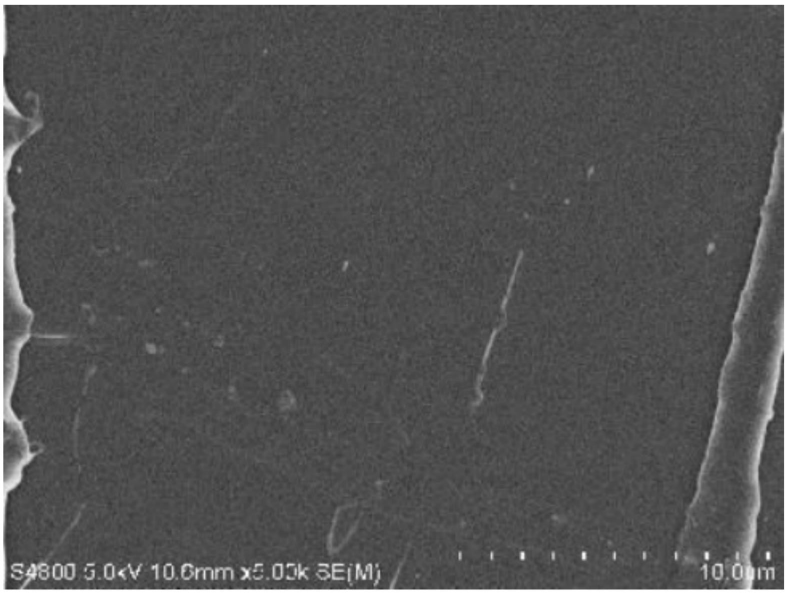


Fig. 24

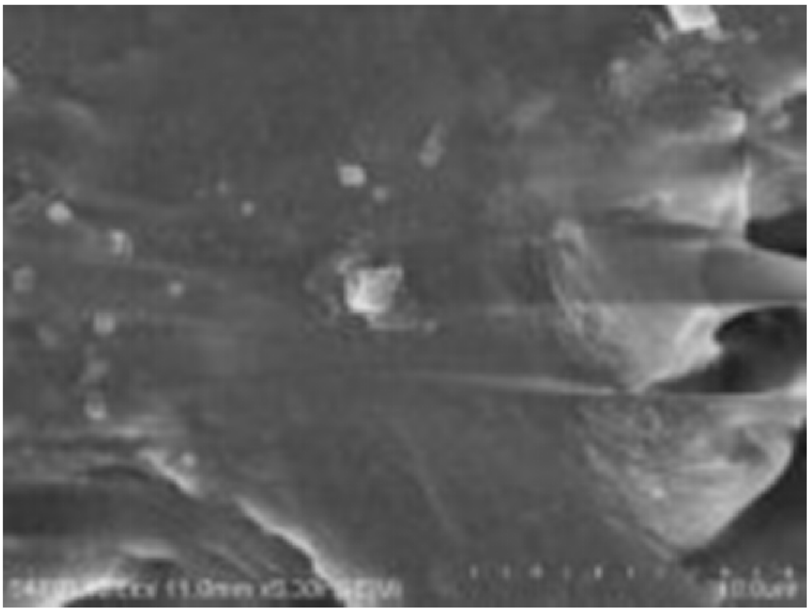

Fig. 25

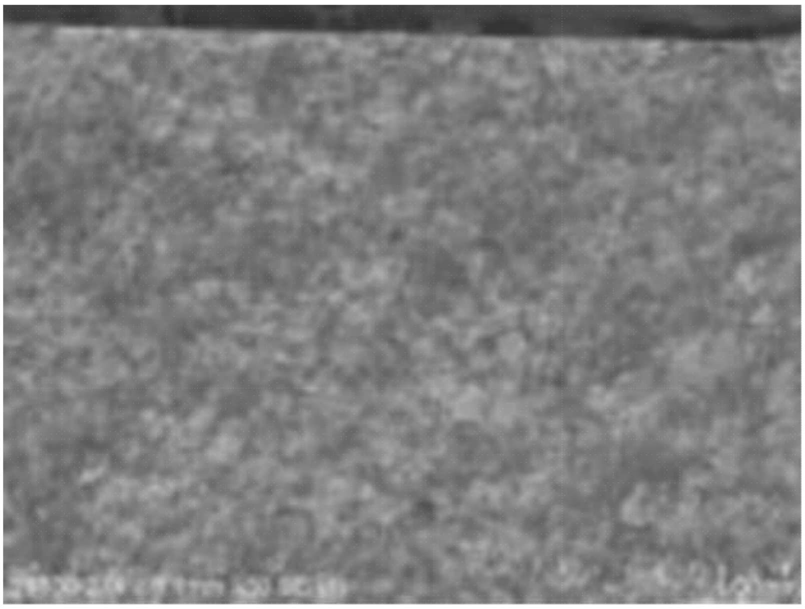

Fig. 26

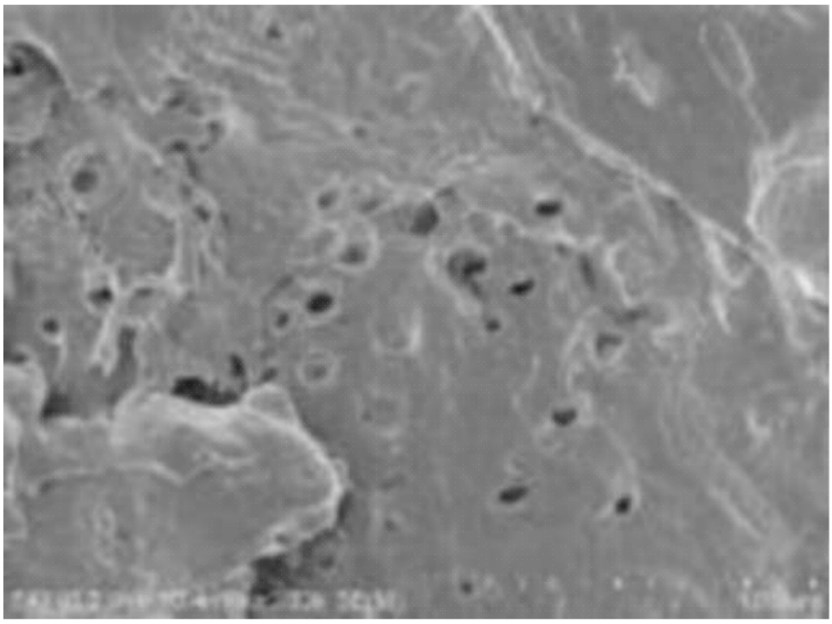

Fig. 27

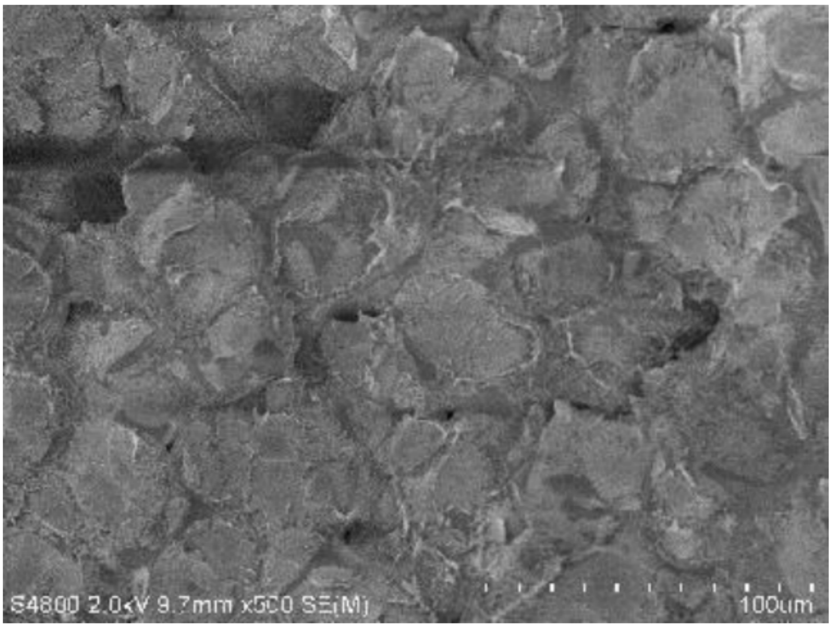


Fig. 28

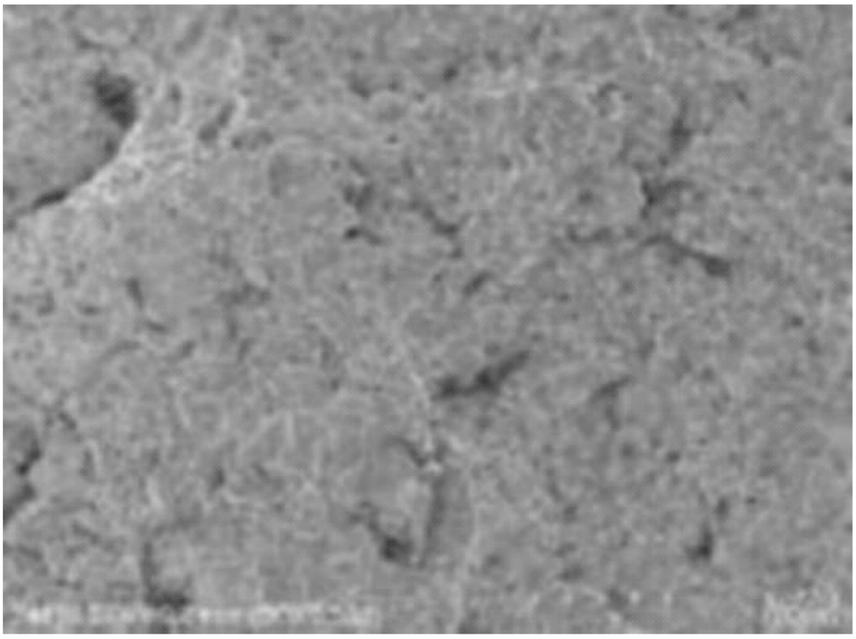

Fig. 29

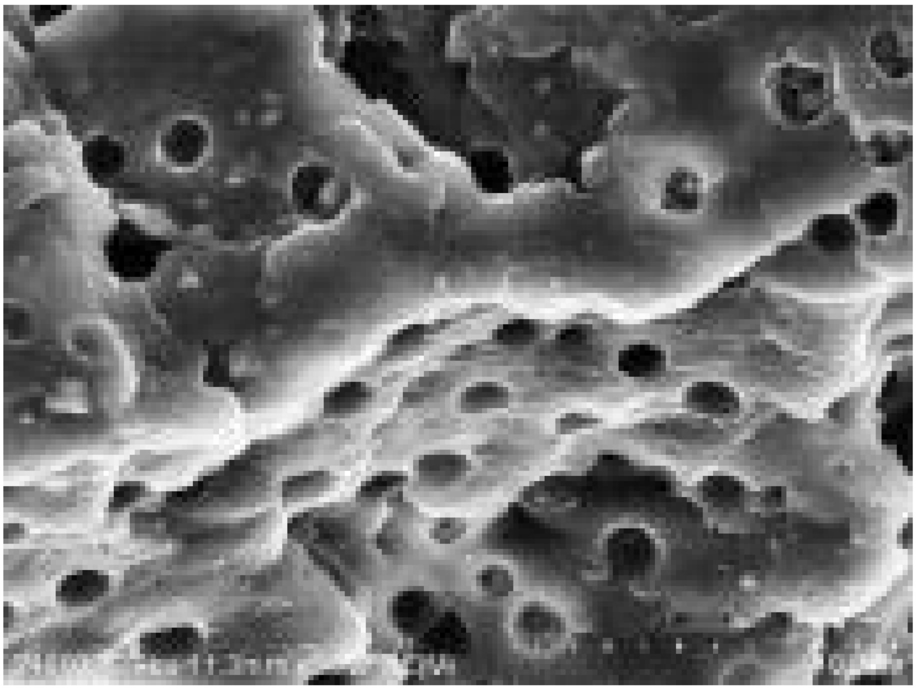

Fig. 30

## INTERNATIONAL SEARCH REPORT

International application No.
**PCT/CN2024/093180**

**A. CLASSIFICATION OF SUBJECT MATTER**

C12N15/80(2006.01)i; C12N1/15(2006.01)i; C12P7/48(2006.01)i; C07C67/08(2006.01)i; C07C69/604(2006.01)i; C08L27/06(2006.01)i

According to International Patent Classification (IPC) or to both national classification and IPC

**B. FIELDS SEARCHED**

Minimum documentation searched (classification system followed by classification symbols)

IPC:C12N C12P C07C C08L

Documentation searched other than minimum documentation to the extent that such documents are included in the fields searched

Electronic data base consulted during the international search (name of data base and, where practicable, search terms used)

CNABS; CNTXT; VEN; DWPI; SIPOABS; ENTXT; ENTXTC; CJFD; WOTXT; EPTXT; USTXT; CNKI; STN-REG; STN-CAPLUS; Genbank; ISI Web of Science; 万方, WANFANG; 超星读秀, CHAOXING DUXIU: 中国科学院青岛生物能源与过程研究所, 山东鲁抗医药股份有限公司, 吕雪峰, 王庆刚, 黄雪年, 彭欣, 侯鸿斌, 郭强, 王敏, 李继彬, 谷猛, 杜志强, 武茂成, 耿策, 司学见, 徐广强, 郭健, 张云鹏, 尹吉金, 郭勍, 乌头酸, 异构酶, 乌头酸酶, 线粒体, 定位, 信号, 溶解氧, 双氧水, 过氧化氢, 脱色, aconitic acid, aconitate, aconitase, isomerase, mitochondria, localization, signal, hydrogen peroxide, dissolved oxygen, PVC, structural formula search, 序列检索SEQ ID NO.1, SEQ ID NO.2, SEQ ID NO.5, SEQ ID NO.7, SEQ ID NO.8, SEQ ID NO.9

**C. DOCUMENTS CONSIDERED TO BE RELEVANT**

| Category* | Citation of document, with indication, where appropriate, of the relevant passages | Relevant to claim No. |
|---|---|---|
| X | CN 112011469 A (QINGDAO INSTITUTE OF BIOENERGY AND BIOPROCESS TECHNOLOGY, CHINESE ACADEMY OF SCIENCES et al.) 01 December 2020 (2020-12-01)<br>description, paragraphs 42-67, 91, and 92 | 1-3, 11, 12, 19 |
| A | CN 112011469 A (QINGDAO INSTITUTE OF BIOENERGY AND BIOPROCESS TECHNOLOGY, CHINESE ACADEMY OF SCIENCES et al.) 01 December 2020 (2020-12-01)<br>description, paragraphs 42-67, 91, and 92 | 4-10, 13-18 |
| X | CN 112029671 A (QINGDAO INSTITUTE OF BIOENERGY AND BIOPROCESS TECHNOLOGY, CHINESE ACADEMY OF SCIENCES et al.) 04 December 2020 (2020-12-04)<br>description, paragraphs 38-91 | 1-3, 11, 12, 19 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **16 August 2024** | **21 August 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2024/093180

C. DOCUMENTS CONSIDERED TO BE RELEVANT

| Category* | Citation of document, with indication, where appropriate, of the relevant passages | Relevant to claim No. |
|---|---|---|
| A | CN 112029671 A (QINGDAO INSTITUTE OF BIOENERGY AND BIOPROCESS TECHNOLOGY, CHINESE ACADEMY OF SCIENCES et al.) 04 December 2020 (2020-12-04)<br>description, paragraphs 38-91 | 4-10, 13-18 |
| X | CN 112011468 A (QINGDAO INSTITUTE OF BIOENERGY AND BIOPROCESS TECHNOLOGY, CHINESE ACADEMY OF SCIENCES et al.) 01 December 2020 (2020-12-01)<br>description, paragraphs 40 and 64-67 | 1, 11, 12 |
| A | CN 112011468 A (QINGDAO INSTITUTE OF BIOENERGY AND BIOPROCESS TECHNOLOGY, CHINESE ACADEMY OF SCIENCES et al.) 01 December 2020 (2020-12-01)<br>description, paragraphs 40 and 64-67 | 2-10, 13-19 |
| X | CN 110527637 A (QINGDAO INSTITUTE OF BIOENERGY AND BIOPROCESS TECHNOLOGY, CHINESE ACADEMY OF SCIENCES) 03 December 2019 (2019-12-03)<br>description, paragraphs 50-53, 60, and 61 | 1, 11-18 |
| X | CN 113817223 A (QINGDAO INSTITUTE OF BIOENERGY AND BIOPROCESS TECHNOLOGY, CHINESE ACADEMY OF SCIENCES) 21 December 2021 (2021-12-21)<br>description, paragraphs 30-32 and 45-80 | 20-41 |
| X | CN 114773748 A (SHANDONG ONE TWO POLYMER MATERIALS CO., LTD.) 22 July 2022 (2022-07-22)<br>description, paragraphs 4-16 | 27-41 |
| X | BROWN, P. et al. "New Catanionic Surfactants with Ionic Liquid Properties"<br>*Journal of Colloid and Interface Science*, Vol. 395, 04 January 2013 (2013-01-04), 185-189<br>pages 185-189 | 20-31, 33, 34 |
| X | EASTOE, J. et al. "Surfactants for CO2"<br>*Langmuir*, Vol. 22, No. 24, 27 October 2006 (2006-10-27), 9832-9842<br>page S1, paragraph 2 | 20-33 |
| A | HUANG, Xuenian et al. "Improving Itaconic Acid Production Through Genetic Engineering of an Industrial Aspergillus terreus Strain"<br>*Microbial Cell Factories*, Vol. 13, 11 August 2014 (2014-08-11), 119<br>pages 1-9 | 5-10 |
| A | STEIGER, M. G. et al. "Characterizing MttA as a Mitochondrialcis-aconitic Acid Transporter by Metabolic Engineering"<br>*Metabolic Engineering*, Vol. 35, 10 February 2016 (2016-02-10), 95-104<br>pages 95-104 | 7-10 |

Form PCT/ISA/210 (second sheet) (July 2022)

**INTERNATIONAL SEARCH REPORT**

International application No.
**PCT/CN2024/093180**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/093180**

| Patent document cited in search report | | | Publication date (day/month/year) | Patent family member(s) | | | Publication date (day/month/year) |
|---|---|---|---|---|---|---|---|
| CN | 112011469 | A | 01 December 2020 | CN | 112011469 | B | 05 July 2022 |
| CN | 112029671 | A | 04 December 2020 | CN | 112029671 | B | 05 July 2022 |
| CN | 112011468 | A | 01 December 2020 | CN | 112011468 | B | 05 July 2022 |
| CN | 110527637 | A | 03 December 2019 | CN | 110527637 | B | 15 October 2021 |
| CN | 113817223 | A | 21 December 2021 | | None | | |
| CN | 114773748 | A | 22 July 2022 | | None | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311144969
- CN 202310580689
- CN 202310546966
- CN 202310575243
- CN 202310580674
- CN 202310573624
- CN 202311140630
- CN 202311140617
- CN 110527637 B
- CN 112011468 B
- CN 112011469 B
- CN 112011470 B
- CN 112029671 B
- US 20210163966 A1
- CN 101391957 A
- CN 110527637 A
- CN 104894165 A
- CN 112011469 A
- CN 112029671 A
- CN 104894L6


### Non-patent literature cited in the description


- *CHEMICAL ABSTRACTS*, 4023-65-8
- *CHEMICAL ABSTRACTS*, 585-84-2
- **LI et al.** *Microb Cell Fact*, 2020, vol. 19, 174
- **KIM, J.G.** ; **CHOI, Y.D** ; **CHANG, Y.J** ; **KIM, S.U.** Genetic transformation of Monascus purpureus DSM1379. *Biotechnology Letters*, 2003, vol. 25, 1509-1514
- **KIM, J.G.** ; **CHOI, Y.D.** ; **CHANG, Y.J.** ; **KIM, S.U.** Genetic transformation of Monascus purpureus DSM1379. *Biotechnology Letters*, 2003, vol. 25, 1509-1514
- **PUNT P. J.** ; **ZEGERS N. D.** ; **BUSSCHER M.** ; **POUWELS P. H** ; **VAN DEN HONDEL C. A.** *Journal of Biotechnology*, 1991, vol. 7, 19-33
- **KIM, J.G.** ; **CHOI, Y.D.** ; **CHANG, Y.J** ; **KIM, S.U.** Genetic transformation of Monascus purpureus DSM1379. *Biotechnology Letters*, 2003, vol. 25, 1509-1514